# EUROPEAN PATENT APPLICATION

(11) **EP 4 464 704 A1**
(43) Date of publication of application: **20.11.2024**
(21) Application number: 23739989.4
(22) Date of filing: 09.01.2023
(51) Int. Cl.: C07D 471/04, C07D 471/12, C07D 487/04, C07D 491/048, C07D 491/147, A61K 31/495, A61P 35/00

(54) **PIPERAZINO RING-CONTAINING DERIVATIVE, PHARMACEUTICALLY ACCEPTABLE SALT THEREOF, PREPARATION METHOD THEREFOR, AND APPLICATION THEREOF**

(30) Priority: 13.01.2022 CN 202210039229; 07.06.2022 CN 202210640587; 30.12.2022 CN 202211742481
(71) Applicant: Acerand Therapeutics (Hong Kong) Limited, Hong Kong (HK)
(72) Inventor: WEI, Yi, Shanghai 201203 (CN); LI, Wenming, Shanghai 201203 (CN); YUAN, Hongbin, Shanghai 201203 (CN); ZHOU, Guoqiang, Shanghai 201203 (CN); LIU, Kun Chin, Shanghai 201203 (CN); KANG, Kai, Shanghai 201203 (CN); LI, Manhua, Shanghai 201203 (CN)
(74) Representative: dompatent von Kreisler Selting Werner - Partnerschaft von Patent- und Rechtsanwälten mbB
(86) International application number: PCT/CN2023/071439
(87) International publication number: WO 2023/134647

(57) **Abstract**

Provided are a piperazino ring derivative represented by formula (I), a pharmaceutically acceptable salt thereof, a preparation method therefor, and a pharmaceutical composition containing said compound, as well as the use of the compound as a polyadenosine diphosphate ribose (ADP-ribose) polymerase (PARP) inhibitor in the treatment of a cancer, inflammation, a metabolic disease, a cardiovascular disease, an immunological disease, a mental disorder, or a related disease.

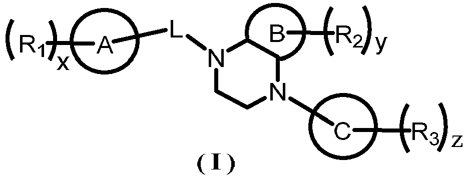

## Description

The present application claims the right of the priorities of Chinese patent application 2022100392290 filed on January 13, 2022, Chinese patent application 2022106405877 filed on June 7, 2022, and Chinese patent application 2022117424814 filed on December 30, 2022. The contents of the above Chinese patent applications are incorporated herein by reference in their entireties.

### TECHNICAL FIELD

The present disclosure belongs to the field of drug synthesis. Specifically, it relates to a piperazino ring-containing derivative, a pharmaceutically acceptable salt thereof, and a preparation method therefor, as well as a use thereof as a PARP inhibitor in the manufacture of a medicament for the treatment of cancer.

### BACKGROUND

During the course of life, DNA in the cells of an organism is affected by a variety of *in vivo* factors and *in vitro* environment on a daily basis, resulting in DNA damage. Under normal circumstances, such damage can be addressed by a variety of intracellular DNA repair pathways to maintain normal cell proliferation and apoptosis. The intracellular DNA damage response (DDR) pathway is a complex protein network system and an important pathway for maintaining genome integrity. An important feature of tumor cells is genome instability, which is mainly manifested by changes in chromosome number and/or structure, as well as microsatellite instability (MSI). Therefore, DDR has become an important approach to tumor drug development.

In mammalian cells, the PARP enzyme family consists of at least 17 members, among which PARP1 and PARP2 are the main members involved in cellular DNA damage repair. PARP acts as an important molecular sensor for DNA breaks in the DDR pathway that is activated after DNA damage. PARP can sense the DNA single-strand breakage and bind to the DNA break site. The catalytic activity is increased more than 500-fold by binding to PARP. PARP catalyzes the decomposition of nicotinamide adenine dinucleotide (NAD⁺) into nicotinamide and ADP ribose through its own glycosylation, and then takes ADP ribose as a substrate to "PARylate" the receptor protein and PARP1 itself, forming PARP-ADP ribose branched chain [poly(ADP-ribose) polymer, pADPr]. On the one hand, it can prevent DNA molecules in the vicinity of the damage site from recombining with the damaged DNA, while recruiting DNA repair proteins, histone H1, and a series of transcription factors to bind at the DNA damage site. On the other hand, it can reduce the affinity of PARP1 for DNA, causing PARP1 to dissociate from the DNA break, and then the DNA repair proteins bind to the DNA breakage to repair the damage site. The "PARylation" of PARP1 is removed by other enzymes, allowing PARP1 to regain its activity.

For tumor cells with BRCA mutations or tumor cells with other defects in DNA damage repair pathways, inhibition of PARP or deletion of BRCA alone is not fatal, but the two together lead to cell death. This synthetic lethality effect has brought a new direction to tumor drug development. To date, several PARP1/2 inhibitors have been approved for tumor treatment and have achieved good clinical results. At present, one of the main side effects of PARP1/2 inhibitors is anemia, which increases the pain of patients, causes patients to discontinue treatment, and limits the possibility of combination with other drugs. PARP1 accounts for 85% to 95% of PARP activity in cells. PARP2 is involved in the regulation of erythrocyte differentiation, and inhibition of PARP2 function leads to shortened lifespan of erythrocytes, decreased hematocrit, and decreased hemoglobin levels. Studies have confirmed that PARP2-deficient mice develop chronic anemia. Therefore, the development of selective PARP1 inhibitors is beneficial to maintain anti-tumor effects while reducing hematological side effects such as anemia.

The present disclosure identifies a series of novel PARP1 inhibitor compounds with selective inhibition of PARP1 that can be potentially used in tumor treatment.

### CONTENT OF THE PRESENT INVENTION

The object of the present disclosure is to provide a compound of general formula (I), a stereoisomer thereof, or a pharmaceutically acceptable salt thereof, wherein the general formula (I) has a structure as follows: 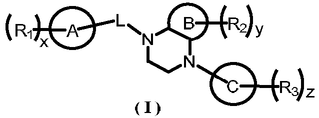 wherein
ring A is 3- to 18-membered heterocyclyl or 5- to 18-membered heteroaryl, wherein the 3- to 18-membered heterocyclyl and 5- to 18-membered heteroaryl are optionally further substituted;
ring B is C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted;
ring C is C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted;
R₁ is independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, 5- to 14-membered heteroaryloxy, C₃₋₁₂ cycloalkyl-C₁₋₆ alkyl, 3- to 12-membered heterocyclyl-C₁₋₆ alkyl, C₆₋₁₄ aryl-C₁₋₆ alkyl, or 5- to 14-membered heteroaryl-C₁₋₆ alkyl, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
or, any two R₁ together with the atom to which they are attached form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, and 5- to 14-membered heteroaryloxy;
R₂ is independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, 5- to 14-membered heteroaryloxy, C₃₋₁₂ cycloalkyl-C₁₋₆ alkyl, 3- to 12-membered heterocyclyl-C₁₋₆ alkyl, C₆₋₁₄ aryl-C₁₋₆ alkyl, or 5- to 14-membered heteroaryl-C₁₋₆ alkyl, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
R₃ is independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, 5- to 14-membered heteroaryloxy, -(CH₂)ₙ₁Rₐ, -(CH₂)ₙ₁ORₐ, -(CH₂)ₙ₁SRₐ, - (CH₂)ₙ₁NR_{b}Rₐ, -(CH₂)ₙ₁C(O)Rₐ, -(CH₂)ₙ₁C(O)NR_{b}Rₐ, -(CH₂)ₙ₁NR_{b}C(O)Rₐ, -(CH₂)ₙ₁S(O)ₘ₁Rₐ, -(CH₂)ₙ₁S(O)ₘ₁NR_{b}Rₐ, -(CH₂)ₘS(O)(=NR_{b})Rₐ, -(CH₂)ₘN=S(=O)RₐR_{b}, or - (CH₂)ₙ₁NR_{b}S(O)ₘ₁Rₐ, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, and 5- to 14-membered heteroaryloxy;
or, any two R₃ together with the atom to which they are attached form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, and 5- to 14-membered heteroaryloxy;
Rₐ and R_{b} are each independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, or 5- to 14-membered heteroaryloxy, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
L is -(CR₄R₅)ₙ₂-, -C(O)-, -C(O)NR₄-, or -(CR₄R₅)₂O;
R₄ and R₅ are each independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, or 5- to 14-membered heteroaryloxy, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
or, R₄ and R₅ together with the atom to which they are attached form C₃₋₁₂ cycloalkyl or 3- to 12-membered heterocyclyl, wherein the C₃₋₁₂ cycloalkyl and 3- to 12-membered heterocyclyl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
x, y, and z are each independently an integer of 0 to 10;
n1 is an integer of 0 to 10;
n2 is 0, 1, 2, or 3; and
m1 is 0, 1, or 2.

In a preferred embodiment of the present disclosure, the ring A is 5- to 6-membered monocyclic heteroaryl, 6- to 10-membered bicyclic fused heterocyclyl, 8- to 10-membered bicyclic fused heteroaryl, 8- to 14-membered tricyclic fused heterocyclyl, or 10- to 14-membered tricyclic fused heteroaryl;
the ring A is optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy.

In a further preferred embodiment of the present disclosure, the ring A is

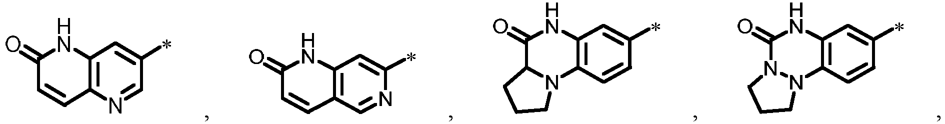

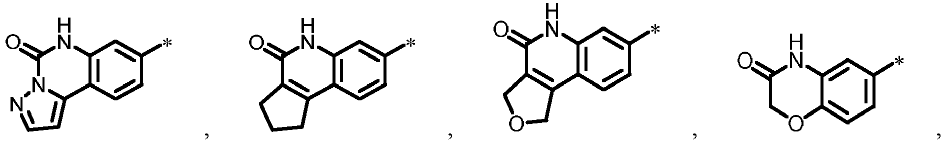

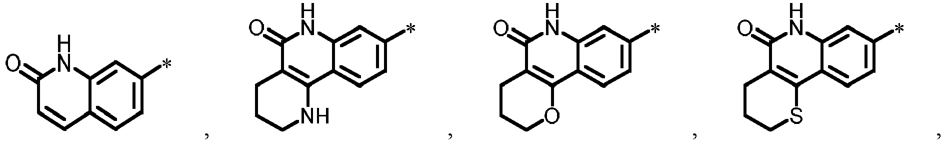

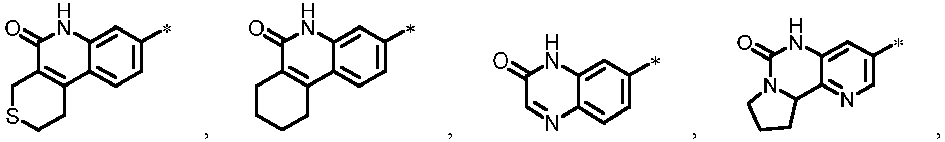

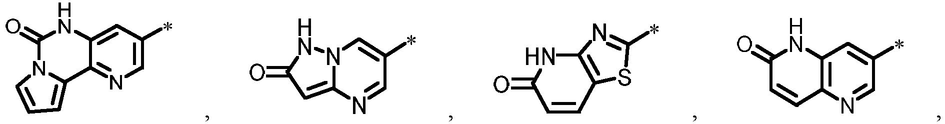

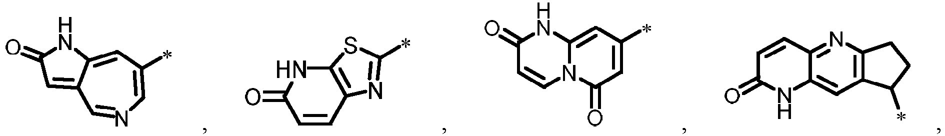

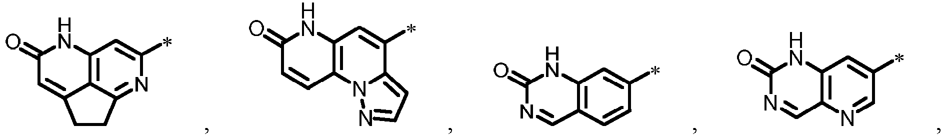

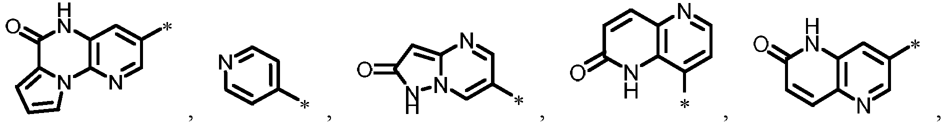

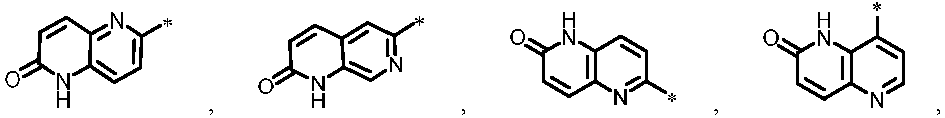

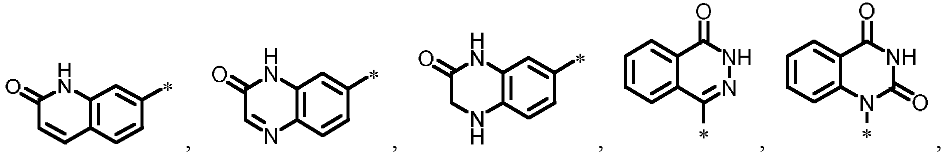

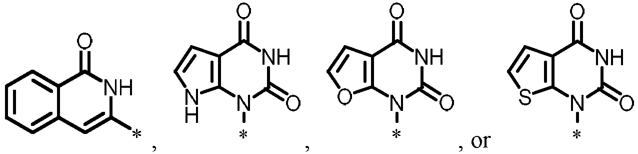

The present disclosure also provides a preferred embodiment in which the compound, the stereoisomer thereof, or the pharmaceutically acceptable salt thereof has a specific structure of general formula (II): 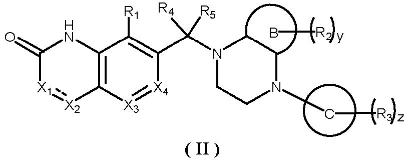 wherein
X₁ is O, S, N, NR₆, CR₆, or CR₆R₇;
X₂ is O, S, N, C(O), NR₈, CR₈, or CR₈R₉;
X₃ is N or CR₁₀;
X₄ is Nor CR₁₁;
R₆, R₇, R₈, R₉, R₁₀, and R₁₁ are each independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, 5- to 14-membered heteroaryloxy, or - (CH₂)ₙ₃R_{c}, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
or, any two of R₆, R₇, R₈, R₉, R₁₀, and R₁₁ together with the atom to which they are attached form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
R_{c} is hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, or 5- to 14-membered heteroaryloxy, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy; and
n3 is 0, 1, 2, 3, or 4;
ring B, ring C, R₂, R₃, R₄, R₅, y, and z are as defined in general formula (I).

In a preferred embodiment of the present disclosure, the ring B is C₃₋₆ cycloalkyl or 3- to 6-membered heterocyclyl;
the ring B is optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy.

The present disclosure also provides a preferred embodiment in which the compound, the stereoisomer thereof, or the pharmaceutically acceptable salt thereof has a specific structure of general formula (III): 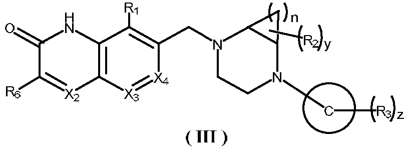 wherein
n is 1, 2, 3, 4, 5, or 6;
X₂ is Nor CR₈;
R₆ and R₈ are each independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, 5- to 14-membered heteroaryloxy, or -(CH₂)ₙ₃R_{c}, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
or, R₆ and R₈ together with the atom to which they are attached form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, and 5- to 14-membered heteroaryloxy;
X₂, X₃, X₄, ring C, R₁, R₂, R₃, y, and z are as defined in general formula (I).

In a further preferred embodiment of the present disclosure, the compound of general formula (I) is not 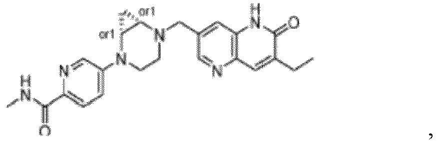 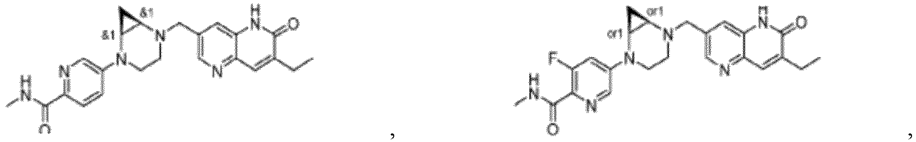 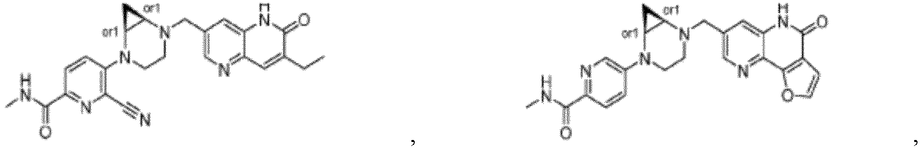 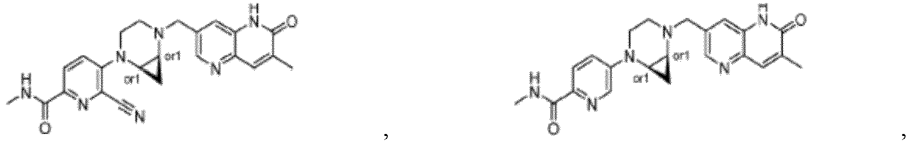 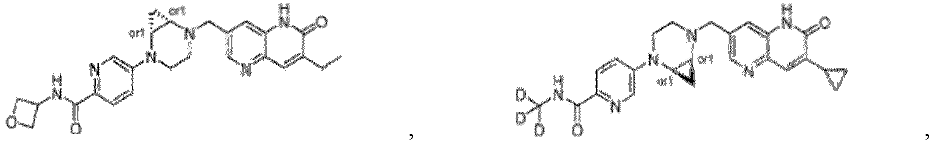 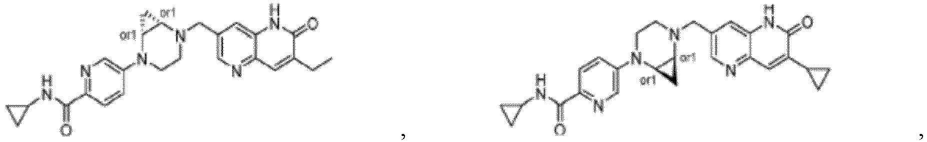 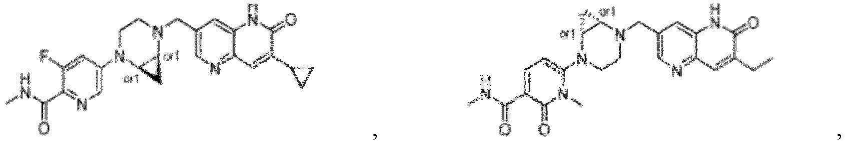 or

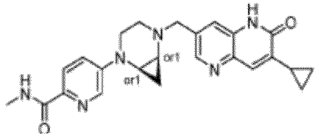

In a further preferred embodiment of the present disclosure, the ring B is selected from C₄₋₁₂ cycloalkyl, 3- to 12-membered heterocyclyl, C₆₋₁₄ aryl, or 5- to 14-membered heteroaryl, wherein the C₄₋₁₂ cycloalkyl, 3- to 12-membered heterocyclyl, C₆₋₁₄ aryl, and 5- to 14-membered heteroaryl are optionally further substituted.

In a further preferred embodiment of the present disclosure, ring C is

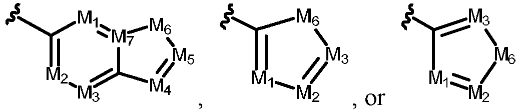

The present disclosure also provides a preferred embodiment in which the compound, the stereoisomer thereof, or the pharmaceutically acceptable salt thereof has a specific structure of general formula (IIIA) and general formula (IIIB): 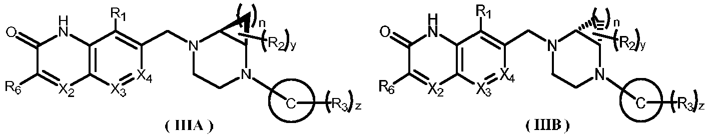 wherein
X₂, X₃, X₄, ring C, R₁, R₂, R₃, R₆, n, y, and z are as defined in general formula (I).

The present disclosure also provides a preferred embodiment in which the compound, the stereoisomer thereof, or the pharmaceutically acceptable salt thereof has a specific structure of general formula (IIIA) and general formula (IIIB): 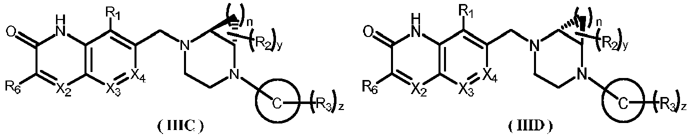 wherein
X₂, X₃, X₄, ring C, R₁, R₂, R₃, R₆, n, y, and z are as defined in general formula (I).

The present disclosure also provides a preferred embodiment in which the compound, the stereoisomer thereof, or the pharmaceutically acceptable salt thereof has a specific structure of general formula (IV): 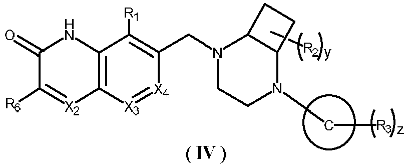 wherein
X₂, X₃, X₄, ring C, R₁, R₂, R₃, R₆, y, and z are as defined in general formula (I).

The present disclosure also provides a preferred embodiment in which the compound, the stereoisomer thereof, or the pharmaceutically acceptable salt thereof has a specific structure of general formula (IVA) and general formula (IVB): 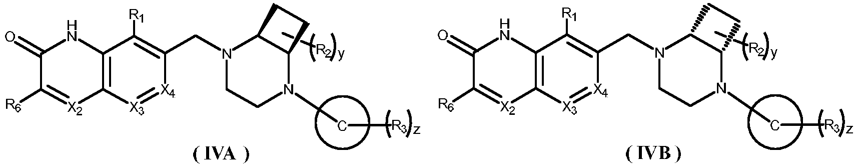 wherein
X₂, X₃, X₄, ring C, R₁, R₂, R₃, R₆, y, and z are as defined in general formula (I).

The present disclosure also provides a preferred embodiment in which the compound, the stereoisomer thereof, or the pharmaceutically acceptable salt thereof has a specific structure of general formula (IVC) and general formula (IVD): 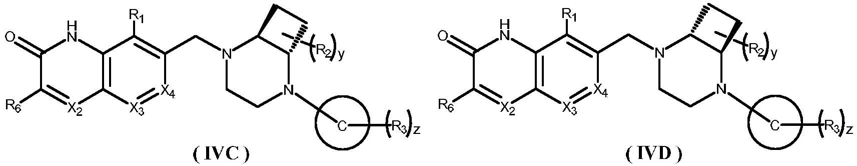 wherein
X₂, X₃, X₄, ring C, R₁, R₂, R₃, R₆, y, and z are as defined in general formula (I).

The present disclosure also provides a preferred embodiment in which the compound, the stereoisomer thereof, or the pharmaceutically acceptable salt thereof has a specific structure of general formula (V): 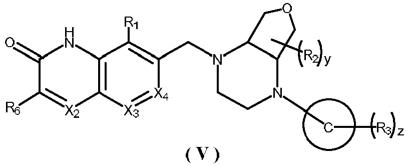 wherein
X₂, X₃, X₄, ring C, R₁, R₂, R₃, R₆, y, and z are as defined in general formula (I).

The present disclosure also provides a preferred embodiment in which the compound, the stereoisomer thereof, or the pharmaceutically acceptable salt thereof has a specific structure of general formula (VI): 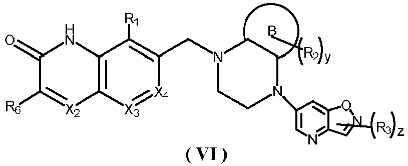 wherein
X₂, X₃, X₄, ring B, R₁, R₂, R₃, R₆, y, and z are as defined in general formula (I).

The present disclosure also provides a preferred embodiment in which the compound, the stereoisomer thereof, or the pharmaceutically acceptable salt thereof has a specific structure of general formula (VII): 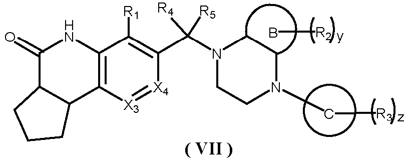 X₃, X₄, ring C, ring B, R₁, R₂, R₃, R₄, R₅, R₆, y, and z are as defined in general formula (I).

The present disclosure also provides a preferred embodiment in which the compound, the stereoisomer thereof, or the pharmaceutically acceptable salt thereof has a specific structure of general formula (VIII): 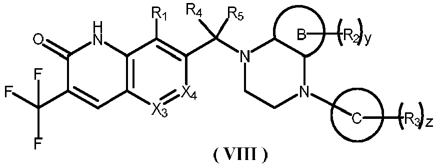 X₃, X₄, ring B, ring C, R₁, R₂, R₃, R₄, R₅, y, and z are as defined in general formula (I).

The present disclosure also provides a preferred embodiment in which the compound, the stereoisomer thereof, or the pharmaceutically acceptable salt thereof has a specific structure of general formula (IX): 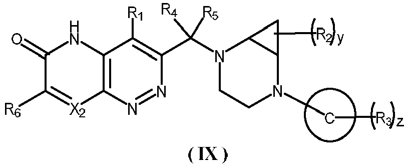 X₂, ring C, R₁, R₂, R₃, R₄, R₅, R₆, y, and z are as defined in general formula (I).

In a preferred embodiment of the present disclosure, the ring C is 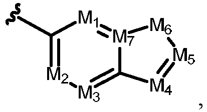 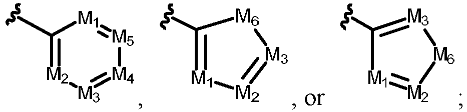 wherein
M₁, M₂, M₃, M₄, and M₅ are each independently N or CR₃;
each M₆ is independently N, NR₃, or CR₃R₃;
each M₇ is independently N or C;
R₃ is as defined in general formula (I).

In a further preferred embodiment of the present disclosure, the ring C is phenyl, furyl, thienyl, pyrrolyl, imidazolyl, pyrazolyl, triazolyl, tetrazolyl, oxazolyl, isoxazolyl, thiazolyl, isothiazolyl, pyridyl, pyrimidinyl, pyrazinyl, pyridazinyl, benzothiazolyl, benzisothiazolyl, benzoxazolyl, benzisoxazolyl, pyridopyrazolyl, pyridooxazolyl, pyridoisoxazolyl, pyridothiazolyl, pyridoisothiazolyl, or pyridopyrrolyl.

In a further preferred embodiment of the present disclosure, the ring C is phenyl, furyl, thienyl, pyrrolyl, imidazolyl, pyrazolyl, triazolyl, tetrazolyl, oxazolyl, isoxazolyl, thiazolyl, isothiazolyl, pyrimidinyl, pyrazinyl, pyridazinyl, benzothiazolyl, benzisothiazolyl, benzoxazolyl, benzisoxazolyl, pyridopyrazolyl, pyridooxazolyl, pyridoisoxazolyl, pyridothiazolyl, pyridoisothiazolyl, or pyridopyrrolyl.

In a further preferred embodiment of the present disclosure, the ring C is 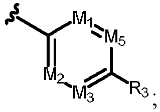 M₁, M₂, M₃, M₄, and M₅ are each independently N or CR₃;
each M₆ is independently N, NR₃, or CR₃R₃;
each M₇ is independently N or C;
R₃ is as defined in general formula (I).

In a further preferred embodiment of the present disclosure, the ring C is 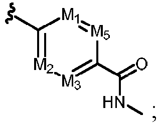 wherein
M₁, M₂, M₃, M₄, and M₅ are each independently N or CR₃;
each M₆ is independently N, NR₃, or CR₃R₃;
each M₇ is independently N or C;
R₃ is as defined in general formula (I).

In a preferred embodiment of the present disclosure, the R₃ is independently hydrogen, deuterium, halogen, hydroxyl, mercapto, nitro, cyano, amino, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, 5- to 10-membered heteroaryl, -ORₐ, - SRₐ, -NR_{b}Rₐ, -C(O)Rₐ, -C(O)NR_{b}Rₐ, -NR_{b}C(O)Rₐ, -S(O)Rₐ, -S(O)₂Rₐ, -S(O)₂NR_{b}Rₐ, - S(O)(=NR_{b})Rₐ, -N=S(=O)RₐR_{b}, or -NR_{b}S(O)₂Rₐ, wherein the amino, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, and 5- to 10-membered heteroaryl are optionally further substituted by one or more substituents selected from deuterium, halogen, hydroxyl, mercapto, nitro, cyano, amino, oxo, thio, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, 5- to 10-membered heteroaryl, C₃₋₈ cycloalkyloxy, 3- to 8-membered heterocyclyloxy, C₆₋₁₀ aryloxy, and 5- to 10-membered heteroaryloxy;
or, any two R₃ together with the atom to which they are attached form C₃₋₈ cycloalkyl, 3- to 8-membered heterocyclyl, C₆₋₁₀ aryl, or 5- to 10-membered heteroaryl, wherein the C₃₋₈ cycloalkyl, 3- to 8-membered heterocyclyl, C₆₋₁₀ aryl, and 5- to 10-membered heteroaryl are optionally further substituted by one or more substituents selected from deuterium, halogen, hydroxyl, mercapto, nitro, cyano, amino, oxo, thio, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, 5- to 10-membered heteroaryl, C₃₋₈ cycloalkyloxy, 3- to 8-membered heterocyclyloxy, C₆₋₁₀ aryloxy, and 5- to 10-membered heteroaryloxy;
Rₐ and R_{b} are each independently hydrogen, deuterium, halogen, hydroxyl, mercapto, nitro, cyano, amino, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, and 5- to 10-membered heteroaryl are optionally further substituted by one or more substituents selected from deuterium, halogen, hydroxyl, mercapto, nitro, cyano, amino, oxo, thio, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, 5- to 10-membered heteroaryl, C₃₋₈ cycloalkyloxy, 3- to 8-membered heterocyclyloxy, C₆₋₁₀ aryloxy, and 5- to 10-membered heteroaryloxy.

In a further preferred embodiment of the present disclosure, ring A is 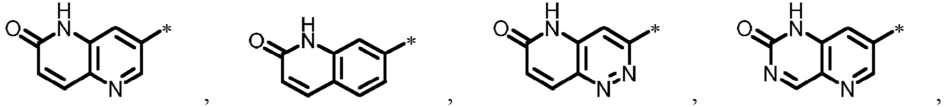 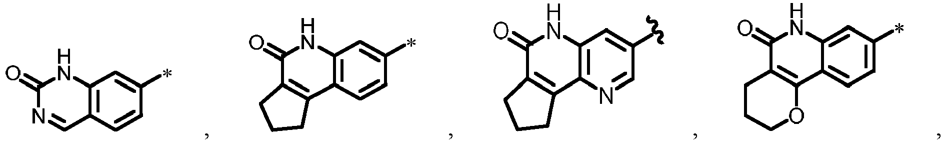 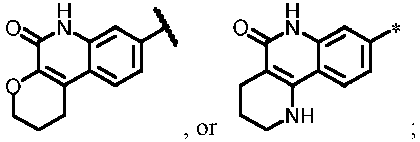
ring B is C₄₋₆ cycloalkyl or 5- to 6-membered heterocyclyl, the heteroatom in the 5- to 6-membered heterocyclyl is N, O, or S, and the number of heteroatoms is 1;
ring C is pyridyl or pyridoisoxazolyl;
R₁ is independently hydrogen, deuterium, halogen, C₁₋₆ alkyl, C₁₋₆ alkyl substituted by one or more halogens, C₃₋₆ cycloalkyl, or C₁₋₆ alkoxy;
or, any two R₁ together with the atom to which they are attached form C₃₋₆ cycloalkyl or 3- to 6-membered heterocyclyl, the heteroatom in the 3- to 6-membered heterocyclyl is N or O, and the number of heteroatoms is 1;
R₂ is independently hydrogen or deuterium;
R₃ is independently hydrogen, deuterium, halogen, C₁₋₆ alkyl, 5- to 6-membered heteroaryl, -(CH₂)ₙ₁C(O)NR_{b}Rₐ, or -(CH₂)ₙ₁NR_{b}Rₐ, wherein the 5- to 6-membered heteroaryl is optionally substituted by C₁₋₆ alkyl; the heteroatom in the 5- to 6-membered heterocyclyl is N and/or O, and the number of heteroatoms is 1 or 2;
Rₐ and R_{b} are each independently hydrogen, C₁₋₆ alkyl, or C₃₋₆ cycloalkyl;
L is -(CR₄R₅)ₙ₂-;
R₄ and R₅ are each independently hydrogen or deuterium;
x, y, and z are each independently an integer of 0 to 3;
n1 is an integer of 0 to 3;
n2 is 1; and
m1 is 0, 1, or 2.

In a further preferred embodiment of the present disclosure, ring B is C₄₋₆ cycloalkyl or 5- to 6-membered heterocyclyl, the heteroatom in the 5- to 6-membered heterocyclyl is N, O, or S, and the number of heteroatoms is 1.

In a further preferred embodiment of the present disclosure, ring C is phenyl, pyridyl, or pyridoisoxazolyl.

In a further preferred embodiment of the present disclosure, R₁ is independently halogen, C₁₋₆ alkyl, C₁₋₆ alkyl substituted by one or more halogens, C₃₋₆ cycloalkyl, or C₁₋₆ alkoxy;
or, any two R₁ together with the atom to which they are attached form C₃₋₆ cycloalkyl or 3- to 6-membered heterocyclyl, the heteroatom in the 3- to 6-membered heterocyclyl is N or O, and the number of heteroatoms is 1.

In a further preferred embodiment of the present disclosure, R₁ is independently hydrogen, deuterium, halogen, C₁₋₆ alkyl, C₁₋₆ alkyl substituted by one or more halogens, C₃₋₆ cycloalkyl, or C₁₋₆ alkoxy;
or, any two R₁ together with the atom to which they are attached form C₅ cycloalkyl or 6-membered heterocyclyl, the heteroatom in the 6-membered heterocyclyl is O, and the number of heteroatoms is 1.

In a further preferred embodiment of the present disclosure, R₁ is independently halogen, C₁₋₆ alkyl, C₁₋₆ alkyl substituted by one or more halogens, C₃₋₆ cycloalkyl, or C₁₋₆ alkoxy;
or, any two R₁ together with the atom to which they are attached form C₅ cycloalkyl or 6-membered heterocyclyl, the heteroatom in the 6-membered heterocyclyl is O, and the number of heteroatoms is 1.

In a further preferred embodiment of the present disclosure, R₁ is independently C₁₋₆ alkyl or C₃₋₆ cycloalkyl;
or, any two R₁ together with the atom to which they are attached form C₅ cycloalkyl or 6-membered heterocyclyl, the heteroatom in the 6-membered heterocyclyl is O, and the number of heteroatoms is 1.

In a further preferred embodiment of the present disclosure, R₁ is independently C₁₋₆ alkyl or trifluoromethyl; or, any two R₁ together with the atom to which they are attached form C₅ cycloalkyl.

In a further preferred embodiment of the present disclosure, R₂ is independently hydrogen or deuterium.

In a further preferred embodiment of the present disclosure, R₃ is independently hydrogen, deuterium, halogen, C₁₋₆ alkyl, 5- to 6-membered heteroaryl, -(CH₂)ₙ₁C(O)NR_{b}Rₐ, or -(CH₂)ₙ₁NR_{b}Rₐ, wherein the 5- to 6-membered heteroaryl is optionally substituted by C₁₋₆ alkyl; the heteroatom in the 5- to 6-membered heterocyclyl is N and/or O, and the number of heteroatoms is 1 or 2.

In a further preferred embodiment of the present disclosure, R₃ is independently hydrogen, halogen, or (CH₂)ₙ₁C(O)NR_{b}Rₐ.

In a further preferred embodiment of the present disclosure, R₃ is independently hydrogen or (CH₂)ₙ₁C(O)NR_{b}Rₐ.

In a further preferred embodiment of the present disclosure, Rₐ and R_{b} are each independently hydrogen, C₁₋₆ alkyl, or C₃₋₆ cycloalkyl; preferably, Rₐ and R_{b} are each independently hydrogen or C₁₋₆ alkyl, such as hydrogen or methyl.

In a further preferred embodiment of the present disclosure, x, y, and z are each independently an integer of 0 to 3.

In a preferred embodiment of the present disclosure, the ring B is cyclopropyl, cyclobutyl, cyclopentyl, or tetrahydrofuryl, and, when X₂ is CH, X₃ is N, and X₄ is CH, then ring B is not cyclopropyl.

In a preferred embodiment of the present disclosure, the n is 1, 2, 3, 4, 5, or 6, and, when X₂ is CH, X₃ is N, and X₄ is CH, then n is not 1.

In a further preferred embodiment of the present disclosure, R₁ is independently ethyl, methoxy, trifluoromethyl, difluoromethyl, cyclopropyl, fluorine, or methyl; or, any two R₁ together with the atom to which they are attached form cyclopentyl or 6-membered heterocyclyl; the heteroatom in the 6-membered heterocyclyl is N or O, and the number of heteroatoms is 1.

In a further preferred embodiment of the present disclosure, R₃ is independently hydrogen, 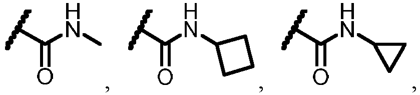 fluorine, methyl,

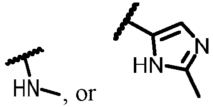

In a further preferred embodiment of the present disclosure, x is an integer of 0 to 3.

In a further preferred embodiment of the present disclosure, L is -(CR₄R₅)ₙ₂-; n2 is 2.

In a further preferred embodiment of the present disclosure, L is -(CR₄R₅)ₙ₂-; n2 is 1.

In a further preferred embodiment of the present disclosure, 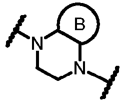 is 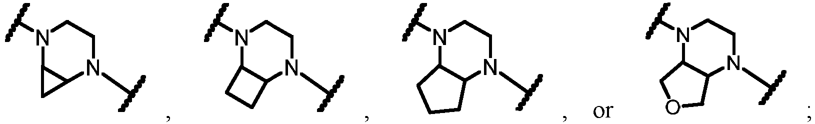 preferably 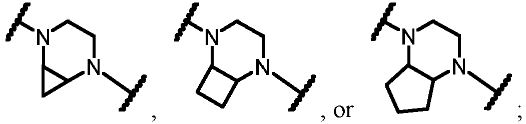 for example,

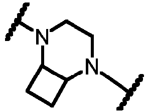

In a further preferred embodiment of the present disclosure, y is 0.

In a further preferred embodiment of the present disclosure, ring C is 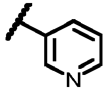 or

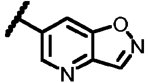

In a further preferred embodiment of the present disclosure, ring C is 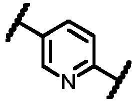 or

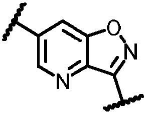

In a further preferred embodiment of the present disclosure, z is 1 or 2.

In a further preferred embodiment of the present disclosure, n1 is 0.

In a further preferred embodiment of the present disclosure, 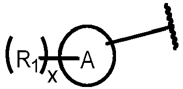 is 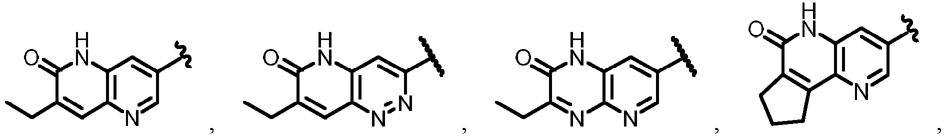 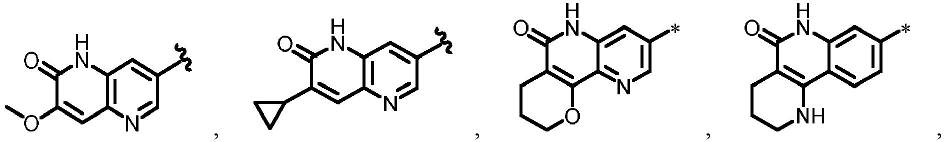 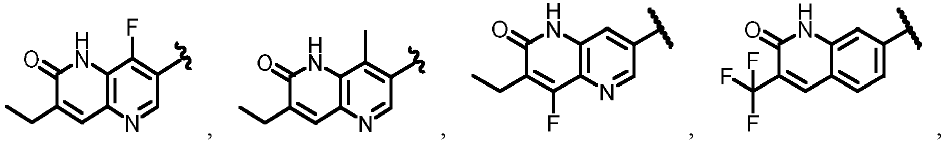 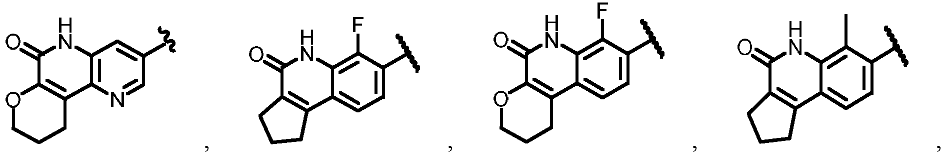 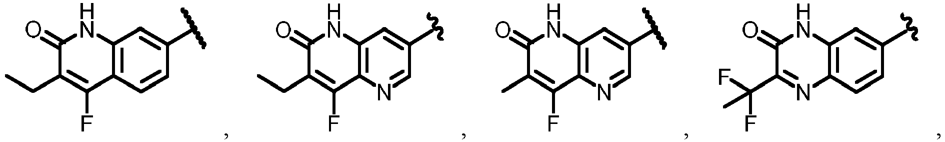 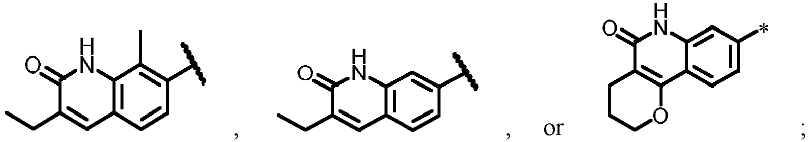 preferably 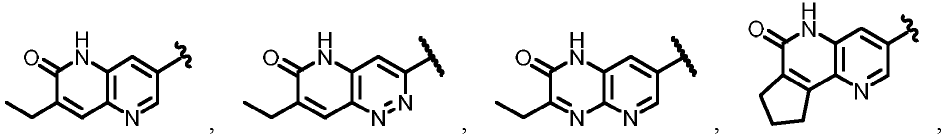 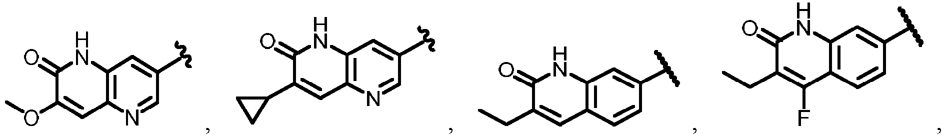 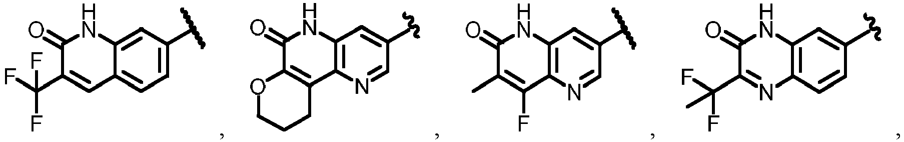 or 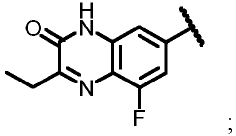 also preferably 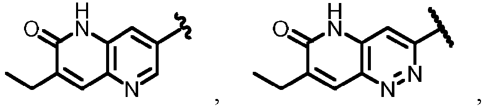 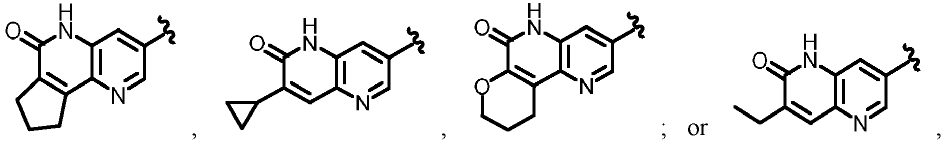 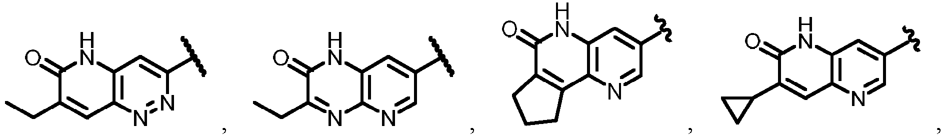 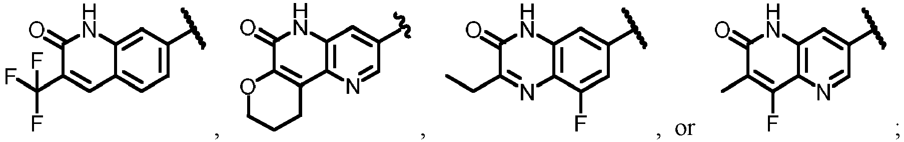 for example,

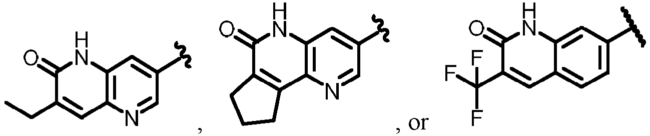

In a further preferred embodiment of the present disclosure, 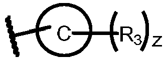 is 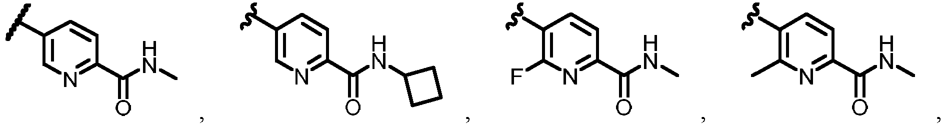 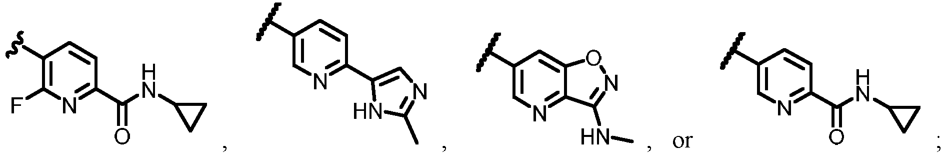 preferably 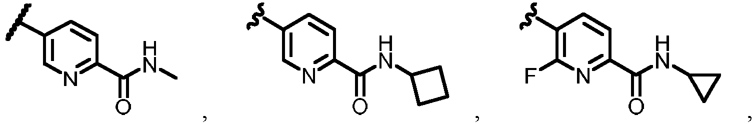 or 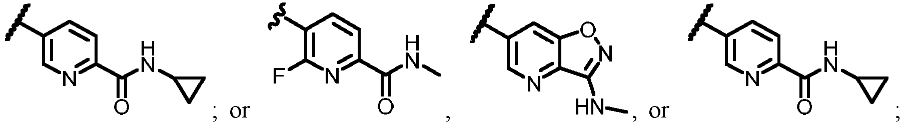 for example,

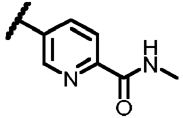

In a preferred embodiment of the present disclosure, the general formula (I), general formula (II), general formula (III), general formula (IV), general formula (V), general formula (VI), general formula (V), general formula (VI), general formula (VII), general formula (VIII), and/or general formula (IX) is in a cis-configuration or trans-configuration.

In a further preferred embodiment of the present disclosure, the general formula (I), general formula (II), general formula (III), general formula (IV), general formula (V), general formula (VI), general formula (V), general formula (VI), general formula (VII), general formula (VIII), and/or general formula (IX) is in a cis-configuration.

The present disclosure also provides a preferred embodiment in which the compound of each general formula as shown above, the stereoisomer thereof, or the pharmaceutically acceptable salt thereof is specifically selected from the following compounds:

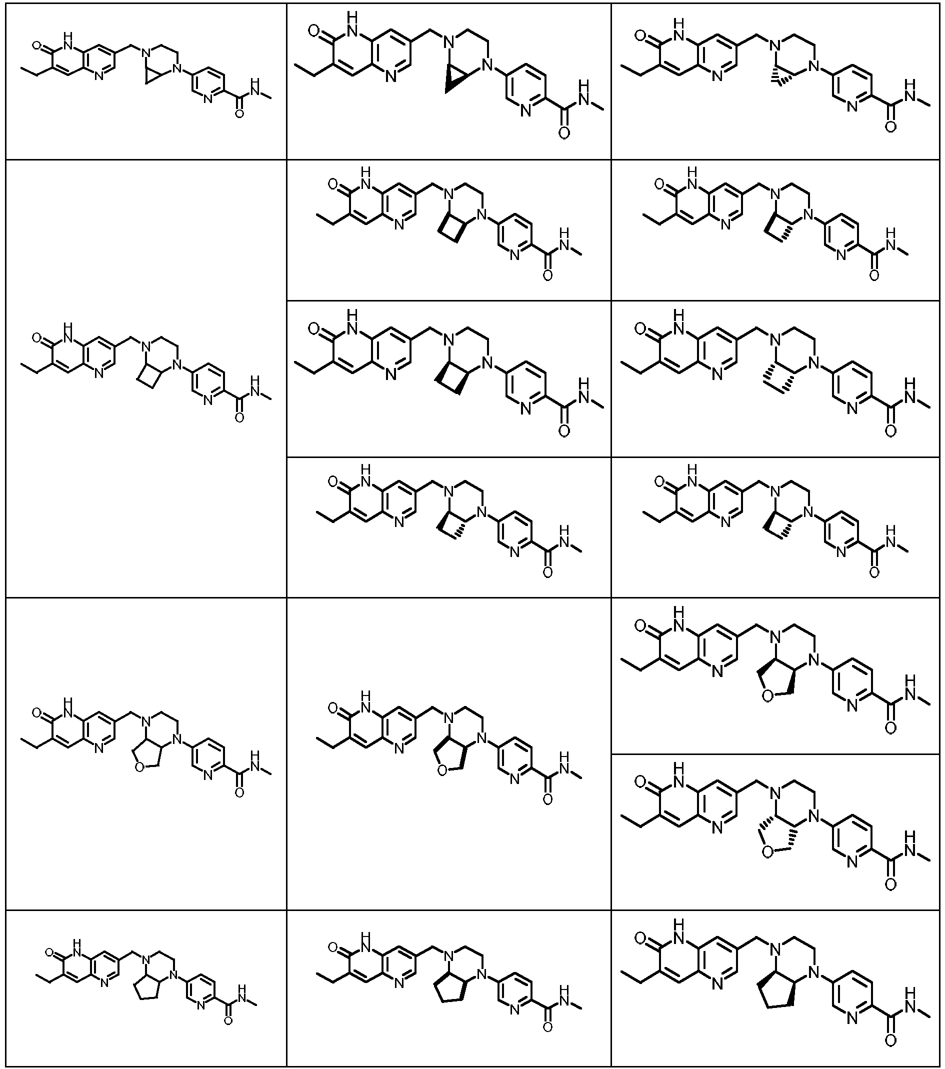

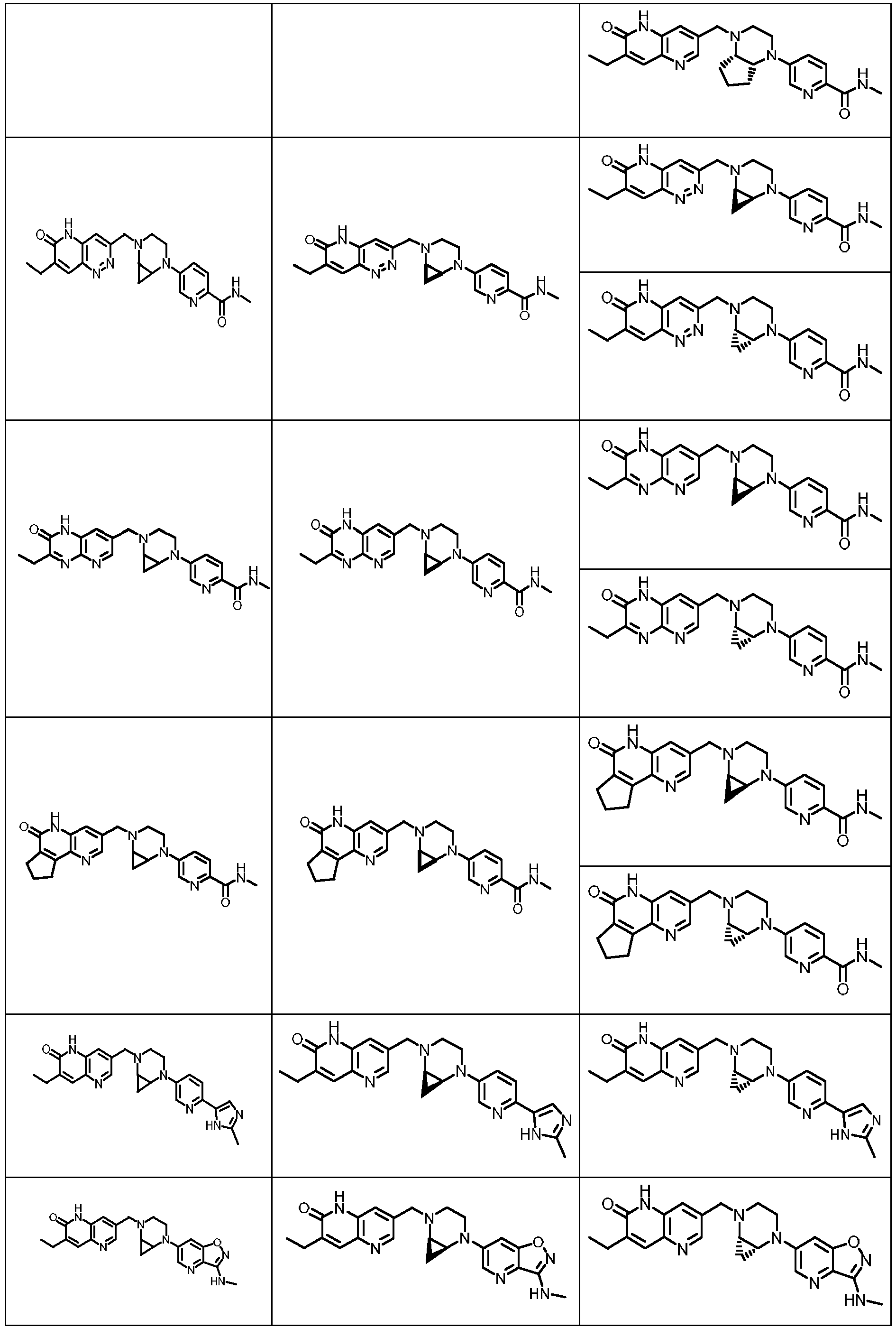

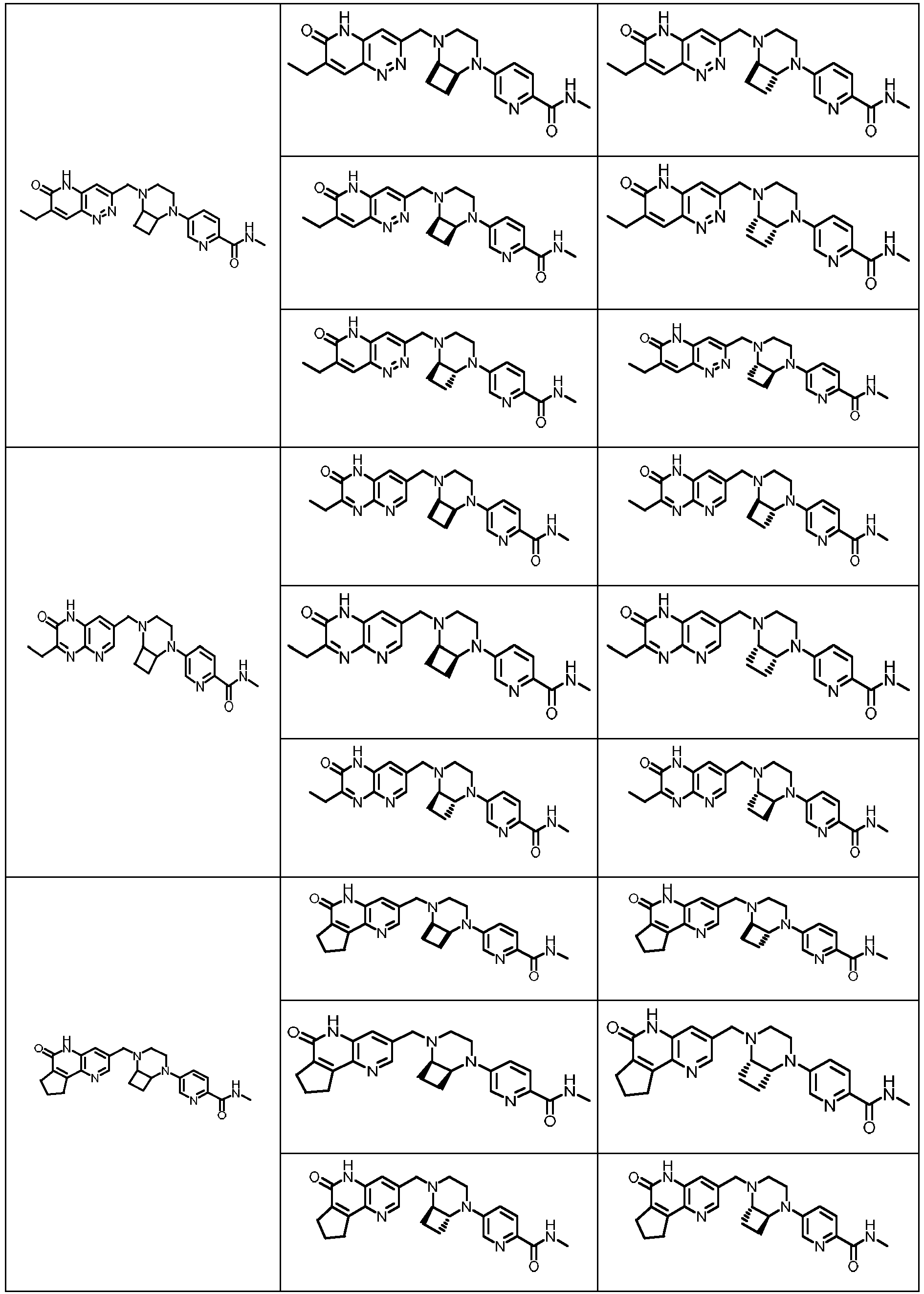

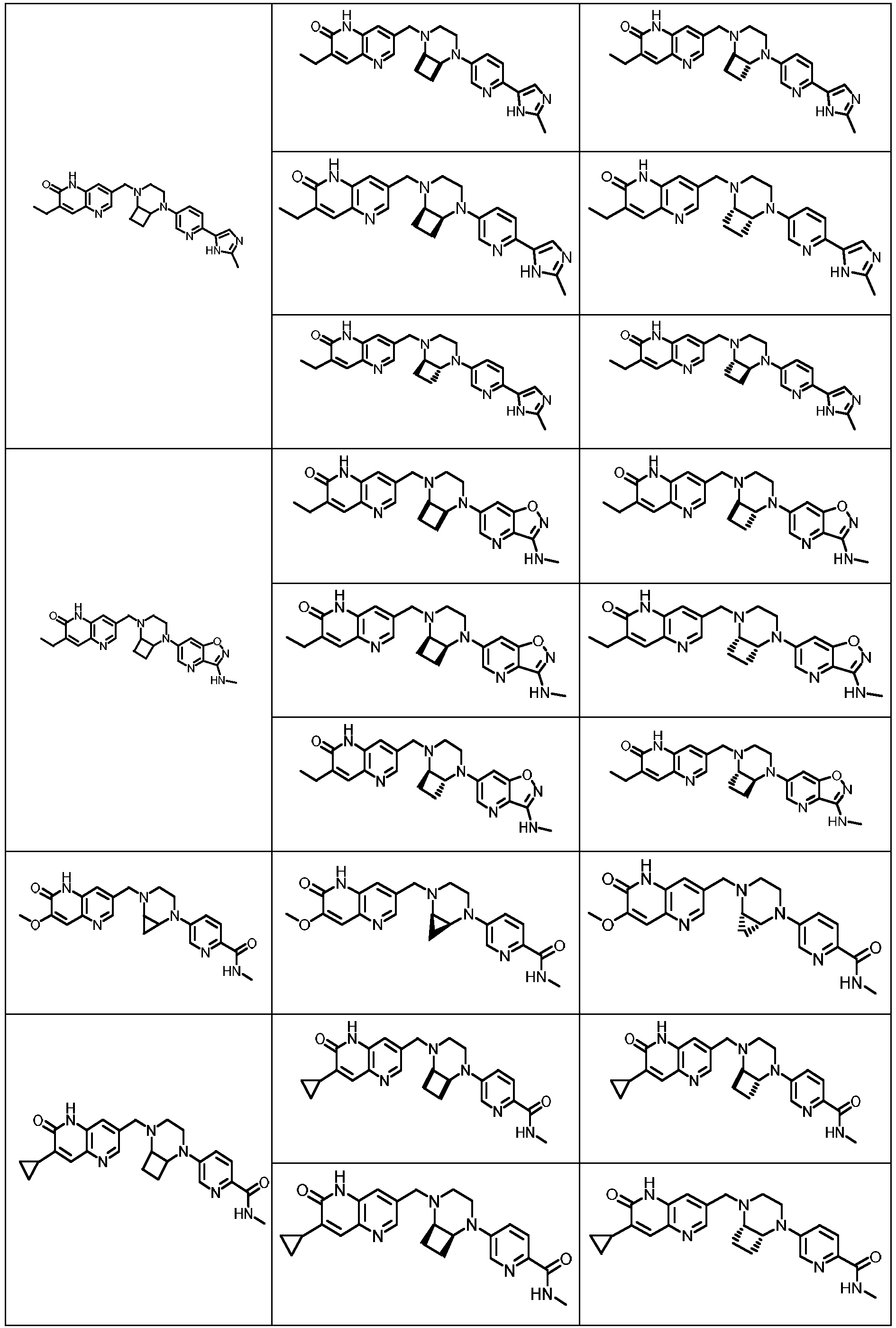

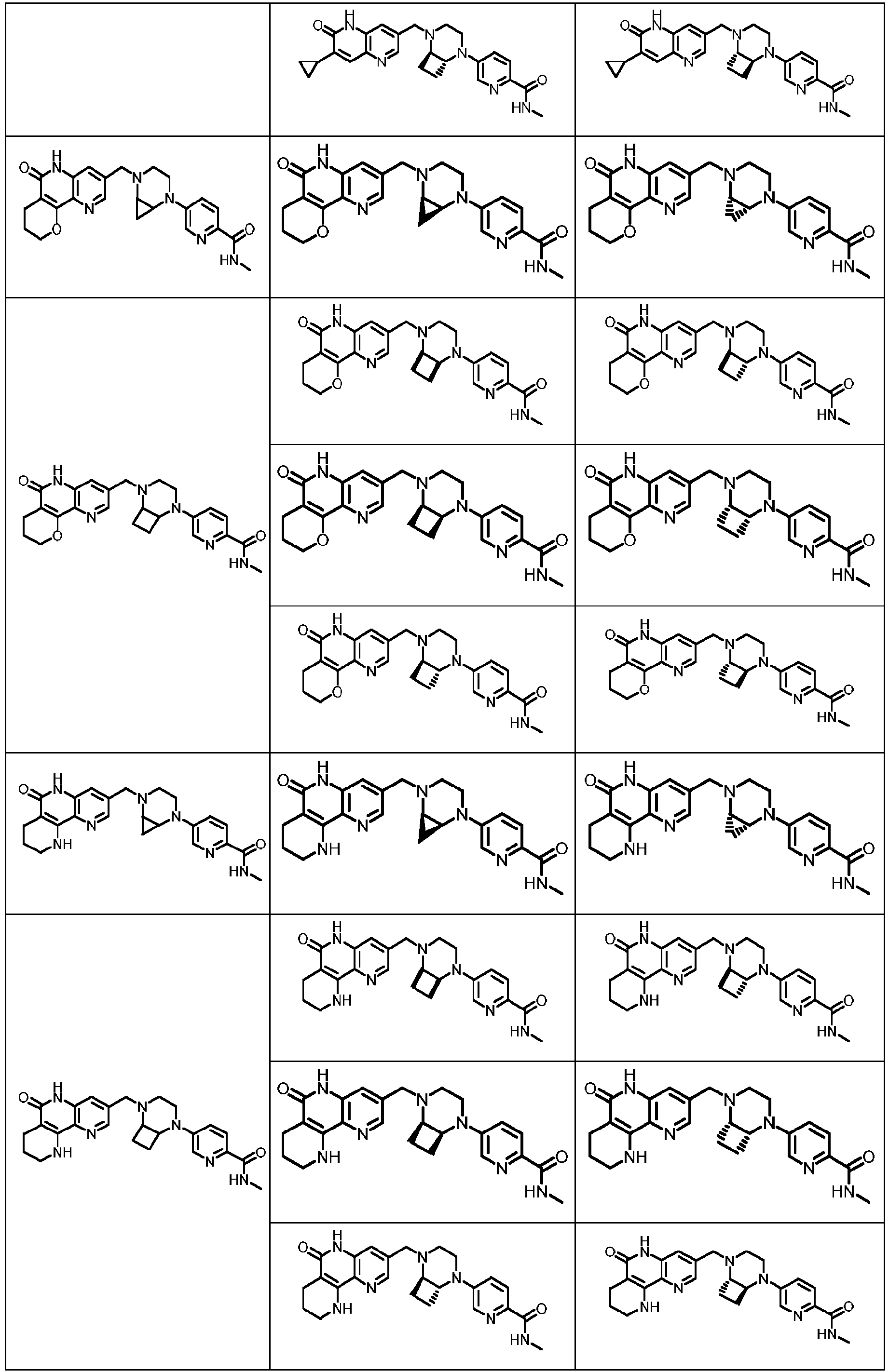

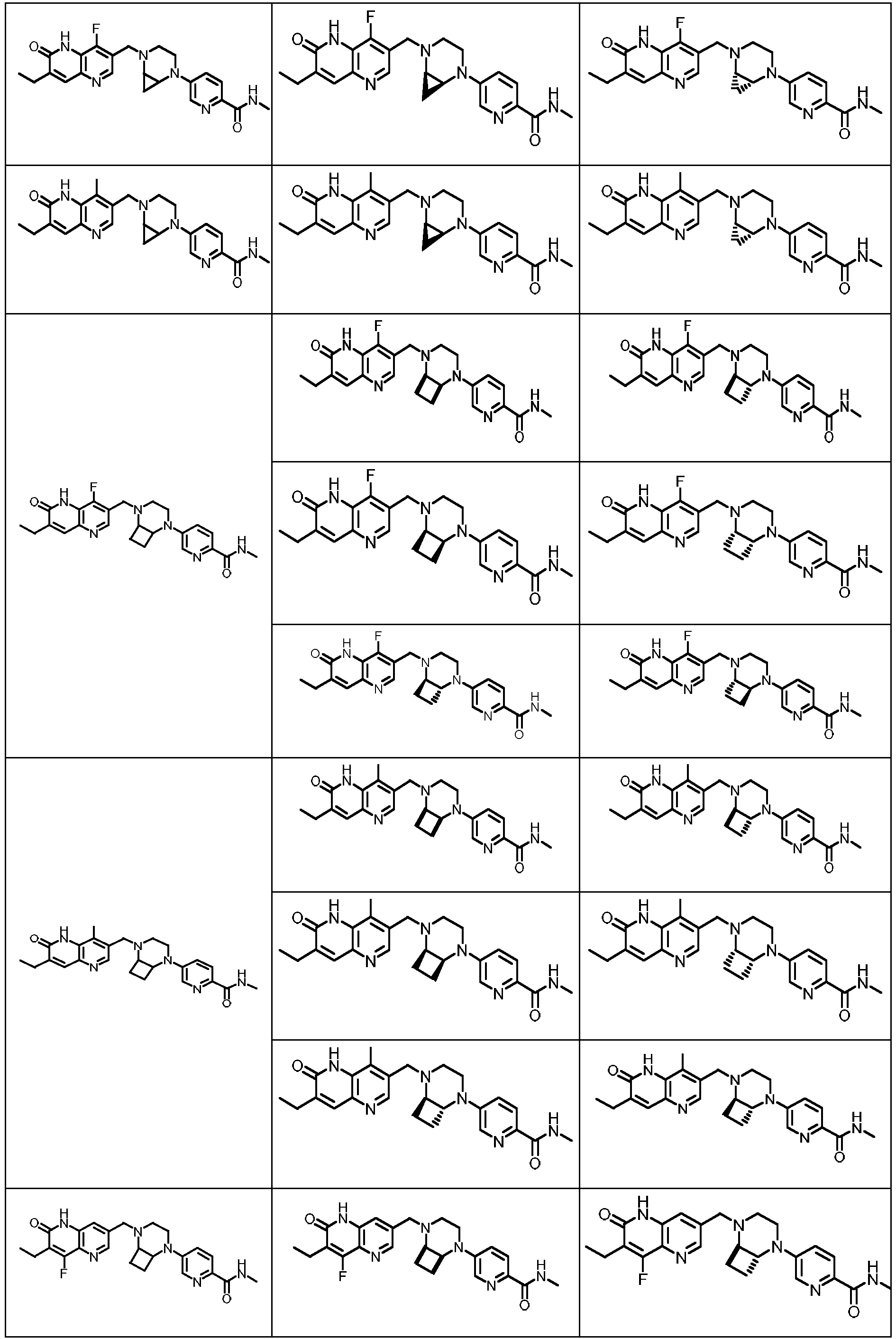

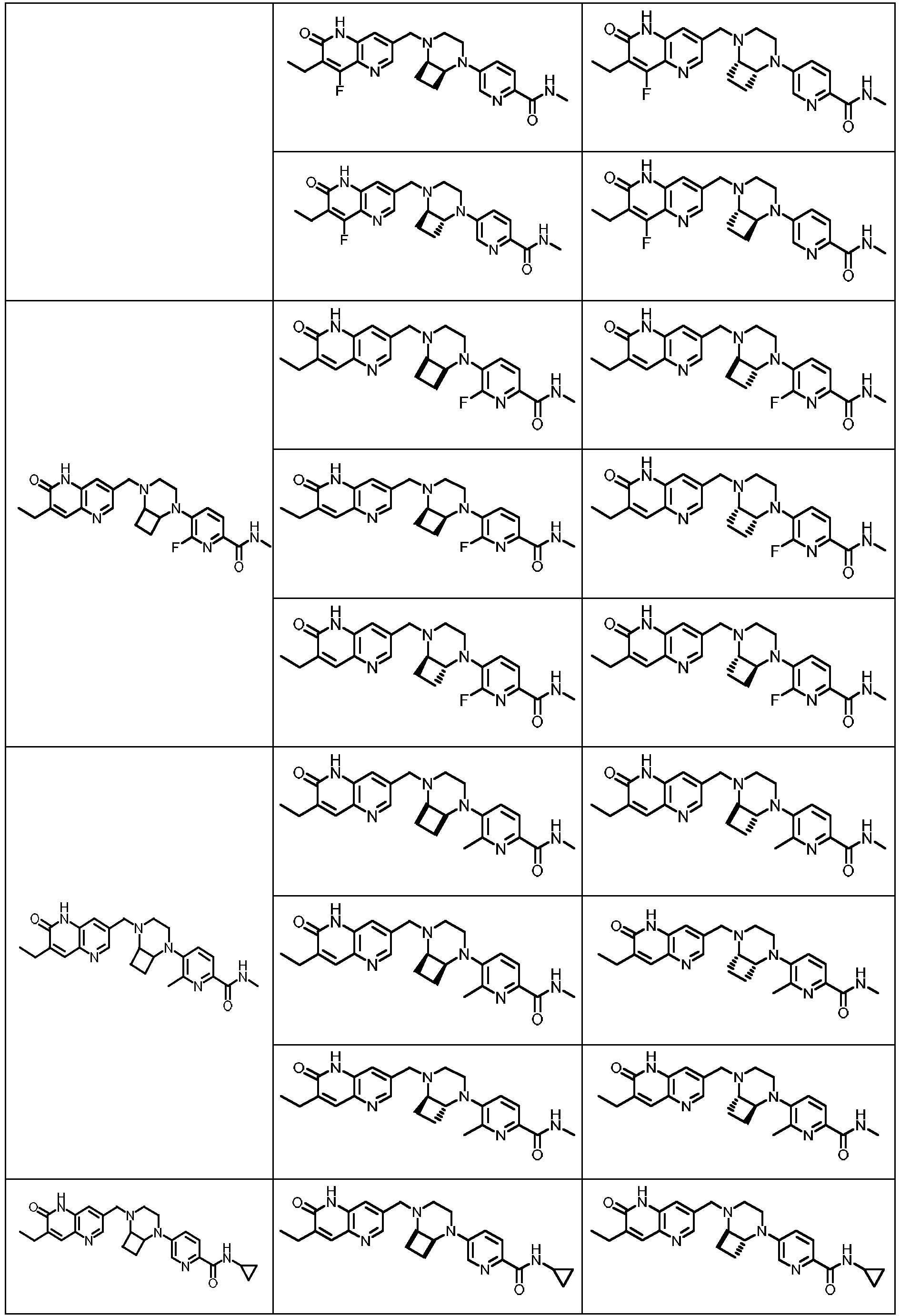

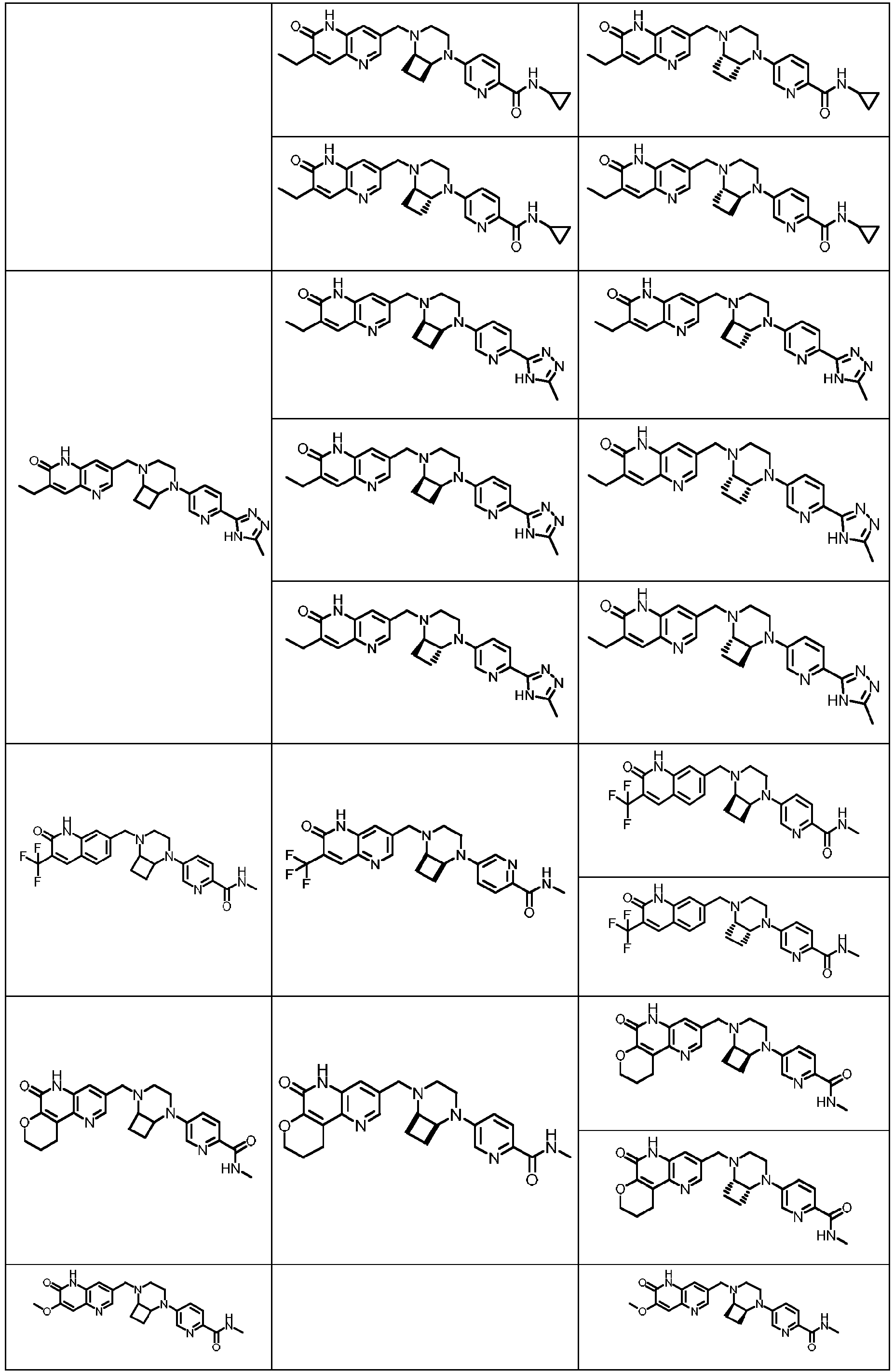

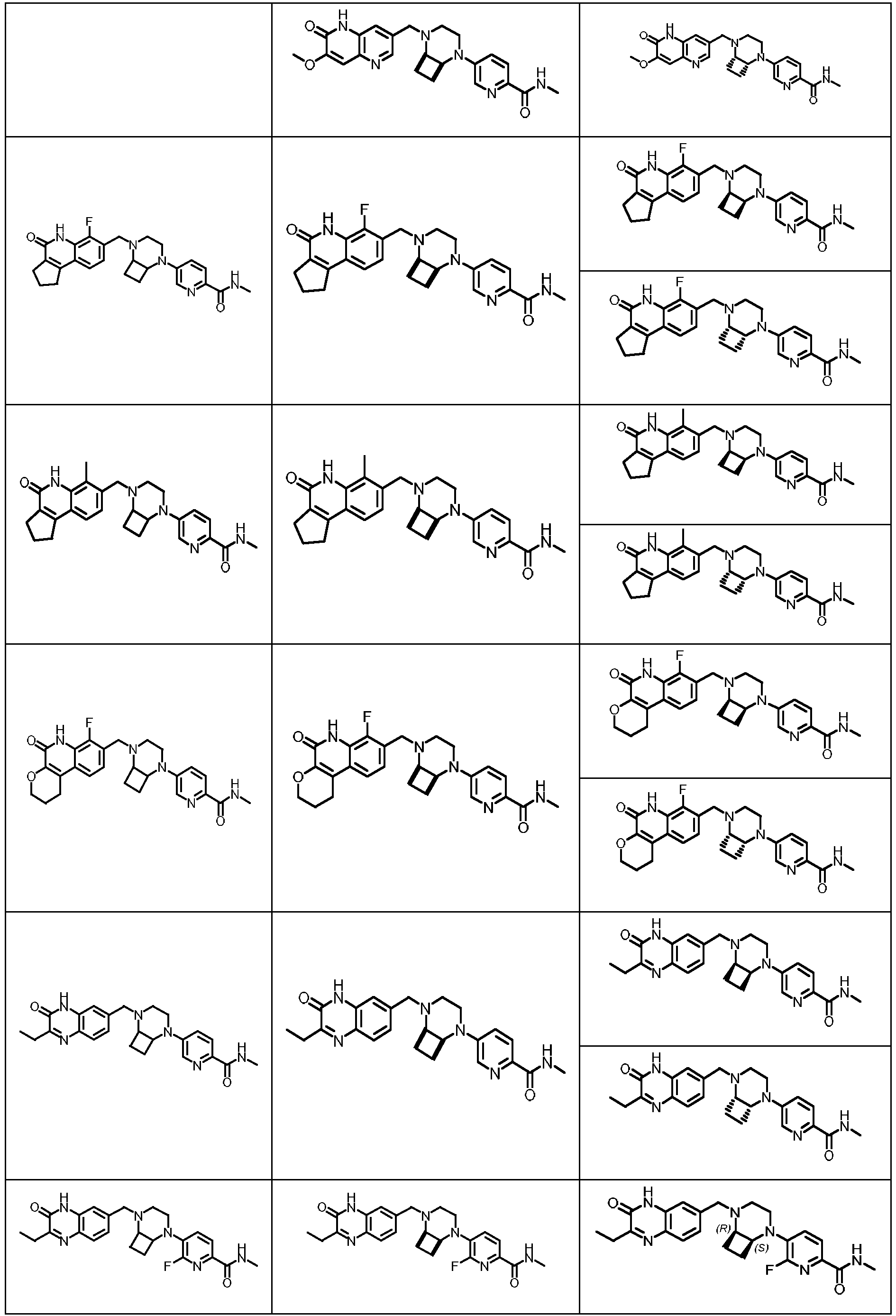

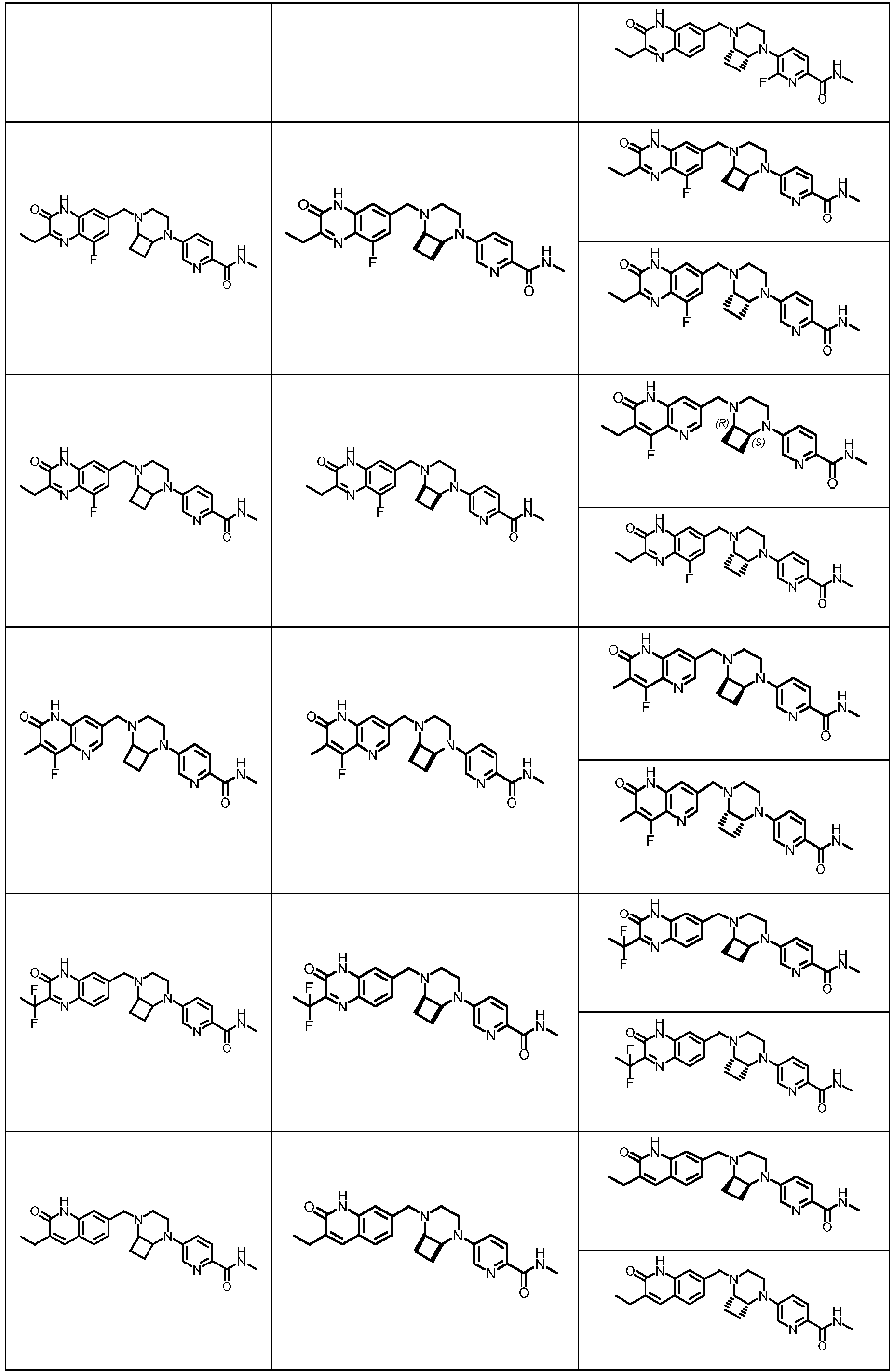

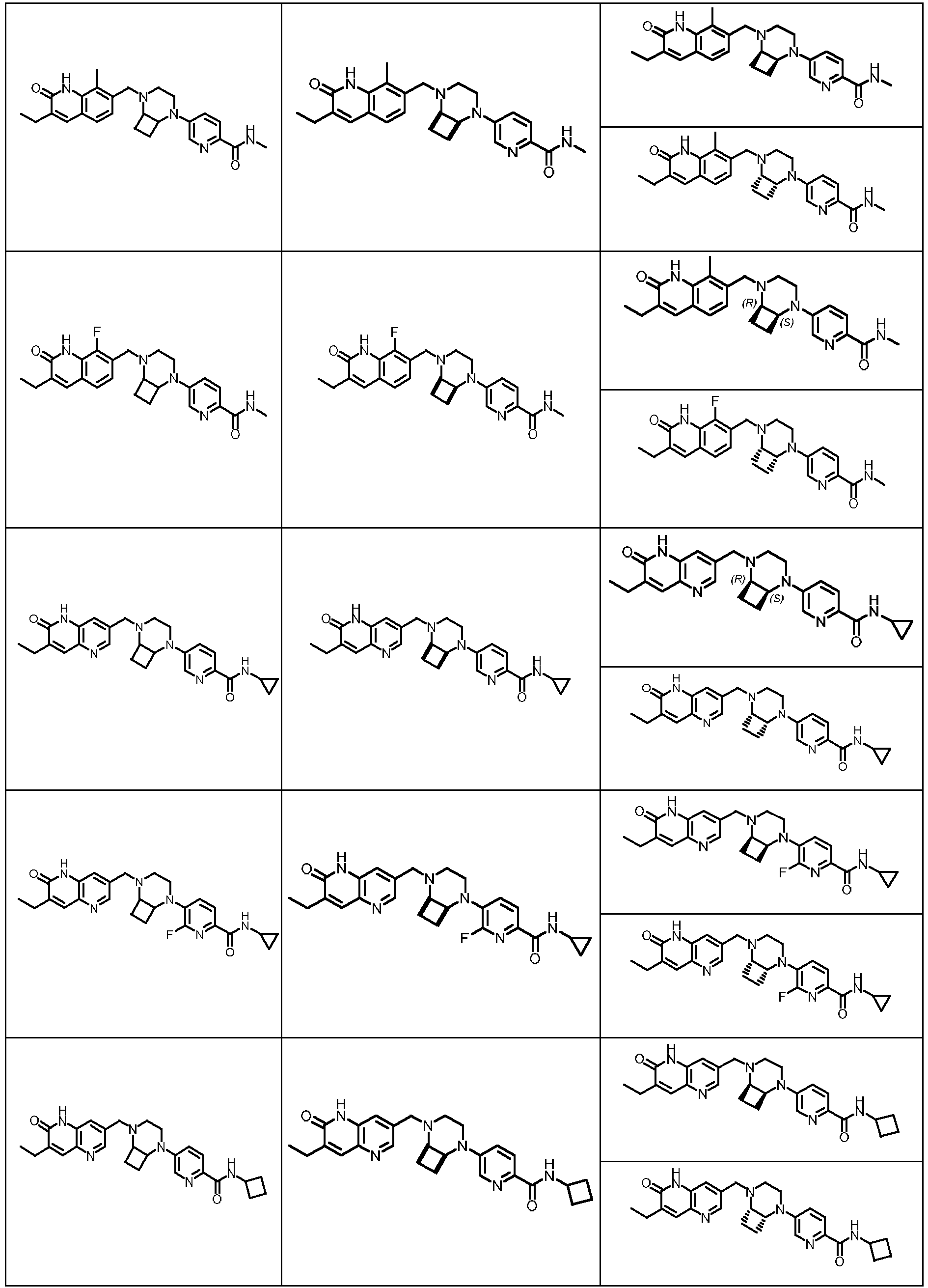

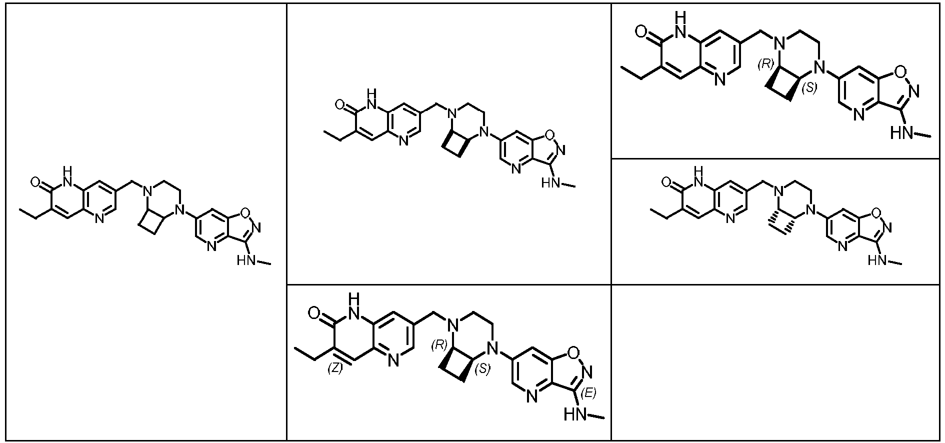

The present disclosure also relates to a method for preparing the compound of general formula (I), the stereoisomer thereof, or the pharmaceutically acceptable salt thereof, comprising the following steps: 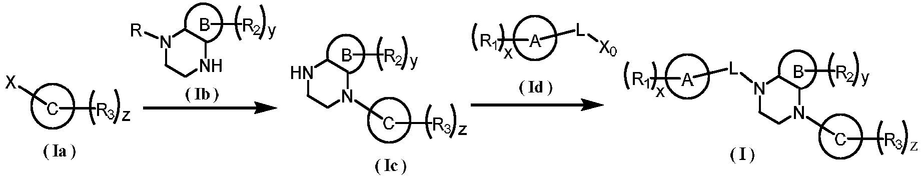
obtaining a compound of general formula (Ic), a prodrug thereof, a stereoisomer thereof, or a pharmaceutically acceptable salt thereof through a one-step or multi-step nucleophilic substitution reaction, coupling reaction, or other reaction between formula (Ia) and formula (Ib), either directly or through a further deprotection reaction;
obtaining the compound of general formula (I), the prodrug thereof, the stereoisomer thereof, or the pharmaceutically acceptable salt thereof through a one-step or multi-step nucleophilic substitution reaction, coupling reaction, or other reaction between formula (Ic) and formula (Id); optionally, the compound of general formula (I) is further subjected to chiral resolution to obtain a single-configuration compound, a prodrug thereof, a stereoisomer thereof, or a pharmaceutically acceptable salt thereof;
wherein
X is selected from halogen; preferably, X is fluorine, chlorine, bromine, or iodine;
X₀ is selected from halogen; preferably, X₀ is fluorine, chlorine, bromine, or iodine;
R is hydrogen or an amino protecting group; the amino protecting group includes, but is not limited to, *tert*-butoxycarbonyl (Boc), benzyloxycarbonyl (Cbz), *etc.*;
when R is hydrogen, formula (Ia) reacts with formula (Ib) to obtain the compound of general formula (Ic), the prodrug thereof, the stereoisomer thereof, or the pharmaceutically acceptable salt thereof directly;
when R is an amino protecting group, formula (Ia) reacts with formula (Ib) followed by a further deprotection reaction to obtain the compound of general formula (Ic), the prodrug thereof, the stereoisomer thereof, or the pharmaceutically acceptable salt thereof;
ring A, ring B, ring C, L, R₁, R₂, R₃, x, y, and z are as described in general formula (I).

The present disclosure also relates to a method for preparing a compound of general formula (IV), a prodrug thereof, a stereoisomer thereof, or a pharmaceutically acceptable salt thereof, wherein the method comprises the following steps: 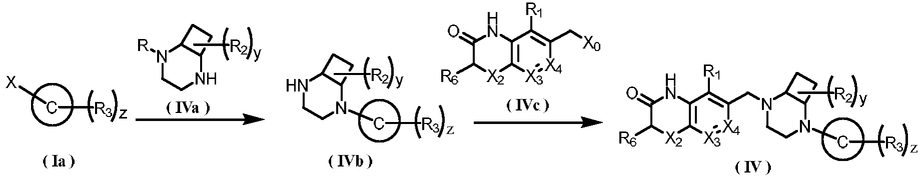
obtaining a compound of general formula (IVb), a prodrug thereof, a stereoisomer thereof, or a pharmaceutically acceptable salt thereof through a one-step or multi-step nucleophilic substitution reaction between formula (Ia) and formula (IVa);
obtaining the compound of general formula (IV), the prodrug thereof, the stereoisomer thereof, or the pharmaceutically acceptable salt thereof through a nucleophilic substitution reaction between formula (IVb) and formula (IVc); optionally, the compound of general formula (IV) is further subjected to chiral resolution to obtain a single-configuration compound, a prodrug thereof, a stereoisomer thereof, or a pharmaceutically acceptable salt thereof;
wherein
X is selected from halogen; preferably, X is fluorine, chlorine, bromine, or iodine;
X₀ is selected from halogen or hydroxyl; preferably, X₀ is fluorine, chlorine, bromine, or hydroxyl;
R is hydrogen or an amino protecting group; the amino protecting group includes, but is not limited to, *tert*-butoxycarbonyl (Boc), benzyloxycarbonyl (Cbz), *etc.*;
when R is hydrogen, formula (Ia) reacts with formula (Ib) to obtain the compound of general formula (Ic), the prodrug thereof, the stereoisomer thereof, or the pharmaceutically acceptable salt thereof directly;
when R is an amino protecting group, formula (Ia) reacts with formula (Ib) followed by a further deprotection reaction to obtain the compound of general formula (Ic), the prodrug thereof, the stereoisomer thereof, or the pharmaceutically acceptable salt thereof;
ring C, X₂, X₃, X₄, R₁, R₂, R₃, R₆, y, and z are as defined in general formula (IV).

The present disclosure also relates to a compound of formula (Ic), a stereoisomer thereof, or a salt thereof: 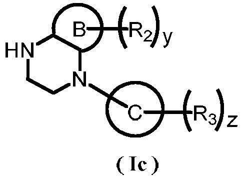 ring B, ring C, R₂, R₃, y, and z are as described in any embodiment of the present disclosure.

The present disclosure also protects a compound having any one of the following structures: 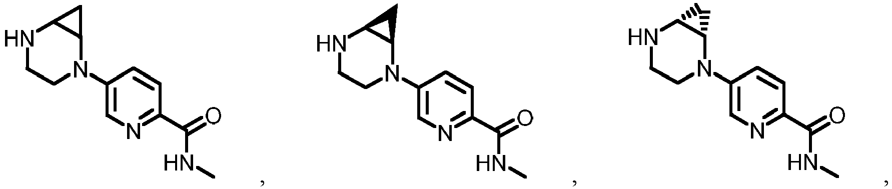 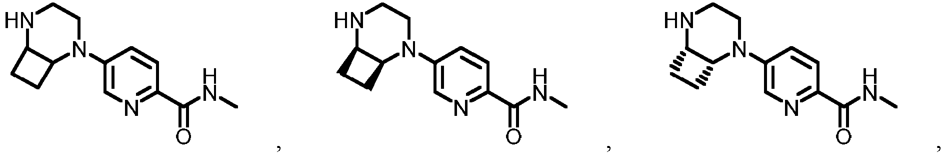 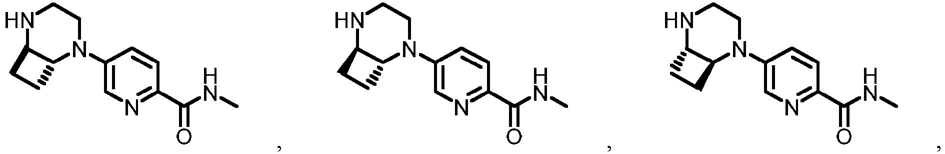 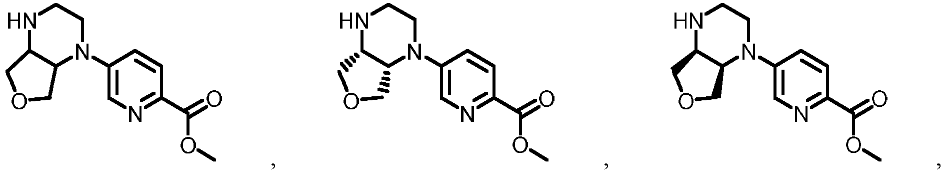 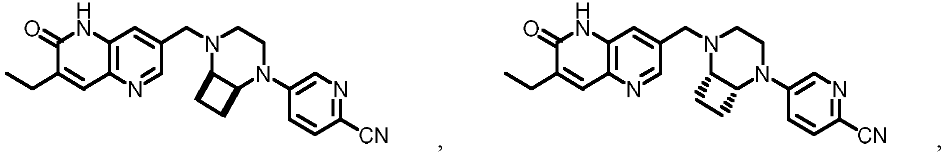 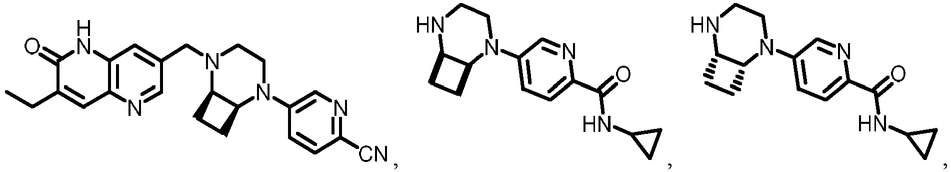 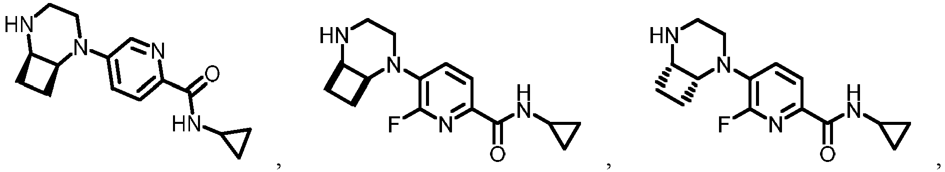 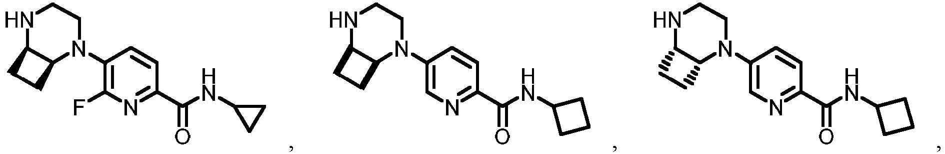 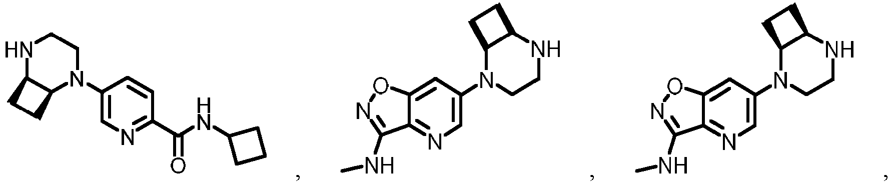 or

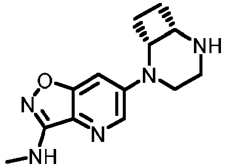

The present disclosure also relates to a pharmaceutical composition comprising a therapeutically effective dose of the compound of each general formula as shown above, the prodrug thereof, the stereoisomer thereof, or the pharmaceutically acceptable salt thereof, and one or more pharmaceutically acceptable carriers or excipients.

The present disclosure further relates to a use of the compound of general formula (I), the stereoisomer thereof, or the pharmaceutically acceptable salt thereof, and the pharmaceutical composition in the manufacture of a medicament for the treatment of a disease mediated by polyadenosine diphosphate ribose (ADP-ribose) polymerase (PARP).

The present disclosure further relates to a use of the compound of general formula (I), the stereoisomer thereof, or the pharmaceutically acceptable salt thereof, and the pharmaceutical composition in the manufacture of a medicament for the treatment of a disease related to a PARP1 inhibitor.

The present disclosure further relates to a use of the compound of general formula (I), the prodrug thereof, the stereoisomer thereof, or the pharmaceutically acceptable salt thereof, and the pharmaceutical composition in the manufacture of a medicament for the treatment of abnormal cell growth.

The present disclosure further relates to a use of the compound of general formula (I), the prodrug thereof, the stereoisomer thereof, or the pharmaceutically acceptable salt thereof, and the pharmaceutical composition in the manufacture of a medicament for the treatment of cancer, inflammation, a metabolic disease, a cardiovascular disease, an immunological disease, a mental disorder, and a related disease.

In another aspect, the present disclosure further relates to a method of the compound of general formula (I), the stereoisomer thereof, or the pharmaceutically acceptable salt thereof, and the pharmaceutical composition thereof for the treatment of a disease mediated by polyadenosine diphosphate ribose (ADP-ribose) polymerase (PARP).

The present disclosure further relates to a use of the compound of general formula (I), the stereoisomer thereof, or the pharmaceutically acceptable salt thereof, and the pharmaceutical composition in the manufacture of a medicament for the treatment of a disease related to a PARP1 inhibitor.

In another aspect, the present disclosure further relates to a method of the compound of general formula (I), the stereoisomer thereof, or the pharmaceutically acceptable salt thereof, and the pharmaceutical composition thereof for the treatment of abnormal cell growth.

In another aspect, the present disclosure further relates to a use of the compound of general formula (I), the stereoisomer thereof, or the pharmaceutically acceptable salt thereof, and the pharmaceutical composition thereof for the treatment of cancer, inflammation, a metabolic disease, a cardiovascular disease, an immunological disease, a mental disorder, and a related disease.

In another aspect, the present disclosure further relates to a use of the compound of general formula (I), the stereoisomer thereof, or the pharmaceutically acceptable salt thereof, and the pharmaceutical composition thereof for the treatment of a disease mediated by polyadenosine diphosphate ribose (ADP-ribose) polymerase (PARP).

In another aspect, the present disclosure further relates to a use of the compound of general formula (I), the stereoisomer thereof, or the pharmaceutically acceptable salt thereof, and the pharmaceutical composition thereof for the treatment of a disease related to a PARP1 inhibitor.

In another aspect, the present disclosure further relates to a use of the compound of general formula (I), the stereoisomer thereof, or the pharmaceutically acceptable salt thereof, and the pharmaceutical composition thereof for the treatment of abnormal cell growth.

In another aspect, the present disclosure further relates to a use of the compound of general formula (I), the stereoisomer thereof, or the pharmaceutically acceptable salt thereof, and the pharmaceutical composition thereof for the treatment of cancer, inflammation, a metabolic disease, a cardiovascular disease, an immunological disease, a mental disorder, and a related disease.

### Detailed description of the invention

Unless stated to the contrary, technical terms used in the specification and claims have the following meanings.

The term "alkyl" refers to a hydrocarbon group lacking one hydrogen atom among saturated aliphatic chain hydrocarbon groups, which is a linear or branched group containing 1 to 20 carbon atoms, preferably an alkyl group containing 1 to 8 carbon atoms, more preferably an alkyl group containing 1 to 6 carbon atoms, most preferably an alkyl group containing 1 to 3 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferred is a lower alkyl group containing 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *etc.* The aryl may be substituted or unsubstituted, and when it is substituted, a substituent may be substituted at any available point of attachment. The substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylic ester group, preferably methyl, ethyl, isopropyl, *tert*-butyl, haloalkyl, deuteroalkyl, alkoxy-substituted alkyl, and hydroxyl-substituted alkyl in the present disclosure.

The term "alkylene" refers to an alkyl group in which one hydrogen atom is further substituted, that is, a hydrocarbon group lacking two hydrogen atoms among saturated aliphatic chain hydrocarbon groups, which is a linear or branched group containing 1 to 20 carbon atoms, preferably an alkylene group containing 1 to 8 carbon atoms, more preferably an alkylene group containing 1 to 6 carbon atoms, most preferably an alkylene group containing 1 to 3 carbon atoms. Non-limiting examples include "methylene" (-CH₂-), "ethylene" (-(CH₂)₂-), "*n-*propylene" (-(CH₂)₃-), "isopropylene" (-(CH)(CH₃)(CH₂)-), "n-butylene" (-(CH₂)₄-), *etc.*

The term "alkenyl" refers to an unsaturated aliphatic hydrocarbon group containing at least two carbon atoms and at least one carbon-carbon double bond, which is a linear or branched group containing 2 to 20 carbon atoms, preferably an alkenyl group containing 2 to 8 carbon atoms, more preferably an alkenyl group containing 2 to 6 carbon atoms, most preferably an alkenyl group containing 2 to 4 carbon atoms. For example, vinyl, 1-propenyl, 2-propenyl, 1-butenyl, 2-butenyl, or 3-butenyl. The alkenyl may be substituted or unsubstituted, and when it is substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio.

The term "alkynyl" refers to an unsaturated aliphatic alkynyl group containing at least two carbon atoms and at least one carbon-carbon triple bond, which is a linear or branched group containing 2 to 20 carbon atoms, preferably an alkynyl group containing 2 to 8 carbon atoms, more preferably an alkynyl group containing 2 to 6 carbon atoms, most preferably an alkynyl group containing 2 to 4 carbon atoms. For example, ethynyl, 1-propynyl, 2-propynyl, 1-butynyl, 2-butynyl, or 3-butynyl. The alkynyl may be substituted or unsubstituted, and when it is substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio.

The term "cycloalkyl" refers to a saturated or partially unsaturated monocyclic or polycyclic non-aromatic cyclic hydrocarbon substituent. The ring atoms of cycloalkyl contain 3 to 20 carbon atoms, preferably 3 to 12 carbon atoms, more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, *etc.*, preferably cyclopropyl, cyclobutyl, cyclohexyl, cyclopentyl, and cycloheptyl; polycyclic cycloalkyl includes spirocycloalkyl, fused cycloalkyl, bridged cycloalkyl, *etc.*

The term "spirocycloalkyl" refers to a polycyclic group containing 5 to 20 carbon atoms with one carbon atom (called spiro atom) shared between monocyclic rings. It may contain one or more double bonds and is not aromatic as a whole (*i.e*., inability to form a conjugated π-electron system as a whole), but one or more of the rings may have a conjugated π-electron system. The ring atoms of spirocycloalkyl are preferably 6 to 14 carbon atoms, more preferably 7 to 10 carbon atoms. Spirocycloalkyl is divided into monospirocycloalkyl, bispirocycloalkyl, or polyspirocycloalkyl according to the number of spiro atoms shared between rings, preferably monospirocycloalkyl and bispirocycloalkyl. It is more preferably 4-membered/4-membered, 4-membered/5-membered, 4-membered/6-membered, 5-membered/5-membered, or 5-membered/6-membered monospirocycloalkyl. Non-limiting examples of spirocycloalkyl include: 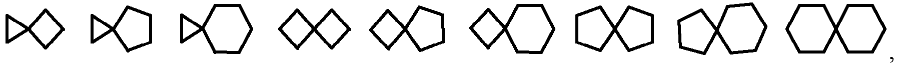 *etc.*

The term "fused cycloalkyl" refers to a all-carbon polycyclic group containing 5 to 20 carbon atoms in which each ring in the system shares an adjacent pair of carbon atoms with the other ring in the system. It may contain one or more double bonds in one or more of the rings and is not aromatic as a whole (*i.e.*, inability to form a conjugated π-electron system as a whole), but one or more of the rings may have a conjugated π-electron system. The ring atoms of fused cycloalkyl are preferably 6 to 14 carbon atoms, more preferably 7 to 10 carbon atoms. According to the number of constituent rings, it can be divided into bicyclic, tricyclic, tetracyclic, or polycyclic fused cycloalkyl, preferably bicyclic or tricyclic, more preferably 5-membered/5-membered or 5-membered/6-membered bicycloalkyl. Non-limiting examples of fused cycloalkyl include: 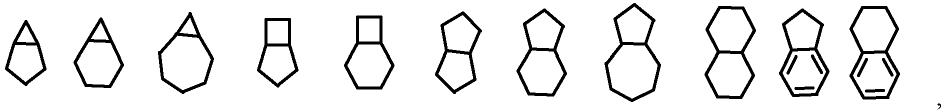 *etc.*

The term "bridged cycloalkyl" refers to a all-carbon polycyclic group containing 5 to 20 carbon atoms in which any two rings share two non-directly attached carbon atoms. It may contain one or more double bonds and is not aromatic as a whole (*i.e*., inability to form a conjugated π-electron system as a whole), but one or more of the rings may have a conjugated π-electron system. The ring atoms of bridged cycloalkyl are preferably 6 to 14 carbon atoms, more preferably 7 to 10 carbon atoms. According to the number of constituent rings, it can be divided into bicyclic, tricyclic, tetracyclic, or polycyclic bridged cycloalkyl, preferably bicyclic, tricyclic, or tetracyclic, more preferably bicyclic or tricyclic bridged cycloalkyl. Non-limiting examples of bridged cycloalkyl include: 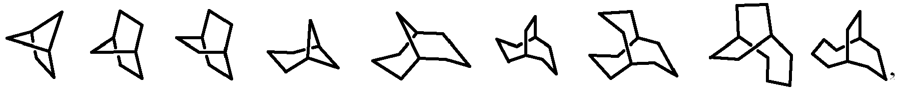 *etc.*

The cycloalkyl ring can be fused to a cycloalkyl ring or an aryl ring, wherein the ring attached to the parent structure may be a cycloalkyl ring or an aryl ring. Non-limiting examples include indanyl, tetrahydronaphthyl, benzocycloheptyl, *etc.* The cycloalkyl may be substituted or unsubstituted, and when it is substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylic ester group.

The term "heterocyclyl" refers to a saturated or partially unsaturated monocyclic or polycyclic non-aromatic cyclic hydrocarbon substituent containing 3 to 20 ring atoms, wherein one or more of the ring atoms are heteroatoms selected from nitrogen, oxygen, phosphorus, boron, or S(O)ₘ (where m is an integer of 0 to 2), but excluding the ring moiety of -O-O-, -O-S-, or -S-S-, and the remaining ring atoms are carbon. The heterocyclyl preferably contains 3 to 12 ring atoms, 1 to 4 of which are heteroatoms; more preferably, it contains 3 to 8 ring atoms; most preferably, it contains 3 to 8 ring atoms. Non-limiting examples of monocyclic heterocyclyl include oxetanyl, tetrahydropyranyl, azepanyl, pyrrolidinyl, imidazolidinyl, tetrahydrofuryl, tetrahydropyranyl, tetrahydrothienyl, dihydroimidazolyl, dihydrofuryl, dihydropyrazolyl, dihydropyrrolyl, piperidinyl, pyridonyl, piperazinyl, morpholinyl, thiomorpholinyl, homopiperazinyl, pyranyl, *etc.*, preferably oxetanyl, tetrahydrofuryl, tetrahydropyranyl, azepanyl, piperidinyl, pyridonyl, and piperazinyl. Polycyclic heterocyclyl includes spiroheterocyclyl, fused heterocyclyl, bridged heterocyclyl, *etc*; wherein the spiroheterocyclyl, fused heterocyclyl, and bridged heterocyclyl groups involved are optionally attached to other groups via a single bond, or further fused to other cycloalkyl, heterocyclyl, aryl, and heteroaryl groups via any two or more atoms on the ring.

The term "spiroheterocyclyl" refers to a polycyclic heterocyclic group containing 5 to 20 ring atoms with one atom (called spiro atom) shared between monocyclic rings, wherein one or more of the ring atoms are heteroatoms selected from nitrogen, oxygen, phosphorus, boron, or S(O)ₘ (where m is an integer of 0 to 2), and the remaining ring atoms are carbon. It may contain one or more double bonds and is not aromatic as a whole (i.e., inability to form a conjugated π-electron system as a whole), but one or more of the rings may have a conjugated π-electron system. The ring atoms of spiroheterocyclyl are preferably 6- to 14-membered, more preferably 7- to 10-membered. Spiroheterocyclyl is divided into monospiroheterocyclyl, bispiroheterocyclyl, or polyspiroheterocyclyl according to the number of spiro atoms shared between rings, preferably monospiroheterocyclyl and bispiroheterocyclyl. It is more preferably 4-membered/4-membered, 4-membered/5-membered, 4-membered/6-membered, 5-membered/5-membered, or 5-membered/6-membered monospiroheterocyclyl. Non-limiting examples of spiroheterocyclyl include: 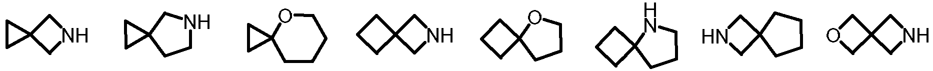 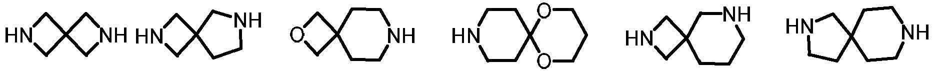 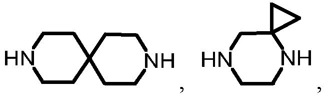 *etc.*

The term "fused heterocyclyl" refers to a polycyclic heterocyclic group containing 5 to 20 ring atoms in which each ring in the system shares an adjacent pair of atoms with the other ring in the system, wherein one or more of the ring atoms are heteroatoms selected from nitrogen, oxygen, phosphorus, boron, or S(O)ₘ (where m is an integer of 0 to 2), and the remaining ring atoms are carbon. It may contain one or more double bonds in one or more of the rings and is not aromatic as a whole (*i.e.*, inability to form a conjugated π-electron system as a whole), but one or more of the rings may have a conjugated π-electron system. The ring atoms of fused heterocyclyl are preferably 6- to 14-membered, more preferably 7- to 10-membered. According to the number of constituent rings, it can be divided into bicyclic, tricyclic, tetracyclic, or polycyclic fused heterocyclyl, preferably bicyclic or tricyclic, more preferably 5-membered/5-membered or 5-membered/6-membered bicyclic fused heterocyclyl. Non-limiting examples of fused heterocyclyl include: 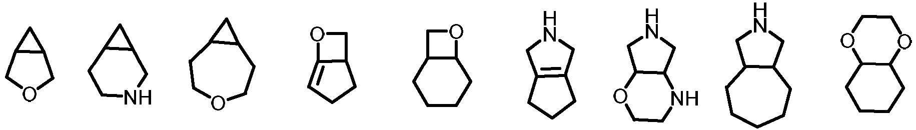 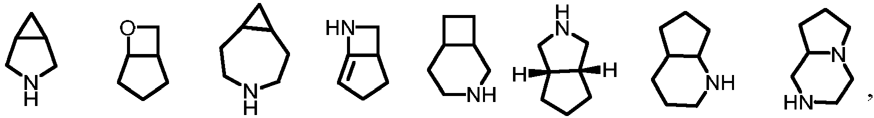 *etc.*

The term "bridged heterocyclyl" refers to a polycyclic heterocyclic group containing 5 to 20 ring atoms in which any two rings share two non-directly attached atoms, wherein one or more of the ring atoms are heteroatoms selected from nitrogen, oxygen, phosphorus, boron, or S(O)ₘ (where m is an integer of 0 to 2), and the remaining ring atoms are carbon. It may contain one or more double bonds and is not aromatic as a whole (*i.e*., inability to form a conjugated π-electron system as a whole), but one or more of the rings may have a conjugated π-electron system. The ring atoms of bridged heterocyclyl are preferably 6- to 14-membered, more preferably 7- to 10-membered. According to the number of constituent rings, it can be divided into bicyclic, tricyclic, tetracyclic, or polycyclic bridged heterocyclyl, preferably bicyclic, tricyclic, or tetracyclic, more preferably bicyclic or tricyclic bridged heterocyclyl. Non-limiting examples of bridged heterocyclyl include: 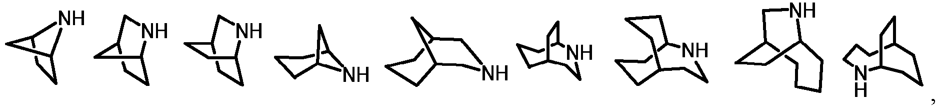 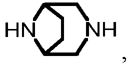 *etc.*

The heterocyclyl ring can be fused to a cycloalkyl ring, a heterocyclyl ring, an aryl ring, or a heteroaryl ring, wherein the ring attached to the parent structure may be a cycloalkyl ring, a heterocyclyl ring, an aryl ring, or a heteroaryl ring. Non-limiting examples include: 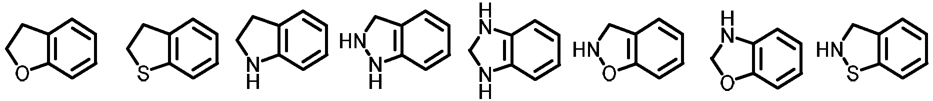 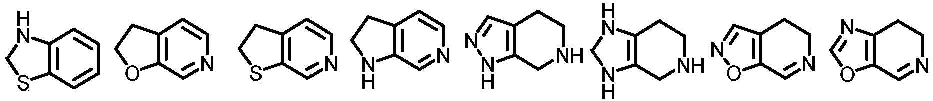 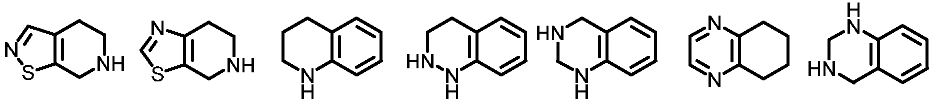 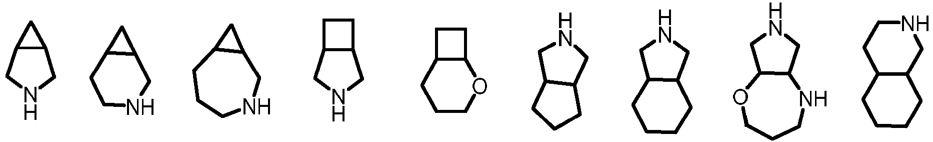 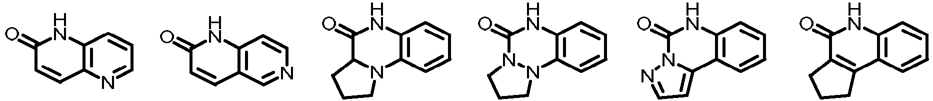 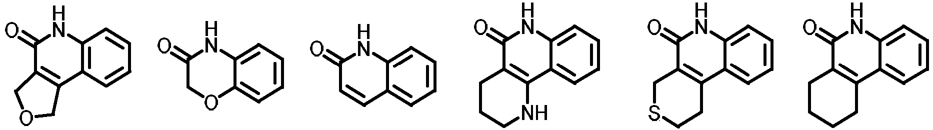 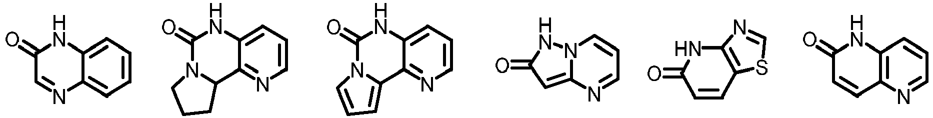 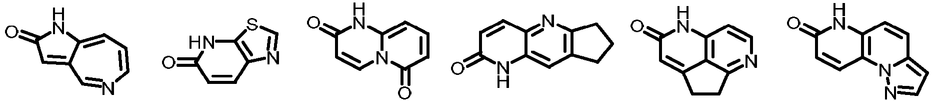 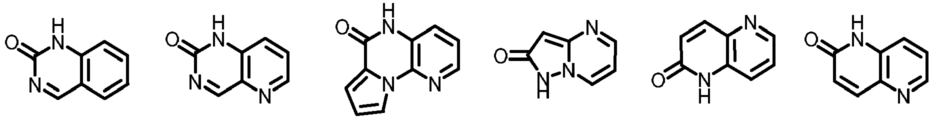 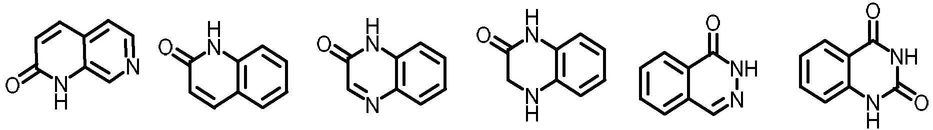 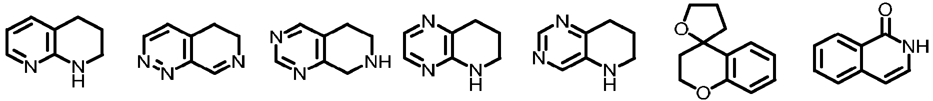 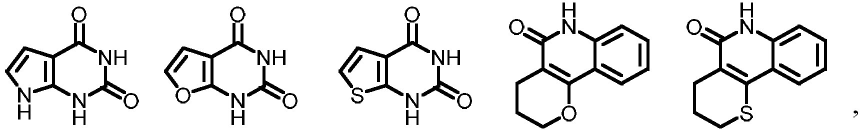 *etc.*

The heterocyclyl may be substituted or unsubstituted, and when it is substituted, the substituent is preferably one or more of the following groups independently selected from oxo (=O), thio (=S), alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylic ester group.

The term "aryl" refers to a 6- to 20-membered all-carbon monocyclic or fused polycyclic (*i.e.*, a ring sharing an adjacent pair of carbon atoms) group having a conjugated π-electron system. The ring atoms of aryl are preferably 6- to 14-membered, more preferably 6- to 10-membered, such as phenyl and naphthyl. It is more preferably phenyl.

The aryl may be substituted or unsubstituted, and when it is substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylic ester group.

The term "heteroaryl" refers to a heteroaromatic system containing 1 to 4 heteroatoms and 5 to 20 ring atoms, wherein the heteroatoms are selected from oxygen, sulfur, nitrogen, *etc.* Heteroaryl is divided into monocyclic heteroaryl and polycyclic heteroaryl; the ring atoms of heteroaryl are preferably 5- to 14-membered, more preferably 5- to 10-membered; the ring atoms of monocyclic heteroaryl are preferably 5-membered or 6-membered, such as imidazolyl, furyl, thienyl, thiazolyl, pyrazolyl, oxazolyl, isoxazolyl, pyrrolyl, triazolyl, tetrazolyl, pyridyl, pyrimidinyl, thiadiazolyl, pyrazinyl, pyridazinyl, oxadiazolyl, *etc.*, preferably pyridyl, oxazolyl, isoxazolyl, pyridyl, pyrimidinyl, pyrazolyl, thiazolyl, thiadiazolyl, and oxadiazolyl. Polycyclic heteroaryl generally refers to a polycyclic fused heteroaryl group formed by the heteroaryl ring be fused to an aryl or heteroaryl group, wherein the ring attached to the parent structure may be an aryl ring or a heteroaryl ring. Polycyclic fused heteroaryl is preferably bicyclic fused heteroaryl. Non-limiting examples of bicyclic fused heteroaryl include: 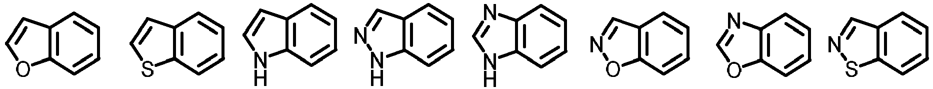 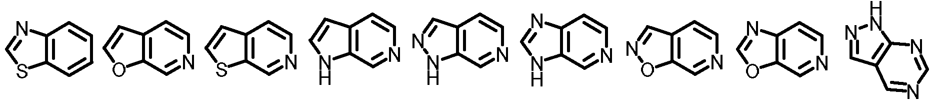 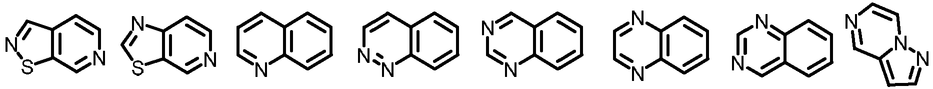 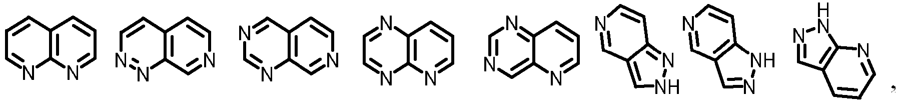 *etc.*

The heteroaryl may be substituted or unsubstituted, and when it is substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylic ester group.

The term "haloalkyl" refers to an alkyl group in which the hydrogen is substituted by one or more halogens, wherein the alkyl is as defined above. Non-limiting examples of haloalkyl include: monofluoromethyl, difluoromethyl, trifluoromethyl, monochloromethyl, dichloromethyl, trichloromethyl, 1-fluoroethyl, 2-fluoroethyl, 1,1-difluoroethyl, 1,2-difluoroethyl, *etc.* The haloalkyl group may be substituted or unsubstituted, and when it is substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylic ester group.

The term "hydroxyalkyl" refers to an alkyl group in which the hydrogen is substituted by one or more hydroxyl, wherein the alkyl is as defined above. Non-limiting examples of hydroxyalkyl include: hydroxymethyl, 1-hydroxyethyl, 2-hydroxyethyl, 1,2-dihydroxyethyl, 1-hydroxypropyl, 1-hydroxybutyl, *etc.* The hydroxyalkyl group may be substituted or unsubstituted, and when it is substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylic ester group.

The term "alkoxy" refers to -O-(alkyl), wherein the alkyl is as defined above. Non-limiting examples of alkoxy include: methoxy, ethoxy, propoxy, or butoxy. The alkoxy group may be substituted or unsubstituted, and when it is substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or carboxylic ester group.

The term "alkylthio" refers to -S-(alkyl), wherein the alkyl is as defined above. Non-limiting examples of alkylthio include: methylthio, ethylthio, propylthio, or butylthio. The alkylthio group may be substituted or unsubstituted, and when it is substituted, the substituent is preferably one or more of the following groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylic ester group.

"Hydroxyl" refers to -OH.

"Halogen" refers to fluorine (F), chlorine (Cl), bromine (Br), or iodine (I).

"Amino" refers to -NH₂.

"Cyano" refers to -CN.

"Nitro" refers to -NO₂.

"Carboxyl" refers to -C(O)OH.

"THF" refers to tetrahydrofuran.

"PE" refers to petroleum ether.

"EA" refers to ethyl acetate.

"IPA" refers to isopropyl alcohol.

"MeOH" refers to methanol.

"DMF" refers to *N,N-*dimethylformamide.

"TFA" refers to trifluoroacetic acid.

"ACN" refers to acetonitrile.

"DMA" refers to *N*,*N-*dimethylacetamide.

"Et₂O" refers to diethyl ether.

"DCM" refers to dichloromethane.

"DCE" refers to 1,2-dichloroethane.

"DIPEA" refers to *N*,*N-*diisopropylethylamine.

"NBS" refers to *N*-bromosuccinimide.

"NIS" refers to *N*-iodosuccinimide.

"Cbz-Cl" refers to benzyl chloroformate.

"Pd₂(dba)₃" refers to tris(dibenzylideneacetone)dipalladium.

"Dppf" refers to 1,1'-bis(diphenylphosphino)ferrocene.

"HATU" refers to 2-(7-azabenzotriazol-1-yl)-*N,N,N',N'*-tetramethyluronium hexafluorophosphate.

"KHMDS" refers to potassium bis(trimethylsilyl)amide.

"LiHMDS" refers to lithium bis(trimethylsilyl)amide.

"MeLi" refers to methyllithium.

"n-BuLi" refers to n-butyllithium.

"NaBH(OAc)₃" refers to sodium triacetoxyborohydride.

The different terms such as "X is selected from A, B, or C", "X is selected from A, B, and C", "X is A, B, or C", and "X is A, B, and C" all express the same meaning, *i.e.,* X can be any one or more of A, B, and C.

The hydrogen described herein can be substituted by its isotope deuterium, and any hydrogen in the compounds of the examples involved in the present disclosure can also be substituted by deuterium.

"Optional" or "optionally" means that the subsequently described event or circumstance may, but need not occur, and the description includes instances where the event or circumstance does or does not occur. For example, "a heterocyclic group optionally substituted by an alkyl group" means that an alkyl group may be, but not necessarily present, and the description includes instances where the heterocyclic group is substituted by an alkyl group and instances where the heterocyclic group is not substituted by an alkyl group.

"Substituted" means that one or more hydrogen in a group, preferably 5, more preferably 1 to 3 hydrogen, are independently of each other substituted by a corresponding number of substituents. It is self-evident that the substituents are only at their possible chemical positions, and those skilled in the art are able to determine the possible or impossible substitutions (either experimentally or theoretically) without undue effort. For example, an amino group or a hydroxyl group having a free hydrogen may be unstable when combined with a carbon atom having an unsaturated (*e.g*., olefinic) bond.

"Pharmaceutical composition" means a mixture containing one or more of the compounds described herein or their physiologically/pharmaceutically acceptable salts or prodrugs, and other chemical components, such as physiologically/pharmaceutically acceptable carriers and excipients. The purpose of the pharmaceutical composition is to promote the administration to the organism and facilitate the absorption of the active ingredients to exert their biological activity.

"Pharmaceutically acceptable salt" refers to a salt of the compounds of the present disclosure, which is safe and effective when used in a mammal, and has appropriate biological activity.

### DETAILED DESCRIPTION OF THE PREFERRED EMBODIMENT

### Chemical synthesis method:

The compounds described herein can be synthesized by a variety of methods well known to those skilled in the art of organic synthesis using starting materials that are commercially available or can be prepared by known experimental methods. The compounds of the present disclosure can be synthesized by the methods described below, or synthesis methods known in the art of synthetic organic chemistry, together with variations of the methods available to those skilled in the art. Preferred synthesis methods include, but are not limited to, those methods described below. For a more detailed description of the individual reaction steps, see the "Examples" section below.

Unless otherwise specified, the starting materials in the synthesis step can be synthesized using or according to methods known in the art, or can be purchased from Sigma-Aldrich Co. Ltd., Bide Pharmatech Ltd., Accela ChemBio Co. Ltd., and other companies.

Unless otherwise specified, all reactions in the present disclosure are carried out under continuous magnetic stirring and dry nitrogen or argon atmosphere. The solvent is a dry solvent, and the unit of reaction temperature is Celsius.

### Analytical methods and instrumentation:

LCMS data used for the characterization of examples were obtained using an Agilent 1260-6120/6125 MSD system coupled with a DAD detector. Test methods include:
LCMS method A:
Column: HALO C18 4.6 × 30 mm, 2.7 µm
Column temperature: 45°C
Mobile phase: solvent A: 0.025% trifluoroacetic acid + 99.975% water; solvent B: 0.025% trifluoroacetic acid + 99.975% acetonitrile
Flow rate: 1.8 mL/min
Gradient: linearly increase from 5% to 95% solvent Bin 0.8 minutes, then hold at 95% solvent B for 0.8 minutes, and finally hold at 5% solvent B to 2.0 minutes

### LCMS method B:

Column: HALO C18 4.6 × 30 mm, 2.7 µm
Column temperature: 45°C
Mobile phase: solvent A: 0.1% formic acid + 99.9% water; solvent B: 0.1% formic acid + 99.9% acetonitrile
Flow rate: 1.8 mL/min
Gradient: linearly increase from 5% to 95% solvent Bin 0.8 minutes, then hold at 95% solvent B for 0.8 minutes, and finally hold at 5% solvent B to 2.0 minutes

### LCMS method C:

Column: Xbrigde^{®} C18 4.6 × 50 mm, 2.5 µm
Column temperature: 40°C
Mobile phase: solvent A: 0.05% ammonia water + 99.5% water; solvent B: 100% acetonitrile
Flow rate: 1.8 mL/min
Gradient: linearly increase from 5% to 95% solvent B to 1 minute in 2.5 minutes, then hold at 95% solvent B to 2 minutes, and finally hold at 5% solvent B from 2.05 minutes to 2.5 minutes

NMR data used for the characterization of examples were obtained with a Bruker Fourier transform spectrometer (¹H NMR: 400 MHz). Data were reported in the format of chemical shift (multiplicity, number of hydrogen atoms). Chemical shifts were specified in terms of a tetramethylsilane internal standard (*δ*_{tetramethylsilane} = 0 ppm) and/or referenced to solvent peaks, which in ¹H NMR spectra appeared at 2.49 ppm for deuterated dimethyl sulfoxide (DMSO-*d₆*), 3.30 ppm for deuterated methanol (CD₃OD), 1.94 ppm for deuterated acetonitrile (CD₃CN), and 7.24 ppm for deuterated chloroform (CDCl₃).

### Purification method:

Purification of examples and intermediates was performed by silica gel chromatography, reversed-phase silica gel chromatography, and/or supercritical fluid chromatography (SFC). Silica gel chromatography was generally carried out using silica gel or pre-packed silica gel columns as carriers, and systems such as petroleum ether/ethyl acetate or dichloromethane/methanol as eluents. Reversed-phase silica gel chromatography was generally carried out using C18 silica gel columns as carriers, and using UV detectors (214 nm and 254 nm) and preparative LCMS for detection, with the mobile phases including systems such as acetonitrile/water (0.1% formic acid), acetonitrile/water (0.1% trifluoroacetic acid), and acetonitrile/water (0.1% ammonia water). Supercritical fluid chromatography (SFC) was generally carried out using different types of columns as carriers, and systems such as CO₂/methanol containing 0.2% NH₃ (a 7 M solution of ammonia in methanol) as the mobile phases.

### Intermediate a: cis-Diazabicyclo[4.2.0]octane

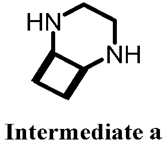

### Synthesis steps:

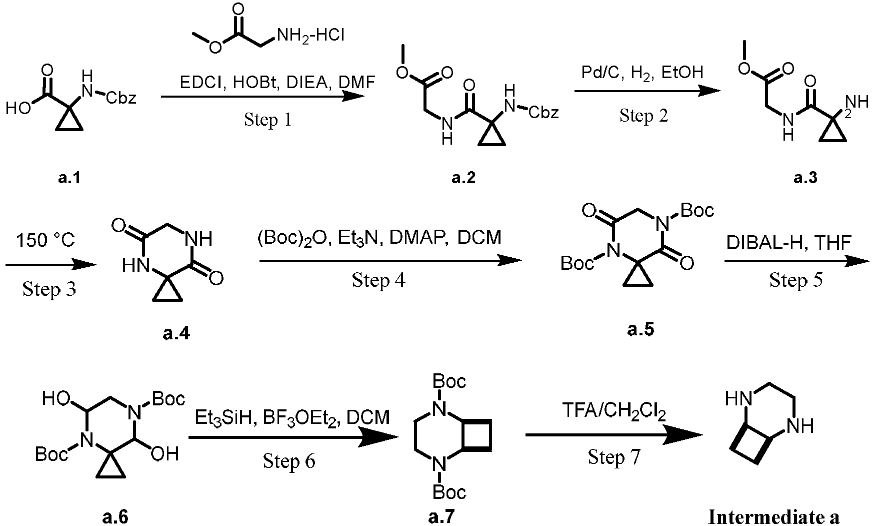

### Step 1: Synthesis of methyl 2-((1-((3-((formyl(formyloxy))methyl)phenyl)amino)cyclopropyl)formamido)acetate (a.2)

To a solution of methyl 1-(benzyloxycarbonyl)cyclopropanecarboxylic acid (100 g, 0.425 mol) in *N*,*N*-dimethylformamide (500 mL) were sequentially added *N-*(3-dimethylaminopropyl)-*N*-ethylcarbodiimide hydrochloride (99 g, 0.64 mol), 1-hydroxybenzotriazole (86 g, 0.64 mol), and *N,N-*diisopropylethylamine (165 g, 1.28 mol) under nitrogen atmosphere. A solution of methyl glycinate hydrochloride (80 g, 0.64 mol) in *N*,*N*-dimethylformamide (80 mL) was then added dropwise to the above mixture. After the dropwise addition was completed, the resulting mixture was stirred at room temperature for 24 hours. After the reaction was completed, the reaction mixture was concentrated under reduced pressure to remove the solvent. The resulting residue was diluted with water (2 L) and extracted with ethyl acetate (1 L × 3). The combined organic phases were dried over anhydrous sodium sulfate and filtered to remove the solid. The filtrate was concentrated under reduced pressure to obtain the crude product of the title compound **a.2** (120 g), which was directly used for the next reaction step. LC-MS (ESI), m/z: [M+H]⁺ = 307.0.

### Step 2: Synthesis of methyl 2-((1-aminocyclopropyl)formamido)acetate (a.3)

To a solution of compound **a.2** (120 g, 0.392 mol) in ethanol (1200 mL) was added Pd/C (12 g). After the reaction system was replaced with hydrogen three times, the reaction mixture was stirred at room temperature for 5 hours under hydrogen atmosphere. After the reaction was completed, the reaction mixture was filtered through diatomite, and the filtrate was concentrated under vacuum to obtain the crude product of the title compound **a.3** (65 g), which was directly used for the next reaction step. LC-MS (ESI), m/z [M+H]⁺ = 173.1.

### Step 3: Synthesis of 4,7-diazaspiro[2.5]octane-5,8-dione (a.4)

Compound **a.3** (65 g, 0.38 mol) was stirred at 150°C for 30 minutes, and the resulting reaction mixture was slurried with ethyl acetate to obtain the title compound **a.4** (28 g, yield: 67%).

¹H NMR (400 MHz, DMSO-*d₆*) δ 8.27 (s, 1H), 8.03 (s, 1H), 3.85 (d, *J* = 2.0 Hz, 2H), 1.14 (q, *J* = 4.3 Hz, 2H), 0.91 (q, *J* = 4.4 Hz, 2H).

### Step 4: Synthesis of tert-butyl(7-(tert-butyl-3-oxo)-5,8-dicarbonyl-4,7-diazaspiro[2.5]octan-4-yl)carboxyl ate (a.5)

To a solution of compound **a.4** (30 g, 0.21 mol) in dichloromethane (500 mL) were sequentially added di-*tert*-butyl dicarbonate (141 g, 0.647 mol), triethylamine (65 g, 0.64 mol), and 4-dimethylaminopyridine (7.84 g, 0.0643 mol). The reaction mixture was stirred at room temperature for 12 hours. After the reaction was completed, the reaction mixture was diluted with water (300 mL), and the resulting mixture was extracted with ethyl acetate (100 mL × 3). The combined organic phases were dried over anhydrous sodium sulfate, filtered to remove the solid, and concentrated under reduced pressure. The resulting residue was slurried and purified (EA/PE = 10/1, 200 mL) to obtain the title compound **a.5** (52 g, yield: 73%). LC-MS (ESI), m/z [2M+Na]⁺ = 702.8.

### Step 5: Synthesis of tert-butyl(7-(tert-butyl-3-oxo)-5,8-dihydroxy-4,7-diazaspiro[2.5]octan-4-yl)carboxyl ate (a.6)

A solution of compound **a.5** (44 g, 0.13 mol) in tetrahydrofuran (1000 mL) was cooled to -78°C under nitrogen atmosphere, and a solution of diisobutylaluminium hydride in tetrahydrofuran (1.0 mol/L, 643 mL, 0.64 mol) was slowly added dropwise to the reaction system. After the addition was completed, the reaction mixture was stirred at -78°C for 2 hours. After the reaction was completed, the reaction mixture was added with ethyl acetate (2 L) and stirred at room temperature for 1 hour. The reaction system was quenched with potassium sodium tartrate solution (2 L), and the quenched mixture was filtered. The filtrate was extracted with ethyl acetate (500 mL × 3). The combined organic phases were washed with saturated brine (500 mL), dried over anhydrous sodium sulfate, filtered to remove the solid, and concentrated under reduced pressure. The resulting residue was slurried and purified (ethyl acetate, 100 mL) to obtain the title compound **a.6** (20 g, yield: 42%).

LC-MS (ESI), m/z [2M+Na]⁺ = 711.2.

### Step 6: Synthesis of tert-butyl cis-(5-(tert-butyl-3-oxo)-2,5-diazabicyclo[4.2.0]octan-2-yl)carboxylate (a.7)

A solution of compound **a.6** (25 g, 0.073 mol) in dichloromethane (250 mL) was cooled to -78°C under nitrogen atmosphere, and triethylsilane (21 g, 0.18 mol) and boron trifluoride diethyl etherate solution (217 mL, 0.217 mol) were slowly added to the reaction system. The reaction mixture was stirred at -78°C for 2 hours. After the reaction was completed, the reaction system was added with water (200 mL), and the resulting mixture was extracted with ethyl acetate (50 mL × 3). The combined organic phases were washed with saturated brine (50 mL), dried over anhydrous sodium sulfate, filtered to remove the solid, and concentrated under reduced pressure. The resulting residue was purified by flash silica gel column chromatography (PE/EA= 100/1 to 5/1) to obtain the title compound **a.7** (12 g, yield: 55%). LC-MS (ESI), m/z [M-Boc]⁺ = 213.0.

### Step 7: cis-2,5-Diazabicyclo[4.2.0]octane (intermediate a)

To a solution of compound **a.7** (24 g, 0.077 mol) in dichloromethane (250 mL) was added trifluoroacetic acid (60 mL), and the reaction mixture was stirred at room temperature for 3 hours. After the reaction was completed, the reaction mixture was concentrated under reduced pressure. The resulting residue was dissolved in dichloromethane (400 mL), and methanol (80 mL) was added to the mixture with stirring. The resulting mixture was filtered to collect a solid precipitate, and the resulting solid was subjected to reduced pressure to remove the solvent to obtain the crude product of **intermediate a** (20 g). LC-MS (ESI), m/z [M+1]⁺ = 113.2.

¹H NMR (400 MHz, DMSO-*d₆*) δ 9.44 (s, 4H), 4.01 - 3.96 (m, 2H), 3.41 - 3.31 (m, 2H), 3.24 - 3.14 (m, 2H), 2.33 - 2.22 (m, 2H), 2.21 - 2.12 (m, 2H).

### Intermediate b: cis-Octahydrofuro[3,4-b]pyrazine

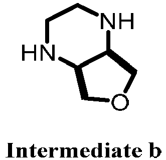

### Synthetic route:

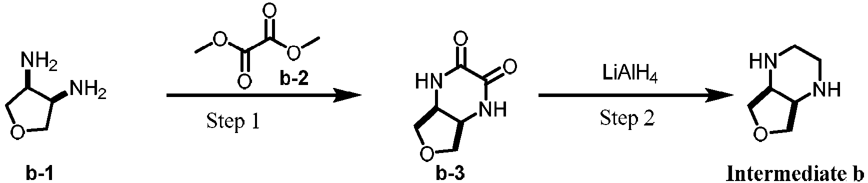

### Step 1: cis-Tetrahydrofuro[3,4-b]pyrazine-2,3(1H,4H)-dione (b-3)

*cis*-Tetrahydrofuran-3,4-diamine (1.0 g, 9.8 mmol) and dimethyl oxalate (1.2 g, 9.8 mmol) were dissolved in methanol (100 mL) at room temperature, and the reaction system was heated to 60°C and stirred for 16 hours. After the reaction was completed, the reaction mixture was concentrated under reduced pressure to obtain 1.6 g of light pink solid. The crude product was directly used for the next step. LC-MS (ESI), m/z: [M+H]⁺ = 157.1.

¹HNMR (400 MHz, DMSO-*d₆*) *δ* 8.58 (s, 2H), 4.13 - 4.06 (m, 2H), 3.88 - 3.82 (m, 2H), 3.66 (dd, *J =* 8.9, 4.2, 2H).

### Step 2: cis-Octahydrofuro[3,4-b]pyrazine (intermediate b)

Compound **b-3** (1.6 g, 10.3 mmol) was dissolved in anhydrous tetrahydrofuran (100 mL) at room temperature, and lithium aluminum hydride (2.1 g, 51.5 mmol) was added thereto in batches at 0°C under nitrogen atmosphere. After the addition was completed, the reaction system was stirred at 65°C for 10 hours under nitrogen atmosphere. The reaction mixture was cooled to room temperature, added with anhydrous sodium sulfate (10 g) and ethyl acetate (50 mL), then added dropwise with water (1 mL) at 0°C, stirred at room temperature for half an hour, filtered, and evaporated to dryness by rotary evaporation to obtain the crude product of **intermediate b** (1.5 g), which was directly used for the next step. LC-MS (ESI), m/z: [M+H]⁺ = 129.2.

### Intermediate A: 3-(hydroxymethyl)-5,7,8,9-tetrahydro-6H-cyclopenta[c][1,5]naphthyridin-6-one

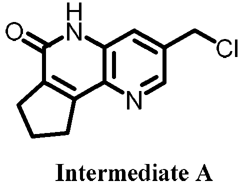

### Synthetic route:

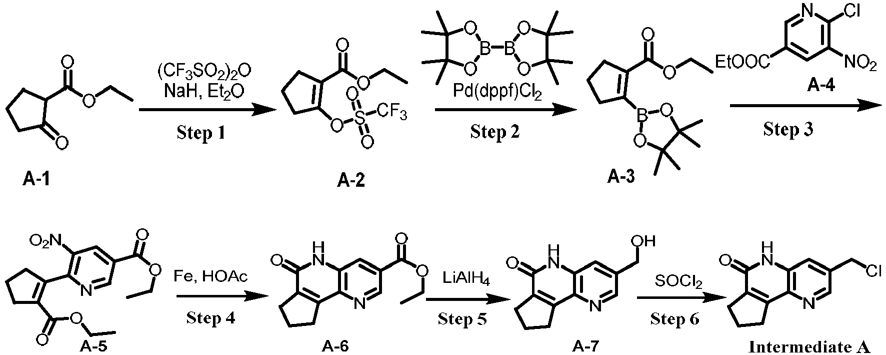

### Step 1: Ethyl 2-(((trifluoromethyl)sulfonyl)oxy)cyclopent-1-ene-1-carboxylate (A-2)

To a solution of ethyl 2-carbonylcyclopentane-1-carboxylate (20.0 g, 0.13 mol) in diethyl ether (200 mL) was added sodium hydride (6.40 g, 0.16 mol, 60%) in batches at 0°C under nitrogen atmosphere. After the addition was completed, the reaction mixture was stirred at 0°C for 0.5 hours, and trifluoromethanesulfonic anhydride (47.0 g, 0.17 mol) was slowly added thereto at 0°C. After the addition was completed, the reaction mixture was warmed to 25°C and stirred for 3 hours. The reaction system was cooled to 0°C, quenched with ice water (700 mL), and the mixture was extracted with EA (300 mL × 3). The combined organic phases were washed with saturated brine, dried over anhydrous sodium sulfate, filtered, and concentrated. The resulting residue was purified by silica gel chromatography (PE: EA = 9:1) to obtain the title compound **A-2** (16.3 g, yield: 44%). LC-MS (ESI), m/z: [M+H]⁺ = 288.9.

### Step 2: Ethyl 2-(4,4,5,5-tetramethyl-1,3,2-dioxaborolan-2-yl)cyclopent-1-ene-1-carboxylate (A-3)

Compound **A-2** (16.3 g, 0.056 mol) was dissolved in 1,4-dioxane (163 mL) at room temperature. 4,4,4',4',5,5,5',5'-Octamethyl-2,2'-bi(1,3,2-dioxaborolane) (17.1 g, 0.067 mol), bis(triphenylphosphine)palladium(II) chloride (1.97 g, 2.8 mmol), and potassium carbonate (15.5 g, 0.11 mol) were added thereto under nitrogen atmosphere. The reaction mixture was stirred at 80°C for 16 hours under nitrogen atmosphere. The reaction mixture was cooled to room temperature, filtered, and concentrated. The resulting residue was purified by silica gel chromatography (PE: EA = 5:1) to obtain the crude product of the title compound **A-3** (15.4 g). LC-MS (ESI), m/z: [M+H]⁺ = 267.0.

### Step 3: Ethyl 6-(2-(ethoxycarbonyl)cyclopent-1-en-1-yl)-5-nitronicotinate (A-5)

To a solution of compound **A-3** (8.90 g, 9.40 mmol) and compound **A-4** (2.16 g, 9.40 mmol) in 1,4-dioxane (25 mL) were added [1,1'-bis(diphenylphosphino)ferrocene]dichloropalladium(II) (0.34 g, 0.47 mmol) and cesium carbonate (6.13 g, 18.8 mmol) at room temperature. The reaction mixture was stirred at 100°C for 16 hours under nitrogen atmosphere. The reaction mixture was cooled to room temperature, filtered, and concentrated. The resulting residue was purified by silica gel chromatography (PE: EA = 4:1) to obtain the crude product of the title compound **A-5** (1.90 g).

LC-MS (ESI), m/z: [M+H]⁺ = 335.1.

### Step 4: Ethyl 6-carbonyl-6,6a,7,8,9,9a-hexahydro-5H-cyclopenta[c][1,5]naphthyridine-3-carboxylate (A-6)

Compound **A-5** (1.90 g, 5.69 mmol) was dissolved in a solution of glacial acetic acid (19 mL) at room temperature, then iron powder (2.55 g, 45.5 mmol) was added thereto, and the reaction mixture was stirred at 30°C for 16 hours. After the reaction was completed, the reaction mixture was filtered and concentrated. The concentrate was slurried with a mixed solvent of dichloromethane and methanol, and filtered to obtain the crude product of the title compound **A-6** (0.90 g). LC-MS (ESI), m/z: [M+H]⁺ = 259.0.

### Step 5: 3-(Hydroxymethyl)-5,7,8,9-tetrahydro-6H-cyclopenta[c][1,5]naphthyridin-6-one (A-7)

To a solution of compound **A-6** (0.90 g, 3.49 mmol) in tetrahydrofuran (22.5 mL) was added lithium aluminum hydride (0.27 g, 6.98 mmol) in batches at 0°C under nitrogen atmosphere, and after the addition was completed, the reaction mixture was stirred at 0°C for 2 hours. Water (0.27 mL) was then slowly added dropwise thereto at 0°C, and the reaction mixture was stirred for 10 minutes. 15% sodium hydroxide solution (0.27 mL) was then added dropwise thereto, and the reaction mixture was stirred for 10 minutes. Finally, water (0.80 mL) was added dropwise thereto, and the reaction mixture was stirred for 10 minutes. A small amount of anhydrous magnesium sulfate powder was then added thereto, and the reaction mixture was filtered and concentrated to obtain the crude product of the title compound **A-7** (0.70 g), which was directly used for the next reaction step. LC-MS (ESI), m/z: [M+H]⁺ = 217.2.

### Step 6: 3-(Chloromethyl)-5,7,8,9-tetrahydro-6H-cyclopenta[c][1,5]naphthyridin-6-one (intermediate A)

Compound **A-7** (0.70 g, 3.24 mmol) was suspended in dichloromethane (20 mL) at room temperature, then thionyl chloride (7.71 g, 64.8 mmol) was added thereto, and the reaction mixture was reacted at room temperature for 5 hours. After the reaction was completed, the reaction mixture was concentrated under reduced pressure. The resulting residue was added with a mixed solvent (35 mL) of dichloromethane and petroleum ether to precipitate a solid, and filtered to obtain the crude product of the title compound **intermediate A** (0.40 g). LC-MS (ESI), m/z: [M+H]⁺ = 235.0.

### Intermediate B: 3-(hydroxymethyl)-9,10-dihydro-5H-pyrano[2,3-c][1,5]naphthyridin-6(8H)-one 3-(chloromethyl)-9,10-dihydro-6H-pyrano[22,3-c],1,5]

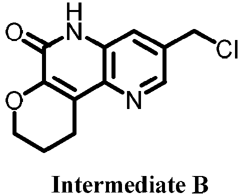

### Synthetic route:

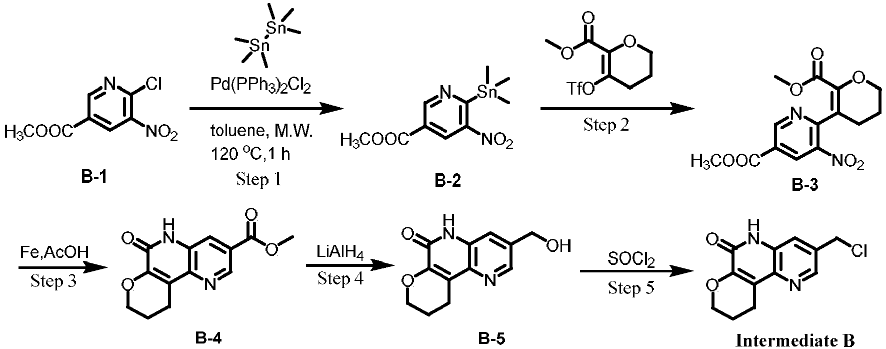

### Step 1: Synthesis of methyl 5-nitro-6-(trimethylstannyl)nicotinate (B-2)

To a solution of compound **B-1** (1.08 g, 4.99 mmol) in toluene (15 mL) were added hexamethyldistannane (2.13 g, 6.50 mmol) and bis(triphenylphosphine)palladium(II) chloride (110 mg, 0.150 mmol) under nitrogen atmosphere. After the addition was completed, the reaction mixture was stirred at 120°C for 1 hour under microwave irradiation. After the reaction was completed, the reaction system was added with n-hexane (150 mL), and the mixture was stirred at room temperature for 3 minutes. The resulting mixture was filtered, and the filtrate was concentrated to obtain the crude product of the title compound **B-2** (1.40 g). LC-MS (ESI), m/z: [M+H]⁺ = 346.9.

### Step 2: Synthesis of methyl 6-(6-(methoxycarbonyl)-3,4-dihydro-2H-pyran-5-yl)-5-nitronicotinate (B-3)

To a solution of compound **B-2** (1.50 g, 2.00 mmol) in toluene (20 mL) were added methyl 5-(((trifluoromethyl)sulfonyl)oxy)-3,4-dihydro-2*H*-pyran-6-carboxylate (580 mg, 2.00 mmol), tetrakis(triphenylphosphine)palladium (230 mg, 0.200 mmol), and cuprous iodide (19.0 mg, 0.100 mmol) at room temperature. After the addition was completed, the reaction mixture was stirred at 140°C for 0.5 hours under microwave irradiation. After the reaction was completed, the reaction system was diluted with water (50 mL), and the resulting mixture was extracted with ethyl acetate (30 mL × 3). The combined organic phases were washed with saturated brine, dried over anhydrous sodium sulfate, filtered, and concentrated. The resulting residue was purified by silica gel chromatography (PE: EA = 5:1) to obtain the title compound **B-3** (70.0 mg, yield: 11%). LC-MS (ESI), m/z: [M+H]⁺ = 323.0.

### Step 3: Synthesis of methyl 6-oxo-6,8,9,10-tetrahydro-5H-pyrano[2,3-c][1,5]naphthyridine-3-carboxylate (B-4)

To a solution of compound **B-3** (480 mg, 1.49 mmol) in acetic acid (20 mL) was added iron powder (805 mg, 14.9 mmol) at room temperature, and the reaction mixture was stirred for 16 hours. After the reaction was completed, the solvent was concentrated under reduced pressure, and the resulting residue was diluted with dichloromethane (100 mL) and methanol (10 mL). The resulting mixture was filtered and concentrated, and the residue was purified by silica gel chromatography (DCM: MeOH = 20:1) to obtain the title compound **B-4** (250 mg, yield: 65%). LC-MS (ESI), m/z: [M+H]⁺ = 261.1.

### Step 4: Synthesis of 3-(hydroxymethyl)-9,10-dihydro-5H-pyrano[2,3-c][1,5]naphthyridin-6(8H)-one (B-5)

A solution of compound **B-4** (150 mg, 0.577 mmol) in tetrahydrofuran (5 mL) was cooled to 0°C under nitrogen atmosphere, then a solution of lithium aluminum hydride in tetrahydrofuran (1.0 mol/L, 1.15 mL, 1.15 mmol) was added thereto, and the reaction mixture was stirred at 0°C for 2 hours. After the reaction was completed, the reaction mixture was diluted with ethyl acetate (20 mL), and quenched with water (40 mg). The quenched system was added with solid silica gel and concentrated to remove the solvent, and purified by silica gel chromatography (DCM: MeOH = 10:1) to obtain the title compound **B-5** (70.0 mg, yield: 52%). LC-MS (ESI), m/z: [M+H]⁺ = 233.0.

### Step 5: Synthesis of 3-(hydroxymethyl)-9,10-dihydro-5H-pyrano[2,3-c][1,5]naphthyridin-6(8H)-one 3-(chloromethyl)-9,10-dihydro-6H-pyrano[22,3-c],1,5] (Intermediate B)

Compound **B-5** (35.0 mg, 0.150 mmol) was dissolved in thionyl chloride (1.00 mL), and the resulting reaction mixture was stirred at 80°C for 2 hours. After the reaction was completed, the reaction mixture was concentrated, and the resulting residue was the crude product of the title compound **intermediate B** (40.0 mg), which was directly used for the next reaction step. LC-MS (ESI), m/z: [M+H]⁺ = 251.0.

### Intermediate C: 7-(chloromethyl)-3-(trifluoromethyl)-1,5-naphthyridin-2(1H)-one

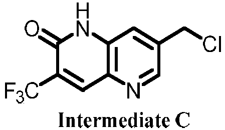

### Synthetic route:

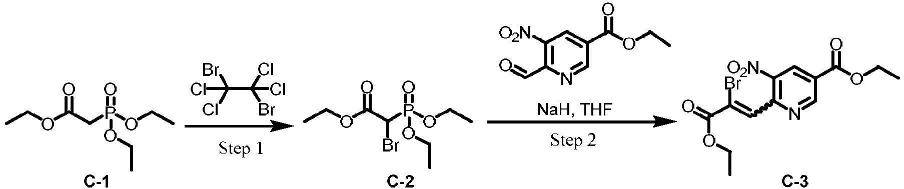

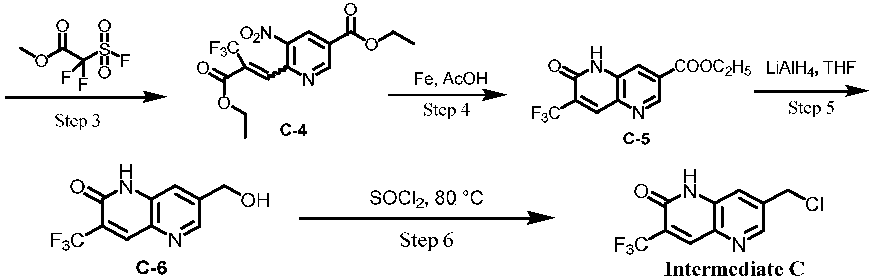

### Step 1: Synthesis of ethyl 2-bromo-2-(diethoxyphosphoryl)acetate (C-2)

To a solution of compound **C-1** (4.00 g, 17.8 mmol) in tetrahydrofuran (50 mL) was added sodium hydride (428 mg, 17.8 mmol) under nitrogen atmosphere. After the addition was completed, the reaction mixture was stirred at 0°C for 0.5 hours, then 1,2-dibromo-1,1,2,2-tetrachloroethane (6.97 g, 21.4 mmol) was added thereto, and the reaction mixture was stirred at room temperature for 16 hours. After the reaction was completed, the reaction system was added with water (50 mL), and the resulting mixture was extracted with ethyl acetate (50 mL × 3). The combined organic phases were washed with saturated brine, dried over anhydrous sodium sulfate, filtered to remove the solid, and the filtrate was concentrated under reduced pressure. The resulting residue was purified by silica gel column chromatography (PE/EA = 5/1) to obtain the title compound **C-2** (2.59 g, yield: 43%). LC-MS (ESI), m/z: [M+H]⁺ = 303.0, 305.0.

### Step 2: Synthesis of ethyl 6-(2-bromo-3-ethoxy-3-oxopropionyl)-5-nitronicotinate (C-3)

To a solution of compound **C-2** (2.59 g, 8.54 mmol) in tetrahydrofuran (40 mL) was added sodium hydride (307 mg, 12.8 mmol) under nitrogen atmosphere. After the addition was completed, the reaction mixture was stirred at 0°C for 0.5 hours, then ethyl 6-formyl-5-nitro-pyridine-3-carboxylate (1.9 g, 8.5 mmol) was added thereto, and the reaction mixture was stirred at room temperature for 16 hours. After the reaction was completed, the reaction system was added with water (40 mL), and the resulting mixture was extracted with ethyl acetate (40 mL × 3). The combined organic phases were washed with saturated brine, dried over anhydrous sodium sulfate, filtered to remove the solid, and the filtrate was concentrated under reduced pressure. The resulting residue was purified by silica gel column chromatography (PE/EA = 10/1) to obtain the title compound **C-3** (2.28 g, yield: 72%). LC-MS (ESI), m/z: [M+H]⁺ = 373.0, 375.0.

### Step 3: Synthesis of ethyl 6-(2-(ethoxycarbonyl)-3,3,3-trifluoroprop-1-en-1-yl)-5-nitronicotinate (C-4)

To a solution of compound **C-3** (1.47 g, 3.93 mmol) in *N,N*-dimethylformamide (30 mL) were added methyl 2,2-difluoro-2-(fluorosulfonyl)acetate (1.51 g, 7.88 mmol) and cuprous iodide (1.50 g, 7.88 mmol). After the addition was completed, the reaction mixture was stirred at 100°C for 4 hours. After the reaction was completed, the reaction system was added with water (30 mL), and the resulting mixture was extracted with ethyl acetate (50 mL × 3). The combined organic phases were washed with saturated brine, dried over anhydrous sodium sulfate, filtered to remove the solid, and the filtrate was concentrated under reduced pressure. The resulting residue was purified by silica gel column chromatography (PE/EA = 8/1) to obtain the title compound **C-4** (1.02 g, yield: 64%). LC-MS (ESI), m/z: [M+H]⁺ = 363.1.

### Step 4: Synthesis of ethyl 6-oxo-7-(trifluoromethyl)-5,6-dihydro-1,5-naphthyridine-3-carboxylate (C-5)

To a solution of compound **C-4** (1.02 g, 2.81 mmol) in acetic acid (10 mL) was added iron powder (627 mg, 11.2 mmol), and the reaction mixture was stirred at room temperature for 2 hours. After the reaction was completed, the reaction mixture was concentrated under reduced pressure. The resulting residue was diluted with dichloromethane (100 mL) and methanol (100 mL), filtered to remove the solvent, and the filtrate was concentrated under reduced pressure. The resulting residue was purified by silica gel column chromatography (DCM/MeOH = 20/1) to obtain the title compound **C-5** (434 mg, yield: 48%). LC-MS (ESI), m/z: [M+H]⁺ = 287.1.

### Step 5: Synthesis of 7-(hydroxymethyl)-3-(trifluoromethyl)-1,5-naphthyridin-2(1H)-one (C-6)

A solution of compound **C-5** (434 mg, 1.51 mmol) in tetrahydrofuran (5 mL) was cooled to 0°C under nitrogen atmosphere, then a solution of lithium aluminum hydride in tetrahydrofuran (1.0 mol/L, 1.81 mL, 1.81 mmol) was added thereto, and the mixture was stirred at 0°C for 2 hours. After the reaction was completed, the reaction system was added with ethyl acetate (20 mL) and water (40 mg), and the mixture was concentrated under reduced pressure. The resulting residue was purified by silica gel column chromatography (DCM/MeOH = 10/1) to obtain the title compound **C-6** (239 mg, yield: 58%). LC-MS (ESI), m/z: [M+H]⁺ = 245.1.

### Step 6: Synthesis of 7-(chloromethyl)-3-(trifluoromethyl)-1,5-naphthyridin-2(1H)-one (intermediate C)

A solution of compound **C-6** (100 mg, 0.408 mmol) in thionyl chloride (5 mL) was stirred at 80°C for 2 hours. After the reaction was completed, the reaction mixture was concentrated under reduced pressure, and the resulting residue was the crude product of the title compound **intermediate C** (93 mg), which was directly used for the next reaction step. LC-MS (ESI), m/z: [M+H]⁺ = 263.0.

### Intermediate D: 3-(chloromethyl)-7-ethylpyrido[3,2-c]pyridazin-6(5H)-one

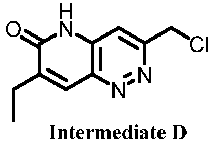

### Synthetic route:

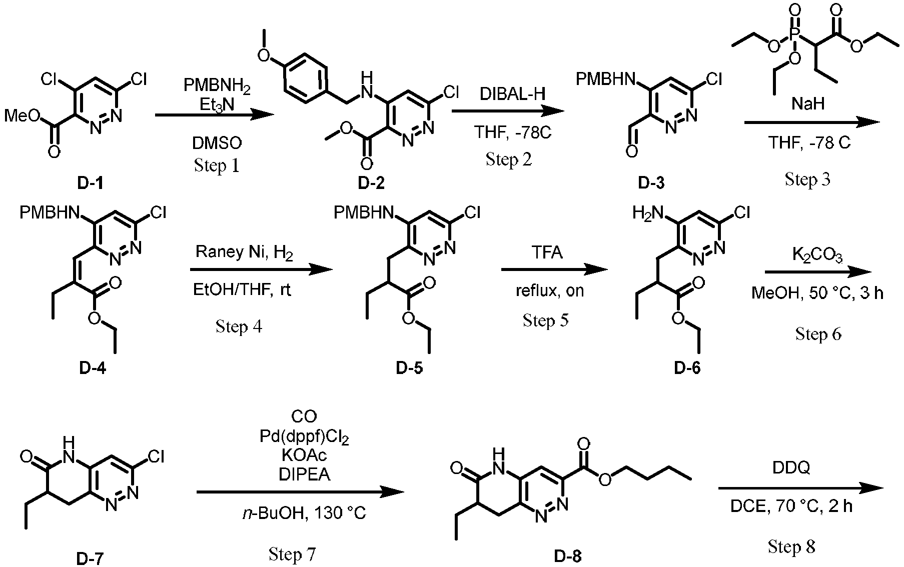

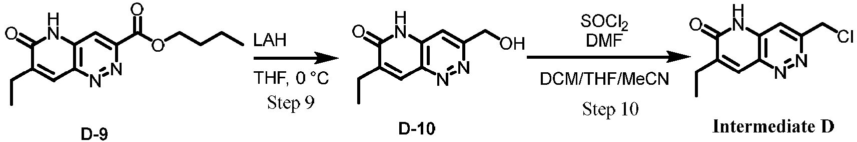

### Step 1: Synthesis of methyl 6-chloro-4-((4-methoxybenzyl)amino)pyridazine-3-carboxylate (D-2)

To a solution of compound **D-1** (27.5 g, 133 mmol) in dimethyl sulfoxide (200 mL) were added 4-methoxybenzylamine (19.1 mL, 146 mmol) and triethylamine (37.0 mL, 266 mmol), and the reaction mixture was stirred at room temperature overnight. After the reaction was completed, the reaction system was added with water (800 mL), filtered to collect the precipitated solid, and the filter cake was washed with water. The resulting solid was dissolved (dichloromethane/methanol = 95/5, 200 mL), dried over anhydrous sodium sulfate, filtered to remove the solid, and concentrated under reduced pressure to obtain the title compound **D-2** (36.9 g, yield: 90%). LC-MS (ESI), m/z: [M+H]⁺ = 308.1.

### Step 2: Synthesis of 6-chloro-4-((4-methoxybenzyl)amino)pyridazine-3-carbaldehyde (D-3)

A solution of compound **D-2** (36.9 g, 120 mol) in dry tetrahydrofuran (500 mL) was cooled to -78°C under nitrogen atmosphere, and a solution of DIBAL-H in *n*-hexane (1.0 mol/L, 180 mL, 180 mmol) was added dropwise thereto. After the dropwise addition was completed, the mixture was stirred at -78°C for 2 hours. After the reaction was completed, the reaction mixture was quenched with isopropanol (10 mL), then saturated potassium sodium tartrate aqueous solution (300 mL) and ethyl acetate (300 mL) were added thereto, and the mixture was stirred at room temperature for 2 hours. The resulting mixture was extracted with ethyl acetate (150 mL × 3). The combined organic phases were washed with saturated brine, dried over anhydrous sodium sulfate, filtered to remove the solid, and concentrated under reduced pressure. The resulting residue was subjected to silica gel column chromatography (dichloromethane/methanol = 95/5) to obtain the title compound **D-3** (26.6 g, yield: 80%). LC-MS (ESI), m/z: [M+H]⁺ = 278.3.

### Step 3: Synthesis of ethyl (Z)-2-((6-chloro-4-((4-methoxybenzyl)amino)pyridazin-3-yl)methylene)butanoate (D-3)

A suspension of sodium hydride (60% wt, 3.3 g, 83 mmol) in tetrahydrofuran (300 mL) was cooled to 0°C under nitrogen atmosphere, and triethyl 2-phosphonobutyrate (21 g, 82 mmol) was added thereto. The reaction mixture was stirred at room temperature for 1 hour. A solution of compound **D-3** (19.3 g, 69.4 mmol) in tetrahydrofuran (300 mL) was added dropwise to the reaction system at -78°C, and the mixture was stirred for 1 hour. After the reaction was completed, the reaction mixture was quenched with saturated ammonium chloride aqueous solution (500 mL), and the resulting mixture was quenched with ethyl acetate (150 mL × 3). The combined organic phases were washed with saturated brine (100 mL), dried over anhydrous sodium sulfate, filtered to remove the solid, and concentrated under reduced pressure. The resulting residue was purified by reversed-phase chromatography (water (0.05% trifluoroacetic acid)/acetonitrile = 20/80 to 5/95) to obtain the title compound **D-4** (19 g, yield: 73%). LC-MS (ESI), m/z: [M+H]⁺ = 376.2.

### Step 4: Synthesis of ethyl 2-((6-chloro-4-((4-methoxybenzyl)amino)pyridazin-3-yl)methyl)butanoate (D-5)

Compound **D-4** (19 g, 51 mmol) was dissolved in ethanol/tetrahydrofuran (1/1, 600 mL), and Raney nickel (0.7 g) was added to the mixture. The mixture was stirred at room temperature overnight under hydrogen atmosphere. After the reaction was completed, the mixture was filtered to remove the solid, and the filtrate was concentrated under reduced pressure to obtain the title compound **D-5** (19.1 g, yield: 100%). LC-MS (ESI), m/z: [M+H]⁺ = 378.2.

### Step 5: Synthesis of ethyl 2-((4-amino-6-chloropyridazin-3-yl)methyl)butanoate (D-6)

Compound **D-5** (19 g, 51 mmol) was dissolved in trifluoroacetic acid (100 mL), and the mixture was heated to reflux for 24 hours. After the reaction was completed, the mixture was concentrated under reduced pressure to obtain the crude product of the title compound **D-6** (10.6 g). LC-MS (ESI), m/z: [M+H]⁺ = 258.1.

### Step 6: Synthesis of 3-chloro-7-ethyl-7,8-dihydropyrido[3,2-c]pyridazin-6(5H)-one (D-7)

To a solution of compound **D-6** (12.9 g, 50.1 mmol) in methanol (300 mL) was added potassium carbonate (14.0 g, 101 mmol), and the mixture was stirred at 50°C for 3 hours. After the reaction was completed, the reaction system was added with dichloromethane (300 mL) and water (300 mL), and the resulting mixture was extracted with dichloromethane (300 mL × 3). The combined organic phases were washed with saturated brine (100 mL), dried over anhydrous sodium sulfate, filtered to remove the solid, and concentrated under reduced pressure. The resulting residue was purified by silica gel column chromatography (dichloromethane/methanol = 100/0 to 95/5) to obtain the title compound **D-7** (8.7 g, yield: 81%). LC-MS (ESI), m/z: [M+H]⁺ = 212.1.

### Step 7: Synthesis of butyl 7-ethyl-6-oxo-5,6,7,8-tetrahydropyrido[3,2-c]pyridazine-3-carboxylate (D-8)

To a mixture of compound **D-7** (0.45 g, 2.1 mmol), 1,1'-bis(diphenylphosphino)ferrocene (153 mg, 0.210 mmol), potassium acetate (0.31 g, 3.2 mmol), and diisopropylethylamine (557 mL, 3.20 mmol) was added n-butanol (5 mL) under argon atmosphere, and the resulting mixture was stirred at 130°C for 1 hour under carbon monoxide atmosphere. After the reaction was completed, the reaction mixture was cooled to room temperature and concentrated under reduced pressure to remove the solvent. The resulting residue was purified by silica gel column chromatography (dichloromethane/methanol = 100/0 to 95/5) to obtain the title compound **D-8** (0.50 g, yield: 86%). LC-MS (ESI), m/z: [M+H]⁺ = 278.2.

### Step 8: Synthesis of butyl 7-ethyl-6-oxo-5,6-dihydropyrido[3,2-c]pyridazine-3-carboxylate (D-9)

To a solution of compound **D-8** (0.50 g, 1.80 mmol) in 1,2-dichloroethane (10 mL) was added dichlorodicyanobenzoquinone (409 mg, 1.80 mmol), and the reaction mixture was stirred at 70°C for 2 hours. After the reaction was completed, the reaction mixture was added with saturated sodium bicarbonate aqueous solution and extracted with dichloromethane (20 mL × 4). The combined organic phases were washed with saturated brine, dried over anhydrous sodium sulfate, filtered to remove the solid, and concentrated under reduced pressure. The resulting residue was the title compound **D-9** (0.38 g, yield: 77%). LC-MS (ESI), m/z: [M+H]⁺ = 276.2.

### Step 9: Synthesis of 7-ethyl-3-(hydroxymethyl)pyrido[3,2-c]pyridazin-6(5H)-one (D-10)

A solution of compound **D-9** (0.38 g, 1.4 mmol) in tetrahydrofuran (5 mL) was cooled to 0°C, and lithium aluminum hydride (2.5 mol/L in tetrahydrofuran, 607 mL, 1.52 mmol) was added thereto. The reaction mixture was stirred at 0°C until LC-MS indicated the completion of the reaction. After the reaction was completed, sodium sulfate decahydrate (0.2 g) was added to the reaction system. The reaction mixture was concentrated under reduced pressure to remove the solvent, and the resulting residue was purified by silica gel column chromatography (dichloromethane/methanol = 95/5) to obtain the title compound **D-10** (0.24 g, yield: 85%). LC-MS (ESI), m/z: [M+H]⁺ = 206.1.

### Step 10: Synthesis of 3-(chloromethyl)-7-ethylpyrido[3,2-c]pyridazin-6(5H)-one (intermediate D)

To a suspension of compound **D-9** (327 mg, 1.60 mmol) in dichloromethane (4 mL) were added tetrahydrofuran (4 mL) and acetonitrile (4 mL), and the mixture was cooled to 0°C. The mixture was added with thionyl chloride (0.58 mL, 8.0 mmol) and *N,N-*dimethylformamide (5 mL), stirred at 0°C for 10 minutes, and then stirred at room temperature for 1 hour. After the reaction was completed, the reaction mixture was concentrated under reduced pressure, and the resulting residue was the crude product of **intermediate D** (357 mg), which was directly used for the next reaction step. LC-MS (ESI), m/z: [M+H]⁺ = 224.1.

### Intermediate E: 3-ethyl-7-(hydroxymethyl)pyrido[2,3-b]pyrazin-2(1H)-one

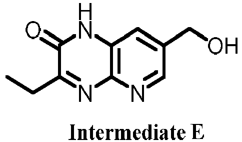

### Synthetic route:

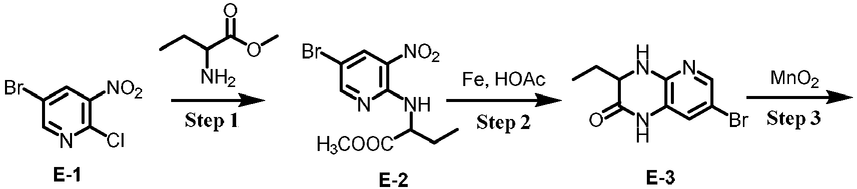

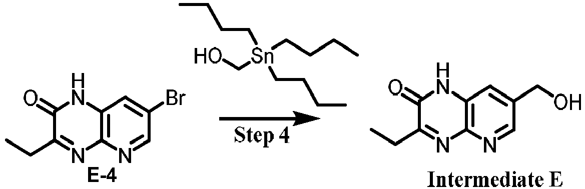

### Step 1: Synthesis of methyl 2-((5-bromo-3-nitropyridin-2-yl)amino)butanoate (E-2)

To a solution of compound **E-1** (20.0 g, 84.0 mmol) in *N,N-*dimethylformamide (200 mL) were added methyl 2-aminobutanoate (14.2 g, 92.4 mmol) and diisopropylethylamine (32.5 g, 25.2 mmol) under nitrogen atmosphere. After the addition was completed, the reaction mixture was stirred at 45°C for 1 hour. After the reaction was completed, the system was added with water (600 mL), and the resulting mixture was extracted with ethyl acetate (300 mL × 3). The combined organic phases were washed with saturated brine, dried over anhydrous sodium sulfate, filtered to remove the solid, and concentrated under reduced pressure. The resulting residue was purified by silica gel column chromatography (PE/EA = 10/1) to obtain the title compound **E-2** (15.7 g, yield: 59%). LC-MS (ESI), m/z: [M+H]⁺ = 317.8, 319.8.

### Step 2: Synthesis of 7-bromo-3-ethyl-3,4-dihydropyrido[2,3-b]pyrazin-2(1H)-one (E-3)

To a solution of compound **E-2** (15.7 g, 49.5 mmol) in glacial acetic acid (300 mL) was added reduced iron powder (22.2 g, 396 mmol) under nitrogen atmosphere. After the addition was completed, the reaction mixture was stirred at room temperature for 16 hours. After the reaction was completed, the reaction mixture was concentrated under reduced pressure. The resulting residue was dissolved in dichloromethane (500 mL) and methanol (50 mL), filtered to remove the solid, and the filtrate was concentrated again under reduced pressure. The resulting residue was purified by silica gel column chromatography (DCM/MeOH = 20/1) to obtain the crude product of the title compound **E-3** (11.5 g), which was directly used for the next reaction step. LC-MS (ESI), m/z: [M+H]⁺ = 255.9, 257.9.

### Step 3: Synthesis of 7-bromo-3-ethylpyrido[2,3-b]pyrazin-2(1H)-one (E-4)

To a solution of compound **E-3** (11.5 g, 45.1 mmol) in tetrahydrofuran (230 mL) was added manganese dioxide (118 g, 1.35 mol) under nitrogen atmosphere, and the mixture was stirred at room temperature for 1 hour. After the reaction was completed, the reaction mixture was concentrated under reduced pressure. The resulting residue was diluted with dichloromethane (500 mL) and methanol (50 mL), and filtered to remove the solid. The resulting filtrate was concentrated, and the resulting residue was the crude product of the title compound E-4 (7.0 g), which was directly used for the next reaction step. LC-MS (ESI), m/z: [M+H]⁺ = 254.0, 256.0.

### Step 4: Synthesis of 3-ethyl-7-hydroxymethylpyrido[2,3-b]pyrazin-2(1H)-one (intermediate E)

To a solution of compound **E-4** (2.01 g, 7.91 mmol) in dioxane (40 mL) were added (tributylstannyl)methanol (3.82 mg, 11.9 mmol) and (2-dicyclohexylphosphino-2',4',6'-triisopropyl-1,1'-biphenyl)(2'-amino-1,1'-biphenyl-2-yl)palladium(II) chloride (311 mg, 0.395 mmol) under nitrogen atmosphere. After the addition was completed, the reaction mixture was stirred at 80°C for 1 hour. After the reaction was completed, the reaction mixture was concentrated under reduced pressure, and the resulting residue was purified by silica gel column chromatography (DCM/MeOH = 30/1) to obtain **intermediate E** (570 mg, yield: 35%). LC-MS (ESI), m/z: [M+H]⁺ = 206.1.

### Intermediate F: 3-cyclopropyl-7-(hydroxymethyl)-1H-1,5-naphthyridin-2-one

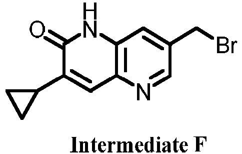

### Synthetic route:

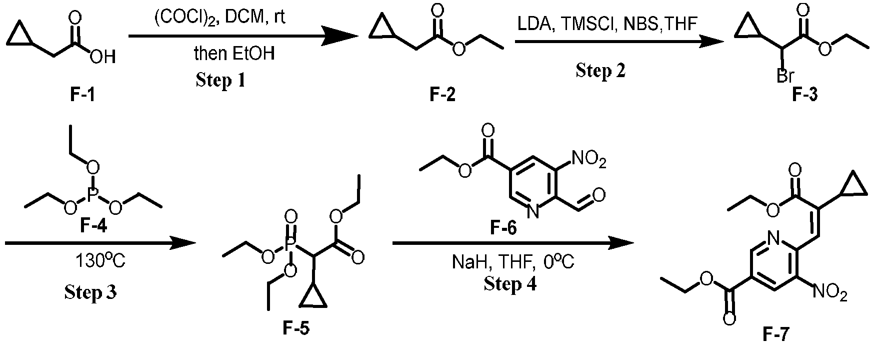

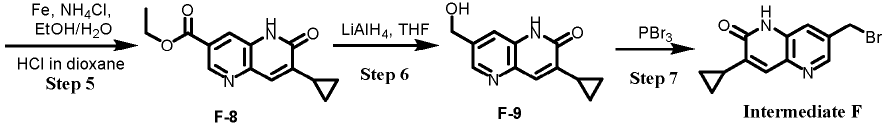

### Synthesis steps:

### Step 1: Ethyl 2-cyclopropylacetate (F-2):

2-Cyclopropylacetic acid (21.5 g, 0.21 mol) was dissolved in DCM (150 mL), and the mixture was cooled to 0°C. The mixture was added with a solution of oxalyl chloride (32.7 g, 0.26 mol) in DCM (50 mL) at 0°C, and stirred at room temperature for 4 hours. The resulting mixture was concentrated under reduced pressure. The resulting mixture was slowly diluted with 200 mL of ethanol at 0°C, and stirred at room temperature for 2 hours. The reaction mixture was concentrated under reduced pressure to obtain the title compound **F-2** (18.5 g, yield: 67%).

¹H NMR (400 MHz, DMSO-*d₆*) δ 4.05 (q, *J =* 7.2 Hz, 2H), 2.20 (d, *J =* 7.2 Hz, 2H), 1.18 (t, *J* = 7.2 Hz, 3H), 0.96 - 0.92 (m, 1H), 0.48 - 0.44 (m, 2H), 0.14 - 0.10 (m, 2H).

### Step 2: Ethyl 2-bromo-2-cyclopropylacetate (F-3):

Ethyl 2-cyclopropylacetate (15.0 g, 0.12 mol) was dissolved in dry THF (150 mL) under nitrogen atmosphere, and the reaction mixture was cooled to -78°C. LDA (0.15 mmol, 2.0 M in THF, 76.1 mL) was slowly added dropwise to the reaction mixture. After 1 hour, TMSCl (12.7 g, 0.12 mmol) was added thereto. The mixture was stirred at room temperature for 3 hours, then cooled to -78°C, added with N-bromosuccinimide (25.0 g, 0.14 mmol), and stirred at room temperature for 2 hours. The mixture was quenched with saturated NH₄Cl solution, and extracted with n-hexane (200 mL × 3). The combined organic phases were dried over anhydrous sodium sulfate, and evaporated under reduced pressure to remove the solvent to obtain the title compound **F-3** (22.0 g, yield: 90%).

¹H NMR (400 MHz, CDCl₃) δ 4.24 (q, *J =* 7.1 Hz, 2H), 3.57 (d, *J =* 10.4 Hz, 1H), 1.62 - 1.51 (m, 1H), 1.30 (t, *J* = 7.1 Hz, 3H), 0.90 - 0.78 (m, 2H), 0.56 - 0.51 (m, 1H), 0.45 - 0.39 (m, 1H).

### Step 3: Ethyl 2-cyclopropyl-2-(diethoxyphosphoryl)acetate (F-5):

Ethyl 2-bromo-2-cyclopropylacetate (3.0 g, 14.5 mmol) was dissolved in triethyl phosphite (3.61 g, 21.7 mmol), and the mixture was stirred at 130°C for 12 hours. After the reaction was cooled to room temperature, the reaction mixture was concentrated under reduced pressure to obtain the title compound **F-5** (3.50 g, yield: 90%).

¹H NMR (400 MHz, CDCl₃) δ 4.22 - 4.14 (m, 6H), 2.24 - 2.15 (m, 1H), 1.31 - 1.26 (m, 10H), 0.76 - 0.68 (m, 1 Hz), 0.65 - 0.54 (m, 1H), 0.48 - 0.37 (m, 1H), 0.28 - 0.19 (m, 1H).

### Step 4: Ethyl 6-((1Z)-2-cyclopropyl-3-ethoxy-3-oxoprop-1-en-1-yl)-5-nitropyridine-3-carboxylate (F-7):

A solution of NaH (0.20 g, 8.40 mmol) in THF (25.0 mL) was cooled to 0°C. Ethyl 2-cyclopropyl-2-(diethoxyphosphoryl)acetate (1.40 g, 5.30 mmol) was added to the above mixture at 0°C. The mixture was stirred at room temperature for 30 minutes and then cooled to 0°C. Ethyl 6-formyl-5-nitropyridine-3-carboxylate (1.78 g, 7.90 mmol) was added to the above mixture at 0°C. The resulting mixture was stirred at room temperature for 30 minutes under argon atmosphere. NH₄Cl (aq., 20 mL) was slowly added dropwise to the reaction mixture at 0°C. The resulting mixture was diluted with water (50 mL) and extracted with EA (50 mL × 2). The combined organic phases were washed with saturated brine (20 mL × 2) and dried over anhydrous sodium sulfate. After filtration, the filtrate was concentrated under reduced pressure. The resulting crude product was purified by silica gel chromatography (PE: EA = 4:1) to obtain the title compound **F-7** (0.85 g, yield: 47%). LC-MS (ESI), m/z: [M+H]⁺ = 335.1.

### Step 5: Ethyl 7-cyclopropyl-6-oxo-5,6-dihydro-1,5-naphthyridine-3-carboxylate (F-8):

A mixed solution of ethyl 6-[(1*Z*)-2-cyclopropyl-3-ethoxy-3-oxoprop-1-en-1-yl]-5-nitropyridine-3-carboxylate (0.85 g, 2.54 mmol), Fe (0.71 g 12.7 mmol), and AcOH (2.0 mL) was stirred at room temperature for 1 hour. After filtration, the filtrate was concentrated under reduced pressure. The mixture was diluted with a solution of HCl in 1,4-dioxane (2.0 M) at room temperature and stirred at 80°C for 12 hours. The mixture was cooled to room temperature and concentrated under reduced pressure to obtain the title compound **F-8** (0.40 g, yield: 60%) as a yellow solid. LC-MS (ESI), m/z: [M+H]⁺ = 259.1.

### Step 6: 3-Cyclopropyl-7-(hydroxymethyl)-1H-1,5-naphthyridin-2-one (F-9)

A solution of LiAlH₄ (162.6 mg, 4.07 mmol) in THF (5.0 mL) was cooled to 0°C. Ethyl 7-cyclopropyl-6-oxo-5*H*-1,5-naphthyridine-3-carboxylate (0.35 g, 1.36 mol) was added dropwise to the above mixture at 0°C. After the dropwise addition was completed, the reaction mixture was stirred at 0°C for 4 hours. Sodium sulfate decahydrate (0.20 g) was slowly added to the reaction mixture at 0°C. The resulting mixture was diluted with EA (10.0 mL). After filtration, the filter cake was rinsed with EA (10 mL). The filtrates were combined and dried over anhydrous magnesium sulfate. The filtrate was filtered, dried, and concentrated to obtain the title compound **F-9** (0.29 g, yield: 91%). LC-MS (ESI), m/z: [M+H]⁺ = 217.1.

### Step 7: 7-(Bromomethyl)-3-cyclopropyl-1H-1,5-naphthyridin-2-one (intermediate F):

3-Cyclopropyl-7-(hydroxymethyl)-1*H*-1,5-naphthyridin-2-one (0.10 g, 0.46 mmol) was dissolved in THF (1.50 mL), and the mixture was cooled to 0°C. PBr₃ (187.8 mg, 0.70 mmol) was slowly added dropwise to the above mixture at 0°C. The mixture was stirred at 0°C for 2 hours. Saturated sodium bicarbonate solution (5.0 mL) was added dropwise to the reaction mixture at 0°C. The resulting mixture was diluted with water (5.0 mL) and extracted with EA (10 mL × 2). The combined organic phases were washed with saturated brine (10 mL × 2) and dried over anhydrous sodium sulfate. After filtration, the filtrate was concentrated under reduced pressure. The resulting residue was purified by silica gel chromatography (PE: EA = 4:1) to obtain the title compound **intermediate F** (45.0 mg, yield: 33%). LC-MS (ESI), m/z: [M+H]⁺ = 278.9.

### Intermediate G: 7-(hydroxymethyl)-3-methoxy-l,5-naphthyridin-2(1H)-one

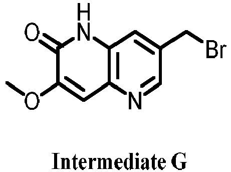

### Synthetic route:

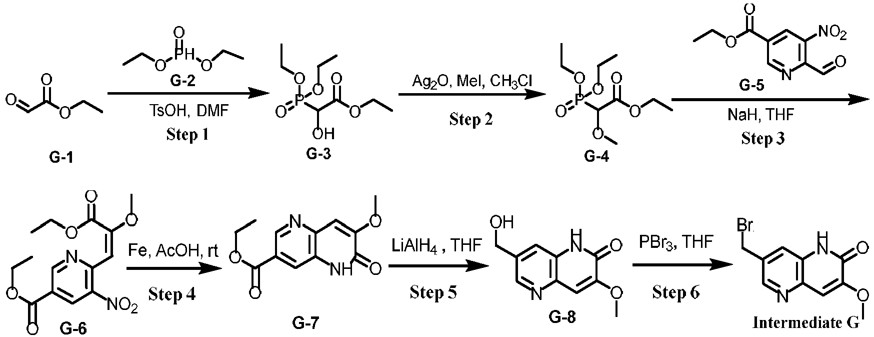

### Step 1: Ethyl 2-(diethoxyphosphoryl)-2-hydroxyacetate (G-3):

To 150 mL of toluene were added ethyl 2-carbonylacetate (15.0 g, 0.15 mol), diethyl phosphonate (22.0 g, 0.16 mol), and 4-methylbenzenesulfonic acid (0.25 g, 1.40 mmol), and the resulting mixture was stirred at 110°C for 16 hours. After the reaction system was cooled to room temperature, the reaction mixture was added with 100 mL of water and then extracted with EA (100 mL × 2). The combined organic phases were washed with saturated brine (150 mL × 2) and dried over anhydrous sodium sulfate. After filtration, the filtrate was concentrated under reduced pressure, and the residue was purified by silica gel column chromatography (using EA/PE, EA: 0% to 80% EA) to obtain the title compound **G-3** (15.0 g, yield: 43%).

¹H NMR (400 MHz, DMSO-*d₆*) δ 4.59 (d, *J* = 11.2 Hz, 1H), 4.17 (q, *J* = 7.2 Hz, 1H), 4.09 - 4.00 (m, 5H), 3.32 (s, 1H), 1.27 - 1.21 (m, 9H).

### Step 2: Ethyl 2-(diethoxyphosphoryl)-2-methoxyacetate (G-4):

To dichloromethane (20 mL) were added ethyl 2-(diethoxyphosphoryl)-2-hydroxyacetate (2.0 g, 8.30 mmol), silver oxide (2.89 g, 12.4 mmol), and iodomethane (11.8 g, 0.083 mol), and the reaction mixture was stirred at 0°C to room temperature for 16 hours. The reaction mixture was diluted with water (20 mL) and extracted with EA (15 mL × 2). The extracted organic phases were combined, washed with saturated brine (15 mL × 2), and dried over anhydrous sodium sulfate. After filtration, the filtrate was concentrated under reduced pressure. The resulting residue was purified by silica gel chromatography (gradient elution of EA in a mixed solvent with petroleum ether from 0% to 50%) to obtain the title compound **G-4** (1.60 g, yield: 72%).

¹H NMR (400 MHz, DMSO-*d₆*) δ 4.52 (d, *J* = 18.8 Hz, 1H), 4.23 - 4.15 (m, 2H), 4.11 - 4.04 (m, 4H), 3.37 (s, 3H), 1.25 - 1.21 (m, 9H).

### Step 3: Ethyl (E)-6-(3-ethoxy-2-methoxy-3-carbonylprop-1-en-1-yl)-5-nitronicotinate (G-6):

A suspension of sodium hydride (0.12 g, 5.0 mmol) in tetrahydrofuran (10 mL) was cooled to 0°C, then ethyl 2-(diethoxyphosphoryl)-2-methoxyacetate (1.0 g, 3.9 mmol) was slowly added dropwise thereto at a maintained temperature of 0°C, and the reaction mixture was stirred at 0°C for 30 minutes. 1,3-Dimethyltetrtahydropyrimidin-2(1*H*)-one (1.50 g, 0.012 mol) and ethyl 6-formyl-5-nitronicotinate (1.05 g, 4.60 mol) were slowly added dropwise thereto, and the reaction system was gradually warmed to room temperature and stirred for 4 hours. After the reaction was completed, the reaction mixture was quenched with saturated ammonium chloride aqueous solution (10 mL), diluted with water (20 mL), and extracted with EA (15 mL × 2). The extracted organic phases were combined, washed with saturated brine (15 mL × 2), and dried over anhydrous sodium sulfate. After filtration, the filtrate was concentrated under reduced pressure. The resulting residue was purified by silica gel chromatography (gradient elution of EA in a mixed solvent with petroleum ether from 0% to 80%) to obtain the title compound **G-6** (0.29 g, yield: 23%). LC-MS (ESI), m/z: [M+H]⁺ = 325.1.

### Step 4: Ethyl 7-methoxy-6-carbonyl-5,6-dihydro-1,5-naphthyridine-3-carboxylate (G-7):

To acetic acid (30 mL) were added ethyl (E)-6-(3-ethoxy-2-methoxy-3-carbonylprop-1-en-1-yl)-5-nitronicotinate (2.50 g, 7.7 mmol) and iron powder (3.44 g, 0.062 mol), and the reaction mixture was stirred at room temperature for 2 hours under argon atmosphere. After the reaction was completed, the reaction mixture was filtered, and the filtrate was concentrated under reduced pressure to obtain the crude product of the title compound **G-7** (2.0 g), which was directly used for the next step. LC-MS (ESI), m/z: [M+H]⁺ = 249.0.

### Step 5: 7-(Hydroxymethyl)-3-methoxy-1,5-naphthyridin-2(1H)-one (G-8):

A suspension of lithium aluminum hydride (1.46 g, 38.5 mmol) in tetrahydrofuran (50 mL) was cooled to 0°C. A solution of ethyl 7-methoxy-6-carbonyl-5,6-dihydro-1,5-naphthyridine-3-carboxylate (1.90 g, 7.7 mmol) in tetrahydrofuran was slowly added dropwise to the suspension at 0°C. The mixture was warmed to room temperature and stirred for 1 hour. After the reaction was completed, the reaction system was added with sodium sulfate decahydrate (1.30 g) to quench the reaction. After filtration, a solid was collected, then dissolved in water (100 mL), added with dilute HCl to adjust the pH to 6 to 7, and extracted with EA (150 mL × 2). The extracted suspended aqueous layers were then combined, and filtered to collect a solid. The resulting residue was purified by silica gel chromatography (gradient elution of MeOH in a mixed solvent with dichloromethane from 0% to 12.5%) to obtain the title compound **G-8** (0.25 g, yield: 16%). LC-MS (ESI), m/z: [M+H]⁺ = 207.1.

### Step 6: 7-(Bromomethyl)-3-methoxy-1,5-naphthyridin-2(1H)-one (intermediate G):

A solution of 7-(hydroxymethyl)-3-methoxy-1,5-naphthyridin-2(1*H*)-one (50.0 mg, 0.24 mmol) in tetrahydrofuran (1.0 mL) was cooled to 0°C, and then phosphorus tribromide (98.5 mg, 0.36 mmol) was slowly added dropwise thereto. The resulting mixture was stirred at room temperature for 3 hours. The reaction mixture was concentrated under reduced pressure to obtain the crude product of the title compound **intermediate G** (30.0 mg), which was directly used for the next step. LC-MS (ESI), m/z: [M+H]⁺ = 269.0.

### Intermediate H: 7-(hydroxymethyl)-6-fluoro-1,2,3,5-tetrahydro-1,2,3,5-tetrahydro-4H-cyclopenta[c]quinolin-4-one

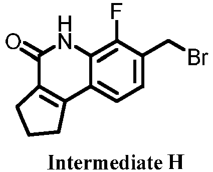

### Synthetic route:

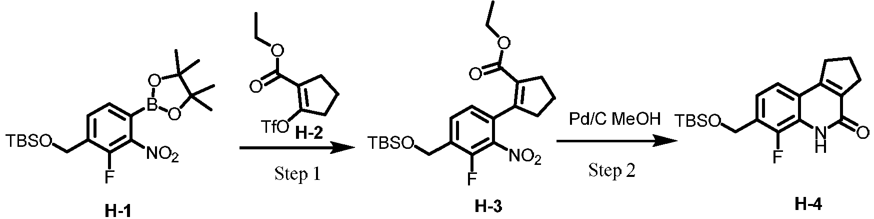

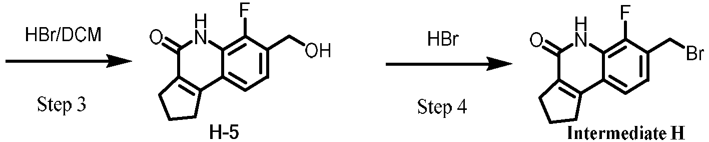

### Step 1: Synthesis of methyl 2-(4-(((tert-butyldimethylsilyl)oxy)methyl)-3-fluoro-2-nitrophenyl)cyclopent-1-ene-1-carboxylate (H-3)

To a solution of compound **H-1** (1.28 g, 3.12 mmol) in dioxane (10 mL) were added ethyl 2-(((trifluoromethyl)sulfonyl)oxy)cyclopent-1-ene-1-carboxylate (600 mg, 2.08 mmol), tetrakis(triphenylphosphine)palladium (241 mg, 0.210 mmol), and sodium carbonate (662 mg, 6.24 mmol) under nitrogen atmosphere. After the addition was completed, the reaction mixture was stirred at 100°C for 12 hours. After the reaction was completed, the reaction system was added with ethyl acetate (20 mL) and water (15 mL). The organic phase was washed with saturated brine, dried over anhydrous sodium sulfate, filtered to remove the solid, and the filtrate was concentrated under reduced pressure. The resulting residue was purified by silica gel column chromatography (PE/EA = 20/1) to obtain the title compound **H-3** (350 mg, yield: 26%). LC-MS (ESI), m/z: [M+H]⁺ = 424.0.

### Step 2: 7-(((tert-butyldimethylsilyl)oxy)methyl)-6-fluoro-1,2,3,5-tetrahydro-4H-cyclopenta[c]quinolin-4-one (H-4)

To a solution of compound **H-3** (300 mg, 0.709 mmol) in methanol (10 mL) was added palladium on carbon (60 mg), and the reaction mixture was stirred at room temperature for 16 hours under hydrogen atmosphere. After the reaction was completed, the reaction mixture was filtered to remove the solid, and the filtrate was concentrated under reduced pressure. The resulting residue was purified by silica gel column chromatography (PE/EA = 5/1) to obtain the title compound **H-4** (220 mg, yield: 89%). LC-MS (ESI), m/z: [M+H]⁺ = 348.1.

### Step 3: 7-(Hydroxymethyl)-6-fluoro-1,2,3,5-tetrahydro-1,2,3,5-tetrahydro-4H-cyclopenta[c]quinolin-4-one (H-5)

To a solution of compound **H-4** (200 mg, 0.576 mmol) in dichloromethane (5 mL) was added hydrobromic acid aqueous solution (40%, 2 mL), and the reaction mixture was stirred at room temperature for 2 hours. After the reaction was completed, the reaction mixture was concentrated under reduced pressure to obtain **H-5** (120 mg, yield: 89%). LC-MS (ESI), m/z: [M+H]⁺ = 234.0.

### Step 4: 7Bromo-6-fluoro-1,2,3,5-tetrahydro-4H-cyclopenta[c]quinolin-4-one (intermediate H)

To compound **H-5** (120 mg, 0.513 mmol) was added hydrobromic acid (40% aqueous solution, 5.00 mL) at room temperature, and the reaction mixture was stirred at 80°C for 2 hours. After the reaction was completed, the reaction mixture was diluted with ethyl acetate (10.0 mL). The organic phase was washed with water and saturated brine respectively, dried over anhydrous sodium sulfate, filtered, and concentrated to obtain the title compound **intermediate H** (140 mg, yield: 93%). LC-MS (ESI), m/z: [M+H]⁺ = 295.9, 297.9.

### Intermediate I: 7-(hydroxymethyl)-6-methyl-1,2,3,5-tetrahydro-4H-cyclopenta[c]quinolin-4-one

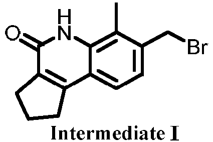

### Synthetic route:

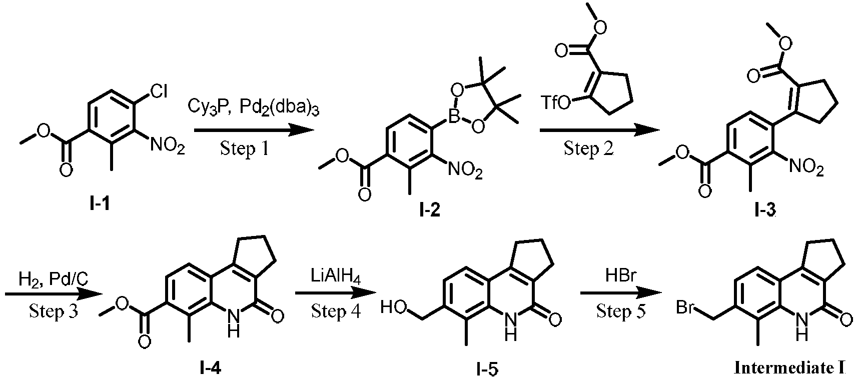

### Step 1: Synthesis of methyl 2-methyl-3-nitro-4-(4,4,5,5-tetramethyl-1,3,2-dioxaborolan-2-yl)benzoate (1-2)

To a solution of compound **1-1** (1.00 g, 4.40 mmol) in 1,4-dioxane (20 mL) were added bis(pinacolato)diboron (2.23 g, 8.80 mmol), tris(dibenzylideneacetone)dipalladium (0.400 g, 0.440 mmol), tricyclohexylphosphine (0.250 g, 0.880 mmol), and potassium acetate (1.30 g, 13.2 mmol) under nitrogen atmosphere. After the addition was completed, the mixture was stirred at 90°C for 16 hours. After the reaction was completed, the reaction mixture was cooled to room temperature and directly used for the next reaction step.

LC-MS (ESI), m/z: [M+HCOO]⁻ = 284.1.

### Step 2: Synthesis of methyl 4-(2-(methoxycarbonyl)cyclopent-1-en-1-yl)-2-methyl-3-nitrobenzoate (I-3)

To the reaction mixture from the previous step were added ethyl 2-(((trifluoromethyl)sulfonyl)oxy)cyclopent-1-ene-1-carboxylate (880 mg, 3.20 mmol), tetrakis(triphenylphosphine)palladium (180 mg, 0.160 mmol), and sodium carbonate (510 mg, 4.80 mmol) under nitrogen atmosphere, and the reaction mixture was stirred at 100°C for 1 hour. After the reaction was completed, the reaction mixture was concentrated under reduced pressure, and the resulting residue was the crude product of the title compound **1-3** (2.50 g). LC-MS (ESI), m/z: [M+³⁵Cl-H]⁻ = 354.0.

### Step 3: Synthesis of methyl 6-methyl-4-oxo-2,3,4,5-tetrahydro-1H-cyclopenta[c]quinoline-7-carboxylate (1-4)

To a solution of compound **1-3** (200 mg, 0.626 mmol) in methanol (10 mL) was added palladium on carbon (40 mg), and the reaction mixture was stirred at room temperature for 16 hours. After the reaction was completed, the reaction mixture was filtered, and the filtrate was concentrated under reduced pressure. The resulting residue was purified by silica gel column chromatography (PE/EA= 1/1) to obtain the title compound **1-4** (100 mg, yield: 62%). LC-MS (ESI), m/z: [M+H]⁺ = 258.0.

### Step 4: Synthesis of 7-(hydroxymethyl)-6-methyl-1,2,3,5-tetrahydro-4H-cyclopenta[c]quinolin-4-one (1-5)

A solution of compound **1-4** (100 mg, 0.388 mmol) in tetrahydrofuran (3 mL) was cooled to 0°C, then a solution of lithium aluminum hydride in tetrahydrofuran (1.0 mol/L, 0.77 mL, 0.77 mmol) was added to the reaction system, and the reaction mixture was stirred for 1 hour. After the reaction was completed, the reaction system was added with water (0.1 mL), and the resulting mixture was concentrated under reduced pressure. The resulting residue was purified by silica gel column chromatography (DCM/MeOH = 10/1) to obtain **1-5** (50 mg, yield: 50%). LC-MS (ESI), m/z: [M+H]⁺ = 229.3.

### Step 5: 7-chloromethyl-6-methyl-1,2,3,5-tetrahydro-4H-cyclopenta[c]quinolin-4-one (intermediate I)

An aqueous solution (30.0%) of compound **1-5** (50.0 mg, 0.218 mmol) in hydrobromic acid (2.00 mL) was heated to 80°C, and the reaction mixture was stirred at 80°C for 2 hours. After the reaction was completed, the solvent was concentrated under reduced pressure to obtain the crude product of **intermediate I** (50.0 mg), which was directly used for the next step. LC-MS (ESI), m/z: [M+H]⁺ = 292.0, 294.0.

### Intermediate J: 7-fluoro-8-(hydroxymethyl)-2,3-dihydro-1H-pyrano[2,3-c]quinolin-5(6H)-one

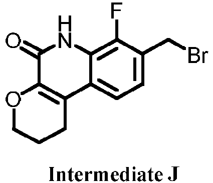

### Synthetic route:

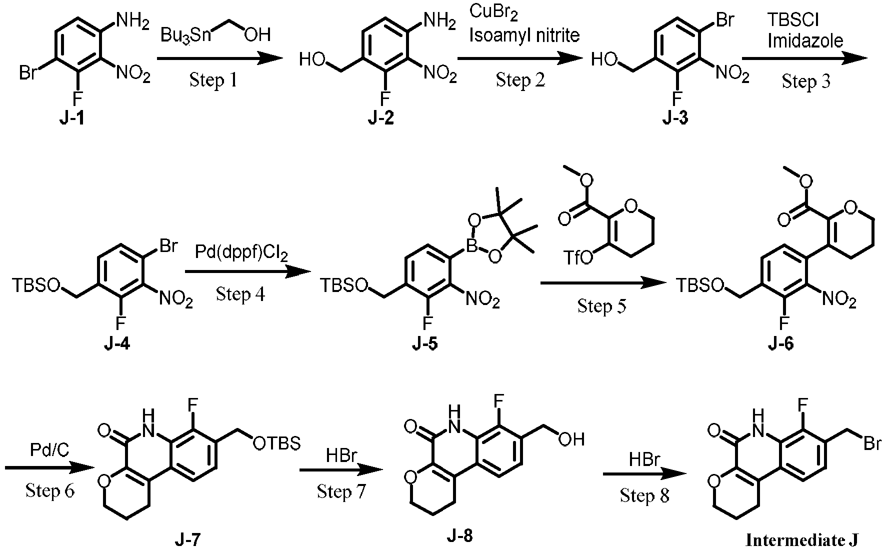

### Step 1: Synthesis of (4-amino-2-fluoro-3-nitrophenyl)methanol (J-2)

To a solution of 4-bromo-3-fluoro-2-nitroaniline (5.00 g, 21.3 mmol) in 1,4-dioxane (50 mL) were added (tributylstannyl)methanol (10.2 g, 31.9 mmol) and (2-dicyclohexylphosphino-2',4',6'-triisopropyl-1,1'-biphenyl)(2'-amino-1,1'-biphenyl-2-yl)palladium(II) chloride (0.840 g, 1.06 mmol) under nitrogen atmosphere. After the addition was completed, the reaction mixture was stirred at 80°C for 16 hours. After the reaction was completed, the reaction system was added with saturated potassium fluoride aqueous solution (150 mL) and stirred at room temperature for 3 minutes. The reaction mixture was filtered to remove the solid, and the filtrate was extracted with ethyl acetate (100 mL × 3). The combined organic phases were washed with saturated brine, dried over anhydrous sodium sulfate, filtered to remove the solid, and the filtrate was concentrated under reduced pressure. The resulting residue was purified by silica gel column chromatography (PE/EA = 5/1) to obtain the title compound **J-2** (3.00 g, yield: 76%). LC-MS (ESI), m/z: [M-H]⁻ = 185.1.

### Step 2: Synthesis of (4-bromo-2-fluoro-3-nitrophenyl)methanol (J-3)

To a solution of isoamyl nitrite (2.28 g, 19.4 mmol) in acetonitrile (50 mL) was added copper bromide (4.34 g, 19.4 mmol), and the reaction mixture was stirred at 25°C for 1 hour. The reaction system was added with compound **J-2** (3.00 g, 16.1 mmol) and stirred for another 0.5 hours. The reaction system was heated to 70°C and stirred for 2 hours. After the reaction was completed, the reaction mixture was concentrated under reduced pressure. The resulting residue was diluted with water (50 mL) and extracted with ethyl acetate (30 mL × 3). The combined organic phases were washed with saturated brine, dried over anhydrous sodium sulfate, filtered to remove the solid, and the filtrate was concentrated under reduced pressure. The resulting residue was purified by silica gel column chromatography (PE/EA = 5/1) to obtain the title compound **J-3** (2.00 g, yield: 50%).

¹H (400 MHz, DMSO-*d₆*) δ 7.76 (dd, *J =* 8.4, 1.2 Hz, 1H), 7.69 (t, *J =* 7.9 Hz, 1H), 5.63 (s, 1H), 4.60 (s, 2H).

### Step 3: Synthesis of ((4-bromo-2-fluoro-3-nitrobenzyl)oxy)(tert-butyl)dimethylsilane (J-4)

To a solution of compound **J-3** (2.00 g, 8.00 mmol) in *N*,*N*-dimethylformamide (20 mL) were added imidazole (1.09 g, 16.0 mmol) and *tert*-butyldimethylchlorosilane (2.41 g, 16.0 mmol), and the reaction mixture was stirred at room temperature for 16 hours. After the reaction was completed, the reaction system was diluted with water (100 mL), and the mixture was extracted with ethyl acetate (50.0 mL × 3). The combined organic phases were washed with saturated brine, dried over anhydrous sodium sulfate, filtered to remove the solid, and the filtrate was concentrated under reduced pressure. The resulting residue was purified by silica gel column chromatography (PE/EA = 20/1) to obtain the title compound **J-4** (2.30 g, yield: 79%).

### Step 4: Synthesis of tert-butyl((2-fluoro-3-nitro-4-(4,4,5,5-tetramethyl-1,3,2-dioxaborolan-2-yl)benzyl)oxy)dimethylsilane (J-5)

To a solution of compound **J-4** (2.00 g, 5.49 mmol) in 1,4-dioxane (30 mL) were added bis(pinacolato)diboron (2.79 g, 11.0 mmol), [1,1'-bis(diphenylphosphino)ferrocene]dichloropalladium(II) (0.402 g, 0.549 mmol), and potassium acetate (1.61 g, 16.5 mmol) under nitrogen atmosphere. After the addition was completed, the reaction mixture was stirred at 100°C for 16 hours. After the reaction was completed, a mixture containing the title compound **J-5** was obtained and directly used for the next reaction step. LC-MS (ESI), m/z: [M+HCOO]⁻ = 374.1.

### Step 5: Synthesis of methyl 5-(4-(((tert-butyldimethylsilyl)oxy)methyl)-3-fluoro-2-nitrophenyl)-3,4-dihydro-2H-pyran-6-carboxylate (J-6)

To the mixture containing compound **J-5** were added methyl 5-(((trifluoromethyl)sulfonyl)oxy)-3,4-dihydro-2*H*-pyran-6-carboxylate (565 mg, 1.95 mmol), tetrakis(triphenylphosphine)palladium (112 mg, 0.0972 mmol), and sodium carbonate (309 mg, 2.92 mmol) under nitrogen atmosphere, and the reaction mixture was stirred at 100°C for 10 hours. After the reaction was completed, the reaction mixture was concentrated under reduced pressure, and the resulting residue was the crude product of the title compound **J-6** (1.20 g). LC-MS (ESI), m/z: [M-TBS+³⁵Cl]⁻ = 346.0.

### Step 6: Synthesis of 8-(((tert-butyldimethylsilyl)oxy)methyl)-7-fluoro-2,3-dihydro-1H-pyrano[2,3-c]quinolin-5(6H)-one (J-7)

To a solution of compound **J-6** (300 mg, 0.706 mmol) in methanol (10 mL) was added palladium on carbon (50 mg), and the reaction mixture was stirred at 25°C for 16 hours. After the reaction was completed, the reaction mixture was filtered, and the filtrate was concentrated under reduced pressure. The resulting residue was purified by silica gel chromatography (PE/EA = 1/1) to obtain the title compound **J-7** (200 mg, yield: 78%). LC-MS (ESI), m/z: [M+H]⁺ = 364.2.

### Step 7: Synthesis of 7-fluoro-8-(hydroxymethyl)-2,3-dihydro-1H-pyrano[2,3-c]quinolin-5(6H)-one (J-8)

Compound **J-7** (200 mg, 0.550 mmol) was dissolved in hydrobromic acid aqueous solution (30%, 5 mL), and the reaction mixture was stirred at room temperature for 0.5 hours. After the reaction was completed, a mixture containing the title compound **J-8** was obtained and directly used for the next reaction step. LC-MS (ESI), m/z: [M+H]⁺ = 250.0.

### Step 8: Synthesis of 8-(bromomethyl)-7-fluoro-2,3-dihydro-1H-pyrano[2,3-c]quinolin-5(6H)-one (intermediate J)

The mixture containing the title compound **J-8** from the previous reaction step was heated to 80°C and stirred at this temperature for 2 hours. After the reaction was completed, the reaction mixture was concentrated under reduced pressure, diluted with water (20 mL), slurried, purified, and filtered. A solid was collected and washed with ethyl acetate (20 mL). The resulting solid was concentrated under reduced pressure to remove the solvent to obtain the title compound **intermediate J** (60.0 mg, yield: 48%). LC-MS (ESI), m/z: [M+H]⁺ = 311.9, 313.9.

### Intermediate K: 8-(bromomethyl)-7-methyl-1H,2H,3H,6H-pyrano[2,3-c]quinolin-5-one

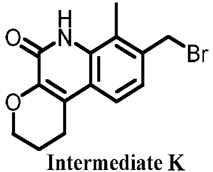

### Synthetic route:

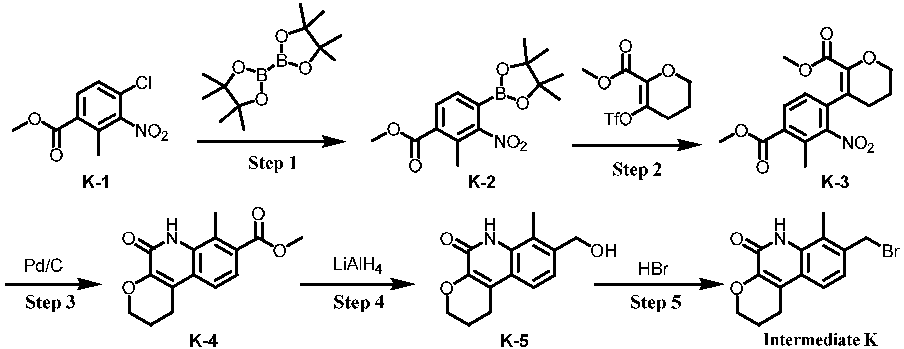

### Step 1: Synthesis of methyl 2-methyl-3-nitro-4-(4,4,5,5-tetramethyl-1,3,2-dioxaborolan-2-yl)benzoate (K-2)

To a solution of compound **K-1** (500 mg, 2.18 mmol) in 1,4-dioxane (2 mL) were added bis(pinacolato)diboron (1.11 g, 4.35 mmol), bis(dibenzylideneacetone)palladium (125 mg, 0.218 mmol), tricyclohexylphosphine (122 mg, 0.435 mmol), and potassium acetate (320 mg, 3.26 mmol) under nitrogen atmosphere, and the reaction mixture was stirred at 90°C for 16 hours. After the reaction was completed, the reaction mixture was concentrated under reduced pressure to obtain the crude product of the title compound **K-2** (1.40 g), which was directly used for the next reaction step.

LC-MS (ESI), m/z: [M+³⁵Cl]⁺ = 274.1.

### Step 2: Synthesis of methyl 3-(4-(methoxycarbonyl)-3-methyl-2-nitrophenyl)-5,6-dihydro-4H-pyran-2-carboxylate (K-3)

To a solution of compound **K-2** (500 mg, 1.56 mmol) in 1,4-dioxane (10 mL) were added methyl 5-(((trifluoromethyl)sulfonyl)oxy)-3,4-dihydro-2*H*-pyran-6-carboxylate (904 mg, 3.11 mmol), tetrakis(triphenylphosphine)palladium (180 mg, 0.156 mmol), and sodium carbonate (330 mg, 3.11 mmol) under nitrogen atmosphere, and the reaction mixture was stirred at 100°C for 16 hours. After the reaction was completed, the reaction mixture was concentrated under reduced pressure to obtain the crude product of the title compound **K-3** (2.0 g), which was directly used for the next reaction step. LC-MS (ESI), m/z: [M+H]⁺ = 336.1.

### Step 3: Synthesis of methyl 7-methyl-5-oxo- 1H,2H,3H,6H-pyrano[2,3-c]quinoline-8-carboxylate (K-4)

To a solution of compound **K-3** (500 mg, 1.49 mmol) in methanol (10 mL) was added palladium on carbon (100 mg) under nitrogen atmosphere, and the reaction mixture was stirred at room temperature for 16 hours under hydrogen atmosphere. After the reaction was completed, the reaction mixture was filtered to remove the solid, and the filtrate was concentrated under reduced pressure. The resulting residue was purified by silica gel column chromatography (DCM/MeOH = 100/1) to obtain the title compound **K-4** (590 mg, yield: 94%). LC-MS (ESI), m/z: [M+H]⁺ = 274.1.

### Step 4: Synthesis of 8-(hydroxymethyl)-7-methyl-1H,2H,3H,6H-pyrano[2,3-c]quinolin-5-one (K-5)

A solution of compound **K-4** (540 mg, 2.00 mmol) in tetrahydrofuran (10 mL) was cooled to 0°C under nitrogen atmosphere, then a solution of lithium aluminum hydride in tetrahydrofuran (1.0 mol/L, 6.0 mL, 6.0 mmol) was added thereto, and the reaction mixture was stirred for 2 hours. After the reaction was completed, the reaction system was added with ethyl acetate (20 mL) and water (240 mg). The resulting mixture was concentrated under reduced pressure, and the resulting residue was purified by silica gel column chromatography (DCM/MeOH = 10/1) to obtain the title compound **K-5** (174 mg, yield: 32%). LC-MS (ESI), m/z: [M+H]⁺ = 246.1.

### Step 5: Synthesis of 8-(bromomethyl)-7-methyl-1H,2H,3H,6H-pyrano[2,3-c]quinolin-5-one (intermediate K)

An aqueous solution of compound **K-5** (30.0 mg, 0.122 mmol) in hydrogen bromide (2 mL) was stirred at 80°C for 2 hours. After the reaction was completed, the reaction system was extracted with ethyl acetate (20 mL). The organic phase was concentrated under reduced pressure, and the resulting residue was the crude product of **intermediate K** (32 mg). LC-MS (ESI), m/z: [M+H]⁺ = 308.0, 310.0.

### Intermediate L: 3-ethyl-7-(hydroxymethyl)quinoxalin-2(1H)-one

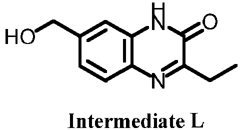

### Synthetic route:

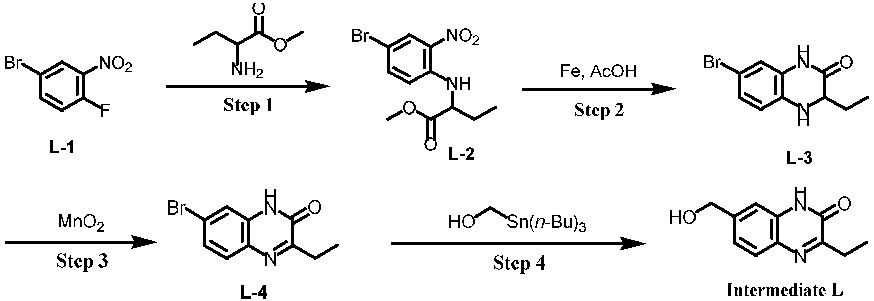

### Step 1: Synthesis of methyl 2-((4-bromo-2-nitrophenyl)amino)butanoate (L-2)

To a solution of compound **L-1** (10.0 g, 45.5 mmol) in *N*,*N*-dimethylformamide (40 mL) were added methyl 2-aminobutanoate (8.0 g, 68 mmol) and diisopropylethylamine (17.6 g, 136 mmol) under nitrogen atmosphere. After the addition was completed, the reaction mixture was stirred at room temperature for 16 hours. After the reaction was completed, the system was added with water (300 mL), and the resulting mixture was extracted with ethyl acetate (100 mL × 3). The combined organic phases were washed with saturated brine, dried over anhydrous sodium sulfate, filtered to remove the solid, and concentrated under reduced pressure. The resulting residue was purified by silica gel column chromatography (DCM/MeOH = 20/1) to obtain the title compound **L-2** (14.4 g, yield: 90%). LC-MS (ESI), m/z: [M+H]⁺ = 317.0.

### Step 2: Synthesis of 7-bromo-3-ethyl-3,4-dihydroquinoxalin-2(1H)-one (L-3)

To a solution of compound **L-2** (14.4 g, 45.4 mmol) in glacial acetic acid (250 mL) was added reduced iron powder (20.3 g, 363 mmol) under nitrogen atmosphere. After the addition was completed, the reaction mixture was stirred at room temperature for 2 hours. After the reaction was completed, the reaction mixture was concentrated under reduced pressure, diluted with water (200 mL), and the resulting mixture was extracted with ethyl acetate (100 mL × 3). The combined organic phases were washed with saturated brine, dried over anhydrous sodium sulfate, filtered to remove the solid, and concentrated under reduced pressure. The resulting residue was purified by silica gel column chromatography (DCM/MeOH = 30/1) to obtain the title compound **L-3** (13.2 g, yield: 96%). LC-MS (ESI), m/z: [M+H]⁺ = 255.1.

### Step 3: Synthesis of 7-bromo-3-ethylquinoxalin-2(1H)-one (L-4)

To a solution of compound **L-3** (2.0 g, 7.8 mmol) in tetrahydrofuran (100 mL) was added manganese dioxide (6.78 g, 78.0 mmol) under nitrogen atmosphere, and the mixture was stirred at room temperature for 1 hour. After the reaction was completed, the reaction mixture was concentrated under reduced pressure. The resulting residue was diluted with dichloromethane (100 mL) and methanol (10 mL), and filtered to remove the solid. The resulting filtrate was concentrated, and the resulting residue was the title compound **L-4** (1.50 g, yield: 68%).

LC-MS (ESI), m/z: [M+H]⁺ = 253.0.

### Step 4: 3-ethyl-7-(hydroxymethyl)quinoxalin-2(1H)-one (intermediate L)

To a solution of compound **L-4** (600 mg, 2.37 mmol) in dioxane (10 mL) were added (tributylstannyl)methanol (913 mg, 2.84 mmol) and (2-dicyclohexylphosphino-2',4',6'-triisopropyl-1,1'-biphenyl)(2'-amino-1,1'-biphenyl-2-yl)palladium(II) chloride (186 mg, 0.237 mmol) under nitrogen atmosphere. After the addition was completed, the reaction mixture was stirred at 80°C for 16 hours. After the reaction was completed, the reaction mixture was concentrated under reduced pressure, and the resulting residue was purified by silica gel column chromatography (DCM/MeOH = 20/1) to obtain **intermediate L** (360 mg, yield: 67%). LC-MS (ESI), m/z: [M+H]⁺ = 205.1.

### Intermediate M: 3-methyl-4-fluoro-7-(hydroxymethyl)-1,5-naphthyridin-2(1H)-one

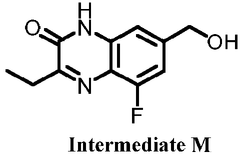

### Synthetic route:

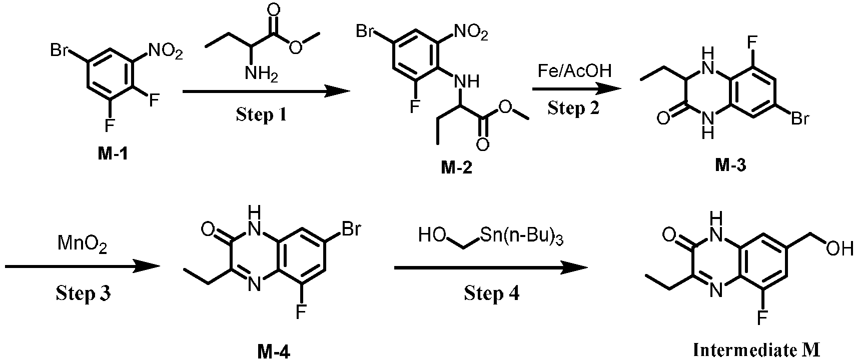

### Step 1: Synthesis of methyl 1-2-[(4-bromo-2-fluoro-6-nitrophenyl)amino]butanoate (M-2)

To a solution of compound **M-1** (10.0 g, 45.5 mmol) in acetonitrile (50 mL) were added methyl 2-aminobutanoate (2.70 g, 22.6 mmol) and potassium carbonate (7.84 g, 56.7 mmol) under nitrogen atmosphere. After the addition was completed, the reaction mixture was stirred at 80°C for 12 hours. After the reaction was completed, the reaction mixture was concentrated under reduced pressure. The resulting residue was dissolved in ethyl acetate (100 mL), washed with saturated brine (100 mL), dried over anhydrous sodium sulfate, and concentrated under reduced pressure. The resulting residue was purified by silica gel column chromatography (PE/EA = 5/1) to obtain the title compound **M-2** (4.10 g, yield: 58%). LC-MS (ESI), m/z: [M+H]⁺ = 334.9.

### Step 2: Synthesis of 7-bromo-3-ethyl-5-fluoro-3,4-dihydro-1H-quinoxalin-2-one (M-3)

To a solution of compound **M-2** (4.01 g, 11.9 mmol) in glacial acetic acid (20 mL) was added reduced iron powder (3.31 g, 59.5 mmol) under nitrogen atmosphere. After the addition was completed, the reaction mixture was stirred at room temperature for 12 hours. After the reaction was completed, the reaction mixture was filtered to remove the solid, and concentrated under reduced pressure. The resulting residue was the crude product of the title compound **M-3** (2.80 g), which was directly used for the next reaction step. LC-MS (ESI), m/z: [M+H]⁺ = 273.0.

### Step 3: Synthesis of 7-bromo-3-ethyl-5-fluoro-1H-quinoxalin-2-one (M-4)

To a solution of compound **M-3** (2.70 g, 9.90 mmol) in tetrahydrofuran (15 mL) was added manganese dioxide (8.60 g, 9.90 mmol) under nitrogen atmosphere. After the addition was completed, the reaction mixture was stirred at room temperature for 12 hours. After the reaction was completed, the reaction mixture was filtered to remove the solid, and concentrated under reduced pressure. The resulting residue was the crude product of the title compound **M-4** (2.30 g), which was directly used for the next reaction step. LC-MS (ESI), m/z: [M+H]⁺ = 270.9.

### Step 4: Synthesis of 3-ethyl-5-fluoro-7-(hydroxymethyl)-1H-quinoxalin-2-one (intermediate M)

To a solution of compound **M-4** (500 mg, 1.80 mmol) in dioxane (10 mL) were added (tributylstannyl)methanol (888, 2.80 mmol) and (2-dicyclohexylphosphino-2',4',6'-triisopropyl-1,1'-biphenyl)(2'-amino-1,1'-biphenyl-2-yl)palladium(II) chloride (145 mg, 0.200 mmol) under nitrogen atmosphere. After the addition was completed, the reaction mixture was stirred at 80°C for 12 hours. After the reaction was completed, the reaction mixture was concentrated under reduced pressure, and the resulting residue was purified by silica gel column chromatography (PE/EA = 1/1) to obtain **intermediate M** (230 mg, yield: 52%). LC-MS (ESI), m/z: [M+H]⁺ = 223.1.

### Intermediate N: 3-ethyl-4-fluoro-7-(hydroxymethyl)-1,5-naphthyridin-2(1H)-one

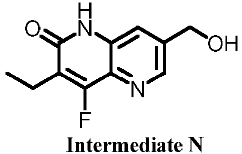

### Synthetic route:

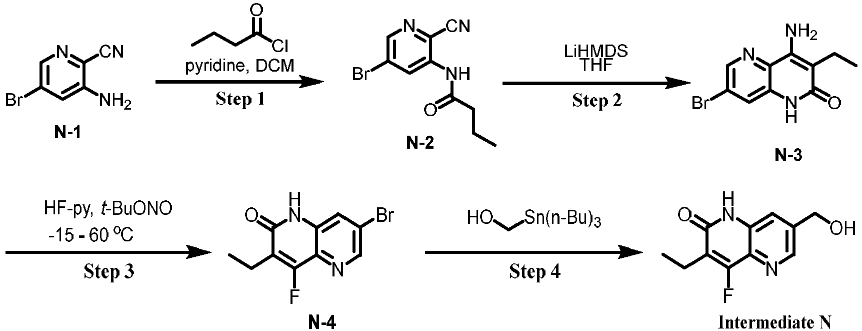

### Step 1: Synthesis of N-(5-bromo-2-cyanopyridin-3-yl)butyramide (N-2)

To a solution of compound **N-1** (10.0 g, 50.5 mmol) in dichloromethane (100 mL) were added dry pyridine (4.79 g, 60.6 mmol) and butyryl chloride (6.46 g, 60.6 mmol) under nitrogen atmosphere. After the addition was completed, the reaction mixture was stirred at room temperature for 2 hours. After the reaction was completed, the reaction system was added with water (100 mL), and the resulting mixture was extracted with ethyl acetate (100 mL × 2). The combined organic phases were washed with saturated brine, dried over anhydrous sodium sulfate, filtered to remove the solid, and concentrated under reduced pressure. The resulting residue was purified by silica gel column chromatography (PE/EA = 10/1) to obtain the title compound **N-2** (12.5 g, yield: 92%). LC-MS (ESI), m/z: [M+H]⁺ = 268.0, 269.9.

### Step 2: Synthesis of 4-amino-7-bromo-3-ethyl-1,5-naphthyridin-2(1H)-one (N-3)

A solution of compound **J-2** (10.0 g, 37.3 mmol) in tetrahydrofuran (100 mL) was cooled to -78°C under nitrogen atmosphere, and lithium bis(trimethylsilyl)amide (1.0 mmol/L, 41.0 mL, 41.0 mmol) was added dropwise thereto. After the dropwise addition was completed, the reaction mixture was stirred at -78°C for 2 hours. Thereafter, lithium bis(trimethylsilyl)amide (1.0 mmol/L, 41.0 mL, 41.0 mmol) was again added to the reaction system. After the addition was completed, the reaction mixture was naturally warmed to room temperature and stirred for another 16 hours. After the reaction was completed, the reaction mixture was quenched with saturated ammonium chloride aqueous solution (200 mL), and the resulting mixture was extracted with ethyl acetate (150 mL × 3). The combined organic phases were washed with saturated brine, dried over anhydrous sodium sulfate, filtered to remove the solid, and concentrated under reduced pressure. The resulting residue was purified by silica gel column chromatography (DCM/MeOH = 20/1) to obtain the title compound **N-3** (2.5 g, yield: 25%). LC-MS (ESI), m/z: [M+H]⁺ = 268.0, 270.0.

### Step 3: Synthesis of 7-bromo-3-ethyl-4-fluoro-1,5-naphthyridin-2(1H)-one (N-4)

A solution of hydrofluoric acid in pyridine (65 to 70%, 27 mL) was cooled to -40°C, and compound **N-3** (2.70 g, 10.1 mmol) and isoamyl nitrite (2.37 g, 20.2 mmol) were added thereto. After the addition was completed, the reaction mixture was slowly warmed to room temperature and stirred for 16 hours. After the reaction was completed, the reaction system was diluted with water (100 mL), and the resulting mixture was extracted with ethyl acetate (70 mL × 3). The combined organic phases were washed with saturated brine, dried over anhydrous sodium sulfate, filtered to remove the solid, and concentrated under reduced pressure. The resulting residue was slurried and purified (EA/MeOH = 2/1) to obtain the title compound **N-4** (270 mg, yield: 10%). LC-MS (ESI), m/z: [M+H]⁺ = 271.0, 273.0.

### Step 4: Synthesis of 3-ethyl-4-fluoro-7-(hydroxymethyl)-1,5-naphthyridin-2(1H)-one (intermediate N)

To a solution of compound **N-4** (240 mg, 0.885 mmol) in 1,4-dioxane (5 mL) were added (tributylstannyl)methanol (313 mg, 0.974 mmol) and (2-dicyclohexylphosphino-2',4',6'-triisopropyl-1,1'-biphenyl)(2'-amino-1,1'-biphenyl-2-yl)palladium(II) chloride (34.8 mg, 0.0443 mmol) under nitrogen atmosphere. After the addition was completed, the reaction mixture was stirred at 80°C for 16 hours. After the reaction was completed, the reaction mixture was concentrated under reduced pressure, and the resulting residue was slurried and purified (EA/MeOH = 2/1) to obtain **intermediate N** (120 mg, yield: 61%). LC-MS (ESI), m/z: [M+H]⁺ = 223.1.

### Intermediate O: 3-methyl-4-fluoro-7-(hydroxymethyl)-1,5-naphthyridin-2(1H)-one

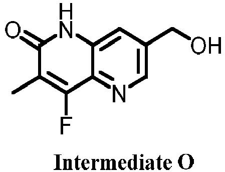

### Synthetic route:

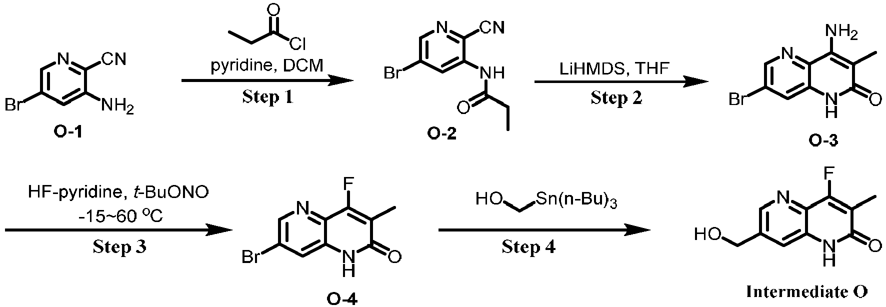

### Step 1: Synthesis of N-(5-bromo-2-cyanopyridin-3-yl)propionamide (O-2)

To a solution of compound **O-1** (10.0 g, 50.5 mmol) in anhydrous dichloromethane (100 mL) were added dry pyridine (4.79 g, 60.6 mmol) and propionyl chloride (5.61 g, 60.6 mmol) under nitrogen atmosphere, and the reaction mixture was stirred at room temperature for 2 hours. After the reaction was completed, the reaction system was added with water (100 mL), and the resulting mixture was extracted with dichloromethane (100 mL × 2). The combined organic phases were washed with saturated brine, dried over anhydrous sodium sulfate, filtered to remove the solid, and concentrated under reduced pressure. The resulting residue was purified by silica gel column chromatography (PE/EA = 10/1) to obtain the title compound **O-2** (12.0 g, yield: 84%). LC-MS (ESI), m/z: [M+H]⁺ = 254.0, 256.0.

### Step 2: Synthesis of 4-amino-7-bromo-3-methyl-1,5-naphthyridin-2(1H)-one (O-3)

A solution of compound **O-2** (1.00 g, 3.90 mmol) in tetrahydrofuran (10 mL) was cooled to -78°C under nitrogen atmosphere, and lithium bis(trimethylsilyl)amide (1.0 mmol/L, 9.0 mL, 9.0 mmol) was added dropwise thereto. After the dropwise addition was completed, the reaction mixture was naturally warmed to room temperature and stirred for 16 hours. After the reaction was completed, the reaction mixture was quenched with saturated ammonium chloride aqueous solution (20 mL), and the resulting mixture was extracted with ethyl acetate (20 mL × 3). The combined organic phases were washed with saturated brine, dried over anhydrous sodium sulfate, filtered to remove the solid, and concentrated under reduced pressure. The resulting residue was purified by silica gel column chromatography (DCM/MeOH = 20/1) to obtain the title compound **O-3** (420 mg, yield: 38%). LC-MS (ESI), m/z: [M+H]⁺ = 253.9, 255.9.

### Step 3: Synthesis of 7-bromo-3-methyl-4-fluoro-1,5-naphthyridin-2(1H)-one (O-4)

A solution of HF-pyridine (65 to 70%, 7.0 mL) was cooled to -40°C, and compound **O-3** (500 mg, 1.97 mmol) and isoamyl nitrite (575 g, 4.92 mmol) were added thereto. After the addition was completed, the reaction mixture was slowly warmed to room temperature and stirred for 16 hours. After the reaction was completed, the reaction system was diluted with water (15 mL), and the resulting mixture was extracted with ethyl acetate (15 mL × 3). The combined organic phases were washed with saturated brine, dried over anhydrous sodium sulfate, filtered to remove the solid, and concentrated under reduced pressure. The resulting residue was purified by silica gel column chromatography (DCM/MeOH = 20/1) to obtain the title compound **O-4** (90 mg, yield: 18%). LC-MS (ESI), m/z: [M+H]⁺ = 257.0, 259.0.

### Step 4: Synthesis of 3-methyl-4-fluoro-7-(hydroxymethyl)-1,5-naphthyridin-2(1H)-one (intermediate O)

To a solution of compound **O-4** (200 mg, 0.778 mmol) in 1,4-dioxane (5 mL) were added (tributylstannyl)methanol (375 mg, 1.17 mmol) and (2-dicyclohexylphosphino-2',4',6'-triisopropyl-1,1'-biphenyl)(2'-amino-1,1'-biphenyl-2-yl)palladium(II) chloride (30.6 mg, 0.00389 mmol) under nitrogen atmosphere. After the addition was completed, the reaction mixture was stirred at 80°C for 16 hours. After the reaction was completed, the reaction mixture was concentrated under reduced pressure, and the resulting residue was slurried and purified (EA/MeOH = 2/1) to obtain **intermediate O** (90 mg, yield: 52%). LC-MS (ESI), m/z: [M+H]⁺ = 209.1.

### Intermediate P: 3-(1,1-difluoroethyl)-7-(hydroxymethyl)quinoxalin-2(1H)-one

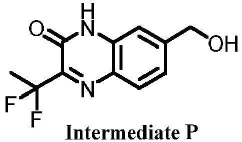

### Synthetic route:

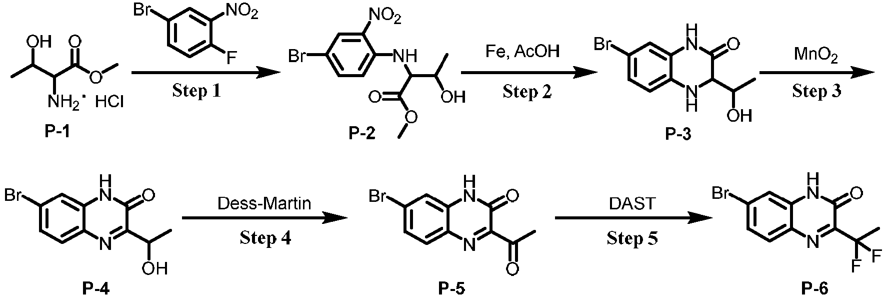

### Step 1: Synthesis of methyl 2-((4-bromo-2-nitrophenyl)amino)-3-hydroxybutanoate (P-2)

To a solution of compound **P-1** (2.00 g, 11.8 mmol) in *N*,*N*-dimethylformamide (30 mL) were added diisopropylethylamine (4.58 g, 35.5 mmol) and 2-fluoro-5-bromonitrobenzene (2.60 g, 11.8 mmol), and the reaction mixture was stirred at 50°C for 3 hours. After the reaction was completed, the reaction mixture was concentrated under reduced pressure, and the resulting residue was purified by silica gel column chromatography (DCM/MeOH = 20/1) to obtain the title compound **P-2** (2.80 g, yield: 76%). LC-MS (ESI), m/z: [M+H]⁺ = 333.0, 335.0.

### Step 2: Synthesis of 7-bromo-3-(1-hydroxyethyl)-3,4-dihydroquinoxalin-2(1H)-one (P-3)

To a solution of compound **P-2** (2.80 g, 8.41 mmol) in glacial acetic acid (250 mL) was added reduced iron powder (3.58 g, 64.0 mmol) under nitrogen atmosphere. After the addition was completed, the reaction mixture was stirred at room temperature for 16 hours. After the reaction was completed, the reaction mixture was concentrated under reduced pressure. The resulting residue was diluted with dichloromethane (500 mL) and methanol (100 mL), filtered to remove the solid, and concentrated again under reduced pressure. The resulting residue was purified by silica gel column chromatography (DCM/MeOH = 20/1) to obtain the title compound **P-3** (2.00 g, yield: 88%). LC-MS (ESI), m/z: [M+H]⁺ = 271.0, 273.0.

### Step 3: Synthesis of 7-bromo-3-(1-hydroxyethyl)quinoxalin-2(1H)-one (P-4)

To a solution of compound **P-3** (271 mg, 1.00 mmol) in tetrahydrofuran (5 mL) was added manganese dioxide (870 mg, 10.0 mmol) under nitrogen atmosphere, and the mixture was stirred at room temperature for 2 hours. After the reaction was completed, the reaction mixture was filtered to remove the solid, and the filtrate was concentrated under reduced pressure. The resulting residue was the crude product of the title compound **P-4** (200 mg), which was directly used for the next reaction step. LC-MS (ESI), m/z: [M+H]⁺ = 269.0, 271.0.

### Step 4: Synthesis of 3-acetyl-7-bromoquinoxalin-2(1H)-one (P-5)

A solution of compound **P-4** (200 mg, 0.743 mmol) in dichloromethane (5 mL) was cooled to 0°C under nitrogen atmosphere, then Dess-Martin periodinane (424 mg, 1.00 mmol) was added thereto, and the reaction mixture was stirred at room temperature for 16 hours. After the reaction was completed, the reaction mixture was concentrated under reduced pressure, and the resulting residue was purified by liquid chromatography (mobile phase: acetonitrile/0.1% formic acid in water; gradient: 10 to 100%) to obtain the title compound **P-5** (130 mg, yield: 66%). LC-MS (ESI), m/z: [M+H]⁺ = 267.0, 269.0.

### Step 5: Synthesis of 7-bromo-3-(1,1-difluoroethyl)quinoxalin-2(1H)-one (P-6)

To a solution of compound **P-5** (130 mg, 0.487 mmol) in dichloromethane (5 mL) was cooled to 0°C under nitrogen atmosphere, then diethylaminosulfur trifluoride (DAST) (391 mg, 2.43 mmol) was added thereto, and the reaction mixture was stirred at room temperature for 16 hours. After the reaction was completed, the reaction mixture was concentrated under reduced pressure, and the resulting residue was purified by liquid chromatography (mobile phase: acetonitrile/0.1% formic acid in water; gradient: 10 to 100%) to obtain the title compound **P-6** (30 mg, yield: 21%). LC-MS (ESI), m/z: [M+H]⁺ = 289.0, 291.0.

### Step 6: Synthesis of 3-(1,1-difluoroethyl)-7-(hydroxymethyl)quinoxalin-2(1H)-one (intermediate P)

To a solution of compound **P-6** (28.9 mg, 0.100 mmol) in 1,4-dioxane (2 mL) were added (tributylstannyl)methanol (35.0 mg, 0.110 mmol) and (2-dicyclohexylphosphino-2',4',6'-triisopropyl-1,1'-biphenyl)(2'-amino-1,1'-biphenyl-2-yl)palladium(II) chloride (8.0 mg, 0.10 mmol) under nitrogen atmosphere. After the addition was completed, the reaction mixture was stirred at 80°C for 16 hours. After the reaction was completed, the reaction mixture was concentrated under reduced pressure, and the resulting residue was purified by silica gel column chromatography (DCM/MeOH = 10/1) to obtain **intermediate P** (20 mg, yield: 83%). LC-MS (ESI), m/z: [M+H]⁺ = 241.1.

### Intermediate Q: 3-(chloromethyl)-4-methyl-5,7,8,9-tetrahydro-6H-cyclopenta[c][1,5]naphthyridin-6-one

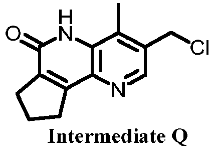

### Synthetic route:

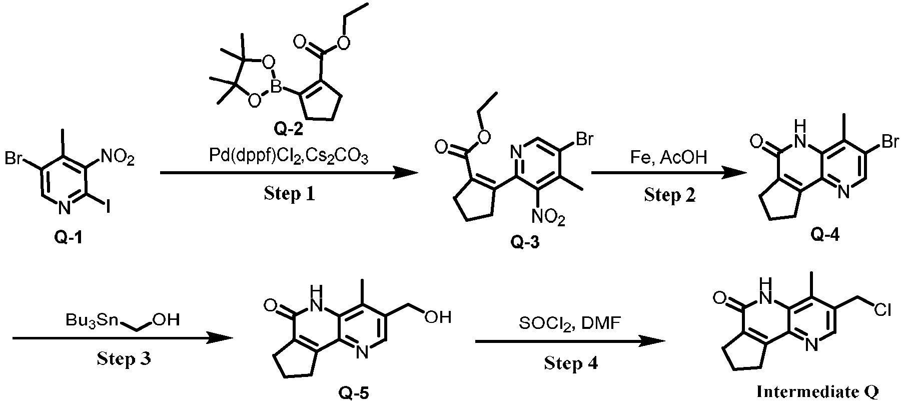

### Step 1: Synthesis of ethyl 2-(5-bromo-4-methyl-3-nitropyridin-2-yl)cyclopent-1-ene-1-carboxylate (Q-3)

To a solution of compound **Q-1** (200 mg, 0.583 mmol) in toluene (2 mL) were added compound **Q-2** (155 mg, 0.583 mmol), [1,1'-bis(diphenylphosphino)ferrocene]dichloropalladium(II) (42.7 mg, 0.0583 mmol), and cesium carbonate (380 mg, 1.17 mmol) under nitrogen atmosphere, and the reaction mixture was stirred at 1 10°C for 1.5 hours under microwave irradiation. After the reaction was completed, the reaction mixture was concentrated under reduced pressure, and the resulting residue was purified by silica gel chromatography (PE/EA= 5/1) to obtain the title compound **Q-3** (60 mg, yield: 29%). LC-MS (ESI), m/z: [M+H]⁺ = 355.0, 357.0.

### Step 2: Synthesis of 3-bromo-4-methyl-5,7,8,9-tetrahydro-6H-cyclopenta[c][1,5]naphthyridin-6-one (Q-4)

To a solution of compound **Q-3** (300 mg, 0.844 mmol) in glacial acetic acid (5 mL) was added reduced iron powder (378 mg, 6.76 mmol) under nitrogen atmosphere. After the addition was completed, the reaction mixture was stirred at 40°C for 16 hours. After the reaction was completed, the reaction mixture was filtered to remove the solid, and the filtrate was concentrated under reduced pressure. The resulting residue was purified by silica gel column chromatography (DCM/MeOH = 20/1) to obtain the title compound **Q-4** (80 mg, yield: 34%). LC-MS (ESI), m/z: [M+H]⁺ = 279.0, 281.1.

### Step 3: Synthesis of 3-(hydroxymethyl)-4-methyl-5,7,8,9-tetrahydro-6H-cyclopenta[c][1,5]naphthyridin-6-one (Q-5)

To a solution of compound **Q-4** (80.0 mg, 0.286 mmol) in 1,4-dioxane (3 mL) were added (tributylstannyl)methanol (184 mg, 0.573 mmol) and (2-dicyclohexylphosphino-2',4',6'-triisopropyl-1,1'-biphenyl)(2'-amino-1,1'-biphenyl-2-yl)palladium(II) chloride (22.6 mg, 0.0290 mmol) under nitrogen atmosphere. After the addition was completed, the reaction mixture was stirred at 80°C for 16 hours. After the reaction was completed, the reaction mixture was concentrated under reduced pressure, and the resulting residue was purified by silica gel chromatography (DCM/MeOH = 10/1) to obtain the title compound **Q-5** (50 mg, yield: 76%). LC-MS (ESI), m/z: [M+H]⁺ = 231.1.

### Step 4: Synthesis of 3-(chloromethyl)-4-methyl-5,7,8,9-tetrahydro-6H-cyclopenta[c][1,5]naphthyridin-6-one (intermediate Q)

A solution of compound **Q-5** (50.0 mg, 0.217 mmol) in thionyl chloride (2 mL) was cooled to 0°C, then *N*,*N-*dimethylformamide (1.6 mg, 0.022 mmol) was added dropwise thereto, and the reaction mixture was stirred at room temperature for 1 hour. After the reaction was completed, the reaction mixture was concentrated under reduced pressure, and the resulting residue was the crude product of the title compound **intermediate Q,** which was directly used for the next reaction step, LC-MS (ESI), m/z: [M+H]⁺ = 249.1.

### Intermediate R: 3-chloromethyl-4-methyl-9,10-dihydropyrano[2,3-c][1,5]naphthyridin-6(8H)-one

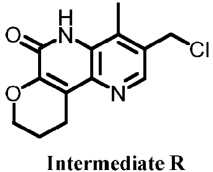

### Synthetic route:

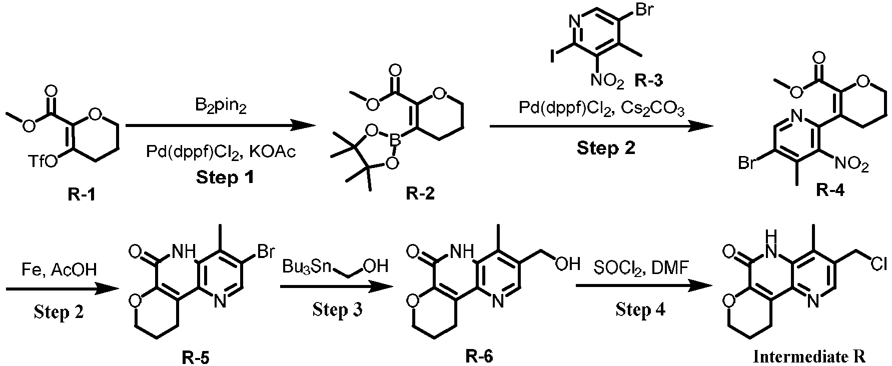

### Step 1: Synthesis of methyl 5-(4,4,5,5-tetramethyl-1,3,2-dioxaborolan-2-yl)-3,4-dihydro-2H-pyran-6-carboxylate (R-2)

To a solution of methyl 5-(((trifluoromethyl)sulfonyl)oxy)-3,4-dihydro-2*H*-pyran-6-carboxylate (2.00 g, 6.90 mmol) in 1,4-dioxane (40 mL) were added bis(pinacolato)diboron (2.10 g, 8.28 mmol), [1,1'-bis(diphenylphosphino)ferrocene]dichloropalladium(II) (500 mg, 0.690 mmol), and potassium acetate (1.40 g, 13.8 mmol) under nitrogen atmosphere, and the reaction mixture was stirred at 80°C for 16 hours. After the reaction was completed, the reaction mixture was cooled to room temperature, diluted with water (50 mL), and the resulting mixture was extracted with ethyl acetate (50 mL × 2). The combined organic phases were washed with saturated brine, dried over anhydrous sodium sulfate, filtered to remove the solid, and concentrated under reduced pressure. The resulting residue was purified by silica gel column chromatography (PE/EA= 5/1) to obtain the title compound **R-2** (1.00 g, yield: 52%). LC-MS (ESI), m/z: [M+H]⁺ = 269.2.

### Step 2: Synthesis of methyl 5-(5-bromo-4-methyl-3-nitropyridin-2-yl)-3,4-dihydropyran-6-carboxylate (R-4)

To a mixture of compound **R-2** (234 mg, 0.875 mmol) in 1,4-dioxane (10 mL) and water (1 mL) were added 5-bromo-2-iodo-4-methyl-3-nitropyridine (300 mg, 0.875 mmol), [1,1'-bis(diphenylphosphino)ferrocene]dichloropalladium(II) (60 mg, 0.088 mmol), and cesium carbonate (570 mg, 1.75 mmol) under nitrogen atmosphere, and the reaction mixture was stirred at 110°C for 4 hours. After the reaction was completed, the reaction system was added with water (10 mL), and the resulting mixture was extracted with ethyl acetate (10 mL × 2). The combined organic phases were washed with saturated brine, dried over anhydrous sodium sulfate, filtered to remove the solid, and concentrated under reduced pressure. The resulting residue was purified by silica gel column chromatography (PE/EA = 5/1) to obtain the title compound **R-4** (90 mg, yield: 28%). LC-MS (ESI), m/z: [M+H]⁺ = 357.0, 359.0.

### Step 3: Synthesis of 3-bromo-4-methyl-9,10-dihydro-5H-pyrano[2,3-c][1,5]naphthyridin-6(8H)-one (R-5)

To a solution of compound **R-4** (90.0 mg, 0.252 mmol) in glacial acetic acid (4 mL) was added reduced iron powder (141 mg, 2.25 mmol) under nitrogen atmosphere. After the addition was completed, the reaction mixture was stirred at 40°C for 16 hours. After the reaction was completed, the reaction mixture was filtered to remove the solid, and the filtrate was concentrated under reduced pressure. The resulting residue was purified by silica gel column chromatography (DCM/MeOH = 20/1) to obtain the title compound **R-5** (40 mg, yield: 54%). LC-MS (ESI), m/z: [M+H]⁺ = 295.0, 297.0.

### Step 4: Synthesis of 3-hydroxymethyl-4-methyl-9,10-dihydropyrano[2,3-c][1,5]naphthyridin-6(8H)-one (R-6)

To a solution of compound **R-5** (40.0 mg, 0.136 mmol) in 1,4-dioxane (3 mL) were added (tributylstannyl)methanol (56.5 mg, 0.176 mmol) and (2-dicyclohexylphosphino-2',4',6'-triisopropyl-1,1'-biphenyl)(2'-amino-1,1'-biphenyl-2-yl)palladium(II) chloride (12.0 mg, 0.0140 mmol) under nitrogen atmosphere. After the addition was completed, the reaction mixture was stirred at 90°C for 16 hours. After the reaction was completed, the reaction mixture was concentrated under reduced pressure, and the resulting residue was purified by silica gel column chromatography (DCM/MeOH = 10/1) to obtain the title compound **R-6** (20 mg, yield: 59%). LC-MS (ESI), m/z: [M+H]⁺ = 247.1.

### Step 5: Synthesis of 3-chloromethyl-4-methyl-9,10-dihydropyrano[2,3-c][1,5]naphthyridin-6(8H)-one (intermediate R):

A solution of compound **R-6** (20.0 mg, 0.0813 mmol) in dichloromethane (2 mL) was cooled to 0°C, and thionyl chloride (0.5 mL) and *N,N*-dimethylformamide (0.59 mg, 0.0081 mmol) were added dropwise to the reaction system. The reaction mixture was stirred at room temperature for 16 hours. After the reaction was completed, the reaction mixture was concentrated under reduced pressure, and the resulting residue was the crude product of **intermediate R** (20 mg), which was directly used for the next reaction step. LC-MS (ESI), m/z: [M+H]⁺ = 265.1, 267.1.

### Intermediate S: 3-ethyl-7-(hydroxymethyl)-1H-quinolin-2-one

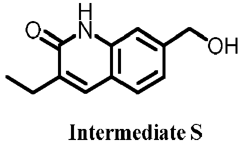

### Synthetic route:

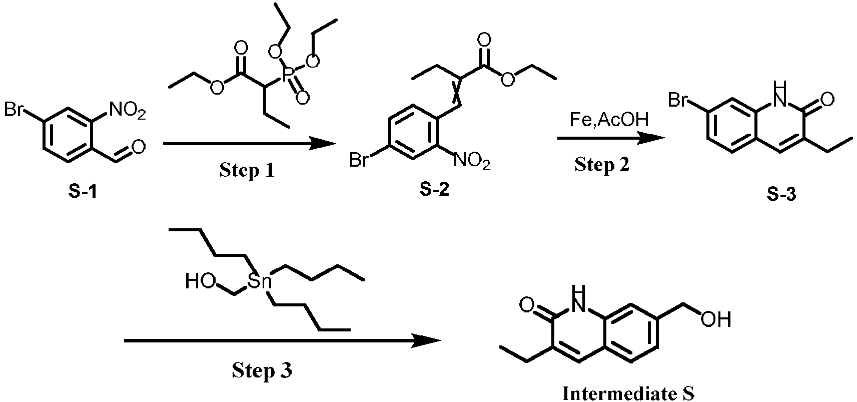

### Step 1: Synthesis of ethyl 2-((4-bromo-2-nitrophenyl)methylene)butanoate (S-2)

A suspension of sodium hydride (0.250 g, 0.0103 mmol) in tetrahydrofuran (20 mL) was cooled to 0°C under nitrogen atmosphere, then ethyl 2-(diethoxyphosphoryl)butanoate (2.60 g, 10.3 mmol) was added thereto, and the reaction mixture was stirred for 0.5 hours. The reaction system was cooled to -78°C, then compound 4-bromo-2-nitrobenzaldehyde (1.00 g, 4.30 mmol) was added thereto, and the reaction mixture was stirred at -78°C for 2 hours. After the reaction was completed, the reaction system was added with saturated ammonium chloride aqueous solution (200 mL) and stirred at room temperature for 3 minutes. After the reaction was completed, the resulting mixture was extracted with ethyl acetate (30 mL × 3). The combined organic phases were washed with saturated brine, dried over anhydrous sodium sulfate, filtered to remove the solid, and concentrated under reduced pressure. The resulting residue was purified by silica gel column chromatography (PE/EA = 20/1) to obtain the title compound **S-2** (1.35 g, yield: 86%). LC-MS (ESI), m/z: [M+H]⁺ = 327.9, 330.0.

### Step 2: Synthesis of 7-bromo-3-ethyl-1H-quinolin-2-one (S-3)

To a solution of compound **S-2** (1.35 g, 4.10 mmol) in glacial acetic acid (40 mL) was added reduced iron powder (1.83 g, 32.8 mmol) under nitrogen atmosphere. After the addition was completed, the reaction mixture was stirred at room temperature for 16 hours. After the reaction was completed, the reaction mixture was concentrated under reduced pressure. The resulting residue was diluted with dichloromethane (100 mL) and methanol (10 mL), filtered to remove the solid, and concentrated again under reduced pressure. The resulting residue was purified by silica gel column chromatography (DCM/MeOH = 20/1) to obtain the title compound **S-3** (0.287 g, yield: 24%). LC-MS (ESI), m/z: [M+H]⁺ = 252.0, 254.0.

### Step 3: Synthesis of 3-ethyl-7-(hydroxymethyl)-1H-quinolin-2-one (intermediate S)

To a solution of compound **S-3** (100 mg, 0.397 mmol) in 1,4-dioxane (10 mL) were added (tributylstannyl)methanol (153 mg, 0.476 mmol) and (2-dicyclohexylphosphino-2',4',6'-triisopropyl-1,1'-biphenyl)(2'-amino-1,1'-biphenyl-2-yl)palladium(II) chloride (32.0 mg, 0.0397 mmol) under nitrogen atmosphere. After the addition was completed, the reaction mixture was stirred at 80°C for 16 hours. After the reaction was completed, the reaction mixture was concentrated under reduced pressure, and the resulting residue was purified by silica gel column chromatography (DCM/MeOH = 10/1) to obtain **intermediate S** (54.0 mg, yield: 60%). LC-MS (ESI), m/z: [M+H]⁺ = 204.1.

### Intermediate T: 8-(hydroxymethyl)-2,3-dihydro-1H-pyrano[2,3-c]quinolin-5(6H)-one

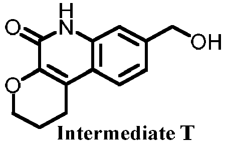

### Synthetic route:

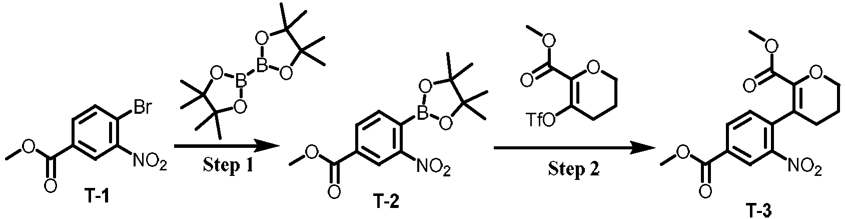

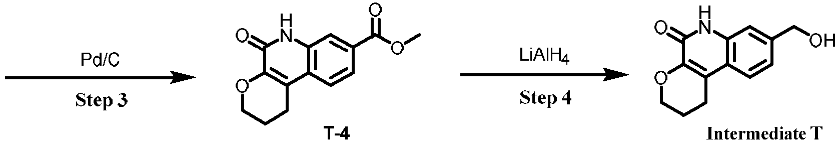

### Step 1: Synthesis of methyl 3-nitro-4-(4,4,5,5-tetramethyl-1,3,2-dioxaborolan-2-yl)benzoate (T-2)

To a solution of compound **T-1** (5.00 g, 19.2 mmol) in 1,4-dioxane (50 mL) were added bis(pinacolato)diboron (7.31 g, 28.8 mmol), [1,1'-bis(diphenylphosphino)ferrocene]dichloropalladium(II) dichloromethane (1.57 g, 1.92 mmol), and potassium acetate (5.65 g, 57.6 mmol) under nitrogen atmosphere, and the reaction mixture was stirred at 80°C for 16 hours. The reaction system was concentrated under reduced pressure. The resulting residue was dissolved in ethyl acetate (100 mL), washed with saturated brine, dried over anhydrous sodium sulfate, filtered to remove the solid, and concentrated under reduced pressure. The resulting residue was purified by silica gel column chromatography (PE/EA = 1/1) to obtain the title compound **T-2** (3.6 g, yield: 61%).

### Step 2: Synthesis of methyl 5-(4-methoxycarbonyl)-2-nitrophenyl-3,4-dihydropyran-6-carboxylate (T-3)

To a solution of compound **T-2** (300 mg, 0.977 mmol) in 1,4-dioxane (10 mL) were added methyl 5-(((trifluoromethyl)sulfonyl)oxy)-3,4-dihydro-2*H*-pyran-6-carboxylate (283 mg, 0.977 mmol), tetrakis(triphenylphosphine)palladium (113 mg, 0.0980 mmol), and sodium carbonate (207 mg, 0.954 mmol) under nitrogen atmosphere, and the reaction mixture was stirred at 110°C for 3 hours. After the reaction was completed, the reaction mixture was concentrated under reduced pressure. The resulting residue was dissolved in ethyl acetate (100 mL), washed with saturated brine, dried over anhydrous sodium sulfate, filtered to remove the solid, and concentrated under reduced pressure. The resulting residue was purified by silica gel column chromatography (PE/EA = 10/1) to obtain the title compound **T-3** (200 mg, yield: 64%). LC-MS (ESI), m/z: [M+Na]⁺ = 344.1.

### Step 3: Synthesis of methyl 5-oxo-2,3,5,6-tetrahydropyrano[2,3-c]quinoline-8-carboxylate (T-4)

To a solution of compound **T-3** (200 mg, 0.623 mmol) in methanol (20 mL) was added palladium on carbon (100 mg) under hydrogen atmosphere, and the reaction mixture was stirred at room temperature for 16 hours. After the reaction was completed, the reaction mixture was filtered to remove the solid, and the filtrate was concentrated under reduced pressure. The resulting residue was the crude product of the title compound **T-4** (130 mg), which was directly used for the next reaction step. LC-MS (ESI), m/z: [M+H]⁺ = 260.1.

### Step 4: Synthesis of 8-(hydroxymethyl)-2,3-dihydro-1H-pyrano[2,3-c]quinolin-5(6H)-one (intermediate T)

A solution of compound **T-4** (130 mg, 0.502 mmol) in tetrahydrofuran (3 mL) was cooled to 0°C under nitrogen atmosphere, then a solution of lithium aluminum hydride in tetrahydrofuran (2.5 mol/L, 1.0 mL, 2.5 mmol) was added thereto, and the reaction mixture was stirred for 2 hours. After the reaction was completed, the reaction system was added with ethyl acetate (10 mL) and water (100 mg). The resulting mixture was concentrated under reduced pressure, and the resulting residue was purified by silica gel column chromatography (DCM/MeOH = 10/1) to obtain **intermediate T** (60 mg, yield: 52%). LC-MS (ESI), m/z: [M+H]⁺ = 232.1.

### Intermediate U: 7-(bromomethyl)-3-ethyl-8-fluoroquinolin-2(1H)-one

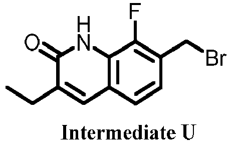

### Synthesis steps:

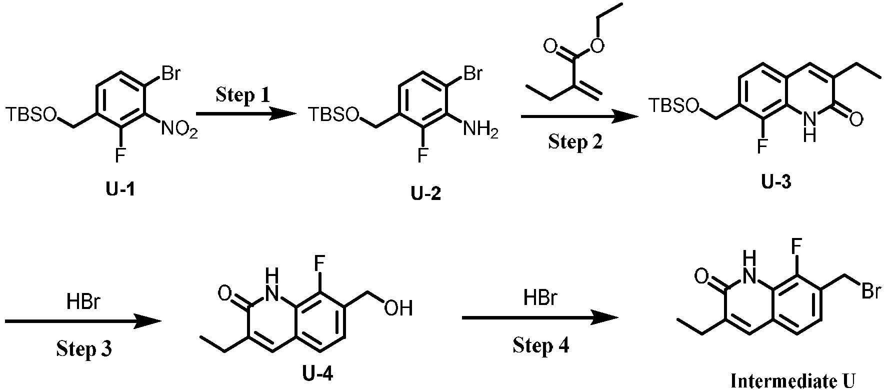

### Step 1: Synthesis of 6-bromo-3-(((tert-butyldimethylsilyl)oxy)methyl)-2-fluoroaniline (U-2)

To a solution of compound **U-1** (500 mg, 1.37 mmol) in ethanol (10 mL) and water (2 mL) were added iron powder (383 mg, 6.86 mmol) and ammonium chloride (734 mg, 13.7 mmol), and the reaction mixture was stirred at 80°C for 16 hours. After the reaction was completed, the reaction mixture was filtered to remove the solid, and the filtrate was concentrated under reduced pressure. The resulting residue was purified by silica gel column chromatography (PE/EA = 5/1) to obtain the title compound **U-2** (300 mg, yield: 66%). LC-MS (ESI), m/z: [M+H]⁺ = 334.1, 336.1.

### Step 2: Synthesis of 7-(((tert-butyldimethylsilyl)oxy)methyl)-3-ethyl-8-fluoroquinolin-2(1H)-one (U-3)

To a solution of compound **U-2** (352 mg, 2.75 mmol) in *N*,*N-*dimethylformamide (5 mL) were added ethyl 2-ethylacrylate (500 mg, 1.37 mmol), triethylamine (417 mg, 4.12 mmol), tris(*o*-tolyl)phosphine (83.6 mg, 0.275 mmol), and palladium acetate (22.9 mg, 0.137 mmol) under nitrogen atmosphere, and the reaction mixture was stirred at 105°C for 12 hours. After the reaction was completed, the reaction mixture was filtered to remove the solid. The filtrate was diluted with ethyl acetate (10 mL), washed with saturated brine, dried over anhydrous sodium sulfate, filtered to remove the solid, and concentrated under reduced pressure. The resulting residue was purified by preparative thin-layer chromatography on silica gel plate (PE/EA = 10/1, R_{f} = 0.1) to obtain the title compound **U-3** (23 mg, yield: 2%). LC-MS (ESI), m/z: [M+H]⁺ = 336.0.

### Step 3: Synthesis of 3-ethyl-8-fluoro-7-(hydroxymethyl)quinolin-2(1H)-one (U-4)

To a solution of compound **U-3** (30 mg, 0.089 mmol) in dichloromethane (5 mL) was added hydrobromic acid aqueous solution (40%, 2.0 mL), and the reaction mixture was stirred at room temperature for 2 hours. After the reaction was completed, the reaction mixture was diluted with ethyl acetate (5 mL), washed with saturated brine, dried over anhydrous sodium sulfate, filtered to remove the solid, and concentrated under reduced pressure. The resulting residue was the crude product of the title compound **U-4** (15 mg), which was directly used for the next reaction step. LC-MS (ESI), m/z: [M+H]⁺ = 222.2.

### Step 4: Synthesis of 7-(bromomethyl)-3-ethyl-8-fluoroquinolin-2(1H)-one (intermediate U)

An aqueous solution of compound **U-4** (15 mg, 0.068 mmol) in hydrobromic acid (40%, 5.0 mL) was stirred at 80°C for 2 hours. After the reaction was completed, the reaction mixture was diluted with ethyl acetate (10 mL), washed with saturated brine, dried over anhydrous sodium sulfate, filtered to remove the solid, and concentrated under reduced pressure. The resulting residue was **intermediate U** (15 mg, yield: 78%). LC-MS (ESI), m/z: [M+H]⁺ = 284.0, 286.0.

### Intermediate V: 7-(bromomethyl)-3-ethyl-8-methylquinolin-2(1H)-one

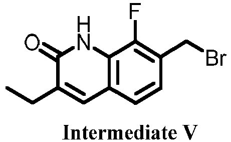

### Synthesis steps:

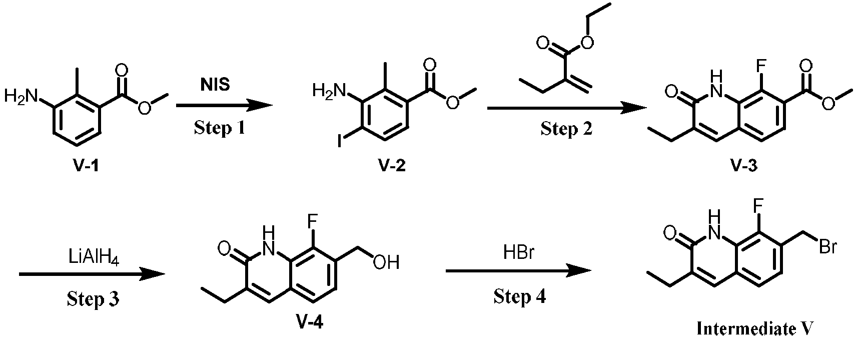

### Step 1: 1-(2-Isopropylphenyl)-1H-pyrazol-3-ol (V-2)

To a solution of compound **V-1** (10.0 g, 60.5 mmol) in acetic acid (100 mL) was added N-iodosuccinimide (14.0 g, 62.3 mmol) in batches at 25°C. The reaction mixture was stirred at room temperature for 0.5 hours. The reaction mixture was diluted with water (200 mL) and extracted with ethyl acetate (200 mL × 3). The combined organic phases were washed with saturated brine, dried over anhydrous sodium sulfate, filtered, and concentrated. The resulting residue was purified by silica gel chromatography (PE: EA = 8:1) to obtain the title compound **V-2** (0.600 g, yield: 3%). LC-MS (ESI), m/z: [M+H]⁺ = 292.0.

### Step 2: Methyl 3-ethyl-8-methyl-2-oxo-1,2-dihydroquinoline-7-carboxylate (V-3)

To a solution of compound **V-2** (500 mg, 1.72 mmol) in *N,N-*dimethylformamide (5.00 mL) were added ethyl 2-ethylacrylate (660 mg, 5.15 mmol), palladium acetate (38.6 mg, 0.170 mmol), tris(o-tolyl)phosphine (52.3 mg, 0.170 mmol), and triethylamine (348 mg, 3.44 mmol) at 25°C under nitrogen atmosphere. After the addition was completed, the reaction system was heated to 100°C and stirred for 10 hours under nitrogen atmosphere. The reaction system was cooled to 25°C, and the reaction mixture was diluted with water (100 mL) and extracted with ethyl acetate (100 mL × 3). The combined organic phases were washed with saturated brine, dried over anhydrous sodium sulfate, filtered, and concentrated. The resulting residue was purified by silica gel chromatography (PE: EA = 3:1) to obtain the title compound **V-3** (0.300 g, yield: 64%). LC-MS (ESI), m/z: [M+H]⁺ = 246.1.

### Step 3: 3-Ethyl-7-(hydroxymethyl)-8-methylquinolin-2(1H)-one (V-4)

A solution of compound **V-3** (300 mg, 1.22 mol) in tetrahydrofuran (5.00 mL) was added dropwise a solution of lithium aluminum hydride in tetrahydrofuran (1 M, 2.40 mL) at 0°C under nitrogen atmosphere. After the addition was completed, the reaction mixture was stirred at 0°C for 2 hours under nitrogen atmosphere. The reaction system was diluted with ice water (50.0 mL), and the mixture was extracted with ethyl acetate (30.0 mL × 3). The combined organic phases were washed with saturated brine, dried over anhydrous sodium sulfate, filtered, and concentrated. The resulting residue was purified by silica gel chromatography (PE: EA = 1:1) to obtain the title compound **V-4** (160 mg, yield: 54%). LC-MS (ESI), m/z: [M+H]⁺ = 218.0.

### Step 4: 7-(Bromomethyl)-3-ethyl-8-methylquinolin-2(1H)-one (intermediate V)

Compound **V-4** (160 mg, 0.740 mmol) was dissolved in a solution of hydrobromic acid (5.00 mL) at room temperature, and the reaction mixture was stirred at 80°C for 2 hours. The reaction mixture was diluted with water (30.0 mL) and extracted with ethyl acetate (30.0 mL × 3). The combined organic phases were washed with saturated brine, dried over anhydrous sodium sulfate, filtered, and concentrated. The resulting residue was purified by silica gel chromatography (PE: EA = 4:1) to obtain **intermediate V** (120 mg, yield: 52%). LC-MS (ESI), m/z: [M+H]⁺ = 280.1/282.1.

### Intermediate W: 5-(2,5-diazabicyclo[4.1.0]heptan-2-yl)-N-methylpyridine-2-carboxamide

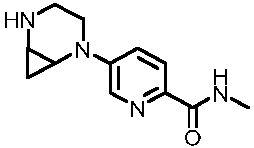

### Synthetic route:

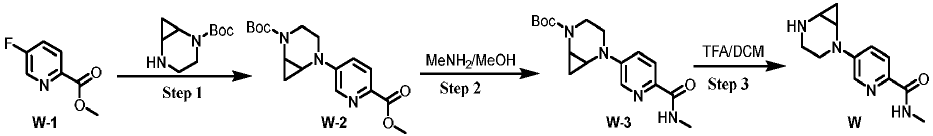

### Step 1: Synthesis of tert-butyl 5-(6-(methoxycarbonyl)pyridin-3-yl)-2,5-diazabicyclo[4.1.0]heptane-2-carboxylate:

To a solution of methyl 5-fluoropyridine-2-carboxylate (300 mg, 1.93 mmol) and *tert-*butyl 2,5-diazabicyclo[4.1.0]heptane-2-carbonate (767 mg, 3.87 mmol) in dimethyl sulfoxide (10 mL) was added potassium carbonate (535 mg, 3.87 mmol) at room temperature. The reaction mixture was stirred at 120°C for 2 hours. The reaction mixture was cooled to room temperature, and added with ethyl acetate (100 mL) and water (10 mL) for phase separation and extraction. The resulting organic phase was dried over anhydrous sodium sulfate, filtered, and concentrated under reduced pressure. The resulting residue was purified by silica gel chromatography (EA: PE = 0:1 to 3:2) to obtain the title compound **W-2** (210 mg, yield: 29%). LC-MS (ESI), m/z: [M+H]⁺ = 334.1.

### Step 2: Synthesis of tert-butyl 5-(6-(methylcarbamoyl)pyridin-3-yl)-2,5-diazabicyclo[4.1.0]heptane-2-carboxylate:

*tert*-Butyl 5-(6-(methoxycarbonyl)pyridin-3-yl)-2,5-diazabicyclo[4.1.0]heptane-2-carboxylate (210 mg, 0.63 mmol) was dissolved in a solution of methylamine in methanol (15 mL) at room temperature. The reaction mixture was reacted at 60°C for 16 hours in a sealed tube. The reaction mixture was cooled to room temperature and concentrated under reduced pressure. The resulting residue was purified by silica gel chromatography (PE: EA = 1:1) to obtain the title compound **W-3** (140 mg, yield: 60%). LC-MS (ESI), m/z: [M+H]⁺ = 333.0.

### Step 3: Synthesis of 5-(2,5-diazabicyclo[4.1.0]heptan-2-yl)-N-methylpyridine-2-carboxamide:

*tert*-Butyl 5-(6-(methylcarbamoyl)pyridin-3-yl)-2,5-diazabicyclo[4.1.0]heptane-2-carboxylate (140 mg, 0.42 mmol) was dissolved in a mixture (15 mL) of dichloromethane/trifluoroacetic acid (10:1) at room temperature. The reaction mixture was stirred at 25°C for 12 hours. The reaction mixture was concentrated. The resulting crude product was dissolved in dichloromethane (100 mL), added with saturated sodium bicarbonate (50 mL), and stirred for 10 minutes. The organic phase was separated, washed with saturated sodium chloride (50 mL), dried over anhydrous sodium sulfate, filtered, and concentrated. The resulting residue was purified by silica gel chromatography (EA: PE = 0: 1 to 4:1) to obtain the title compound **intermediate W** (80 mg, yield: 74%).

LC-MS (ESI), m/z: [M+H]⁺ = 233.1.

### Example 1: 5-(5-((7-ethyl-6-carbonyl-5,6-dihydro-1,5-naphthyridin-3-yl)methyl)-2,5-diazabicyclo[4.1.0]heptan-2-yl)-N methylpicolinamide

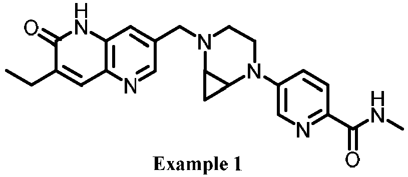

### Synthetic route:

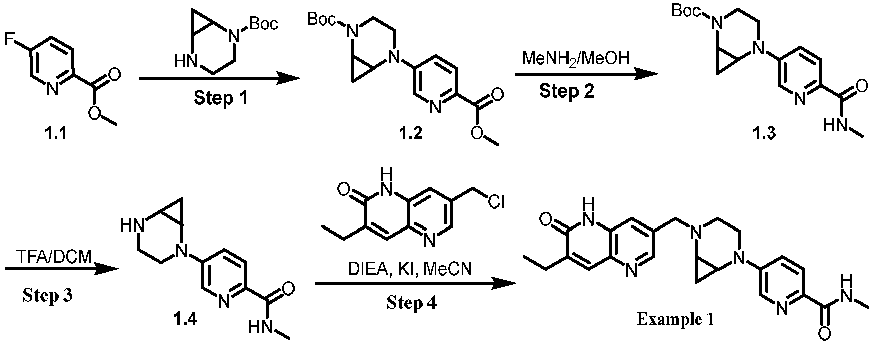

### Step 1: Synthesis of tert-butyl 5-(6-(methoxycarbonyl)pyridin-3-yl)-2,5-diazabicyclo[4.1.0]heptane-2-carb oxylate (1.2):

To a solution of methyl 5-fluoropyridine-2-carboxylate (300 mg, 1.93 mmol) and *tert-*butyl 2,5-diazabicyclo[4.1.0]heptane-2-carbonate (767 mg, 3.87 mmol) in dimethyl sulfoxide (10 mL) was added potassium carbonate (535 mg, 3.87 mmol) at room temperature. The reaction mixture was stirred at 120°C for 2 hours. The reaction mixture was cooled to room temperature, and added with ethyl acetate (100 mL) and water (10 mL) for phase separation and extraction. The resulting organic phase was dried over anhydrous sodium sulfate, filtered, and concentrated under reduced pressure. The resulting residue was purified by silica gel chromatography (EA: PE = 0:1 to 3:2) to obtain the title compound **1.2** (210 mg, yield: 29%).

LC-MS (ESI), m/z: [M+H]⁺ = 334.1.

### Step 2: Synthesis of tert-butyl 5-(6-(methylcarbamoyl)pyridin-3-yl)-2,5-diazabicyclo[4.1.0]heptane-2-carboxylate (1.3):

*tert*-Butyl 5-(6-(methoxycarbonyl)pyridin-3-yl)-2,5-diazabicyclo[4.1.0]heptane-2-carboxylate (210 mg, 0.63 mmol) was dissolved in a solution of methylamine in methanol (15 mL) at room temperature. The reaction mixture was reacted at 60°C for 16 hours in a sealed tube. The reaction mixture was cooled to room temperature and concentrated under reduced pressure. The resulting residue was purified by silica gel chromatography (PE: EA = 1:1) to obtain the title compound **1.3** (140 mg, yield: 60%).

LC-MS (ESI), m/z: [M+H]⁺ = 333.0.

### Step 3: Synthesis of 5-(2,5-diazabicyclo[4.1.0]heptan-2-yl)-N-methylpyridine-2-carboxamide (1.4):

*tert*-Butyl 5-(6-(methylcarbamoyl)pyridin-3-yl)-2,5-diazabicyclo[4.1.0]heptane-2-carboxylate (140 mg, 0.42 mmol) was dissolved in a mixture (15 mL) of dichloromethane/trifluoroacetic acid (10:1) at room temperature. The reaction mixture was stirred at 25°C for 12 hours. The reaction mixture was concentrated. The resulting crude product was dissolved in dichloromethane (100 mL), added with saturated sodium bicarbonate (50 mL), and stirred for 10 minutes. The organic phase was separated, washed with saturated sodium chloride (50 mL), dried over anhydrous sodium sulfate, filtered, and concentrated. The resulting residue was purified by silica gel chromatography (EA: PE = 0:1 to 4:1) to obtain the title compound **1.4** (80 mg, yield: 74%).

LC-MS (ESI), m/z: [M+H]⁺ = 233.1.

### Step 4: Synthesis of 5-(5-((7-ethyl-6-carbonyl-5,6-dihydro-1,5-naphthyridin-3-yl)methyl)-2,5-diazabicyclo[4.1,0]heptan-2-yl)-A-methylpyridine-2-carboxamide (Example 1):

To a solution of 5-(2,5-diazabicyclo[4.1.0]heptan-2-yl)-A-methylpyridine-2-carboxamide (80 mg, 0.34 mmol) and 7-(chloromethyl)-3-ethyl-1,5-naphthyridin-2(1*H*)-one (77 mg, 0.34 mmol) in acetonitrile (10 mL) were added diisopropylethylamine (134 mg, 1.04 mmol) and potassium iodide (11 mg, 0.07 mol) at room temperature, and the reaction system was replaced with nitrogen three times. The reaction mixture was stirred at 80°C for 2 hours under nitrogen atmosphere. The reaction mixture was cooled to room temperature, diluted with ethyl acetate (30 mL), washed with saturated brine, dried over anhydrous sodium sulfate, filtered, and concentrated. The resulting residue was purified by preparative high performance liquid chromatography (chromatographic column model: Gemini-C18 150 × 21.2 mm, 5 µm, mobile phase: acetonitrile/water (0.1% formic acid), gradient: 12 to 18%, flow rate: 20 mL/min) to obtain the title compound **Example 1** (80 mg, yield: 55%).

LC-MS (ESI), m/z: [M+H]⁺ = 419.1.

¹H NMR (400 MHz, DMSO-*d₆*) δ 11.87 (s, 1H), 8.45 (s, 1H), 8.37 (d, *J =* 4.8 Hz, 1H), 8.19 (d, *J =* 2.8 Hz, 1H), 7.86 (d, *J =* 8.8 Hz, 1H), 7.76 (s, 1H), 7.69 (s, 1H), 7.28 (dd, *J =* 8.8 Hz, 2.4, 1H), 3.94 (br, 2H), 3.66 - 3.33 (m, 3H), 3.27 - 3.15 (m, 1H), 2.77 (d, *J =* 4.8 Hz, 3H), 2.90 - 2.70 (m, 1H), 2.66 - 2.52 (m, 3H), 1.18 (t, *J =* 7.2 Hz, 3H), 0.88 - 0.75 (m, 1H), 0.50 - 0.37 (m, 1H).

### Example 1-1 and Example 1-2: 5-((1S,6R)-5-((7-ethyl-6-carbonyl-5,6-dihydro-1,5-naphthyridin-3-yl)methyl)-2,5-diazabicyclo[4.1.0]heptan-2-yl)-N-methylpicolinamide and 5-((1R,6S)-5-((7-ethyl-6-carbonyl-5,6-dihydro-1,5-naphthyridin-3-yl)methyl)-2,5-diazabicyclo[4.1.0]heptan-2-yl)-N-methylpicolinamide

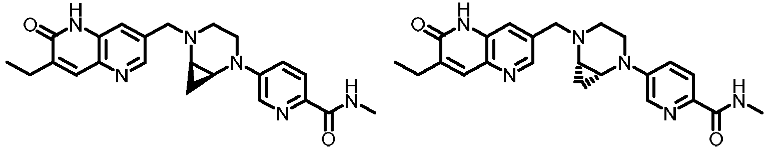

### Synthetic route:

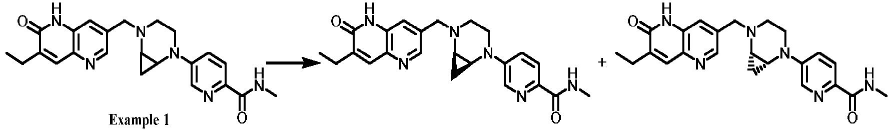

**Example 1** (76 mg, 0.18 mmol) was separated by preparative supercritical fluid chromatography (SFC) (chromatographic column model: CHIRALPAK-OD 250 mm × 20 mm, 5 µm, mobile phase: 50% EtOH (0.2% ammonia water), flow rate: 14 mL/min) to obtain the following:

### Example 1-1 (t_{R} = 10.53 min, 4.2 mg, yield: 5.5 %)

LC-MS (ESI), m/z: [M+H]⁺ = 419.1.

¹H NMR(400 MHz, DMSO-*d₆*) δ 11.87 (s, 1H), 8.45 (s, 1H), 8.37 (d, *J =* 4.8 Hz, 1H), 8.19 (d, *J =* 2.8 Hz, 1H), 7.86 (d, *J =* 8.8 Hz, 1H), 7.76 (s, 1H), 7.69 (s, 1H), 7.28 (dd, *J =* 8.8 Hz, 2.4, 1H), 3.94 (br, 2H), 3.66 - 3.33 (m, 3H), 3.27 - 3.15 (m, 1H), 2.77 (d, *J =* 4.8 Hz, 3H), 2.90 - 2.70 (m, 1H), 2.66 - 2.52 (m, 3H), 1.18 (t, *J =* 7.2 Hz, 3H), 0.88 - 0.75 (m, 1H), 0.50 - 0.37 (m, 1H).

### Example 1-2 (t_{R} = 12.21 min, 3.5 mg, yield: 4.6 %)

LC-MS (ESI), m/z: [M+H]⁺ = 419.2.

¹H NMR(400 MHz, DMSO-*d₆*) δ 11.87 (s, 1H), 8.45 (s, 1H), 8.37 (d, *J =* 4.8 Hz, 1H), 8.19 (d, *J =* 2.4 Hz, 1H), 7.85 (d, *J =* 8.8 Hz, 1H), 7.76 (s, 1H), 7.69 (s, 1H), 7.28 (dd, *J =* 8.8, 2.4 Hz, 1H), 3.93 (br, 2H), 3.66 - 3.33 (m, 3H), 3.27 - 3.15 (m, 1H), 2.78 (d, *J =* 4.8 Hz, 3H), 2.90-2.70 (m, 1H), 2.61 - 2.53 (m, 3H), 1.18 (t, *J =* 7.2 Hz, 3H), 0.90-0.68 (m, 1H), 0.50-0.33 (s, 1H).

### Example 2: 5-(5-((7-ethyl-6-carbonyl-5,6-dihydro-1,5-naphthyridin-3-yl)methyl)-2,5-diazabicyclo[4.2.0]octan-2-yl)-N-methylpyridine-2-carboxamide

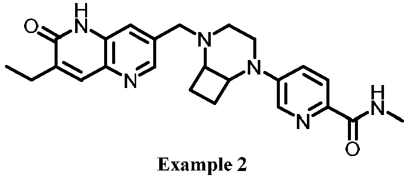

### Synthetic route:

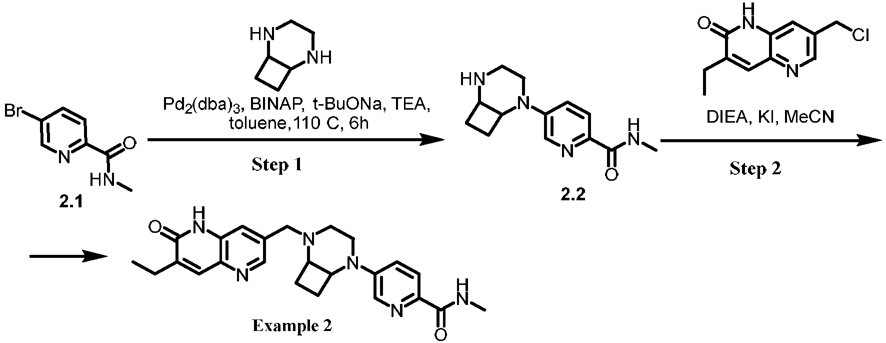

### Step 1: Synthesis of 5-(2,5-diazabicyclo[4.2.0]octan-2-yl)-N-methylpyridine-2-carboxamide (2.1):

To a solution of 2,5-diazabicyclo[4.2.0]octane (200 mg, 1.78 mmol) in toluene (3 mL) were sequentially added 5-bromo-*N*-methylpyridine-2-carboxamide (766 mg, 3.56 mmol), sodium *tert*-butoxide (685 mg, 7.13 mmol), tris(dibenzylideneacetone)dipalladium (163 mg, 0.18 mmol), 2,2'-bis(diphenylphosphino)-1,1'-binaphthyl (222 mg, 0.36 mmol), and triethylamine (1 mL) at room temperature. The reaction system was replaced with nitrogen three times, then heated to 110°C, and stirred for 6 hours. After the reaction was completed, the reaction mixture was quenched with water (20 mL), and the aqueous phase was extracted with ethyl acetate (20 mL × 3). The combined organic phases were washed with brine (20 mL) and then dried over anhydrous sodium sulfate. After filtration, the organic phase was concentrated under vacuum, and the crude product was purified by flash silica gel column chromatography (dichloromethane: methanol = 1:99 to 3:97) to obtain the title compound **2.2** (200 mg, yield: 41%).

LC-MS (ESI), m/z: [M+H]⁺ = 247.1.

### Step 2: Synthesis of 5-(5-((7-ethyl-6-carbonyl-5,6-dihydro-1,5-naphthyridin-3-yl)methyl)-2,5-diazabicyclo[4.2.0]octan-2-yl)-N-methylpyridine-2-carboxamide (Example 2):

To a solution of 7-(chloromethyl)-3-ethyl-1,5-naphthyridin-2(1*H*)-one (90 mg, 0.41 mmol) in acetonitrile (5 mL) were sequentially added *N*,*N*-diisopropylethylamine (156 mg, 1.21 mmol), potassium iodide (7 mg, 0.04 mmol), and 5-(2,5-diazabicyclo[4.2.0]octan-2-yl)-N-methylpyridine-2-carboxamide (100 mg, 0.40 mmol) at room temperature. The reaction mixture was heated to 80°C and stirred for 2 hours. After the reaction was completed, the reaction mixture was diluted with water (20.0 mL), and the aqueous phase was extracted with ethyl acetate (20.0 mL × 3). The combined organic phases were washed with saturated brine (20 mL), dried over anhydrous sodium sulfate, filtered, and the filtrate was concentrated under vacuum. The crude product was purified by preparative high performance liquid chromatography (chromatographic column model: Gemini-C18 150 × 21.2 mm, 5 µm, mobile phase: acetonitrile/water (0.1% formic acid), gradient: 2% to 25%, flow rate: 20 mL/min) 0.1%FA) to obtain the title compound **Example 2** (15.3 mg, yield: 8.7%).

LC-MS (ESI), m/z: [M+H]⁺ = 433.1.

¹H NMR(400 MHz, MeOD) δ 8.49 (d, *J* = 2.0 Hz, 1H), 8.12 (d, *J =* 2.8 Hz, 1H), 7.86 (d, *J =* 8.8 Hz, 1H), 7.82 (s, 1H), 7.81 (s, 1H), 7.19 (dd, *J =* 8.8, 2.8 Hz, 1H), 4.38 - 4.28 (m, 1H), 3.98 (d, *J =* 14.2 Hz, 1H), 3.51 - 3.36 (m, 3H), 3.36 - 3.28 (m, 1H), 2.91 (s, 3H), 2.94 - 2.84 (m, 1H), 2.70 - 2.60 (m, 2H), 2.42 - 2.14 (m, 3H), 1.99 - 1.88 (m, 2H), 1.27 (t, *J* = 7.2 Hz, 3H).

### Example 2-1 and Example 2-2:

### 5-((1S,6R)-5-((7-ethyl-6-carbonyl-5,6-dihydro-1,5-naphthyridin-3-yl)methyl)-2,5-diazabicyclo[4.2.0]octan-2-yl)-N-methylpyridine-2-carboxamide and 5-((1R,6S)-5-((7-ethyl-6-carbonyl-5,6-dihydro-1,5-naphthyridin-3-yl)methyl)-2,5-diazabicyclo[4.2.0]octan-2-yl)-N-methylpyridine-2-carboxamide

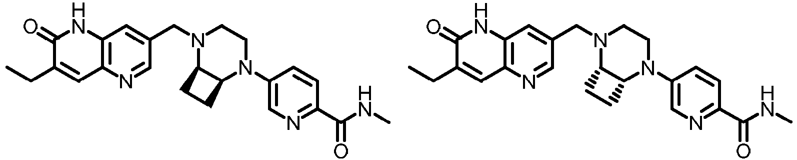

### Synthetic route:

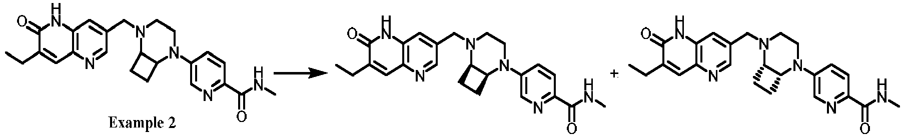

**Example 2** (15.3 mg, 0.04 mmol) was purified by preparative supercritical fluid chromatography (SFC) (column: CHIRALPAK OJ-H 250 mm × 20 mm, 5 µm, 40% EtOH (NH₄OH 0.2%) to obtain the following two compounds:

### Example 2-1 (t_{R} = 6.24 min, 5.80 mg, yield: 38%)

LC-MS (ESI), m/z: [M+H]⁺ = 433.1.

¹H NMR(400 MHz, MeOD) δ 8.49 (d, *J* = 2.0 Hz, 1H), 8.12 (d, *J* = 2.8 Hz, 1H), 7.86 (d, *J =* 8.8 Hz, 1H), 7.82 (s, 1H), 7.81 (s, 1H), 7.19 (dd, *J =* 8.8, 2.8 Hz, 1H), 4.38 - 4.28 (m, 1H), 3.98 (d, *J =* 14.2 Hz, 1H), 3.51 - 3.36 (m, 3H), 3.36 - 3.28 (m, 1H), 2.91 (s, 3H), 2.94 - 2.84 (m, 1H), 2.70 - 2.60 (m, 2H), 2.42 - 2.14 (m, 3H), 1.99 - 1.88 (m, 2H), 1.27 (t, *J* = 7.2 Hz, 3H).

### Example 2-2 (t_{R} = 12.10 min, 6.8 mg, yield: 44%)

LC-MS (ESI), m/z: [M+H]⁺ = 433.1.

¹H NMR(400 MHz, MeOD) δ 8.49 (d, *J* = 1.6 Hz, 1H), 8.12 (d, *J =* 2.8 Hz, 1H), 7.86 (d, *J* = 8.8 Hz, 1H), 7.82 (s, 1H), 7.81 (s, 1H), 7.19 (dd, *J* = 8.8, 2.8 Hz, 1H), 4.38 - 4.28 (m, 1H), 3.98 (d, *J =* 14.2 Hz, 1H), 3.51 - 3.36 (m, 3H), 3.36 - 3.28 (m, 1H), 2.91 (s, 3H), 2.94 - 2.84 (m, 1H), 2.70 - 2.60 (m, 2H), 2.42 - 2.14 (m, 3H), 1.99 - 1.88 (m, 2H), 1.27 (t, *J* = 7.2 Hz, 3H).

### Example 2": cis-5-(5-((7-ethyl-6-carbonyl-5,6-dihydro-1,5-naphthyridin-3-yl)methyl)-2,5-diazabicyclo[4.2.0]octan-2-yl)-N-methylpyridine-2-carboxamide

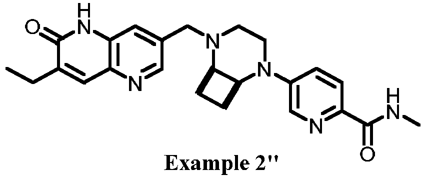

**Example 2"** was obtained with reference to the synthesis of **Example 2** by replacing 2,5-diazabicyclo[4.2.0]octane with *trans*-2,5-diazabicyclo[4.2.0]octane. LC-MS (ESI), m/z: [M+H]⁺ = 433.1.

**Example 2-1** and **Example 2-2** can also be obtained by the following methods:

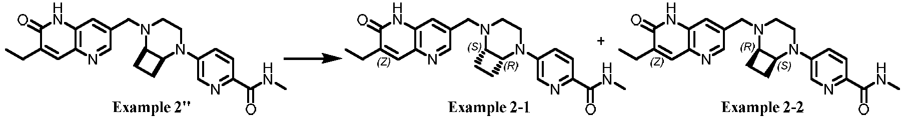

**Example 2"** (15.3 mg, 0.04 mmol) was purified by preparative supercritical fluid chromatography (SFC) (column: CHIRALPAK OJ-H 250 mm × 20 mm, 5 µm, 40% EtOH (NH₄OH 0.2%) to obtain **Example 2-1** (t_{R} = 6.24 min, 5.80 mg) and **Example 2-2** (t_{R} = 12.10 min, 6.8 mg, yield: 44%).

### Example 2': trans-5-(5-((7-ethyl-6-carbonyl-5,6-dihydro-1,5-naphthyridin-3-yl)methyl)-2,5-diazabicyclo[4.2.0]octan-2-yl)-N-methylpyridine-2-carboxamide

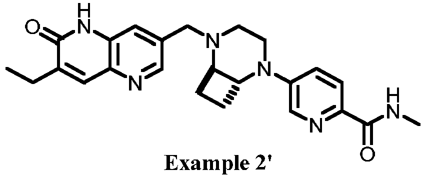

**Example 2'** was obtained with reference to the synthesis of **Example 2** by replacing 2,5-diazabicyclo[4.2.0]octane with *trans*-2,5-diazabicyclo[4.2.0]octane. LC-MS (ESI), m/z: [M+H]⁺ = 433.1.

### Example 2-3 and Example 2-4: 5-((1R,6R)-5-((7-ethyl-6-carbonyl-5,6-dihydro-1,5-naphthyridin-3-yl)methyl)-2,5-diazabicyclo[4.2.0]octan-2-yl)-N-methylpyridine-2-carboxamide and 5-((1S,6S)-5-((7-ethyl-6-carbonyl-5,6-dihydro-1,5-naphthyridin-3-yl)methyl)-2,5-diazabicyclo[4.2.0]octan-2-yl)-N-methylpyridine-2-carboxamide

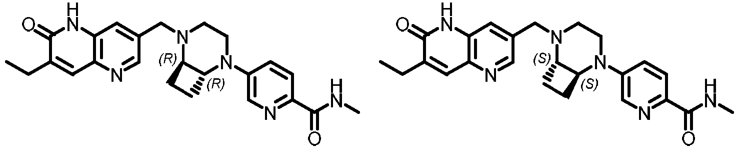

### Synthetic route:

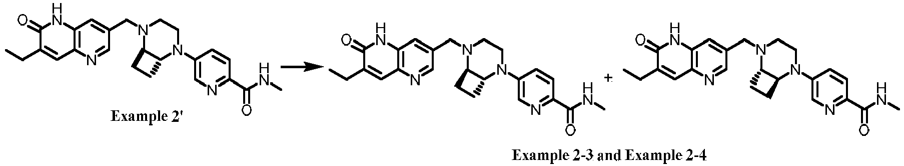

**Example 2'** was separated by chiral column chromatography (chromatographic column model: CHIRALPAKAD-H 250 mm × 20 mm, 5 µm; mobile phase: 40% isopropanol (0.2% ammonia water); flow rate: 40 g/min) to obtain **Example 2-3** (t_{R} = 9.36 min, 20.5 mg, yield: 41%) and **Example 2-4** (t_{R} = 11.08 min, 19.6 mg, yield: 39%).

### Example 2-3: LC-MS (ESI), m/z: [M+H]⁺ = 433.2.

¹H NMR (400 MHz, DMSO-*d₆*) δ 11.84 (s, 1H), 8.46 - 8.39 (m, 1H), 8.11 (m, 1H), 7.81 (d, *J* = 8.6 Hz, 1H), 7.75 (s, 1H), 7.64 (s, 1H), 7.27 (dd, *J =* 8.7, 2.5 Hz, 1H), 3.85 (d, *J=* 12.1 Hz, 1H), 3.59 - 3.51 (m, 2H), 3.02 (d, *J =* 11.3 Hz, 1H), 2.89 - 2.80 (m, 2H), 2.77 (d,*J* = 4.8 Hz, 3H), 2.57 - 2.53 (m, 1H), 2.38 - 2.28 (m, 4H), 2.03 - 1.97 (m, 1H), 1.75 - 1.68 (m, 1H), 1.45 - 1.35 (m, 1H), 1.18 (t, *J* = 7.4 Hz, 3H).

### Example 2-4: LC-MS (ESI), m/z: [M+H]⁺ = 433.2.

¹H NMR (400 MHz, DMSO-*d₆*) δ 11.84 (s, 1H), 8.46 - 8.38 (m, 1H), 8.14 - 8.09 (m, 1H), 7.81 (d, *J =* 8.6 Hz, 1H), 7.75 (s, 1H), 7.64 (s, 1H), 7.27 (dd, *J* = 8.7, 2.5 Hz, 1H), 3.85 (d, *J =* 12.1 Hz, 1H), 3.59 - 3.51 (m, 2H), 3.02 (d, *J* = 11.3 Hz, 1H), 2.89 - 2.77 (m, 2H), 2.76 (d, *J =* 4.8 Hz, 3H), 2.57 - 2.54 (m, 1H), 2.40 - 2.24 (m, 4H), 2.04 - 1.95 (m, 1H), 1.75 - 1.68 (m, 1H), 1.44 - 1.34 (m, 1H), 1.18 (t,*J* = 7.4 Hz, 3H).

### Example 3: cis-5-(4-((7-ethyl-6-oxo-5,6-dihydro-1,5-naphthyridin-3-yl)methyl)hexahydrofuro[3,4-b]pyrazin-1(2H)-y1)-N-methylpyridine-2-carboxamide

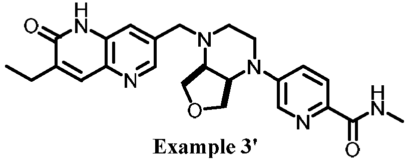

### Synthetic route:

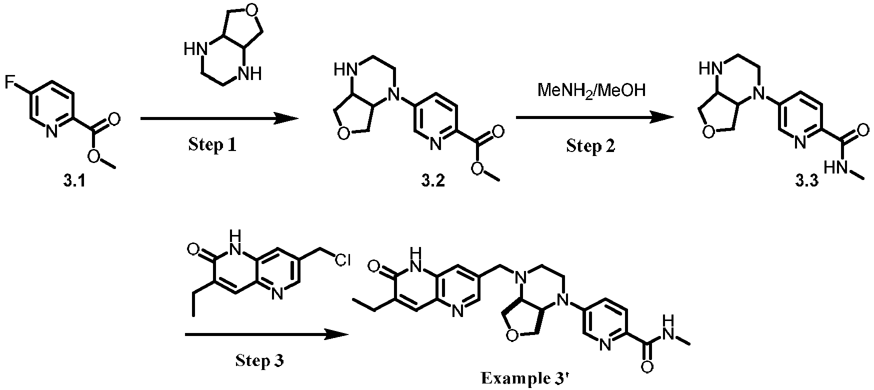

### Step 1: Synthesis of methyl cis-5-(hexahydrofuro[3,4-b]pyrazin-1(2H)-yl)pyridine-2-carboxylate (3.2):

To a solution of methyl 5-fluoropyridine-2-carboxylate (300 mg, 1.93 mmol) and *cis-*decahydrofuro[3,4-*b*]pyrazine (intermediate b, 1.2 g, 9.4 mmol) in dimethyl sulfoxide (10 mL) was added potassium carbonate (535 mg, 3.88 mmol) at room temperature. The reaction mixture was stirred at 120°C for 2 hours. The reaction mixture was cooled to room temperature, filtered, and concentrated. The resulting residue was purified by silica gel chromatography (PE: EA = 5:1) to obtain the title compound **3.2** (275 mg, yield: 55%).

LC-MS (ESI), m/z: [M+H]⁺ = 264.0.

### Step 2: Synthesis of cis-5-(hexahydrofuro[3,4-b]pyrazin-1(2H)-yl)-N-methylpyridine-2-carboxamide (3.3):

Methyl *cis*-5-(hexahydrofuro[3,4-*b*]pyrazin-1(2*H*)-yl)pyridine-2-carboxylate (100 mg, 0.38 mmol) was dissolved in a solution of methylamine in methanol (10 mL) at room temperature. The reaction mixture was reacted at 60°C for 16 hours in a sealed tube. The reaction mixture was cooled to room temperature and concentrated under reduced pressure to obtain the title compound **3.3** (80 mg, yield: 80%).

LC-MS (ESI), m/z: [M+H]⁺ = 263.0.

### Step 3: Synthesis of cis-5-(4-((7-ethyl-6-oxo-5,6-dihydro-1,5-naphthyridin-3-yl)methyl)hexahydrofuro[3,4-b]pyrazin-1(2H)-yl)-N-methylpyridine-2-carboxamide (Example 3):

To a solution of *cis*-5-(hexahydrofuro[3,4-*b*]pyrazin-1(2*H*)-yl)-*N*-methylpyridine-2-carboxamide (80 mg, 0.31 mmol) and 7-(chloromethyl)-3-ethyl-1,5-naphthyridin-2(1*H*)-one (68 mg, 0.31 mmol) in acetonitrile (10 mL) were added diisopropylethylamine (120 mg, 0.93 mmol) and potassium iodide (10 mg, 0.06 mmol) at room temperature, and the reaction system was replaced with nitrogen three times. The reaction mixture was stirred at 80°C for 2 hours under nitrogen atmosphere. The reaction mixture was cooled to room temperature, diluted with ethyl acetate (30 mL), washed with saturated brine, dried over anhydrous sodium sulfate, filtered, and concentrated. The resulting residue was purified by preparative high performance liquid chromatography (chromatographic column model: Gemini-C18 150 × 21.2 mm, 5 µm, mobile phase: acetonitrile/water (0.1% formic acid), gradient: 2 to 30%, flow rate: 20 mL/min) to obtain the title compound **Example 3** (30 mg, yield: 22%).

LC-MS (ESI), m/z: [M+H]⁺ = 448.9.

¹H NMR (400 MHz, DMSO-*d₆*) δ 11.84 (s, 1H), 8.41 (d, *J =* 1.6 Hz, 1H), 8.41 - 8.36 (m, 1H), 8.26 (d, *J =* 2.8, 1H), 7.82 (d, *J =* 8.8 Hz, 1H), 7.76 (s, 1H), 7.66 (s, 1H), 7.40 (dd, *J* = 8.8, 2.8 Hz, 1H), 4.77 - 4.67 (m, 1H), 4.18 - 4.07 (m, 2H), 3.94 - 3.87 (m, 2H), 3.70 - 3.58 (m, 2H), 3.34 - 3.27 (m, 1H), 3.08 - 2.95 (m, 2H), 2.77 (d, *J =* 4.8 Hz, 3H), 2.59 - 2.50 (m, 2H), 2.32 - 2.22 (m, 1H), 2.04 - 1.93 (m, 1H), 1.19 (t, *J =* 6.8 Hz, 3H).

### Example 3-1 and Example 3-2: 5-((4aS,7aR)-4-((7-ethyl-6-oxo-5,6-dihydro-1,5-naphthyridin-3-yl)methyl)hexahydrofuro[3,4-b]pyrazin-1(2H)-yl)-N-methylpyridine-2-carboxamide and 5 -((4aR,7aS)-4-((7-ethyl-6-oxo-5,6-dihydro-1,5-naphthyridin-3-yl)methyl)hexahydrofuro[3,4-b]pyrazin-1(2H)-yl)-N-methylpyridine-2-carboxamide

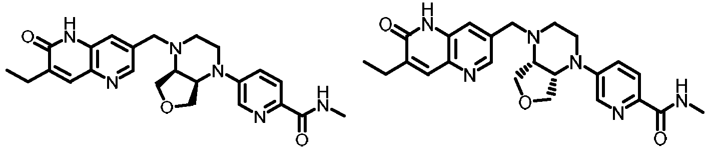

### Synthetic route:

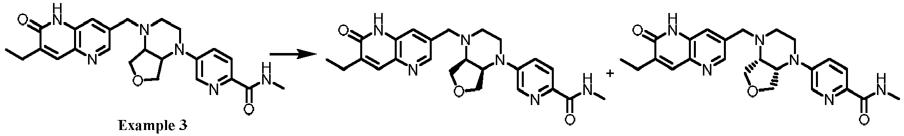

**Example 3** (26 mg, 0.06 mmol) was separated by preparative supercritical fluid chromatography (SFC) (chromatographic column model: CHIRALPAK AD-H 250 × 20 mm, 5 µm, mobile phase: 30% MeOH (0.2% ammonia water), flow rate: 40 g/min) to obtain the following:

### Example 3-1 (t_{R} = 18.40 min, 4.2 mg, yield: 14%)

LC-MS (ESI), m/z: [M+H]⁺ = 448.9.

¹H NMR (400 MHz, DMSO-*d₆*) δ 11.84 (s, 1H), 8.41 (d, *J* = 1.6 Hz, 1H), 8.41 - 8.36 (m, 1H), 8.26 (d, *J =* 2.8, 1H), 7.82 (d, *J =* 8.8 Hz, 1H), 7.76 (s, 1H), 7.66 (s, 1H), 7.40 (dd, *J* = 8.8, 2.8 Hz, 1H), 4.77 - 4.67 (m, 1H), 4.18 - 4.07 (m, 2H), 3.94 - 3.87 (m, 2H), 3.70 - 3.58 (m, 2H), 3.34 - 3.27 (m, 1H), 3.08 - 2.95 (m, 2H), 2.77 (d, *J =* 4.8 Hz, 3H), 2.59 - 2.50 (m, 2H), 2.32 - 2.22 (m, 1H), 2.04 - 1.93 (m, 1H), 1.19 (t, *J =* 6.8 Hz, 3H).

### Example 3-2 (t_{R} = 26.40 min, 3.5 mg, yield: 12%)

LC-MS (ESI), m/z: [M+H]⁺ = 449.0.

¹H NMR (400 MHz, DMSO-*d₆*) δ 11.84 (s, 1H), 8.41 (d, *J* = 1.6 Hz, 1H), 8.41 - 8.36 (m, 1H), 8.26 (d, *J =* 2.8, 1H), 7.82 (d, *J =* 8.8 Hz, 1H), 7.76 (s, 1H), 7.66 (s, 1H), 7.40 (dd, *J* = 8.8, 2.8 Hz, 1H), 4.77 - 4.67 (m, 1H), 4.18 - 4.07 (m, 2H), 3.94 - 3.87 (m, 2H), 3.70 - 3.58 (m, 2H), 3.34 - 3.27 (m, 1H), 3.08 - 2.95 (m, 2H), 2.77 (d, *J =* 4.8 Hz, 3H), 2.59 - 2.50 (m, 2H), 2.32 - 2.22 (m, 1H), 2.04 - 1.93 (m, 1H), 1.19 (t, *J =* 6.8 Hz, 3H).

### Example 4: cis-5-(4-((7-ethyl-6-oxo-5,6-dihydro-1,5-naphthyridin-3-yl)methyl)octahydro-1H-cyclopenta[b]pyrazin-1-yl)-N-methyl-pyridine-2-carboxamide

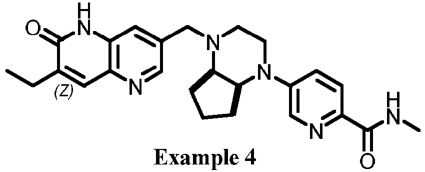

**Example 4** was obtained with reference to the synthesis of **Example 1.** LC-MS (ESI), m/z: [M+H]⁺ = 446.9.

¹H NMR (400 MHz, DMSO-*d₆*) δ 11.81 (s, 1H), 8.40 (d, *J* = 1.8 Hz, 1H), 8.33 (d,*J* = 4.9 Hz, 1H), 8.20 (d, *J =* 2.8 Hz, 1H), 7.80 (d, *J =* 8.8 Hz, 1H), 7.75 (s, 1H), 7.66 (s, 1H), 7.33 (dd, *J =* 8.9, 2.9 Hz, 1H), 4.38 - 4.28 (m, 1H), 4.12 (d, *J =* 14.5 Hz, 1H), 3.57 (d, *J =* 12.0 Hz, 1H), 3.22 (d, *J =* 14.5 Hz, 1H), 3.01 - 2.91 (m, 1H), 2.77 (d, *J =* 4.7 Hz, 3H), 2.74 - 2.69 (m, 2H), 2.57 - 2.52 (m, 1H), 2.25 - 2.17 (m, 1H), 2.07 - 1.95 (m, 1H), 1.93 - 1.76 (m, 2H), 1.75 - 1.57 (m, 2H), 1.18 (t, *J* = 7.4 Hz, 3H).

**Example 4-1** and **Example 4-2:** 5-((4a*S*,7a*R*)-4-((7-ethyl-6-oxo-5,6-dihydro-1,5-naphthyridin-3-yl)methyl)octahydro-1*H*-cyclopenta[*b*]pyrazin-1-yl)-*N*-methyl-pyridine-2-carboxamide and 5-((4a*R*,7a*S*)-4-((7-ethyl-6-oxo-5,6-dihydro-1,5-naphthyridin-3-yl)methyl)octahydro-1*H*-cyclopenta[*b*]pyrazin-1-yl)-*N*-methyl-pyridine-2-carboxamide

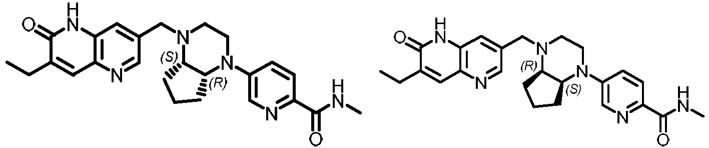

### Synthetic route:

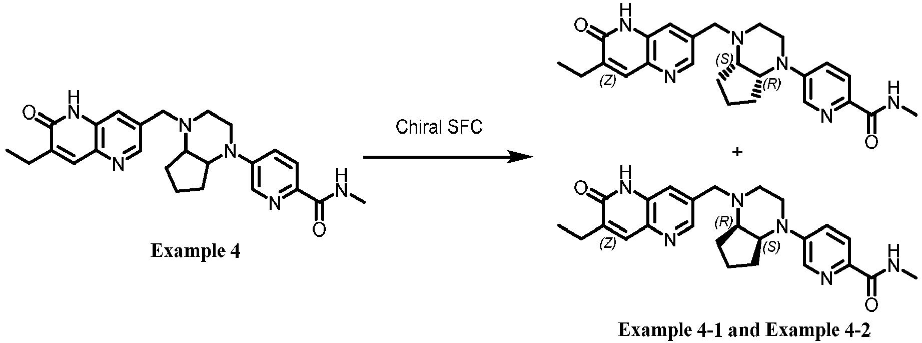

**Example 4** (26 mg, 0.06 mmol) was separated by preparative supercritical fluid chromatography (SFC) (chromatographic column model: CHIRALPAK OJ-H 250 mm × 4.6 mm, 5 µm, mobile phase: 40% EtOH (0.2% ammonia water), flow rate: 2.5 mL/min) to obtain **Example 4-1** (t_{R} = 5.68 min) and **Example 4-2** (t_{R} = 11.04 min).

### Example 4-1: LC-MS (ESI), m/z: [M+H]⁺ = 446.9.

¹H NMR (400 MHz, DMSO-*d₆*) δ 11.83 (s, 1H), 8.40 (d, *J* = 1.8 Hz, 1H), 8.35 (d,*J* = 4.9 Hz, 1H), 8.20 (d, *J =* 2.8 Hz, 1H), 7.81 (d, *J =* 8.8 Hz, 1H), 7.75 (s, 1H), 7.66 (s, 1H), 7.33 (dd, *J =* 8.9, 2.9 Hz, 1H), 4.38 - 4.28 (m, 1H), 4.12 (d, *J =* 14.5 Hz, 1H), 3.57 (d, *J =* 12.0 Hz, 1H), 3.22 (d, *J =* 14.5 Hz, 1H), 3.01 - 2.91 (m, 1H), 2.77 (d, *J =* 4.7 Hz, 3H), 2.74 - 2.69 (m, 2H), 2.57 - 2.52 (m, 1H), 2.25 - 2.17 (m, 1H), 2.07 - 1.95 (m, 1H), 1.93 - 1.76 (m, 2H), 1.75 - 1.57 (m, 2H), 1.18 (t, *J* = 7.4 Hz, 3H).

### Example 4-2: LC-MS (ESI), m/z: [M+H]⁺ = 446.9.

¹H NMR (400 MHz, DMSO-*d₆*) δ 11.83 (s, 1H), 8.40 (d, *J* = 1.8 Hz, 1H), 8.35 (d,*J* = 4.9 Hz, 1H), 8.20 (d, *J =* 2.8 Hz, 1H), 7.81 (d, *J =* 8.8 Hz, 1H), 7.75 (s, 1H), 7.66 (s, 1H), 7.33 (dd, *J =* 8.9, 2.9 Hz, 1H), 4.38 - 4.28 (m, 1H), 4.12 (d, *J =* 14.5 Hz, 1H), 3.57 (d, *J =* 12.0 Hz, 1H), 3.22 (d, *J =* 14.5 Hz, 1H), 3.01 - 2.91 (m, 1H), 2.77 (d, *J =* 4.7 Hz, 3H), 2.74 - 2.69 (m, 2H), 2.57 - 2.52 (m, 1H), 2.25 - 2.17 (m, 1H), 2.07 - 1.95 (m, 1H), 1.93 - 1.76 (m, 2H), 1.75 - 1.57 (m, 2H), 1.18 (t, *J* = 7.4 Hz, 3H).

### Example 5: cis-5-(5-((7-ethyl-6-oxo-5,6-dihydro-pyrido[3,2-c]pyridazine-2,5-diazabicyclo[4.1.0]octan-2-yl)-N-methylpicolinamide

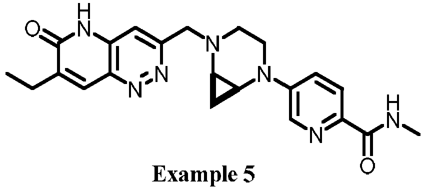

**Example 5** (50 mg, yield: 46%) was obtained with reference to the synthesis of **Example 1:**

LC-MS (ESI), m/z: [M+H]⁺ = 420.2.

¹H NMR (400 MHz, DMSO-*d₆*) δ 12.30 (s, 1H), 8.40 (d, *J =* 4.8 Hz, 1H), 8.21 (d,*J* = 2.6 Hz, 1H), 8.07 (s, 1H), 7.89 (d, *J* = 8.8 Hz, 1H), 7.53 (s, 1H), 7.33 (dd, *J* = 8.8, 2.8 Hz, 1H), 4.54 (m, 2H), 3.72 - 3.66 (m, 1H), 3.35 - 3.27 (m, 1H), 3.23 - 3.16 (m, 1H), 3.02 - 2.88 (m, 3H), 2.79 (d, *J =* 4.7 Hz, 3H), 2.60 (q, *J =* 7.1 Hz, 2H), 1.22 (t, *J =* 7.4 Hz, 3H), 1.03 (q, *J =* 6.3 Hz, 1H), 0.82 (s, 1H).

### Example 5-1 and Example 5-2: 5-((1S,6R)-5-((7-ethyl-6-oxo-5,6-dihydro-pyrido[3,2-c]pyridazine-2,5-diazabicyclo[4.1,0]octan-2-yl)-N-methylpicolinamide and 5-((1R,6S)-5-((7-ethyl-6-oxo-5,6-dihydro-pyrido[3,2-c]pyridazine-2,5-diazabicyclo[4. 1.0]octan-2-yl)-N-methylpicolinamide:

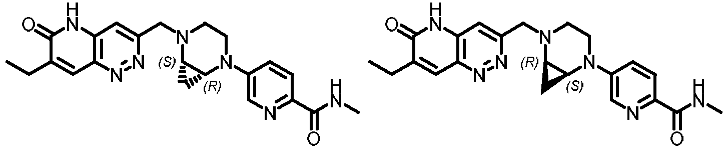

### Synthesis method:

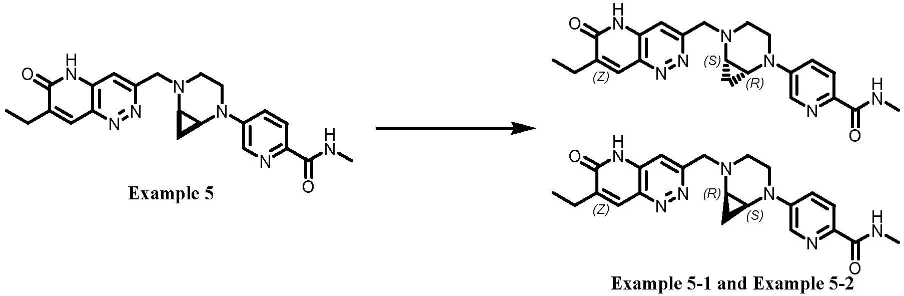

**Example 5** (48 mg) was purified by chiral column chromatography (chromatographic column model: XBridge peptide BEH C18 19 mm × 250 mm, 10 µm, 130 Å; mobile phase: acetonitrile (0.05% TFA)/water (0.05% TFA): 5 to 35%; flow rate: 25 mL/min) to obtain **Example 5-1** (t_{R} = 28.5 min, 8.4 mg, yield: 18%) and **Example 5-2** (t_{R} = 36.5 min, 10.0 mg, yield: 21%).

### Example 5-1: LC-MS (ESI), m/z: [M+H]⁺ = 420.2.

¹H NMR (400 MHz, DMSO-*d₆*) δ 12.30 (s, 1H), 8.40 (d, *J* = 4.8 Hz, 1H), 8.21 (d,*J* = 2.6 Hz, 1H), 8.07 (s, 1H), 7.89 (d, *J =* 8.8 Hz, 1H), 7.53 (s, 1H), 7.33 (dd, *J* = 8.8, 2.8 Hz, 1H), 4.54 (m, 2H), 3.72 - 3.66 (m, 1H), 3.35 - 3.27 (m, 1H), 3.23 - 3.16 (m, 1H), 3.02 - 2.88 (m, 3H), 2.79 (d, *J =* 4.7 Hz, 3H), 2.60 (q, *J =* 7.1 Hz, 2H), 1.22 (t, *J =* 7.4 Hz, 3H), 1.03 (q, *J =* 6.3 Hz, 1H), 0.82 (s, 1H).

### Example 5-2: LC-MS (ESI), m/z: [M+H]⁺ = 420.2.

¹H NMR (400 MHz, DMSO-*d₆*) 12.01 (s, 1H), 8.35 (d, *J =* 4.8 Hz, 1H), 8.19 (d, *J =* 2.5 Hz, 1H), 8.02 (s, 1H), 7.85 (d, *J =* 8.8 Hz, 1H), 7.47 (s, 1H), 7.28 (dd, *J =* 8.8, 2.7 Hz, 1H), 4.35 - 4.05 (q, *J =* 15.8 Hz, 2H), 3.55 - 3.44 (m, 1H), 3.31 (s, 1H), 3.28 - 3.20 (m, 1H), 2.89 - 2.81 (m, 1H).

### Example 6:

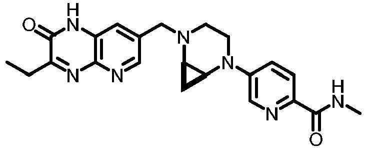

**Example 6** was obtained with reference to the synthesis of **Example 1.** LC-MS (ESI), m/z: [M+H]⁺ = 419.9.

¹H NMR (400 MHz, DMSO-*d₆*) δ 8.47 (d, *J* = 1.8 Hz, 1H), 8.35 (d, *J* = 4.9 Hz, 1H), 8.19 (d, *J =* 2.6 Hz, 1H), 7.85 (d, *J =* 8.8 Hz, 1H), 7.68 (d, *J =* 1.9 Hz, 1H), 7.27 (dd, *J =* 8.8, 2.8 Hz, 1H), 3.55 - 3.47 (m, 1H), 3.26 - 3.18 (m, 1H), 2.84 (q, *J =* 7.4 Hz, 2H), 2.78 (d, *J =* 4.9 Hz, 2H), 2.75 - 2.69 (m, 1H), 2.58 - 2.52 (m, 3H), 1.23 (t, *J=* 7.3 Hz, 2H), 0.80 - 0.74 (m, 2H), 0.41 - 0.35 (m, 1H).

### Example 6-1 and Example 6-2:

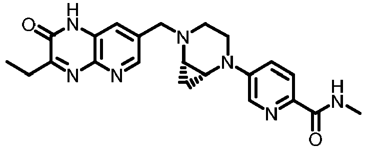

and

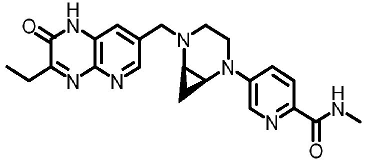

**Example 6** was separated by preparative chiral chromatography (CHIRALPAK IC 0.46 cm I.D. × 15 cm; mobile phase: mobile phase A = *n*-hexane, mobile phase B = methanol (0.1% diethylamine), A/B = 0/100; flow rate: 0.7 mL/min) to obtain **Example 6-1** (t_{R} = 4.02) and **Example 6-2** (t_{R} = 5.02).

### Example 6-1: LC-MS (ESI), m/z: [M+H]⁺ = 420.1.

¹H NMR (400 MHz, DMSO-*d₆*) δ 8.47 (d, *J* = 1.5 Hz, 1H), 8.36 (q, *J* = 4.8 Hz, 1H), 8.19 (d, *J =* 2.6 Hz, 1H), 7.85 (d, *J =* 8.8 Hz, 1H), 7.69 (d, *J =* 1.6 Hz, 1H), 7.27 (dd, *J =* 8.8, 2.7 Hz, 1H), 3.92 (q, *J =* 13.9 Hz, 2H), 3.55 - 3.48 (m, 1H), 3.26 - 3.18 (m, 1H), 2.84 (q, *J =* 7.3 Hz, 2H), 2.78 (d, *J =* 4.8 Hz, 3H), 2.75 - 2.69 (m, 1H), 2.59 - 2.53 (m, 2H), 1.24 (t, *J =* 7.3 Hz, 3H), 0.89 - 0.82 (m, 1H), 0.81 - 0.74 (m, 1H), 0.38 (q, *J =* 4.9 Hz, 1H).

### Example 6-2: LC-MS (ESI), m/z: [M+H]⁺ = 420.1.

¹H NMR (400 MHz, DMSO-*d₆*) δ 8.47 (d, *J* = 1.5 Hz, 1H), 8.35 (q, *J* = 4.8 Hz, 1H), 8.19 (d, *J =* 2.6 Hz, 1H), 7.85 (d, *J =* 8.8 Hz, 1H), 7.68 (d, *J =* 1.6 Hz, 1H), 7.27 (dd, *J =* 8.8, 2.7 Hz, 1H), 3.91 (q, *J =* 13.9 Hz, 2H), 3.55 - 3.48 (m, 1H), 3.26 - 3.18 (m, 1H), 2.84 (q, *J =* 7.3 Hz, 2H), 2.78 (d, *J =* 4.8 Hz, 3H), 2.75 - 2.69 (m, 1H), 2.59 - 2.53 (m, 2H), 1.24 (t, *J =* 7.3 Hz, 3H), 0.89 - 0.82 (m, 1H), 0.81 - 0.74 (m, 1H), 0.38 (q, *J =* 4.9 Hz, 1H).

### Example 7: cis-N-methyl-5-(5-((6-oxo-6,7,8,9-tetrahydro-5H-cyclopenta[c][1,5]naphthyridin-3-yl)methyl)-2,5-diazabicyclo[4.1.0]heptan-2-yl)picolinamide

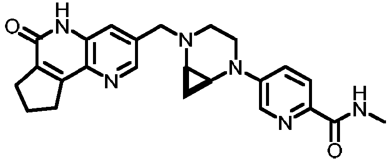

**Example 7** was obtained with reference to the synthesis of **Example 1.** LC-MS (ESI), m/z: [M+H]⁺ = 431.2.

¹H NMR (400 MHz, DMSO-*d₆*) δ 11.67 (s, 1H), 8.45 (d, *J* = 1.6 Hz, 1H), 8.35 (d,*J* = 4.8 Hz, 1H), 8.19 (d, *J =* 2.7 Hz, 1H), 7.85 (d, *J =* 8.8 Hz, 1H), 7.70 (s, 1H), 7.27 (dd, *J =* 8.8, 2.9 Hz, 1H), 3.95 - 3.83 (m, 2H), 3.54 - 3.46 (m, 1H), 3.24 - 3.20 (m, 1H), 3.17 (t, *J =* 7.2 Hz, 3H), 2.83 - 2.79 (m, 2H), 2.78 (d, *J =* 4.8 Hz, 3H), 2.75 - 2.69 (m, 1H), 2.58 - 2.52 (m, 2H), 2.11 (p, *J =* 7.3 Hz, 2H), 0.77 (q, *J =* 6.3 Hz, 1H), 0.38 (q, *J =* 5.0 Hz, 1H).

### Example 10: cis-5-(5-((7-ethyl-6-oxo-5,6-dihydro-pyrido[3,2-c]pyridazine-2,5-diazabicyclo[4.2.0]octan-2-yl)-N-methylpicolinamide

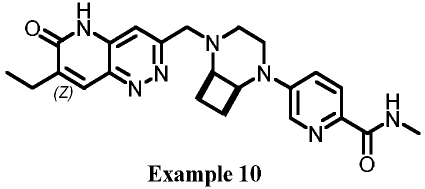

**Example 10** (51 mg, yield: 44%) was obtained with reference to the synthesis of **Example 1.**

LC-MS (ESI), m/z: [M+H]⁺ = 434.2.

### Example 10-1 and Example 10-2: 5-((1S,6R)-(5-((7-ethyl-6-oxo-5,6-dihydro-pyrido[3,2-c]pyridazine-2,5-diazabicyclo[4.2.0]octan-2-yl)-N-methylpicolinamide and 5-((1R,6S)-(5-((7-ethyl-6-oxo-5,6-dihydro-pyrido[3,2-c]pyridazine-2,5-diazabicyclo[4.2.0]octan-2-yl)-N-methylpicolinamide

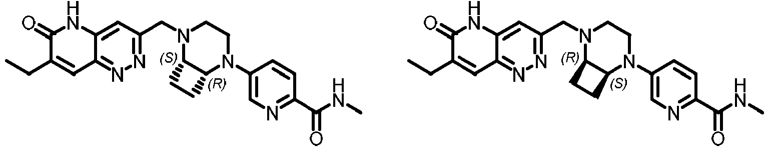

**Example 10** (48 mg) was separated by preparative chiral SCF column chromatography (instrument model: SFC-80 (Thar, Waters); chromatographic column model: CHIRALCEL OD (30 × 250 mm 5 µm); mobile phase: mobile phase A = carbon dioxide, mobile phase B = methanol (0.2% ammonia (7 mol/L ammonia-methanol solution)); total flow rate: 45 mL/min) to obtain **Example 10-1** (t_{R} = 14.0 min, 10.2 mg, yield: 22%) and **Example 10-2** (t_{R} = 21.5 min, 12.0 mg, yield: 25%).

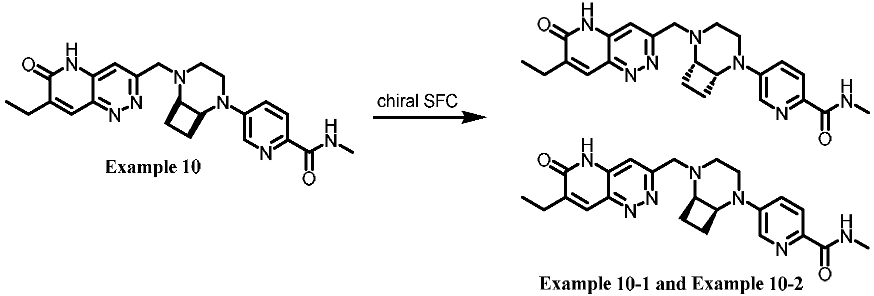

### Example 10-1: LC-MS (ESI), m/z: [M+H]⁺ = 434.2.

¹H NMR (400 MHz, DMSO-*d₆*) δ 11.86 (br, 1H), 8.37 (d, *J* = 4.0 Hz, 1H), 8.11 (s, 1H), 8.03 (s, 1H), 7.83 (d, *J =* 8.7 Hz, 1H), 7.54 (s, 1H), 7.24 (dd, *J =* 8.9, 2.8 Hz, 1H), 4.33 (q, *J =* 6.5 Hz, 1H), 4.11 (d, *J =* 14.4 Hz, 1H), 3.77 (d, *J =* 14.4 Hz, 1H), 3.53 - 3.38 (m, 2H), 3.26 - 3.13 (m, 2H), 2.88 - 2.80 (m, 1H), 2.77 (m, 3H), 2.62 - 2.55 (m, 2H), 2.40 (t, *J =* 10.2 Hz, 1H), 2.29 - 2.21 (m, 1H), 2.13 - 2.00 (m, 1H), 1.82 (m, 2H), 1.21 (t, *J =* 7.2 Hz, 3H).

### Example 10-2: LC-MS (ESI), m/z: [M+H]⁺ = 434.2.

¹H NMR (400 MHz, DMSO-*d₆*) δ 11.74 (br, 1H), 8.36 (d, *J =* 4.0 Hz, 1H), 8.11 (s, 1H), 8.03 (s, 1H), 7.82 (d, *J =* 8.7 Hz, 1H), 7.54 (s, 1H), 7.23 (dd, *J =* 8.9, 2.8 Hz, 1H), 4.33 (q, *J =* 6.9 Hz, 1H), 4.10 (d, *J* = 14.5 Hz, 1H), 3.76 (d, *J =* 14.4 Hz, 1H), 3.51 - 3.45 (m, 1H), 3.43 - 3.38 (m, 1H), 3.27 - 3.17 (m, 2H), 2.86 - 2.80 (m, 1H), 2.77 (d, *J =* 4.4 Hz, 3H), 2.62 - 2.56 (m, 2H), 2.39 (t, *J* = 9.9 Hz, 1H), 2.29 - 2.21 (m, 1H), 2.13 - 2.00 (m, 1H), 1.83 (m, 2H), 1.21 (t, *J* = 7.4 Hz, 3H).

### Example 11: cis-5-(5-((3-ethyl-2-oxo-1,2-dihydropyrido[2,3-b]pyrazin-7-yl)methyl)-2,5-diazabicyclo[4.2.0]octan-2-yl)-N-methylpicolinamide

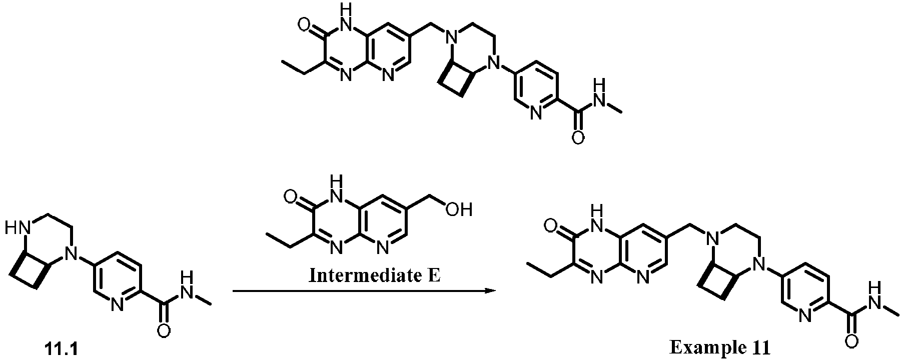

To a solution of compound **11.1** (20.0 mg, 0.810 mmol) in butyronitrile (2.00 mL) were added *N*,*N-*diisopropylethylamine (210 mg, 1.62 mmol), **intermediate E** (16.7 mg, 0.810 mmol), and (cyanomethyl)trimethylammonium phosphonium iodide (78.9 mg, 0.325 mmol) at 25°C. The reaction mixture was stirred at 80°C for 2 hours under nitrogen atmosphere. The reaction system was concentrated, and the resulting residue was purified by preparative liquid chromatography (chromatographic column model: Gemini-C18 150 × 21.2 mm, 5.00 µm, mobile phase: acetonitrile/0.100% formic acid in water, gradient: 2.00% to 21.0%) to obtain **Example 11** (10.0 mg, yield: 27.0%). LC-MS (ESI), m/z: [M+H]⁺ = 433.9.

### Example 11-1 and Example 11-2:

### 5-((1S,6R)-5-((3-ethyl-2-oxo-1,2-dihydropyrido[2,3-b]pyrazin-7-yl)methyl)-2,5-diazabicyclo[4.2.0]octan-2-yl)-N-methylpicolinamide and 5-((1R,6S)-5-((3-ethyl-2-oxo-1,2-dihydropyrido[2,3-b]pyrazin-7-yl)methyl)-2,5-diazabicyclo[4.2.0]octan-2-yl)-N-methylpicolinamide

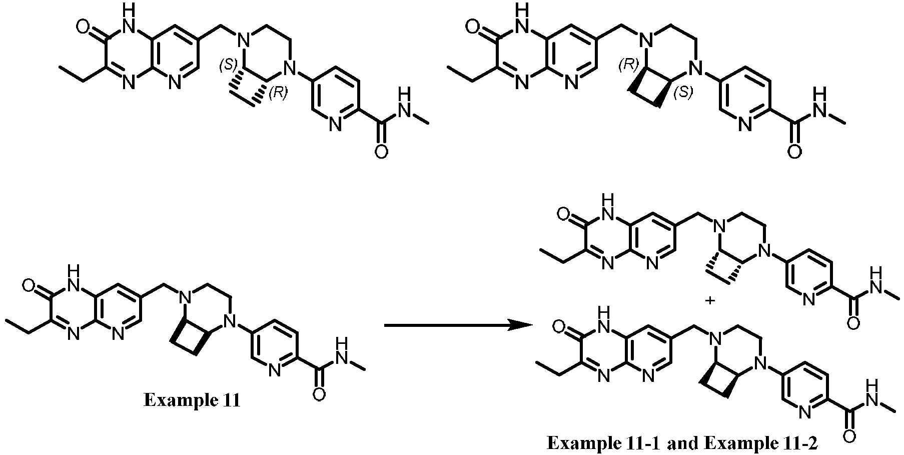

**Example 11** was separated by preparative chiral chromatography (CHIRALPAK OJ-H 250 mm × 4.6 mm, 5 µm; mobile phase: mobile phase A = carbon dioxide, mobile phase B = methanol (0.2% ammonia water), A/B = 60/40; flow rate: 40 g/min) to obtain **Example 11-1** (t_{R} = 10.9) and **Example 11-2** (t_{R} = 19.8).

### Example 11-1: LC-MS (ESI), m/z: [M+H]⁺ = 433.9.

¹H NMR (400 MHz, DMSO-*d₆*) δ 8.45 (s, 1H), 8.37 (q, *J* = 4.9 Hz, 1H), 8.10 (d, *J =* 2.4 Hz, 1H), 7.82 (d, *J =* 8.8 Hz, 1H), 7.64 (s, 1H), 7.23 (dd, *J =* 8.9, 2.6 Hz, 1H), 4.32 (q, *J =* 7.0 Hz, 1H), 3.90 (d, *J =* 14.1 Hz, 1H), 3.52 - 3.40 (m, 3H), 3.26 - 3.17 (m, 1H), 2.85 (q, *J =* 7.3 Hz, 2H), 2.77 (d, *J =* 4.8 Hz, 3H), 2.35 - 2.20 (m, 2H), 2.13 - 1.94 (m, 2H), 1.87 - 1.77 (m, 2H), 1.24 (t, *J =* 7.3 Hz, 3H).

### Example 11-2: LC-MS (ESI), m/z: [M+H]⁺ = 433.9.

¹H NMR (400 MHz, DMSO-*d₆*) δ 8.46 (s, 1H), 8.37 (q, *J* = 4.9 Hz, 1H), 8.10 (d, *J =* 2.4 Hz, 1H), 7.82 (d, *J =* 8.8 Hz, 1H), 7.70 (s, 1H), 7.23 (dd, *J* = 8.9, 2.6 Hz, 1H), 4.32 (q, *J =* 7.0 Hz, 1H), 3.92 (d, *J =* 14.1 Hz, 1H), 3.52 - 3.40 (m, 3H), 3.26 - 3.17 (m, 1H), 2.85 (q, *J =* 7.3 Hz, 2H), 2.77 (d, *J =* 4.8 Hz, 3H), 2.35 - 2.20 (m, 2H), 2.13 - 1.94 (m, 2H), 1.87 - 1.77 (m, 2H), 1.24 (t, *J =* 7.3 Hz, 3H).

### Example 12: cis-N-methyl-5-(5-((6-oxo-6,7,8,9-tetrahydro-5H-cyclopenta[c][1,5]naphthyridin-3-yl)methyl)-2,5-diazabicyclo[4.2.0]octan-2-yl)picolinamide

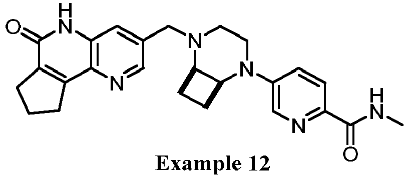

### Synthetic route:

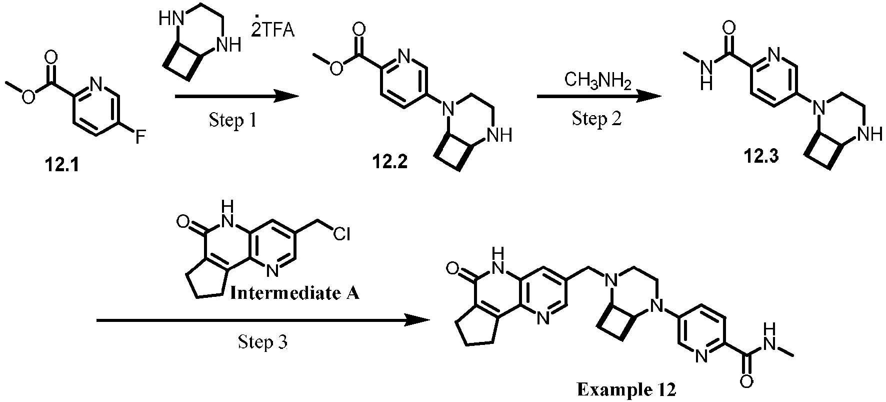

### Step 1: Synthesis of methyl cis-5-(2,5-diazabicyclo[4.2.0]octan-2-yl)pyridine-2-carboxylate (12.2)

To a solution of compound **12.1** (150 mg, 0.970 mmol) in dimethyl sulfoxide (2 mL) were added 2,5-diazabicyclo[4.2.0]octane bis(trifluoroacetate) (355 mg, 1.04 mmol) and triethylamine (489 mg, 4.80 mmol) under nitrogen atmosphere. After the addition was completed, the reaction mixture was stirred at 120°C for 5 hours. After the reaction was completed, the reaction mixture was concentrated under reduced pressure, and the resulting residue was purified by column chromatography (DCM/MeOH = 20/3) to obtain the title compound **12.2** (316 mg, yield: 93%). LC-MS (ESI), m/z: [M+H]⁺ = 248.2.

### Step 2: Synthesis of cis-5-(2,5-diazabicyclo[4.2.0]octan-2-yl)-N-methylpyridine-2-carboxamide (12.3)

To a solution of compound **12.2** (316 mg, 1.30 mmol) in methanol (10 mL) was added methylamine alcohol solution (10 mL) at room temperature. After the addition was completed, the reaction mixture was stirred at room temperature for 16 hours. After the reaction was completed, the reaction mixture was concentrated under reduced pressure, and the resulting residue was purified by column chromatography (DCM/MeOH = 10/1) to obtain the title compound **12.3** (219 mg, yield: 63%). LC-MS (ESI), m/z: [M+H]⁺ = 247.1.

### Step 3: Synthesis of cis-N-methyl-5-(5-((6-oxo-6,7,8,9-tetrahydro-5H-cyclopenta[c][1,5]naphthyridin-3-yl)methyl)-2,5-diazabicyclo[4.2.0]octan-2-yl)picolinamide (Example 12)

To a solution of compound **12.3** (19.8 mg, 0.0800 mmol) in acetonitrile (1 mL) were added **intermediate A** (22.0 mg, 0.0900 mmol), diisopropylethylamine (42.0 mg, 0.300 mmol), and potassium iodide (3.00 mg, 0.0200 mmol) under nitrogen atmosphere. After the addition was completed, the reaction mixture was stirred at 80°C for 16 hours. After the reaction was completed, the reaction system was concentrated under reduced pressure, and the resulting residue was purified by preparative liquid chromatography (chromatographic column model: Gemini-C18 150 × 21.2 mm, 5 µm; mobile phase: acetonitrile/0.1% formic acid in water; gradient: 20 to 40%) to obtain the title compound **Example 12** (40.0 mg, yield: 70%). LC-MS (ESI), m/z: [M+H]⁺ = 445.3.

### Example 12-1 and Example 12-2: N-methyl-5-[(1S,6R)-5-((6-oxo-5H,7H,8H,9H-cyclopenta[c][1,5]naphthyridin-3-yl)methyl)-2,5-diazabicyclo[4.2.0]octan-2-yl]pyridine-2-carboxamide and N-methyl-5-[(1R,6S)-5-((6-oxo-5H,7H,8H,9H-cyclopenta[c][1,5]naphthyridin-3-yl)methyl)-2,5-diazabicyclo[4.2.0]octan-2-yl]pyridine-2-carboxamide

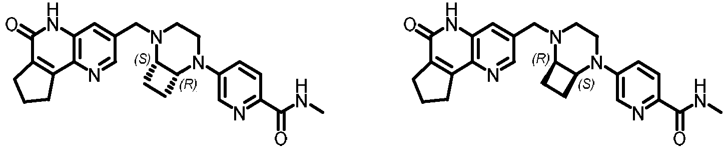

### Synthetic route:

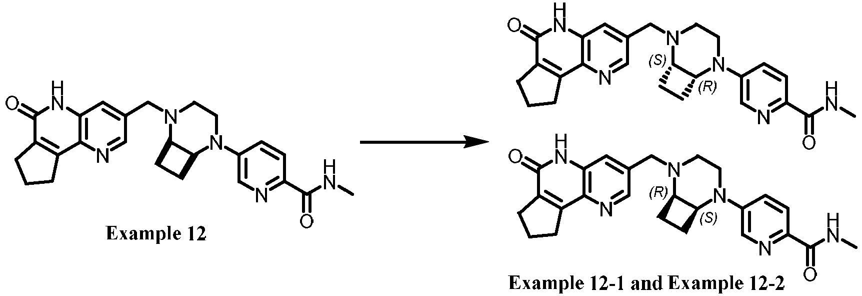

**Example 12** was separated by chiral column chromatography (chromatographic column model: CHIRALPAKAD-H 250 mm × 20 mm, 5 µm; mobile phase: 40% isopropanol (0.2% ammonia water); flow rate: 40 g/min) to obtain **Example 12-1** (t_{R} = 12.01 min, 12.0 mg, yield: 33%) and **Example 12-2** (t_{R} = 14.06 min, 13.8 mg, yield: 37%).

### Example 12-1: LC-MS (ESI), m/z: [M+H]⁺ = 445.1.

¹H NMR (400 MHz, MeOH-*d₄*) δ 8.55 (d, *J =* 1.8 Hz, 1H), 8.16 (d, *J=* 2.8 Hz, 1H), 7.92 - 7.87 (m, 2H), 7.23 (dd, *J =* 8.9, 2.9 Hz, 1H), 4.37 (dd, *J* = 14.7, 6.9 Hz, 1H), 4.03 (d, *J* = 14.2 Hz, 1H), 3.54 - 3.41 (m, 4H), 3.02 - 2.89 (m, 6H), 2.46 - 2.22 (m, 6H), 2.01 - 1.95 (m, 2H).

### Example 12-2: LC-MS (ESI), m/z: [M+H]⁺ = 445.2.

¹H NMR (400 MHz, MeOH-*d₄*) δ 8.55 (d, *J =* 1.7 Hz, 1H), 8.16 (d, *J =* 2.7 Hz, 1H), 7.92 - 7.87 (m, 2H), 7.23 (dd, *J* = 8.9, 2.9 Hz, 1H), 4.37 (dd, *J* = 14.7, 6.9 Hz, 1H), 4.03 (d, *J* = 14.2 Hz, 1H), 3.54 - 3.41 (m, 4H), 3.02 - 2.89 (m, 6H), 2.46 - 2.22 (m, 6H), 2.01 - 1.95 (m, 2H).

### Example 16:

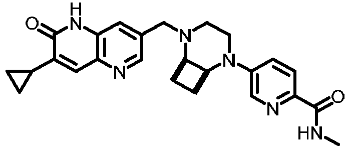

**Example 16** was obtained with reference to the synthesis of **Example 29.** LC-MS (ESI), m/z: [M+H]⁺ = 445.1.

### Example 16-1 and Example 16-2:

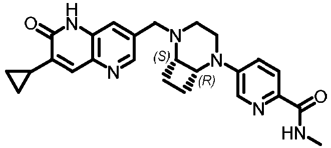

and

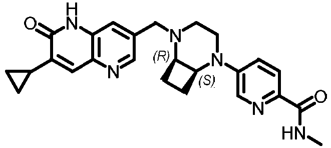

**Example 16** was separated by preparative chiral chromatography (CHIRALPAK OJ-H 250 mm × 20 mm, 5 µm; mobile phase: mobile phase A = carbon dioxide, mobile phase B = ethanol (0.2% ammonia water), A/B = 60/40; flow rate: 40 g/min) to obtain **Example 16-1** (t_{R} = 9.67) and **Example 16-2** (t_{R} = 13.25).

### Example 16-1: LC-MS (ESI), m/z: [M+H]⁺ = 445.1.

¹H NMR (400 MHz, DMSO-*d₆*) δ 11.85 (s, 1H), 8.41 (d, *J* = 1.7 Hz, 1H), 8.36 (q,*J* = 4.9 Hz, 1H), 8.10 (d, *J =* 2.8 Hz, 1H), 7.82 (d, *J =* 8.8 Hz, 1H), 7.66 (d, *J =* 1.6 Hz, 1H), 7.43 (s, 1H), 7.22 (dd, *J* = 8.9, 2.9 Hz, 1H), 4.31 (q, *J* = 6.9 Hz, 2H), 3.89 (d, *J* = 14.1 Hz, 1H), 3.48 - 3.42 (m, 1H), 3.39 (d, *J=* 14.1 Hz, 1H), 3.25 - 3.17 (m, 1H), 2.79 (m, 1H), 2.77 (d, *J =* 4.9 Hz, 3H), 2.67 (p, *J =* 1.9 Hz, 1H), 2.34 - 2.20 (m, 2H), 2.17 - 2.01 (m, 2H), 1.86 - 1.79 (m, 2H), 1.00 - 0.94 (m, 2H), 0.85 - 0.78 (m, 2H).

### Example 16-2: LC-MS (ESI), m/z: [M+H]⁺ = 445.1.

¹H NMR (400 MHz, DMSO-*d₆*) δ 11.86 (s, 1H), 8.41 (d, *J* = 1.7 Hz, 1H), 8.36 (q,*J* = 4.9 Hz, 1H), 8.10 (d, *J =* 2.8 Hz, 1H), 7.82 (d, *J* = 8.8 Hz, 1H), 7.66 (d, *J =* 1.6 Hz, 1H), 7.43 (s, 1H), 7.22 (dd, *J =* 8.9, 2.9 Hz, 1H), 4.31 (q, *J* = 6.9 Hz, 2H), 3.89 (d, *J* = 14.1 Hz, 1H), 3.48 - 3.42 (m, 1H), 3.39 (d, *J =* 14.1 Hz, 1H), 3.25 - 3.17 (m, 1H), 2.79 (m, 1H), 2.77 (d, *J =* 4.9 Hz, 3H), 2.67 (p, *J =* 1.9 Hz, 1H), 2.34 - 2.20 (m, 2H), 2.17 - 2.01 (m, 2H), 1.86 - 1.79 (m, 2H), 1.00 - 0.94 (m, 2H), 0.85 - 0.78 (m, 2H).

### Example 29: cis-3-ethyl-7-((5-(6-(5-methyl-4H-1,2,4-triazol-3-yl)pyridin-3-yl)-2,5-diazabicyclo[4.2.0]octan-2-yl)methyl)-1H-1,5-naphthyridin-2-one:

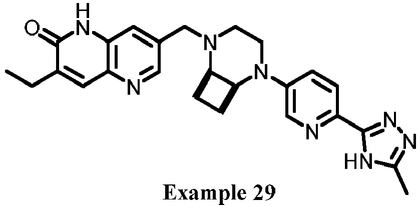

### Synthetic route:

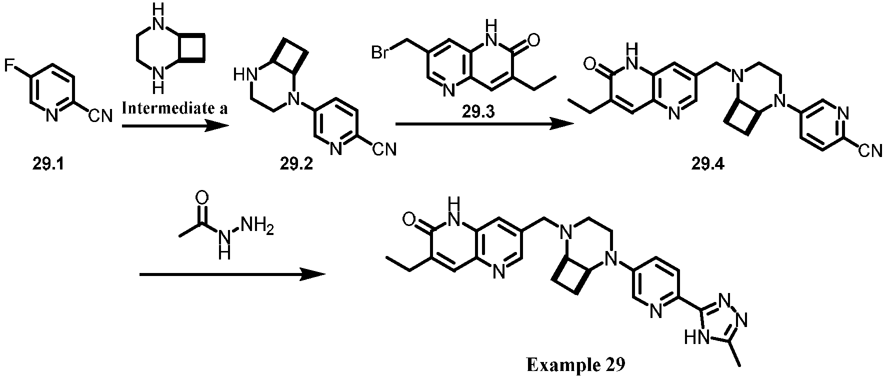

### Step 1: Synthesis of cis-5-(2,5-diazabicyclo[4.2.0]octan-2-yl)pyridine-2-carbonitrile (29.2)

To a solution of **intermediate a** (250 mg, 0.735 mmol) and 5-fluoropyridine-2-carbonitrile (90 mg, 0.735 mmol) in DMSO (15 mL) was added K₂CO₃ (508 mg, 3.675 mmol) at room temperature, and the mixture was stirred at 100°C for 24 hours. After the reaction was completed, the reaction mixture (about 20 mL) was added with dry ice (10 g), stirred for 16 hours, and cooled to room temperature. The reaction mixture was directly used for the next reaction step. LC-MS (ESI), m/z: [M+H]⁺ = 215.1.

### Step 2: Synthesis of cis-5-(5-((7-ethyl-6-oxa-5H-1,5-naphthyridin-3-yl)methyl)-2,5-diazabicyclo[4.2.0]octan-2-yl)pyridine-2-carbonitrile (29.4):

To the reaction mixture containing compound **29.2** was added a solution of compound **29.3** in DCM. The resulting reaction mixture was reacted at room temperature for 30 minutes, then quenched with 30 mL of water, and extracted with EA (30 mL × 2). The combined organic phases were dried over anhydrous sodium sulfate and concentrated under reduced pressure. The resulting residue was purified by pre-TLC to obtain the target compound **29.4** (120 mg, yield: 29%). LC-MS (ESI), m/z: [M+H]⁺ = 401.2.

### Step 3: Synthesis of cis-3-ethyl-7-((5-(6-(5-methyl-4H-1,2,4-triazol-3-yl)pyridin-3-yl)-2,5-diazabicyclo[4.2.0]octan-2-yl)methyl)-1H-1,5-naphthyridin-2-one (Example 29)

To a solution of compound **29.4** (30 mg, 0.075 mmol) in *n*-butanol (2.5 mL) were added acethydrazide (55 mg, 0.75 mmol) and K₂CO₃ (155 mg, 1.13 mmol) at room temperature, and the resulting mixture was stirred at 150°C for 6 hours. After the reaction was completed, the resulting mixture was cooled to room temperature, diluted with water (20 mL), and extracted with ethyl acetate (5 mL × 2). The combined organic phases were dried over anhydrous sodium sulfate and concentrated under reduced pressure. The resulting residue was purified by preparative liquid chromatography (chromatographic column model: Gemini 5 µm C18 150 × 21.2 mm; mobile phase: 10 to 40% acetonitrile/water (0.1% formic acid); flow rate: 20 mL/min) to obtain the title compound **Example 29** (15 mg, yield: 43%).

### Example 29-1 and Example 29-2: 3-ethyl-7-((1R,6S)-5-(6-(5-methyl-4H-1,2,4-triazol-3-yl)pyridin-3-yl)-2,5-diazabicyclo[4.2.0]octan-2-yl)methyl)-1H-1,5-naphthyridin-2-one and 3-ethyl-7-((1S,6R)-5-(6-(5-methyl-4H-1,2,4-triazol-3-yl)pyridin-3-yl)-2,5-diazabicyclo[4.2.0]octan-2-yl)methyl)-1H-1,5-naphthyridin-2-one

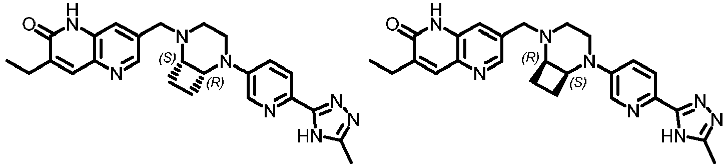

**Example 29** (15 mg, 0.033 mmol) was purified by chiral SFC chromatography (chromatographic column model: CHIRALPAK AD-H 250 mm × 20 mm, 5 µm; mobile phase: 40% isopropanol (0.2% ethylenediamine); flow rate: 40 g/min) to obtain **Example 29-1** (t_{R} = 21.7 min, 2.5 mg, yield: 17%) and **Example 29-2** (t_{R} = 31.5 min, 1.7 mg, yield: 11%).

### Example 29-1: LC-MS (ESI), m/z: [M+H]⁺ = 457.2.

¹H NMR (400 MHz, DMSO-*d₆*) δ 13.89 (s, 1H), 11.84 (s, 1H), 8.44 (d, *J =* 1.7 Hz, 1H), 8.18 (s, 1H), 7.82 (d, *J* = 8.8 Hz, 1H), 7.76 (s, 1H), 7.68 (s, 1H), 7.25 (s, 1H), 4.35 - 4.25 (m, 1H), 3.90 (d, *J =* 14.0 Hz, 1H), 3.49 - 3.37 (m, 3H), 3.26 - 3.18 (m, 1H), 2.82 - 2.76 (m, 1H), 2.58 - 2.52 (m, 2H), 2.40 - 2.30 (m, 2H), 2.30 - 2.20 (m, 3H), 2.13 - 2.03 (m, 1H), 1.88 - 1.78 (m, 2H), 1.19 (t, *J =* 7.4 Hz, 3H).

### Example 29-2: LC-MS (ESI), m/z: [M+H]⁺ = 457.2.

¹H NMR (400 MHz, DMSO-*d₆*) δ 13.89 (s, 1H), 11.84 (s, 1H), 8.44 (d, *J* = 1.7 Hz, 1H), 8.18 (s, 1H), 7.82 (d, *J =* 8.8 Hz, 1H), 7.76 (s, 1H), 7.69 (s, 1H), 7.25 (s, 1H), 4.35 - 4.25 (m, 1H), 3.90 (d, *J =* 14.0 Hz, 1H), 3.49 - 3.37 (m, 3H), 3.27 - 3.18 (m, 1H), 2.83 - 2.76 (m, 1H), 2.59 - 2.52 (m, 2H), 2.39 - 2.30 (m, 2H), 2.30 - 2.20 (m, 3H), 2.13 - 2.03 (m, 1H), 1.88 - 1.78 (m, 2H), 1.19 (t, *J =* 7.4 Hz, 3H).

### Example 30: cis-N-methyl-5-(5-((6-oxo-7-(trifluoromethyl)-5,6-dihydro-1,5-naphthyridin-3-yl)methyl)-2,5-diazabicyclo[4.2.0]octan-2-yl)picolinamide

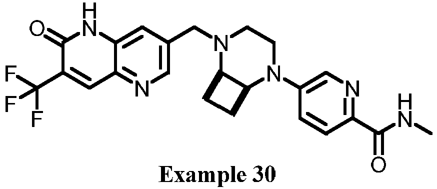

### Synthetic route:

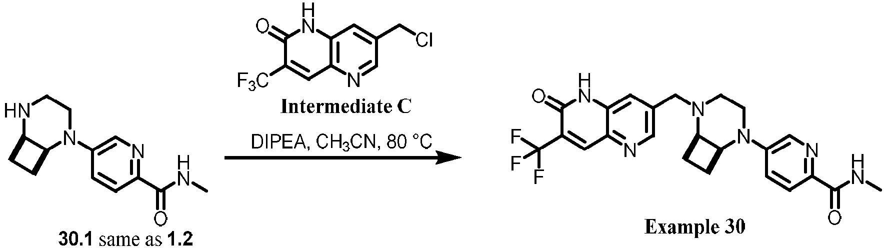

### Synthesis of cis-N-methyl-5-(5-((6-oxo-7-(trifluoromethyl)-5,6-dihydro-1,5-naphthyridin-3-yl)methyl)-2,5-diazabicyclo[4.2.0]octan-2-yl)picolinamide (Example 30):

To a solution of compound **intermediate C** (93 mg, 0.35 mmol) in acetonitrile (5 mL) were added diisopropylethylamine (137 mg, 1.06 mmol), compound **30.1** (87 mg, 0.35 mmol), and potassium iodide (5.8 mg, 0.035 mmol), and the reaction mixture was stirred at 80°C for 2 hours. After the reaction was completed, the reaction mixture was concentrated under reduced pressure, and the resulting residue was purified by preparative liquid chromatography (chromatographic column model: Gemini-C18 150 × 21.2 mm, 5 µm; mobile phase: acetonitrile/water (0.1% formic acid); gradient: 20 to 40%) to obtain the title compound **Example 30** (42 mg, yield: 22%). LC-MS (ESI), m/z: [M+H]⁺ = 473.1.

### Example 30-1 and Example 30-2: N-methyl-5-((1S,6R)-5-((6-oxo-7-(trifluoromethyl)-5,6-dihydro-1,5-naphthyridin-3-yl)methyl)-2,5-diazabicyclo[4.2.0]octan-2-yl)picolinamide and N-methyl-5-((1R,6S)-S-((6-oxo-7-(trifluoromethyl)-5,6-dihydro-1,5-naphthyridin-3-yl)methyl)-2,5-diazabicyclo[4.2.0]octan-2-yl)picolinamide

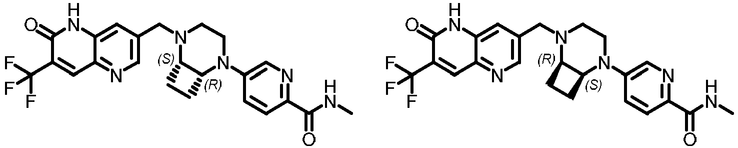

### Synthetic route:

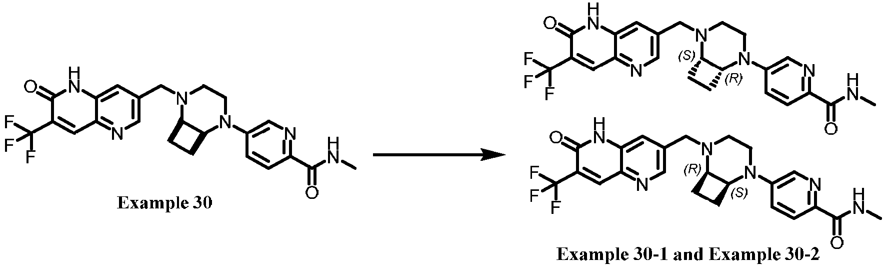

**Example 30** (42 mg) was purified by chiral column chromatography (chromatographic column model: CHIRALPAK AD-H 250 mm × 20 mm, 5 µm; mobile phase: 40% isopropanol (0.2% ammonia water); flow rate: 40 g/min) to obtain **Example 30-1** (t_{R} = 5.02 min, 10.1 mg, yield: 24%) and **Example 30-2** (t_{R} = 11.02 min, 9.50 mg, yield: 23%).

### Example 30-1: LC-MS (ESI), m/z: [M+H]⁺ = 473.2.

¹H NMR (400 MHz, DMSO-*d₆*) δ 12.46 (s, 1H), 8.61 (s, 1H), 8.42 (s, 1H), 8.37 (d, *J* = 4.8 Hz, 1H), 8.11 (d, *J =* 2.5 Hz, 1H), 7.86 - 7.78 (m, 2H), 7.23 (dd, *J =* 8.8, 2.6 Hz, 1H), 4.33 (q, *J =* 6.9 Hz, 1H), 3.96 (d, *J =* 14.7 Hz, 1H), 3.53 - 3.45 (m, 2H), 3.23 (t, *J =* 9.5 Hz, 1H), 2.80 (m, 1H), 2.78 (d, *J=* 4.7 Hz, 3H), 2.33 (t, *J =* 9.9 Hz, 1H), 2.28 - 2.22 (m, 1H), 2.09 (p, *J* = 9.0 Hz, 1H), 1.89 - 1.77 (m, 1H).

### Example 30-2: LC-MS (ESI), m/z: [M+H]⁺ = 473.2.

¹H NMR (400 MHz, DMSO-*d₆*) δ 12.46 (s, 1H), 8.61 (s, 1H), 8.42 (s, 1H), 8.37 (d, *J* = 4.8 Hz, 1H), 8.11 (d, *J =* 2.5 Hz, 1H), 7.84 - 7.78 (m, 2H), 7.23 (dd, *J =* 8.8, 2.6 Hz, 1H), 4.33 (q, *J =* 6.9 Hz, 1H), 3.96 (d, *J=* 14.7 Hz, 1H), 3.53 - 3.45 (m, 2H), 3.23 (t, *J=* 9.5 Hz, 1H), 2.80 (m, 1H), 2.78 (d, *J =* 4.7 Hz, 3H), 2.33 (t, *J =* 9.9 Hz, 1H), 2.28 - 2.22 (m, 1H), 2.15 - 2.05 (m, *J* = 9.0 Hz, 1H), 1.89 - 1.77 (m, 1H).

### Example 31: cis-N-methyl-5-(5-((6-oxo-6,8,9,10-tetrahydro-5H-pyrano[2,3-c][1,5]naphthyridin-3-yl)methyl)-2,5-diazabicyclo[4.2.0]octan-2-yl)picolinamide:

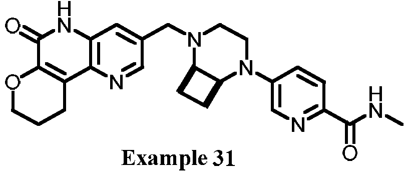

### Synthetic route:

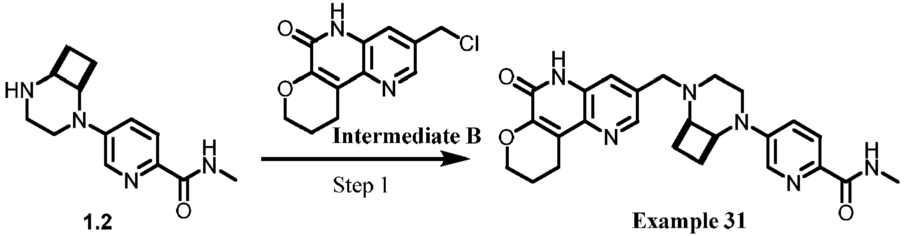

### Step 1: cis-N-methyl-5-(5-((6-oxo-6,8,9,10-tetrahydro-5H-pyrano[2,3-c][1,5]naphthyridin-3-yl)methyl)-2,5-diazabicyclo[4.2.0]octan-2-yl)picolinamide (Example 31)

To a solution of compound **1.2** (34.0 mg, 0.140 mmol) in acetonitrile (2 mL) were added *N*,*N*-diisopropylethylamine (90.0 mg, 0.700 mmol), **intermediate B** (35.0 mg, 0.140 mmol), and potassium iodide (3.00 mg, 0.0200 mmol) at room temperature, and the reaction mixture was stirred at 80°C for 3 hours. After the reaction was completed, the reaction mixture was concentrated, and the resulting residue was purified by preparative liquid chromatography (chromatographic column model: Gemini-C18 150 × 21.2 mm, 5 µm; mobile phase: acetonitrile/0.1% formic acid in water; gradient: 20 to 40%) to obtain the title compound **Example 31** (45 mg, yield: 70%). LC-MS (ESI), m/z: [M+H]⁺ = 461.2.

### Example 31-1 and Example 31-2: N-methyl-5-((1S,6R)-5-((6-oxo-6,8,9,10-tetrahydro-5H-pyrano[2,3-c][1,5]naphthyridin-3-yl)methyl)-2,5-diazabicyclo[4.2.0]octan-2-yl)picolinamide and N-methyl-5-((1R,6S)-5-((6-oxo-6,8,9,10-tetrahydro-5H-pyrano[2,3-c][1,5]naphthyridin-3-yl)methyl)-2,5-diazabicyclo[4.2.0]octan-2-yl)picolinamide

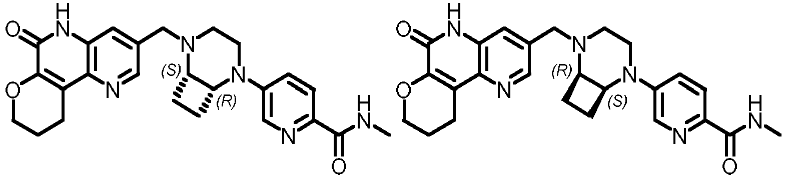

### Synthetic route:

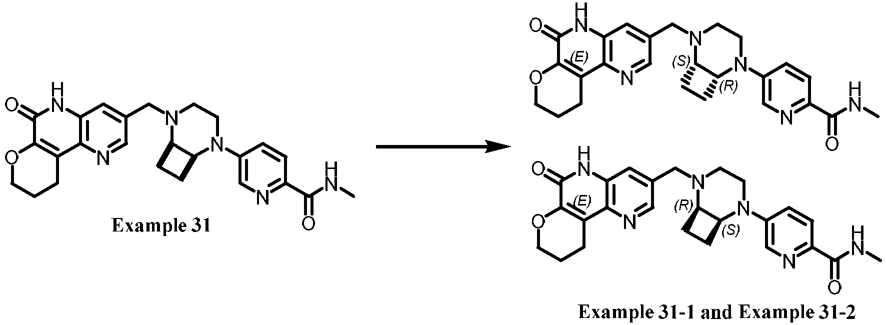

**Example 31** was separated by chiral column chromatography (chromatographic column model: CHIRALPAKAD-H 250 mm × 20 mm, 5 µm; mobile phase: 40% isopropanol (0.2% ammonia water); flow rate: 40 g/min) to obtain compound **Example 31-1** (t_{R} = 28.37 min, 17.5 mg, yield: 39%) and compound **Example 31-2** (t_{R} = 36.19 min, 17.0 mg, yield: 38%).

### Example 31-1: LC-MS (ESI), m/z: [M+H]⁺ = 461.1.

¹H NMR (400 MHz, DMSO-*d₆*) δ 11.82 (s, 1H), 8.43 (d, *J* = 1.7 Hz, 1H), 8.36 (d,*J* = 4.9 Hz, 1H), 8.10 (d, *J =* 2.8 Hz, 1H), 7.82 (d, *J =* 8.8 Hz, 1H), 7.70 (d, *J =* 1.5 Hz, 1H), 7.22 (dd, *J =* 8.9, 2.8 Hz, 1H), 4.93 (s, 2H), 4.30 (dd, *J =* 15.2, 7.1 Hz, 1H), 3.94 - 3.87 (m, 3H), 3.49 - 3.38 (m, 2H), 3.26 - 3.19 (m, 1H), 2.80 - 2.74 (m, 4H), 2.34 - 2.19 (m, 3H), 2.11 - 2.02 (m, 1H), 1.86 - 1.79 (m, 2H).

### Example 31-2: LC-MS (ESI), m/z: [M+H]⁺ = 461.1.

¹H NMR (400 MHz, DMSO-*d₆*) δ 11.82 (s, 1H), 8.42 (d, *J* = 1.7 Hz, 1H), 8.36 (d,*J* = 4.9 Hz, 1H), 8.10 (d, *J =* 2.8 Hz, 1H), 7.82 (d, *J =* 8.8 Hz, 1H), 7.70 (d, *J =* 1.5 Hz, 1H), 7.22 (dd, *J =* 8.9, 2.8 Hz, 1H), 4.93 (s, 2H), 4.30 (dd, *J =* 15.2, 7.1 Hz, 1H), 3.94 - 3.87 (m, 3H), 3.49 - 3.38 (m, 2H), 3.26 - 3.19 (m, 1H), 2.80 - 2.74 (m, 4H), 2.34 - 2.19 (m, 3H), 2.11 - 2.02 (m, 1H), 1.86 - 1.79 (m, 2H).

### Example 33:

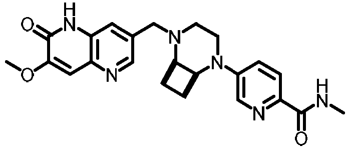

**Example 33** was obtained with reference to the synthesis of **Example 29.** LC-MS (ESI), m/z: [M+H]⁺ = 435.2.

### Example 33-1 and Example 33-2:

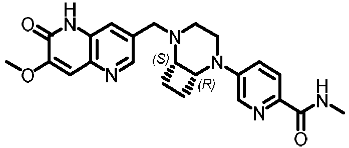

and

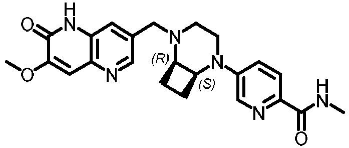

**Example 33** was separated by preparative chiral chromatography (CHIRALPAK OJ-H 250 mm × 20 mm, 5 µm; mobile phase: mobile phase A = carbon dioxide, mobile phase B = ethanol (0.2% ammonia water), A/B = 60/40; flow rate: 40 g/min) to obtain **Example 33-1** (t_{R} = 13.46) and **Example 33-2** (t_{R} = 21.0).

### Example 33-1: LC-MS (ESI), m/z: [M+H]⁺ = 435.2.

¹H NMR (400 MHz, DMSO-*d₆*) δ 11.99 (s, 1H), 8.40 (d, *J* = 1.7 Hz, 1H), 8.39-8.35 (m, 1H), 8.10 (d, *J =* 2.7 Hz, 1H), 7.82 (d, *J =* 8.8 Hz, 1H), 7.64 (d, *J =* 1.6 Hz, 1H), 7.25 - 7.16 (m, 2H), 4.31 (q, *J =* 6.9 Hz, 1H), 3.91 - 3.84 (m, 4H), 3.49 - 3.42 (m, 1H), 3.38 (d, *J =* 14.2 Hz, 1H), 3.25 - 3.17 (m, 1H), 2.80 (m, 1H), 2.77 (d, *J =* 4.8 Hz, 3H), 2.34 - 2.20 (m, 2H), 2.10 - 2.01 (m, 1H), 1.87 - 1.79 (m, 2H).

### Example 33-2: LC-MS (ESI), m/z: [M+H]⁺ = 435.2.

¹H NMR (400 MHz, DMSO-*d₆*) δ 11.99 (s, 1H), 8.40 (d, *J* = 1.7 Hz, 1H), 8.39-8.35 (m, 1H), 8.10 (d, *J =* 2.7 Hz, 1H), 7.82 (d, *J =* 8.8 Hz, 1H), 7.65 (d, *J =* 1.6 Hz, 1H), 7.24 - 7.18 (m, 2H), 4.31 (q, *J =* 6.9 Hz, 1H), 3.92 - 3.84 (m, 4H), 3.49 - 3.42 (m, 1H), 3.38 (d, *J =* 14.2 Hz, 1H), 3.25 - 3.17 (m, 1H), 2.80 (m, 1H), 2.77 (d, *J =* 4.8 Hz, 3H), 2.34 - 2.20 (m, 2H), 2.10 - 2.01 (m, 1H), 1.87 - 1.79 (m, 2H).

### Example 34:

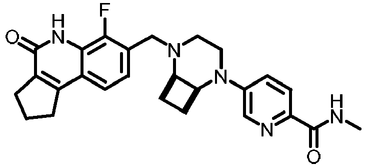

**Example 34** was obtained with reference to the synthesis of **Example 29.** LC-MS (ESI), m/z: [M+H]⁺ = 462.0.

### Example 34-1 and Example 34-2:

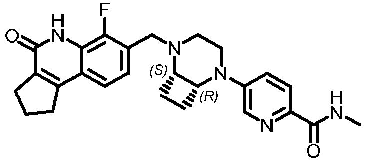

and

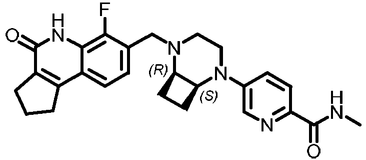

**Example 34** was separated by preparative chiral chromatography (CHIRALPAK OJ-H 250 mm × 20 mm, 5 µm; mobile phase: mobile phase A = carbon dioxide, mobile phase B = methanol (0.2% ammonia water), A/B = 60/40; flow rate: 40 g/min) to obtain **Example 34-1** (t_{R} = 10.8) and **Example 34-2** (t_{R} = 18.96).

### Example 34-1: LC-MS (ESI), m/z: [M+H]⁺ = 462.0.

¹H NMR (400 MHz, DMSO-*d₆*) δ 11.51 (s, 1H), 8.35 (d, *J* = 5.1 Hz, 1H), 8.09 (d,*J* = 2.9 Hz, 1H), 7.81 (d, *J* = 8.8 Hz, 1H), 7.36 (d, *J =* 8.1 Hz, 1H), 7.25 (t, *J* = 7.3 Hz, 1H), 7.20 (dd, *J =* 8.9, 2.9 Hz, 1H), 4.30 (q, *J =* 7.2 Hz, 1H), 3.85 (d, *J =* 13.6 Hz, 1H), 3.55 (d, *J =* 13.5 Hz, 1H), 3.50 - 3.43 (m, 1H), 3.21 - 3.13 (m, 1H), 3.10 (t, *J =* 7.6 Hz, 2H), 2.86 - 2.80 (m, 2H), 2.77 (d, *J =* 4.6 Hz, 3H), 2.35 - 2.17 (m, 2H), 2.16 - 1.98 (m, 4H), 1.89 - 1.77 (m, 2H).

### Example 34-2: LC-MS (ESI), m/z: [M+H]⁺ = 462.0.

¹H NMR (400 MHz, DMSO-*d₆*) δ 11.52 (s, 1H), 8.35 (d, *J =* 5.1 Hz, 1H), 8.09 (d,*J* = 2.9 Hz, 1H), 7.81 (d, *J =* 8.8 Hz, 1H), 7.36 (d, *J =* 8.1 Hz, 1H), 7.23 (t, *J =* 7.3 Hz, 1H), 7.20 (dd, *J =* 8.9, 2.9 Hz, 1H), 4.30 (q, *J =* 7.2 Hz, 1H), 3.85 (d, *J =* 13.6 Hz, 1H), 3.55 (d, *J =* 13.5 Hz, 1H), 3.50 - 3.47 (m, 1H), 3.20 - 3.13 (m, 1H), 3.10 (t, *J =* 7.6 Hz, 2H), 2.86 - 2.80 (m, 2H), 2.77 (d, *J =* 4.6 Hz, 3H), 2.35 - 2.17 (m, 2H), 2.16 - 1.98 (m, 4H), 1.89 - 1.75 (m, 2H).

### Example 35:

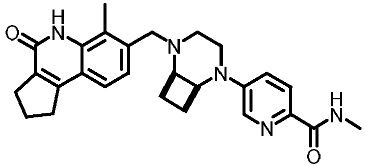

**Example 35** was obtained with reference to the synthesis of **Example 29.** LC-MS (ESI), m/z: [M+H]⁺ = 457.5.

### Example 35-1 and Example 35-2:

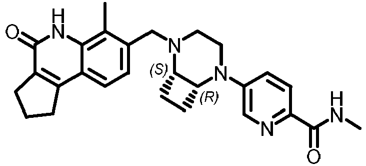

and

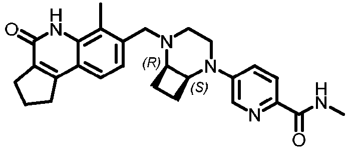

**Example 35** was separated by preparative chiral chromatography (CHIRALPAK OJ-H 250 mm × 20 mm, 5 µm; mobile phase: mobile phase A = carbon dioxide, mobile phase B = methanol (0.2% ammonia water), A/B = 60/40; flow rate: 40 g/min) to obtain **Example 35-1** (t_{R} = 6.09) and **Example 35-2** (t_{R} = 11.55).

### Example 35-1: LC-MS (ESI), m/z: [M+H]⁺ = 457.5.

¹H NMR (400 MHz, DMSO-*d₆*) δ 10.63 (s, 1H), 8.36 (q, *J =* 4.6 Hz, 1H), 8.08 (d,*J* = 2.7 Hz, 1H), 7.81 (d, *J =* 8.8 Hz, 1H), 7.38 (d, *J =* 8.0 Hz, 1H), 7.20 (dd, *J =* 8.5, 3.0 Hz, 2H), 4.31 (q, *J =* 7.1 Hz, 1H), 3.91 (d, *J =* 13.1 Hz, 1H), 3.44 - 3.39 (m, 1H), 3.31 - 3.24 (m, 2H), 3.13 - 3.06 (m, 3H), 2.78 (t, *J =* 7.4 Hz, 2H), 2.77 (d, *J =* 4.8 Hz, 3H), 2.73 - 2.66 (m, 1H), 2.28 - 2.17 (m, 2H), 2.16 - 2.07 (m, 2H), 2.07 - 1.99 (m, 1H), 1.87 - 1.78 (m, 2H).

### Example 35-2: LC-MS (ESI), m/z: [M+H]⁺ = 457.5.

¹H NMR (400 MHz, DMSO-*d₆*) δ 10.62 (s, 1H), 8.36 (q, *J =* 4.6 Hz, 1H), 8.09 (d,*J* = 2.7 Hz, 1H), 7.81 (d, *J =* 8.8 Hz, 1H), 7.37 (d, *J =* 8.0 Hz, 1H), 7.20 (dd, *J =* 8.5, 3.0 Hz, 2H), 4.33 (q, *J* = 7.1 Hz, 1H), 3.91 (d, *J =* 13.1 Hz, 1H), 3.44 - 3.39 (m, 1H), 3.31 - 3.24 (m, 2H), 3.13 - 3.06 (m, 3H), 2.78 (t, *J =* 7.4 Hz, 2H), 2.77 (d, *J =* 4.8 Hz, 3H), 2.73 - 2.66 (m, 1H), 2.28 - 2.17 (m, 2H), 2.16 - 2.07 (m, 2H), 2.07 - 1.99 (m, 1H), 1.87 - 1.79 (m, 2H).

### Example 36:

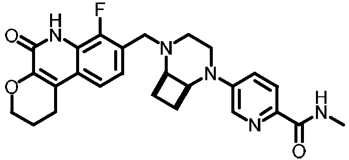

**Example 36** was obtained with reference to the synthesis of **Example 29.** LC-MS (ESI), m/z: [M+H]⁺ = 478.2.

### Example 36-1 and Example 36-2:

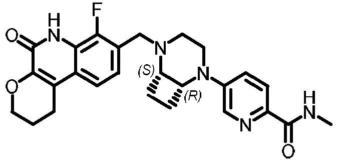

and

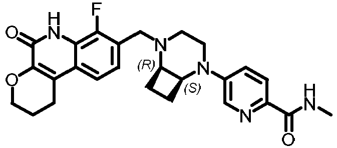

**Example 36** was separated by preparative chiral chromatography (CHIRALPAK OJ-H 250 mm × 20 mm, 5 µm; mobile phase: mobile phase A = carbon dioxide, mobile phase B = ethanol (0.2% ammonia water), A/B = 60/40; flow rate: 40 g/min) to obtain **Example 36-1** (t_{R} = 12.6) and **Example 36-2** (t_{R} = 11.55).

### Example 36-1: LC-MS (ESI), m/z: [M+H]⁺ = 478.2.

¹H NMR (400 MHz, DMSO-*d₆*) δ 11.76 (s, 1H), 8.35 (d, *J* = 5.0 Hz, 1H), 8.09 (d,*J* = 2.6 Hz, 1H), 7.81 (d, *J =* 8.8 Hz, 1H), 7.32 (d, *J =* 8.3 Hz, 1H), 7.25 (d, *J =* 6.6 Hz, 1H), 7.20 (dd, *J =* 9.1, 2.9 Hz, 1H), 4.87 (s, 2H), 4.31 (q, *J =* 7.0 Hz, 1H), 3.89 (t, *J =* 5.5 Hz, 2H), 3.84 (d, *J =* 13.9 Hz, 1H), 3.55 (d, *J =* 13.6 Hz, 1H), 3.47 (d, *J =* 11.7 Hz, 1H), 3.30 - 3.26 (m, 1H), 3.21 - 3.12 (m, 1H), 2.82 (d, *J =* 12.0 Hz, 1H), 2.77 (d, *J* = 4.8 Hz, 3H), 2.34 - 2.17 (m, 3H), 2.10 - 1.98 (m, 2H), 1.86 - 1.79 (m, 2H).

### Example 36-2: LC-MS (ESI), m/z: [M+H]⁺ = 478.2.

¹H NMR (400 MHz, DMSO-*d₆*) δ 11.77 (s, 1H), 8.35 (d, *J* = 5.0 Hz, 1H), 8.12 (d,*J* = 2.6 Hz, 1H), 7.81 (d, *J =* 8.8 Hz, 1H), 7.33 (d, *J =* 8.3 Hz, 1H), 7.25 (d, *J =* 6.6 Hz, 1H), 7.20 (dd, *J =* 9.1, 2.9 Hz, 1H), 4.86 (s, 2H), 4.31 (q, *J =* 7.0 Hz, 1H), 3.89 (t, *J =* 5.5 Hz, 2H), 3.84 (d, *J =* 13.9 Hz, 1H), 3.55 (d, *J =* 13.6 Hz, 1H), 3.47 (d, *J =* 11.7 Hz, 1H), 3.30 - 3.26 (m, 1H), 3.21 - 3.12 (m, 1H), 2.82 (d, *J =* 12.0 Hz, 1H), 2.78 (d, *J =* 4.8 Hz, 3H), 2.34 - 2.17 (m, 3H), 2.11 - 1.98 (m, 2H), 1.86 - 1.80 (m, 2H).

### Example 37:

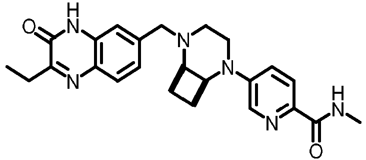

**Example 37** was obtained with reference to the synthesis of **Example 11.** LC-MS (ESI), m/z: [M+H]⁺ = 433.1.

### Example 37-1 and Example 37-2:

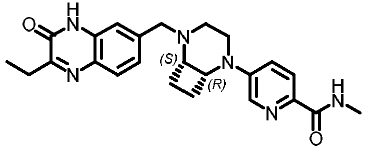

and

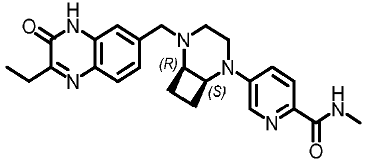

**Example 37** was separated by preparative chiral chromatography (CHIRALPAK OJ-H 250 mm × 20 mm, 5 µm; mobile phase: mobile phase A = carbon dioxide, mobile phase B = ethanol (0.2% ammonia water), A/B = 60/40; flow rate: 40 g/min) to obtain **Example 37-1** (t_{R} = 7.81) and **Example 37-2** (t_{R} = 18.4).

### Example 37-1: LC-MS (ESI), m/z: [M+H]⁺ = 433.1.

¹H NMR (400 MHz, MeOH-*d₄*) δ 8.16 (d, *J =* 2.8 Hz, 1H), 7.90 (d, *J =* 8.8 Hz, 1H), 7.78 (d, *J =* 8.1 Hz, 1H), 7.43 - 7.38 (m, 2H), 7.23 (dd, *J =* 8.9, 2.9 Hz, 1H), 4.35 (q, *J =* 6.8 Hz, 1H), 3.98 (d, *J =* 13.9 Hz, 1H), 3.52 - 3.47 (m, 1H), 3.44 - 3.38 (m, 2H), 2.95 (s, 3H), 2.94 - 2.89 (m, 2H), 2.42 - 2.21 (m, 4H), 1.99 - 1.93 (m, 2H), 1.34 (t, *J =* 7.4 Hz, 3H).

### Example 37-2: LC-MS (ESI), m/z: [M+H]⁺ = 433.1.

¹H NMR (400 MHz, MeOH-*d₄*) δ 8.16 (d, *J =* 2.8 Hz, 1H), 7.90 (d, *J =* 8.8 Hz, 1H), 7.78 (d, *J =* 8.1 Hz, 1H), 7.43 - 7.38 (m, 2H), 7.23 (dd, *J =* 8.9, 2.9 Hz, 1H), 4.35 (q, *J =* 6.8 Hz, 1H), 3.98 (d, *J =* 13.9 Hz, 1H), 3.52 - 3.47 (m, 1H), 3.44 - 3.38 (m, 2H), 2.95 (s, 3H), 2.94 - 2.89 (m, 2H), 2.42 - 2.21 (m, 4H), 1.99 - 1.93 (m, 2H), 1.34 (t, *J =* 7.4 Hz, 3H).

### Example 38:

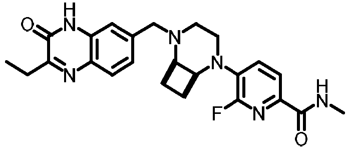

**Example 38** was obtained with reference to the synthesis of **Example 11.** LC-MS (ESI), m/z: [M+H]⁺ = 451.2.

### Example 38-1 and Example 38-2:

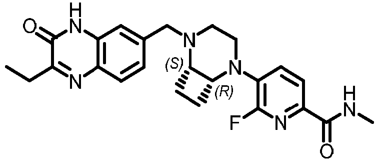

and

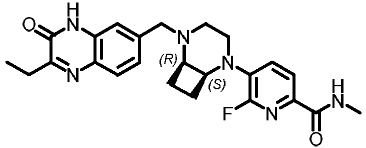

**Example 38** was separated by preparative chiral chromatography (OJ-H 20 × 250 mm, 10 µm; mobile phase: mobile phase A = *n*-hexane, mobile phase B = ethanol (0.2% ammonia water), A/B = 50/50; flow rate: 0.8 mL/min; A/B = 60/40; flow rate: 40 g/min) to obtain **Example 38-1** (t_{R} = 6.93) and **Example 38-2** (t_{R} = 8.84).

### Example 38-1: LC-MS (ESI), m/z: [M+H]⁺ = 451.2.

¹H NMR(400 MHz, DMSO-*d₆*) δ 12.25 (s, 1H), 8.37 (s, 1H), 7.81 (d, *J =* 7.9 Hz, 1H), 7.70 (d, *J =* 7.2 Hz, 1H), 7.53 - 7.15 (m, 3H), 4.19 (m, 1H), 3.79 (d, *J =* 12.6 Hz, 2), 3.49 - 3.39 (m, 3H), 3.22 - 3.13 (m, 1H), 2.86 - 2.77 (m, 2H), 2.76 (d, *J =* 4.8 Hz, 3H), 2.44 - 2.34 (m, 1H), 2.12 - 2.01 (m, 1H), 1.99 - 1.90 (m, 1H), 1.89 - 1.79 (m, 1H), 1.77 - 1.68 (m, 1H), 1.22 (t, *J* = 7.4 Hz, 3H).

### Example 38-2: LC-MS (ESI), m/z: [M+H]⁺ = 451.2.

¹H NMR(400 MHz, DMSO-*d₆*) δ 12.25 (s, 1H), 8.36 (s, 1H), 7.81 (d, *J =* 7.9 Hz, 1H), 7.69 (d, *J =* 7.2 Hz, 1H), 7.44 - 7.26 (m, 3H), 4.19 (m, 1H), 3.79 (d, *J =* 12.6 Hz, 2), 3.49 - 3.39 (m, 3H), 3.22 - 3.13 (m, 1H), 2.86 - 2.77 (m, 2H), 2.76 (d, *J =* 4.8 Hz, 3H), 2.44 - 2.34 (m, 1H), 2.12 - 2.01 (m, 1H), 1.99 - 1.90 (m, 1H), 1.89 - 1.79 (m, 1H), 1.77 - 1.68 (m, 1H), 1.21 (t, *J* = 7.4 Hz, 3H).

### Example 39:

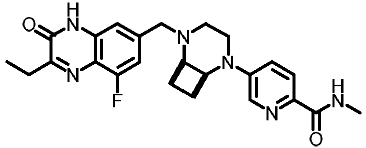

**Example 39** was obtained with reference to the synthesis of **Example 11.** LC-MS (ESI), m/z: [M+H]⁺ = 451.0.

### Example 39-1 and Example 39-2:

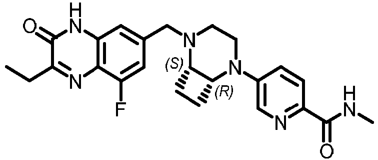

and

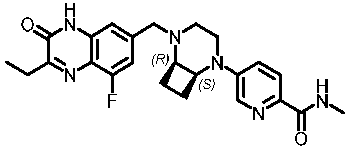

**Example 39** was separated by preparative chiral chromatography (CHIRALPAK OJ-H 250 mm × 20 mm, 5 µm; mobile phase: mobile phase A = carbon dioxide, mobile phase B = ethanol (0.2% ammonia water), A/B = 60/40; flow rate: 40 g/min) to obtain **Example 39-1** (t_{R} = 7.91) and **Example 39-2** (t_{R} = 14.8).

### Example 39-1: LC-MS (ESI), m/z: [M+H]⁺ = 451.0.

¹H NMR (400 MHz, DMSO-*d₆*) δ 12.41 (s, 1H), 8.37 (q, *J* = 4.7 Hz, 1H), 8.11 (d, *J =* 2.6 Hz, 1H), 7.82 (d, *J =* 8.8 Hz, 1H), 7.23 (dd, *J =* 8.9, 2.7 Hz, 1H), 7.18 - 7.10 (m, 2H), 4.32 (q, *J =* 6.9 Hz, 1H), 3.86 (d, *J =* 14.3 Hz, 1H), 3.47 (d, *J =* 11.7 Hz, 1H), 3.35 (m, 2H), 3.28 - 3.19 (m, 1H), 2.85 - 2.76 (m, 5H), 2.34 - 2.20 (m, 2H), 2.10 (p, *J =* 9.2 Hz, 1H), 1.89 - 1.75 (m, 2H), 1.22 (t, *J =* 7.4 Hz, 3H).

### Example 39-2: LC-MS (ESI), m/z: [M+H]⁺ = 451.0.

¹H NMR (400 MHz, DMSO-*d₆*) δ 12.41 (s, 1H), 8.37 (q, *J* = 4.7 Hz, 1H), 8.11 (d, *J =* 2.6 Hz, 1H), 7.82 (d, *J =* 8.8 Hz, 1H), 7.23 (dd, *J =* 8.9, 2.7 Hz, 1H), 7.18 - 7.09 (m, 2H), 4.32 (q, *J =* 6.9 Hz, 1H), 3.86 (d, *J =* 14.3 Hz, 1H), 3.47 (d, *J =* 11.7 Hz, 1H), 3.35 (m, 2H), 3.28 - 3.19 (m, 1H), 2.85 - 2.74 (m, 5H), 2.34 - 2.20 (m, 2H), 2.10 (p, *J =* 9.2 Hz, 1H), 1.89 - 1.75 (m, 2H), 1.22 (t, *J =* 7.4 Hz, 3H).

### Example 40:

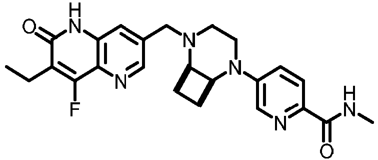

**Example 40** was obtained with reference to the synthesis of **Example 11.** LC-MS (ESI), m/z: [M+H]⁺ = 451.2.

### Example 40-1 and Example 40-2:

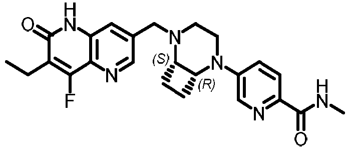

and

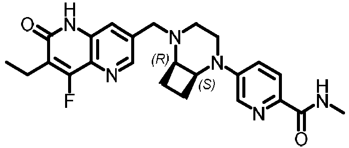

**Example 40** was separated by preparative chiral chromatography (CHIRALPAK OJ-H 250 mm × 20 mm, 5 µm; mobile phase: mobile phase A = carbon dioxide, mobile phase B = isopropanol (0.2% ammonia water), A/B = 60/40; flow rate: 40 g/min) to obtain **Example 40-1** (t_{R} = 6.91) and **Example 40-2** (t_{R} = 18.6).

### Example 40-1: LC-MS (ESI), m/z: [M+H]⁺ = 451.2.

¹H NMR (400 MHz, DMSO-*d₆*) δ 11.99 (s, 1H), 8.51 (d, *J* = 1.6 Hz, 1H), 8.37 (q,*J* = 4.7 Hz, 1H), 8.11 (d, *J =* 2.7 Hz, 1H), 7.82 (d, *J =* 8.8 Hz, 1H), 7.76 (s, 1H), 7.23 (dd, *J* = 8.9, 2.9 Hz, 1H), 4.32 (q, *J =* 6.9 Hz, 1H), 3.93 (d, *J =* 14.4 Hz, 1H), 3.49 - 3.41 (m, 2H), 3.22 (td, *J =* 11.2, 3.2 Hz, 2H), 2.79 (m, 1H), 2.77 (d, *J =* 4.8 Hz, 4H), 2.59 (q, *J =* 7.7 Hz, 2H), 2.35 - 2.21 (m, 2H), 2.08 (p, *J =* 9.0 Hz, 1H), 1.86 - 1.79 (m, 2H), 1.12 (t, *J =* 7.5 Hz, 3H).

### Example 40-2: LC-MS (ESI), m/z: [M+H]⁺ = 451.2.

¹H NMR (400 MHz, DMSO-*d₆*) δ 11.98 (s, 1H), 8.51 (d, *J* = 1.6 Hz, 1H), 8.37 (q,*J* = 4.7 Hz, 1H), 8.11 (d, *J =* 2.7 Hz, 1H), 7.82 (d, *J =* 8.8 Hz, 1H), 7.76 (s, 1H), 7.23 (dd, *J* = 8.9, 2.9 Hz, 1H), 4.32 (q, *J =* 6.9 Hz, 1H), 3.93 (d, *J =* 14.4 Hz, 1H), 3.49 - 3.41 (m, 2H), 3.22 (td, *J =* 11.2, 3.2 Hz, 2H), 2.80 (m, 1H), 2.77 (d, *J =* 4.8 Hz, 4H), 2.59 (q, *J =* 7.7 Hz, 2H), 2.35 - 2.21 (m, 2H), 2.08 (p, *J* = 9.0 Hz, 1H), 1.86 - 1.79 (m, 2H), 1.12 (t, *J =* 7.5 Hz, 3H).

### Example 41:

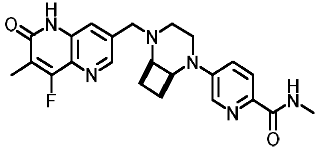

**Example 41** was obtained with reference to the synthesis of **Example 11.** LC-MS (ESI), m/z: [M+H]⁺ = 437.2.

### Example 41-1 and Example 41-2:

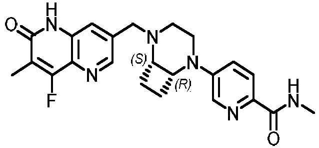

and

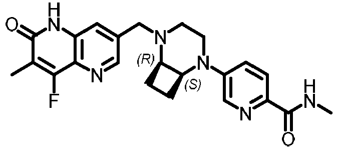

**Example 41** was separated by preparative chiral chromatography (chromatographic column model: OD (AB50)-50 min. 1 cm, mobile phase: 40% ethanol (0.2% ammonia water), flow rate: 40 g/min) to obtain **Example 41-1** (t_{R} = 11.9) and **Example 41-2** (t_{R} = 15.7).

### Example 41-1: LC-MS (ESI), m/z: [M+H]⁺ = 437.2.

¹H NMR (400 MHz, DMSO-*d₆*) δ 12.00 (s, 1H), 8.51 (d, *J =* 1.5 Hz, 1H), 8.37 (q,*J* = 4.8 Hz, 1H), 8.11 (d, *J =* 2.7 Hz, 1H), 7.82 (d, *J =* 8.8 Hz, 1H), 7.76 (s, 1H), 7.23 (dd, *J =* 8.9, 2.8 Hz, 1H), 4.32 (q, *J =* 6.9 Hz, 1H), 3.93 (d, *J =* 14.3 Hz, 1H), 3.49 - 3.41 (m, 2H), 3.27 - 3.18 (m, 1H), 2.80 (s, 1H), 2.77 (d, *J =* 4.8 Hz, 3H), 2.36 - 2.22 (m, 2H), 2.12 - 2.04 (m, 4H), 1.86 - 1.81 (m, 2H).

### Example 41-2: LC-MS (ESI), m/z: [M+H]⁺ = 437.2.

¹H NMR (400 MHz, DMSO-*d₆*) δ 12.00 (s, 1H), 8.50 (d, *J* = 1.5 Hz, 1H), 8.37 (q, *J* = 4.8 Hz, 1H), 8.11 (d, *J =* 2.8 Hz, 1H), 7.82 (d, *J =* 8.8 Hz, 1H), 7.76 (s, 1H), 7.23 (dd, *J =* 8.9, 2.8 Hz, 1H), 4.32 (q, *J =* 7.0 Hz, 1H), 3.93 (d, *J =* 14.3 Hz, 1H), 3.49 - 3.42 (m, 2H), 3.27 - 3.18 (m, 1H), 2.80 (s, 1H), 2.77 (d, *J =* 4.8 Hz, 3H), 2.36 - 2.22 (m, 2H), 2.11 - 2.04 (m, 4H), 1.86 - 1.81 (m, 2H).

### Example 42:

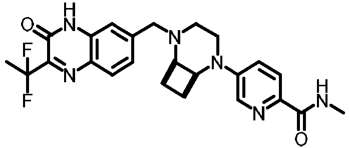

**Example 42** was obtained with reference to the synthesis of **Example 11.** LC-MS (ESI), m/z: [M+H]⁺ = 469.0.

### Example 42-1 and Example 42-2:

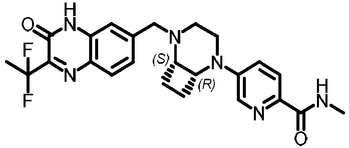

and

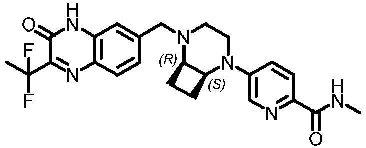

**Example 42** was separated by preparative chiral chromatography (CHIRALPAK OJ-H 250 mm × 20 mm, 5 µm; mobile phase: mobile phase A = carbon dioxide, mobile phase B = ethanol (0.2% ammonia water), A/B = 60/40; flow rate: 40 g/min) to obtain **Example 42-1** (t_{R} = 6.57) and **Example 42-2** (t_{R} = 18.3).

### Example 42-1: LC-MS (ESI), m/z: [M+H]⁺ = 469.0.

¹H NMR (400 MHz, MeOH-*d₄*) δ 8.12 (d, *J =* 2.8 Hz, 1H), 7.85 (t, *J =* 8.4 Hz, 2H), 7.45 - 7.42 (m, 2H), 7.19 (dd, *J =* 8.9, 2.9 Hz, 1H), 4.32 (q, *J =* 6.8 Hz, 1H), 3.97 (d, *J =* 14.3 Hz, 1H), 3.49 - 3.31 (m, 4H), 2.91 - 2.85 (m, 4H), 2.39 - 2.20 (m, 3H), 2.05 (t, *J =* 19.0 Hz, 3H), 1.95 - 1.88 (m, 2H).

### Example 42-2: LC-MS (ESI), m/z: [M+H]⁺ = 469.0.

¹H NMR (400 MHz, MeOH-*d₄*) δ 8.12 (d, *J =* 2.8 Hz, 1H), 7.85 (t, *J =* 8.4 Hz, 2H), 7.45 - 7.42 (m, 2H), 7.19 (dd, *J =* 8.9, 2.9 Hz, 1H), 4.32 (q, *J =* 6.8 Hz, 1H), 3.97 (d, *J =* 14.3 Hz, 1H), 3.48 - 3.31 (m, 4H), 2.91 - 2.85 (m, 4H), 2.39 - 2.20 (m, 3H), 2.05 (t, *J =* 19.0 Hz, 3H), 1.95 - 1.88 (m, 2H).

### Example 43:

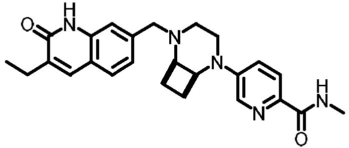

**Example 43** was obtained with reference to the synthesis of **Example 11.** LC-MS (ESI), m/z: [M+H]⁺ = 432.3.

### Example 43-1 and Example 43-2:

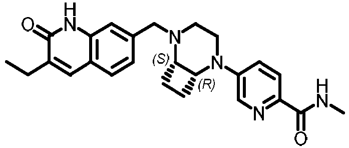

and

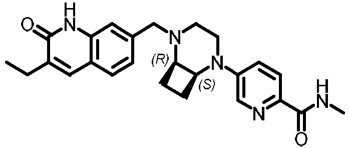

**Example 43** was separated by preparative chiral chromatography (CHIRALPAK OJ-H 250 mm × 20 mm, 5 µm; mobile phase: mobile phase A = carbon dioxide, mobile phase B = ethanol (0.2% ammonia water), A/B = 60/40; flow rate: 40 g/min) to obtain **Example 43-1** (t_{R} = 3.86) and **Example 43-2** (t_{R} = 7.58).

### Example 43-1: LC-MS (ESI), m/z: [M+H]⁺ = 432.3.

¹H NMR (400 MHz, MeOH-*d₄*) δ 8.14 (d, *J =* 2.8 Hz, 1H), 7.88 (d, *J =* 8.8 Hz, 1H), 7.78 (s, 1H), 7.61 (d, *J =* 8.1 Hz, 1H), 7.42 (s, 1H), 7.29 (d, *J =* 8.0 Hz, 1H), 7.20 (dd, *J* = 8.9, 2.9 Hz, 1H), 4.32 (q, *J =* 6.9 Hz, 1H), 3.96 (d, *J =* 13.8 Hz, 1H), 3.50 - 3.44 (m, 1H), 3.42 - 3.33 (m, 3H), 2.93 (s, 3H), 2.90 (m, 1H), 2.62 (q, *J =* 7.5 Hz, 1H), 2.39 - 2.18 (m, 3H), 1.97 - 1.91 (m, 2H), 1.26 (t, *J =* 7.5 Hz, 1H).

### Example 43-2: LC-MS (ESI), m/z: [M+H]⁺ = 432.3.

¹H NMR (400 MHz, MeOH-*d₄*) δ 8.15 (d, *J =* 2.8 Hz, 1H), 7.90 (d, *J =* 8.8 Hz, 1H), 7.78 (s, 1H), 7.63 (d, *J =* 8.1 Hz, 1H), 7.42 (s, 1H), 7.29 (d, *J =* 8.0 Hz, 1H), 7.21 (dd, *J* = 8.9, 2.9 Hz, 1H), 4.33 (q, *J =* 6.9 Hz, 1H), 3.96 (d, *J =* 13.8 Hz, 1H), 3.50 - 3.44 (m, 1H), 3.42 - 3.33 (m, 3H), 2.93 (s, 3H), 2.90 (m, 1H), 2.62 (q, *J =* 7.5 Hz, 1H), 2.39 - 2.18 (m, 3H), 1.98 - 1.94 (m, 2H), 1.26 (t, *J =* 7.5 Hz, 1H).

### Example 44:

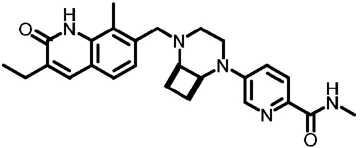

**Example 44** was obtained with reference to the synthesis of **Example 1.** LC-MS (ESI), m/z: [M+H]⁺ = 446.3.

### Example 44-1 and Example 44-2:

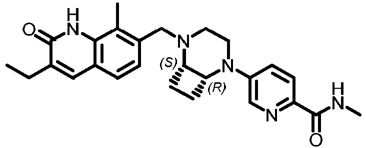

**and**

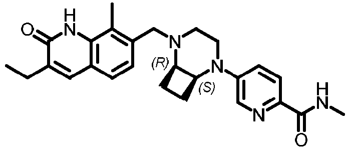

**Example 44** was separated by preparative chiral chromatography (CHIRALPAK OJ-H 250 mm × 20 mm, 5 µm; mobile phase: mobile phase A = carbon dioxide, mobile phase B = methanol (0.2% ammonia water), A/B = 60/40; flow rate: 40 g/min) to obtain **Example 44-1** (t_{R} = 5.7) and **Example 44-2** (t_{R} = 11.2).

### Example 44-1: LC-MS (ESI), m/z: [M+H]⁺ = 446.3.

¹H NMR (400 MHz, DMSO-*d₆*) δ 10.80 (s, 1H), 8.36 (q, *J =* 4.6 Hz, 1H), 8.08 (d,*J* = 2.7 Hz, 1H), 7.81 (d, *J* = 8.8 Hz, 1H), 7.70 (s, 1H), 7.45 (d, *J* = 8.0 Hz, 1H), 7.20 (dd, *J* = 8.9, 2.8 Hz, 1H), 7.17 (d, *J =* 8.0 Hz, 1H), 4.31 (q, *J* = 6.9 Hz, 1H), 3.90 (d, *J =* 13.2 Hz, 1H), 3.42 (d, *J* = 11.7 Hz, 2H), 3.33 (d, *J* = 6.2 Hz, 1H), 3.30 - 3.26 (m, 1H), 3.12 (td, *J =* 11.3, 3.2 Hz, 1H), 2.77 (d, *J =* 4.8 Hz, 3H), 2.74 - 2.66 (m, 1H), 2.56 - 2.52 (m, 2H), 2.47 (s, 3H), 2.26 - 2.17 (m, 2H), 2.07 - 1.99 (m, 1H), 1.88 - 1.78 (m, 2H), 1.18 (t, *J =* 7.4 Hz, 3H).

### Example 44-2: LC-MS (ESI), m/z: [M+H]⁺ = 446.3.

¹H NMR (400 MHz, DMSO-*d₆*) δ 10.80 (s, 1H), 8.36 (d, *J =* 4.6 Hz, 1H), 8.08 (s, 1H), 7.81 (d, *J =* 8.8 Hz, 1H), 7.70 (s, 1H), 7.45 (d, *J =* 8.0 Hz, 1H), 7.22 - 7.14 (m, 2H), 4.31 (q, *J* = 6.9 Hz, 1H), 3.90 (d, *J =* 13.2 Hz, 1H), 3.42 (d, *J =* 11.7 Hz, 2H), 3.33 (d, *J =* 6.2 Hz, 1H), 3.30 - 3.26 (m, 1H), 3.12 (td, *J =* 11.3, 3.2 Hz, 1H), 2.77 (d, *J =* 4.8 Hz, 3H), 2.74 - 2.66 (m, 1H), 2.56 - 2.52 (m, 2H), 2.47 (s, 3H), 2.26 - 2.17 (m, 2H), 2.07 - 1.99 (m, 1H), 1.88 - 1.78 (m, 2H), 1.19 (t, *J =* 7.4 Hz, 3H).

### Example 45:

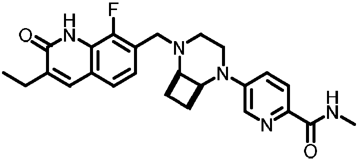

**Example 45** was obtained with reference to the synthesis of **Example 2.** LC-MS (ESI), m/z: [M+H]⁺ = 450.2.

¹H NMR (400 MHz, DMSO-*d₆*) δ 11.71 (s, 1H), 8.36 (q, *J* = 4.6 Hz, 1H), 8.09 (d,*J* = 2.7 Hz, 1H), 7.80 (d, *J =* 8.8 Hz, 1H), 7.76 (s, 1H), 7.44 (d, *J =* 8.1 Hz, 1H), 7.26 - 7.17 (m, 2H), 4.31 (q, *J =* 6.7 Hz, 1H), 3.83 (d, *J =* 13.7 Hz, 1H), 3.53 (d, *J =* 13.4 Hz, 1H), 3.49 - 3.43 (m, 1H), 3.30 - 3.25 (m, 1H), 3.21 - 3.13 (m, 1H), 2.85 - 2.79 (m, 1H), 2.77 (d, *J* = 4.8 Hz, 3H), 2.55 - 2.52 (m, 2H), 2.33 - 2.19 (m, 2H), 2.09 - 1.99 (m, 1H), 1.86 - 1.79 (m, 2H), 1.17 (t, *J* = 7.4 Hz, 3H).

### Example 46: cis-N-cyclopropyl-5-(5-((7-ethyl-6-oxo-5,6-dihydro-1,5-naphthyridin-3-yl)methyl)-2,5-diazabicyclo[4.2.0]octan-2-yl)pyridine-2-carboxamide

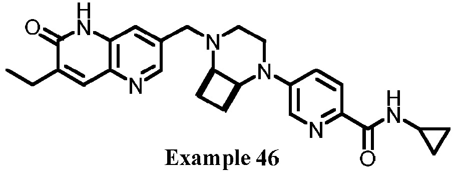

### Synthetic route:

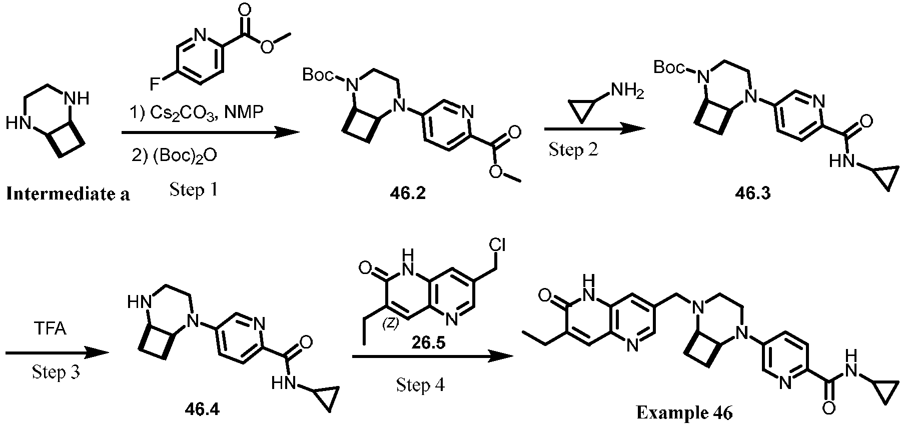

### Step 1: Synthesis of tert-butyl cis-5-(6-(methoxycarbonyl)pyridin-3-yl)-2,5-diazabicyclo[4.2.0]octane-2-carboxylate (46.2)

To a solution of methyl 5-bromopyridine-2-carboxylate (248 mg, 1.15 mmol) in NMP (20 mL) were added *cis*-2,5-diazabicyclo[4.2.0]octane dihydrochloride (425 mg, 2.30 mmol), cesium carbonate (3.0 g, 9.2 mmol), tris(dibenzylideneacetone)dipalladium (105 mg, 0.115 mmol), and 2,2'-bis(diphenylphosphino)-1,1'-binaphthyl (144 mg, 0.230 mmol), and the mixture was stirred at 85°C for 24 hours. After the reaction was completed, the reaction mixture (about 20 mL) was added with dry ice (10 g), stirred for 30 minutes, and then added with tetrahydrofuran (20 mL), saturated sodium bicarbonate aqueous solution (20 mL), and di-*tert*-butyl dicarbonate (1 mL). The resulting mixture was stirred at room temperature overnight. After the reaction was completed, the reaction mixture was concentrated under reduced pressure and extracted (petroleum ether/ethyl acetate = 1/1, 100 mL × 2). The combined organic phases were washed with saturated brine, dried over anhydrous sodium sulfate, filtered to remove the solid, and the filtrate was concentrated under reduced pressure. The resulting residue was purified by silica gel column chromatography (dichloromethane/methanol = 97/3) to obtain the crude product of the title compound **46.2** (330 mg), which was directly used for the next reaction step. LC-MS (ESI), m/z: [M+H]⁺ = 348.2.

### Step 2: Synthesis of tert-butyl cis-5-(6-(cyclopropylcarbamoyl)pyridin-3-yl)-2,5-diazabicyclo[4.2.0]octane-2-carboxylate (46.3)

A solution of cyclopropylamine (149 mg, 2.61 mmol) in tetrahydrofuran (2 mL) was cooled to 0°C, and DIBAL-H (1.0 mol/L, 2.3 mL, 2.3 mmol) was added thereto. The resulting mixture was stirred at room temperature for 1 hour, and then the reaction system was cooled to 0°C. Compound **46.2** (165 mg, crude product, about 0.47 mmol) was added to the reaction system, and the reaction mixture was stirred at room temperature for 3 hours. After the reaction was completed, potassium sodium tartrate aqueous solution (10 mL) and ethyl acetate (20 mL) were added thereto, and the reaction mixture was stirred for 2 hours. The resulting mixture was extracted with ethyl acetate (50 mL × 2), washed with saturated brine, dried over anhydrous sodium sulfate, filtered to remove the solid, and the filtrate was concentrated under reduced pressure. The resulting residue was the crude product of the title compound **46.3** (120 mg), which was directly used for the next reaction step. LC-MS (ESI), m/z: [M+H]⁺ = 373.3.

### Step 3: Synthesis of cis-5-(2,5-diazabicyclo[4.2.0]octan-2-yl)-N-cyclopropyl-2-picolinamide (46.4)

To a solution of compound **46.3** (165 mg, 0.345 mmol) in dichloromethane (2 mL) was added trifluoroacetic acid (2 mL), and the reaction mixture was stirred at room temperature for 2 hours. After the reaction was completed, the reaction mixture was concentrated under reduced pressure, and the resulting residue was purified by silica gel column chromatography (dichloromethane/methanol = 100/0 to 90/10) to obtain the title compound **46.4** (30 mg, yield: 32%). LC-MS (ESI), m/z: [M+H]⁺ = 273.2.

### Step 4: Synthesis of cis-N-cyclopropyl-5-(5-((7-ethyl-6-oxo-5,6-dihydro-1,5-naphthyridin-3-yl)methyl)-2,5-diazabicyclo[4.2.0]octan-2-yl)pyridine-2-carboxamide (Example 46)

To a solution of compound **46.4** (30 mg, 0.11 mmol) in acetonitrile (2 mL) were added 7-(chloromethyl)-3-ethyl-1,5-naphthyridin-2(1*H*)-one (35.0 mg, 0.171 mmol), potassium iodide (5.0 mg, 0.022 mmol), and diisopropylethylamine (94 mL, 0.55 mmol), and the mixture was stirred at 70°C for 16 hours. After the reaction was completed, the reaction mixture was concentrated under reduced pressure, and the resulting residue was purified by preparative liquid chromatography (chromatographic column model: XBridge peptide BEH C18 19 mm × 250 mm, 10 µm, 130 Å; mobile phase: acetonitrile/water (0.05% trifluoroacetic acid); gradient: 5 to 65%, 20 min; flow rate: 25 mL/min) to obtain the title compound **Example 46** (50 mg, yield: 99%). LC-MS (ESI), m/z: [M+H]⁺ = 459.1.

### Example 46-1 and Example 46-2: N-cyclopropyl-5-((1S,6R)-5-((7-ethyl-6-oxo-5,6-dihydro-1,5-naphthyridin-3-yl)methyl)-2,5-diazabicyclo[4.2.0]octan-2-yl)picolinamide and N-cyclopropyl-5-((1R,6S)-5-((7-ethyl-6-oxo-5,6-dihydro-1,5-naphthyridin-3-yl)methyl)-2,5-diazabicyclo[4.2.0]octan-2-yl)picolinamide

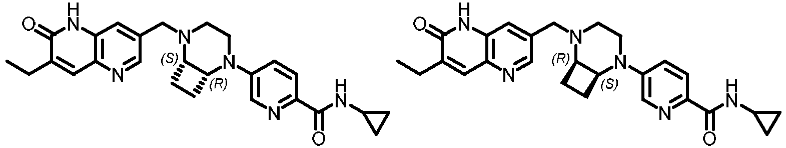

### Synthetic route:

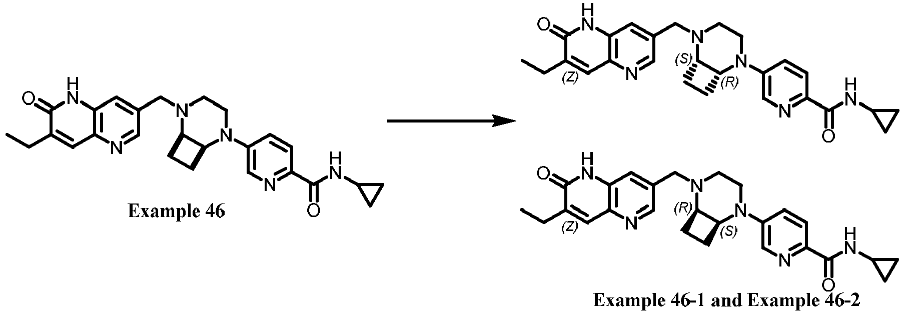

**Example 46** (47 mg) was purified by preparative chiral SCF chromatography (instrument model: SFC-150 (Waters); chromatographic column model: OJ 20 × 250 mm, 10 µm; mobile phase: mobile phase A = carbon dioxide, mobile phase B = methanol (0.2% ammonia (7 mol/L ammonia-methanol solution)), A/B = 50/50; flow rate: 120 g/min) to obtain **Example 46-1** (t_{R} = 1.69 min, 13 mg, yield: 28%) and **Example 46-2** (t_{R} = 2.61 min, 13 mg, yield: 28%).

### Example 46-1: LC-MS (ESI), m/z: [M+H]⁺ = 459.3.

¹H NMR (400 MHz, DMSO-*d₆*) δ 11.84 (s, 1H), 8.43 (d, *J =* 1.6 Hz, 1H), 8.31 (d,*J* = 4.8 Hz, 1), 8.06 (d, *J =* 2.8 Hz,1H), 7.82 (d, *J =* 8.8 Hz, 1H), 7.76 (s, 1H), 7.68 (s, 1H), 7.22 (dd, *J =* 2.8, 8.8 Hz, 1H), 4.30 (dd, *J =* 7.2, 14.4 Hz, 1H), 3.90 (d, *J =* 14.0 Hz, 1H), 3.46 - 3.40 (m, 2H), 3.35 - 3.28 (m, 1H), 3.25 - 3.16 (m, 1H), 2.88 - 2.74 (m, 2H), 2.55 (q, *J =* 7.2 Hz, 2H), 2.35 - 2.18 (m, 2H), 2.12 - 2.00 (m, 1H), 1.87 - 1.78 (m, 2H), 1.19 (t, *J =* 7.2 Hz, 3H), 0.71 - 0.57 (m, 4H).

### Example 46-2: LC-MS (ESI), m/z: [M+H]⁺ = 459.3.

¹H NMR (400 MHz, DMSO-*d₆*) δ 11.83 (s, 1H), 8.43 (d, *J =* 1.6 Hz, 1H), 8.31 (d,*J* = 4.8 Hz, 1), 8.06 (d, *J =* 2.8 Hz,1H), 7.82 (d, *J =* 8.8 Hz, 1H), 7.76 (s, 1H), 7.68 (s, 1H), 7.22 (dd, *J =* 2.8, 8.8 Hz, 1H), 4.30 (dd, *J =* 7.2, 14.4 Hz, 1H), 3.90 (d, *J =* 14.0 Hz, 1H), 3.46 - 3.40 (m, 2H), 3.35 - 3.28 (m, 1H), 3.23 - 3.18 (m, 1H), 2.85 - 2.74 (m, 2H), 2.55 (q, *J =* 7.2 Hz, 2H), 2.35 - 2.18 (m, 2H), 2.12 - 2.00 (m, 1H), 1.87 - 1.78 (m, 2H), 1.19 (t, *J =* 7.2 Hz, 3H), 0.67 - 0.61 (m, 4H).

### Example 47: cis-N-cyclopropyl-5-(5-((7-ethyl-6-oxo-5,6-dihydro-1,5-naphthyridin-3-yl)methyl)-2,5-diazabicyclo[4.2.0]octan-2-yl)-6-fluoropicolinamide

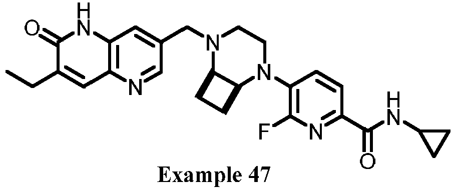

**Example 47** (12.0 mg) was obtained with reference to the synthesis of **Example 2.** LC-MS (ESI), m/z: [M+H]⁺ = 477.1.

### Example 47-1 and Example 47-2: N-cyclopropyl-5-((1R,6S)-5-((7-ethyl-6-oxo-5,6-dihydro-1,5-naphthyridin-3-yl)methyl)-2,5-diazabicyclo[4.2.0]octan-2-yl)-6-fluoropicolinamide and N-cyclopropyl-5-((1S,6R)-5-((7-ethyl-6-oxo-5,6-dihydro-1,5-naphthyridin-3-yl)methyl)-2,5-diazabicyclo[4.2.0]octan-2-yl)-6-fluoropicolinamide

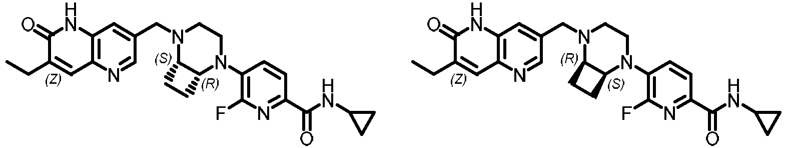

**Example 47** (12.0 mg, 0.0251 mmol) was purified by chiral SFC chromatography (chromatographic column model: CHIRALPAK OJ-H 250 mm × 20 mm, 5 µm; mobile phase: 40% ethanol (0.2% ammonia water); flow rate: 14 mL/min) to obtain **Example 47-1** (t_{R} = 7.59 min, 3.4 mg, yield: 27%) and **Example 47-2** (t_{R} = 9.01 min, 3.6 mg, yield: 30%).

### Example 47-1: LC-MS (ESI), m/z: [M+H]⁺ = 476.9.

¹H NMR (400 MHz, DMSO-*d₆*) δ 11.83 (s, 1H), 8.42 (d, *J =* 1.7 Hz, 11H), 8.31 (d, *J* = 4.8 Hz, 1H), 7.83 - 7.78 (m, 1H), 7.76 (s, 1H), 7.65 (s, 1H), 7.39 (dd, *J=* 10.5, 8.3 Hz, 1H), 4.19 (q, *J =* 6.2 Hz, 1H), 3.81 (d, *J =* 14.1 Hz, 1H), 3.47 (d, *J =* 14.0 Hz, 1H), 3.44 - 3.38 (m, 1H), 3.20 - 3.14 (m, 1H), 2.87 - 2.80 (m, 1H), 2.79 - 2.71 (m, 1H), 2.58 - 2.52 (m, 2H), 2.43 - 2.36 (m, 1H), 2.10 - 1.80 (m, 5H), 1.78 - 1.69 (m, 1H), 1.18 (t, *J =* 7.4 Hz, 3H), 0.67 - 0.65 (m, 1H), 0.65 - 0.62 (m, 2H).

### Example 47-2: LC-MS (ESI), m/z: [M+H]⁺ = 476.9.

¹H NMR (400 MHz, DMSO-*d₆*) δ 11.84 (s, 1H), 8.42 (d, *J =* 1.7 Hz, 11H), 8.31 (d, *J* = 4.8 Hz, 1H), 7.83 - 7.78 (m, 1H), 7.76 (s, 1H), 7.65 (s, 1H), 7.39 (dd, *J =* 10.5, 8.3 Hz, 1H), 4.19 (q, *J* = 6.2 Hz, 1H), 3.81 (d, *J =* 14.1 Hz, 1H), 3.47 (d, *J =* 14.0 Hz, 1H), 3.44 - 3.38 (m, 1H), 3.20 - 3.14 (m, 1H), 2.87 - 2.80 (m, 1H), 2.78 - 2.71 (m, 1H), 2.58 - 2.52 (m, 2H), 2.43 - 2.36 (m, 1H), 2.11 - 1.81 (m, 5H), 1.78 - 1.69 (m, 1H), 1.18 (t, *J* = 7.4 Hz, 3H), 0.67 - 0.65 (m, 1H), 0.65 - 0.62 (m, 2H).

### Example 48: cis-N-cyclobutyl-5-(5-((7-ethyl-6-oxo-5,6-dihydro-1,5-naphthyridin-3-yl)methyl)-2,5-diazabicyclo[4.2.0]octan-2-yl)picolinamide

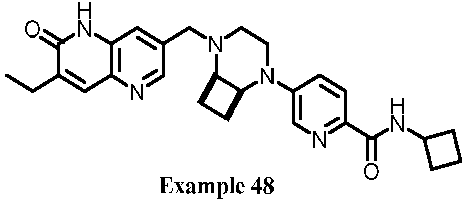

**Example 48** (13.6 mg) was obtained with reference to the synthesis of **Example 2.**

LC-MS (ESI), m/z: [M+H]⁺ = 473.3.

### Example 48-1 and Example 48-2: N-cyclobutyl-5-((1R,6S)-5-((7-ethyl-6-oxo-5,6-dihydro-1,5-naphthyridin-3-yl)methyl)-2,5-diazabicyclo[4.2.0]octan-2-yl)picolinamide and N-cyclobutyl-5-((1S,6R)-5-((7-ethyl-6-oxo-5,6-dihydro-1,5-naphthyridin-3-yl)methyl)-2,5-diazabicyclo[4.2.0]octan-2-yl)picolinamide

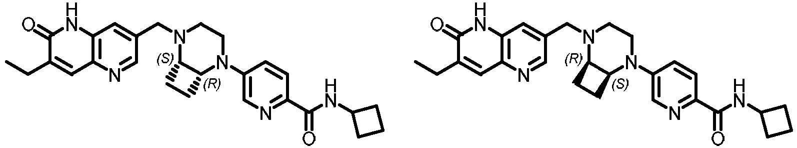

**Example 48** (13.6 mg, 0.288 mmol) was purified by chiral SFC chromatography (chromatographic column model: CHIRALPAK AD-H 250 mm × 20 mm, 5 µm; mobile phase: 40% isopropanol (0.2% ammonia water); flow rate: 40 g/min) to obtain **Example 48-1** (t_{R} = 3.87 min, 3.2 mg, yield: 24%) and **Example 48-2** (t_{R} = 4.96 min, 3.6 mg, yield: 26%).

### Example 48-1: LC-MS (ESI), m/z: [M+H]⁺ = 473.3.

¹H NMR (400 MHz, MeOH-*d₄*) δ 8.41 (d, *J =* 1.7 Hz, 1H), 8.05 (d, *J =* 2.7 Hz, 1H), 7.79 -7.73 (m, 2H), 7.11 (dd, *J =* 8.9, 2.8 Hz, 1H), 4.39 (dt, *J* = 16.2, 7.9 Hz, 1H), 4.25 (q, *J* = 6.9 Hz, 1H), 3.90 (d, *J =* 14.3 Hz, 1H), 3.43 - 3.30 (m, 3H), 3.24 (m, 1H), 2.81 (dt, *J =* 11.9, 3.7 Hz, 1H), 2.57 (q, *J =* 7.0 Hz, 2H), 2.33 - 2.20 (m, 3H), 2.19 - 2.09 (m, 1H), 2.06 - 1.95 (m, 2H), 1.91 - 1.80 (m, 2H), 1.74 - 1.63 (m, 2H), 1.19 (t, *J =* 7.4 Hz, 3H).

### Example 48-2: LC-MS (ESI), m/z: [M+H]⁺ = 473.3.

¹H NMR (400 MHz, MeOH-*d₄*) δ 8.42 (d, *J =* 1.7 Hz, 1H), 8.05 (d, *J =* 2.7 Hz, 1H), 7.79 -7.73 (m, 2H), 7.11 (dd, *J =* 8.9, 2.8 Hz, 1H), 4.39 (dt, *J* = 16.2, 7.9 Hz, 1H), 4.25 (q,*J* = 6.9 Hz, 1H), 3.90 (d, *J =* 14.3 Hz, 1H), 3.43 - 3.30 (m, 3H), 3.24 (m, 1H), 2.81 (dt, *J =* 11.9, 3.7 Hz, 1H), 2.57 (q, *J =* 7.0 Hz, 2H), 2.33 - 2.20 (m, 3H), 2.19 - 2.09 (m, 1H), 2.06 - 1.95 (m, 2H), 1.91 - 1.80 (m, 2H), 1.74 - 1.63 (m, 2H), 1.19 (t, *J =* 7.4 Hz, 3H).

### Example 49: cis-3-ethyl-7-((5-(3-(methylamino)isoxazolo[4,5-b]pyridin-6-yl)-2,5-diazabicyclo[4.2.0]octan-2-yl)methyl)-1,5-naphthyridin-2(1H)-one

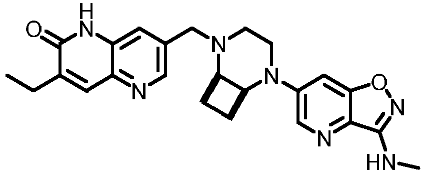

### Example 49

### Synthetic route:

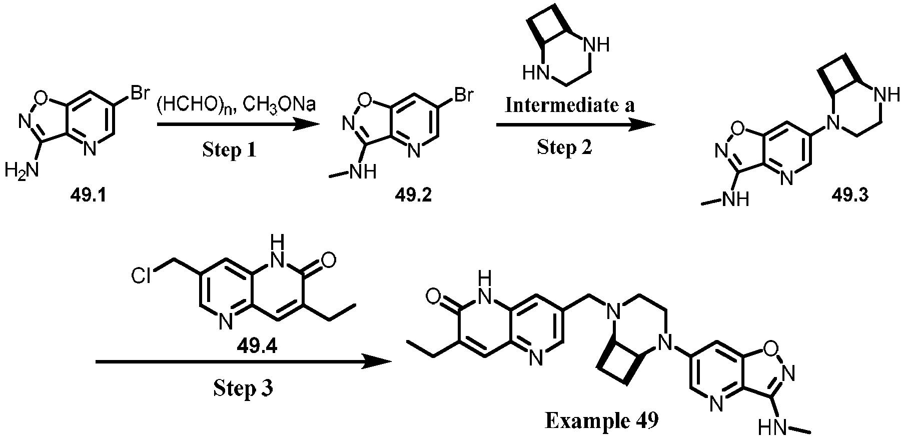

### Step 1: 6-Bromo-N-methylisoxazolo[4,5-b]pyridin-3-amine (49.2)

To a solution of compound **49.1** (510 mg, 2.38 mmol) in methanol (10.0 mL) were added sodium methoxide (5.4 M in MeOH) (2.20 mL, 11.9 mmol) and paraformaldehyde (1.48 g, 23.8 mmol) at 25°C. After the addition was completed, the reaction mixture was stirred at 25°C for 16 hours. Sodium borohydride (271 mg, 7.15 mmol) was then added thereto at 0°C, and the reaction mixture was stirred at 25°C for 1 hour. Sodium borohydride (271 mg, 7.15 mmol) was again added thereto at 0°C, and the reaction mixture was stirred at 25°C for 1 hour. After the reaction was completed, the reaction mixture was diluted with water (20.0 mL) and extracted with ethyl acetate (15.0 mL × 3). The combined organic phases were washed with saturated brine, dried over anhydrous sodium sulfate, filtered, and concentrated. The resulting residue was purified by silica gel chromatography (PE: EA = 5:1) to obtain the title compound **49.2** (280 mg, yield: 52%). LC-MS (ESI), m/z: [M+H]⁺ = 228.0/230.0.

### Step 2: cis-6-(2,5-Diazabicyclo[4.2.0]octan-2-yl)-N-methylisoxazolo[4,5-b]pyridin-3-amine (49.3)

To a solution of compound **49.2** (180 mg, 0.789 mmol) in 1,4-dioxane (5.00 mL) were added compound **intermediate a** (487 mg, 1.58 mmol), cesium carbonate (1.29 g, 3.95 mmol), 2-dicyclohexylphosphino-2',6'-diisopropoxy-1,1'-biphenyl (73.7 mg , 0.158 mmol), and chloro(2-dicyclohexylphosphino-2',6'-diisopropoxy-1,1'-biphenyl)(2-amino-1,1'-biphenyl-2-yl)palladium(II) (61.3 mg, 0.0790 mmol) at 25°C. After the addition was completed, the reaction mixture was stirred at 110°C for 16 hours under nitrogen atmosphere. After the reaction was completed, the reaction mixture was evaporated to dryness under reduced pressure to remove the solvent. The resulting residue was purified by silica gel chromatography (DCM: MeOH = 10: 1) to obtain the title compound **49.3** (40.0 mg, yield: 19.5%). LC-MS (ESI), m/z: [M+H]⁺ = 260.2.

### Step 3: cis-3-Ethyl-7-((5-(3-(methylamino)isoxazolo[4,5-b]pyridin-6-yl)-2,5-diazabicyclo[4.2.0]octan-2-yl)methyl)-1,5-naphthyridin-2(1H)-one (Example 49)

To a solution of compound **49.3** (40.0 mg, 0.154 mmol) in acetonitrile (2.00 mL) were added *N*,*N*-diisopropylethylamine (59.8 mg, 0.463 mmol), compound **5** (68.7 mg, 0.309 mmol), and potassium iodide (5.12 mg, 0.0309 mmol) at 25°C, and the reaction mixture was stirred at 80°C for 2 hours. After the reaction was completed, the reaction mixture was evaporated to dryness under reduced pressure to remove the solvent. The resulting residue was purified by silica gel chromatography (DCM: MeOH = 20:1) to obtain the title compound **Example 49** (30.0 mg, yield: 44%). LC-MS (ESI), m/z: [M+H]⁺ = 446.2.

### Example 49-1 and Example 49-2: 3-ethyl-7-(((1R,6S)-5-(3-(methylamino)isoxazolo[4,5-b]pyridin-6-yl)-2, 5-diazabicyclo[4.2.0] octan-2-yl)methyl)-1, 5-naphthyridin-2(1H)-one and 3-ethyl-7-(((1S,6R)-5-(3-(methylamino)isoxazolo[4,5-b]pyridin-6-yl)-2,5-diazabicyclo[4.2.0]octan-2-yl)methyl)-1,5-naphthyridin-2(1H)-one

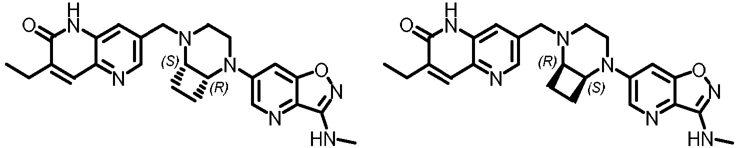

**Example 49** was separated by chiral column chromatography (chromatographic column model: CHIRALPAK OJ-H 250 mm × 20 mm, 5 µm; mobile phase: 40% ethanol (0.2% ammonia water); flow rate: 40 g/min) to obtain **Example 49-1** (3.00 mg, yield: 10%, t_{R} = 8.45 min) and **Example 49-2** (4.70 mg, yield: 16%, t_{R} = 17.57 min).

### Example 49-1: LC-MS (ESI), m/z: [M+H]⁺ = 446.2.

¹HNMR (400 MHz, ) δ 11.85 (s, 1H), 8.45 (s, 1H), 8.22 (s, 1H), 7.77 (s, 1H), 7.69 (s, 1H), 7.09 (s, 1H), 6.76 (d, *J* = 3.8 Hz, 1H), 4.34 (s, 1H), 3.91 (d, *J =* 10.8 Hz, 1H), 3.49 (d,*J* = 10.5 Hz, 3H), 3.24 (s, 1H), 2.80 (dd, *J =* 17.3, 5.1 Hz, 4H), 2.58 - 2.52 (m, 3H), 2.27 (s, 1H), 2.07 (s, 1H), 1.82 (s, 2H), 1.19 (t, *J =* 7.4 Hz, 3H).

### Example 49-2: LC-MS (ESI), m/z: [M+H]⁺ = 446.2.

¹HNMR (400 MHz, ) δ 11.80 (s, 1H), 8.40 (d, *J =* 1.8 Hz, 1H), 8.18 (d, *J =* 2.3 Hz, 1H), 7.72 (s, 1H), 7.64 (d, *J =* 1.2 Hz, 1H), 7.04 (d, *J =* 2.2 Hz, 1H), 6.71 (d, *J =* 50 Hz, 1H), 4.31 (dd, *J =* 14.4, 6.8 Hz, 1H), 3.87 (d, *J =* 14.0 Hz, 1H), 3.40 (dd, *J =* 22.0, 13.0 Hz, 3H), 3.19 (td, *J =* 11.3, 3.1 Hz, 1H), 2.80 - 2.72 (m, 4H), 2.54 - 2.48 (m, 3H), 2.24 (s, 1H), 2.06 - 1.98 (m, 1H), 1.78 (dd, *J =* 8.5, 4.2 Hz, 2H), 1.14 (d, *J =* 7.4 Hz, 3H).

### Biological evaluation

The present disclosure is further described and explained below in conjunction with assays, but these examples are not meant to limit the scope of the present disclosure.

### I. Enzymatic activity assays

### (i) PARP1 and PARP2 enzymatic activity assays

### 1. Experimental purpose:

To test the example compounds of the present disclosure for their enzymatic inhibitory activity against PARP1.

### 2. Experimental instruments and reagents:

### 2.1 Reagents:

PARP1 (BPS, Cat. No. 80501);
PARP2 (BPS, Cat. No. 80502);
Histone (Active Motif, Cat. No. 81167);
Activated DNA (Genscript, Cat. No. L05182-01&02&03);
Secondary antibody (anti-rabbit IgG, HRP-linked Antibody, CST, Cat. No. 7074P2);
Primary antibody (anti-Poly/Mono-ADP Ribose (E6F6A) Rabbit mAb, CST, Cat. No. 83732S);
SuperSignal ELISAFemto Substrate (Thermo Pierce, Cat. No. 37074);
Biotin-NAD+ (R&D, Cat. No. 6573);
NAD+ (TCI, Cat. No. D0919-5G);
Strep-HRP (Thermo Pierce, Cat. No. 21127);
QuantaRed Enhanced Chemifluorescent HRP Substrate Kit (Thermo Pierce, Cat. No. 15159).

### 2.2 Instruments:

| Instrument name | Model | Manufacturer | SN |
|---|---|---|---|
| Automatic micropore pipette | PRC384U | BioTek | 205532 |
| Ultrasonic nanoliter liquid handling system | Echo 550 | Labcyte | E5XX-1045 |
| Microplate reader | Envision | Perkin Elmer | 1041604 |
| Centrifuge | Avanti J-15R | BECKMAN COULTER | JBR20A055 |
| Microplate shaker | Titramax 1000 | Heidolph | 51302444 |
| Multifunctional plate reader | PARADIGM | MOLECULAR DEVICES | 33270-2051 |

### 3. Experimental methods:

### 3.1 PARP1 enzymatic activity assay steps:

1) 50 ng/mL of histone coating buffer was prepared with 1X PBS, and 25 µL of coating buffer was transferred to a 384-well reaction plate, which was coated at 4°C overnight.
2) After the coating was completed, the coating buffer was discarded. The reaction plate was washed with PBST solution (1X PBS, 0.05% Tween-20) by transferring 50 µL of PBST to the 384-well reaction plate, allowing it stand for 5 minutes, discarding the washing buffer, refilling the washing buffer, repeating the washing three times, and finally patting the plate dry for blocking.
3) 50 µL of blocking buffer (1X PBS, 0.05% Tween-20, 5% BSA) was transferred to a 384-well reaction plate, which was allowed to stand for 1 hour. After washing, the blocking buffer was discarded. The reaction plate was washed three times with PBST solution as described above and finally patted dry.
4) 1000X compound was prepared. 1 µL of compound was transferred to a 96-well plate containing 199 µL of reaction buffer (50 mM Tris-HCl (pH 7.5), 0.005% Tween-20, 0.01% BSA) and mixed well. 5 µL of mixed compound was transferred to a 384-well reaction plate.
5) 25/10X PARP1-DNA solution was prepared with reaction buffer. 10 µL of PARP1-DNA solution was transferred to a 384-well reaction plate, and 10 µL of DNA solution was transferred to the negative control wells with a final PARP1 concentration of 0.02 nM and a final DNA concentration of 0.8 nM.
6) 25/10X NAD+ solution was prepared with reaction buffer. 10 µL of NAD+ solution was transferred to a 384-well reaction plate with a final NAD+ concentration of 3.5 µM, and the plate was incubated at room temperature for 60 minutes.
7) After the reaction was completed, the reaction buffer was discarded. The reaction plate was washed three times with PBST solution according to step 2 and finally patted dry.
8) Primary antibody was 2000-fold diluted with the blocking buffer. 20 µL of primary antibody was added to the reaction plate, which was incubated at room temperature for 1.5 hours.
9) The primary antibody was discarded. The reaction plate was washed three times with PBST solution according to step 2 and finally patted dry.
10) Secondary antibody was 2000-fold diluted with the blocking buffer. 20 µL of secondary antibody was added to the reaction plate, which was incubated at room temperature for 1 hour.
11) The secondary antibody was discarded. The reaction plate was washed three times with PBST solution according to step 2 and finally patted dry.
12) Femto-ECL Substrate A and Femto-ECL Substrate B were mixed at a ratio of 1:1, and 25 µL of the mixture was transferred to the 384-well reaction plate. The chemiluminescence value RLU was read with Envision.

### 3.2 PARP2 enzymatic activity assay steps:

1) 100 ng/mL of histone coating buffer was prepared with 1X PBS, and 25 µL of coating buffer was transferred to a 384-well reaction plate, which was coated at 4°C overnight.
2) After the coating was completed, the coating buffer was discarded. The reaction plate was washed with PBST solution (1X PBS, 0.05% Tween-20) by transferring 50 µL of PBST to the 384-well reaction plate, allowing it stand for 5 minutes, discarding the washing buffer, refilling the washing buffer, repeating the washing three times, and finally patting the plate dry for the next step of blocking.
3) 50 µL of blocking buffer was transferred to the 384-well reaction plate, which was allowed to stand for 1 hour.
4) After the blocking was completed, the blocking buffer (1X PBS, 0.05% Tween-20, 5% BSA) was discarded. The reaction plate was washed three times with PBST solution according to step 2 and finally patted dry.
5) 25/10X PARP2 solution was prepared. 10 µL of PARP2 solution was transferred to a 384-well reaction plate, and 10 µL of reaction buffer (50 mM HEPES (pH 7.5), 0.002% Tween-20, 0.1% BSA, 100 mM NaCl, 2 mM DTT) was transferred to the negative control wells with a final PARP2 concentration of 1.5 nM.
6) 2000X compound was prepared. 50 µL of compound was transferred with echo, then added with 19.95 µL of reaction buffer, and mixed well. 5 µL of mixed compound was transferred to a 384-well reaction plate.
7) 25/10X Biotin-NAD+ solution was prepared. 10 µL of NAD+ solution was transferred to a 384-well reaction plate with a final NAD+ concentration of 2 µM, and the plate was incubated at room temperature for 60 minutes.
8) After the reaction was completed, the reaction buffer was discarded. The reaction plate was washed three times with PBST solution according to step 2.
9) Stre-HRP solution was prepared with dilution of blocking buffer. 25 µL of Stre-HRP solution was transferred to the reaction plate with a final Stre-HRP concentration of 0.1 µg/mL, and the plate was incubated at room temperature for 1 hour.
10) The Stre-HRP solution was discarded. The reaction plate was washed three times with PBST solution according to step 2.
11) Femto-ECL Substrate A, Femto-ECL Substrate B, and QuantaRed ADHP were mixed at a ratio of 50:50:1, and 25 µL of the mixture was transferred to the 384-well reaction plate. The plate was incubated at room temperature for 10 minutes, and 2.5 µL of QuantaRed stop solution was added thereto. The fluorescence value (Ex 550/Em 620) was read using Paradigm.

### 4. Experimental results:

The enzymatic inhibitory activity of the compounds of the present disclosure on PARP1 and PARP2 was determined by the above assay, and the measured IC₅₀ values are shown in Table 1.

**Table 1**

| Example number | PARP1 IC₅₀ (nM) | PARP2 IC₅₀ (nM) | PARP1/PARP2 inhibition Selectivity multiple N (N = IC_{50 (PARP2)} / IC_{50 (PARP1)}) |
|---|---|---|---|
| 1 | 1.1 | 15.4 | 14 |
| 1-1 | 0.9 | 26.7 | 31 |
| 1-2 | 0.7 | 11.4 | 16 |
| 2 | 0.9 | 14.0 | 16 |
| 2-2 | 0.6 | 46.4 | 71 |
| 2-3 | 1.5 | 72 | 48 |
| 2-4 | 1.0 | 40 | 40 |
| 4-1 | 12 | 365 | 30 |
| 5 | 0.5 | 31 | 62 |
| 5-1 | 0.9 | 75 | 83 |
| 5-2 | 0.5 | 32 | 64 |
| 6 | 2.5 | 198 | 79 |
| 6-1 | 4.1 | >300 | >73 |
| 6-2 | 1.5 | 152 | 101 |
| 7 | 0.6 | 58 | 97 |
| 10 | 1 | 105 | 105 |
| 10-1 | 0.8 | 36 | 45 |
| 10-2 | 0.9 | 77 | 85 |
| 11-1 | 3.9 | 272 | 69 |
| 11-2 | 1.5 | 202 | 135 |
| 12-1 | 0.3 | 18 | 60 |
| 12-2 | 0.5 | 60 | 120 |
| 16-1 | 0.7 | 118 | 168 |
| 16-2 | 0.3 | 19 | 63 |
| 29-2 | 0.7 | 30 | 43 |
| 30-2 | 1.2 | 47 | 39 |
| 31-1 | 0.3 | 91 | 303 |
| 33-1 | 6.6 | >300 | >45 |
| 33-2 | 2.3 | >300 | >130 |
| 37-2 | 1.9 | 403 | 212 |
| 38-2 | 1.2 | 30 | 25 |
| 39-2 | 2.4 | >1000 | >416 |
| 40-2 | 0.5 | 142 | 284 |
| 41-2 | 0.6 | 159 | 265 |
| 42-2 | 4.3 | 163 | 38 |
| 43-2 | 2.7 | 429 | 159 |
| 46 | 1.0 | 52 | 52 |
| 46-1 | 0.4 | 29 | 72 |
| 46-2 | 0.3 | 81 | 270 |
| AZD5305 | 0.9 | 16 | 18 |

AZD5305 has a structure of

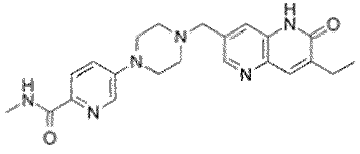

### 5. Experimental conclusion:

It can be seen from the above data that the example compounds of the present disclosure have good inhibitory activity against PARP1 but poor inhibitory activity against PARP2, and have good selectivity for the inhibitory activity against PARP1/PARP2.

### II. Cell proliferation activity assay

### Proliferation activity assay of MDA-MB-436 cells

### 1. Experimental purpose:

To test the example compounds of the present disclosure for their inhibitory activity on the proliferation of MDA-MB-436 cells.

### 2. Experimental instruments and reagents:

### Cell line:

| Cell line | Source | Cell culture medium | Number of cells seeded | Compound incubation time |
|---|---|---|---|---|
| MDA-MB-436 | ATCC | DMEM + 10% FBS | 4000 | 6 days |

### 3. Experimental methods:

### 3.1 Cell plating

Complete culture medium was prepared and mixed thoroughly. The cells were resuscitated and cultured for about two passages to select a cell line in good growth condition. The cell culture flask was removed from the incubator. Cell adherence: the culture medium was aspirated, then the cells were washed once with trypsin to discard the waste liquid, and 3 mL of fresh trypsin was added to the culture flask for digestion. When the cells were loose to be detached from the wall of the flask, 8 mL of complete culture medium was added thereto to stop trypsin digestion and mixed gently. The cell suspension was transferred to a centrifuge tube with a pipette and centrifuged at 1000 rpm for 5 minutes. The supernatant was discarded. An appropriate volume of culture medium was added to the centrifuge tube and pipetted gently to resuspend the cells evenly. The cells were counted using a Vi-Cell XR cell counter. The cell suspension was adjusted to an appropriate concentration. The cell suspension was added to a 96-well plate at 100 µL/well. The details such as cell name, seeding density, and date were labelled, and the culture plate was placed in a CO₂ incubator overnight.

### 3.2 Preparation and addition of compound plate

The compound to be tested was prepared into a 10 mM stock solution, which was aliquoted and stored in a -20°C freezer. The compound solution was added to a 96-well plate using HPD300. The plate was incubated in a CO₂ incubator for a total of 6 days.

### 3.3 Reagent preparation and detection

CellTiter-Glo buffer was melted at room temperature. The lyophilized CellTiter-Glo substrate was equilibrated to room temperature. CellTiter-Glo buffer was added to the CellTiter Glo substrate and mixed thoroughly. A cell plate was taken out and equilibrated to room temperature. 100 µL of mixed CellTiter Glo reagent was added to each well, and the mixture was shaken in the dark for 10 minutes, followed by incubation for 20 minutes. The culture plate was placed into an Enspire reading plate, and the luminescence reading results were recorded. The inhibition rate was calculated according to the following formula: inhibition rate (%) = (1 - (RLU _{compound} - RLU _{blank}) / (RLU _{DMSO} - RLU _{blank})) × 100%. A pharmacodynamic inhibition rate curve was plotted by using XLFit, and the IC₅₀ value was calculated. The 4-parameter model [fit = (A + ((B - A) / (1 + ((C / x) ^ D)))] was used.

### 4. Experimental results:

The proliferation inhibitory activity of the compounds of the present disclosure on MDA-MB-436 cells was determined by the above assay, and the measured IC₅₀ values are shown in Table 1.

**Table 2**

| Example number | MDA-MB-436 proliferation inhibition IC₅₀ (nM) |
|---|---|
| 2 | 5 |
| 2-1 | 4 |
| 2-2 | 1 |
| 2-4 | 2 |
| 7 | 13 |
| 10 | 9 |
| 10-2 | 5 |
| 11-2 | 9 |
| 12-1 | 15 |
| 12-2 | 2 |
| 16-1 | 5 |
| 16-2 | 6 |
| 29-2 | 13 |
| 30-1 | 2 |
| 30-2 | 1 |
| 31-1 | 3 |
| 37-2 | 6 |
| 38-2 | 15 |
| 39-2 | 9 |
| 40-1 | 15 |
| 40-2 | 4 |
| 41-2 | 5 |
| 42-2 | 8 |
| 43-1 | 12 |
| 43-2 | 10 |
| 46 | 5 |
| 46-1 | 8 |
| 46-2 | 2 |
| 47-2 | 3 |
| 48-1 | 12 |
| 48-2 | 2 |

### 5. Experimental conclusion:

It can be seen from the above data that the example compounds of the present disclosure have good cell proliferation inhibitory activity on MDA-MB-436 cells.

### III. Pharmacokinetic study

Experimental purpose: To test the pharmacokinetics of the compounds in ICR (CD-1) mice
Experimental materials: mice (male, ICR (CD-1))
Experimental operation:

ICR (CD-1) male mice, 3 mice per group. One group of mice was injected with the compound to be tested via the tail vein at a dose of 1 mg/kg. The other group of mice was administered the compound to be tested via single-dose oral administration at a dose of 3 mg/kg. In the tail vein injection group, blood was collected from the saphenous vein at 5 minutes, 15 minutes, 30 minutes, 1 hour, 2 hours, 4 hours, 8 hours, and 24 hours post-administration. In the single-dose oral administration group, blood was collected at 15 minutes, 30 minutes, 1 hour, 2 hours, 4 hours, 8 hours, and 24 hours post-administration. The collected blood was placed in an EDTA-K2 anticoagulant tube, and 30 µL of whole blood was collected at each time point. Immediately after collection, blood samples were placed on wet ice for centrifugation. Samples would be centrifuged (maintained at 4600 rpm for 5 minutes at 4°C) within 30 minutes to obtain plasma. The plasma sample was placed into a test tube (a target of 12 to 20 µL) for analysis. 50 µL of plasma was added with 5 µL of MeOH, then added with 200 µL of MeOH/ACN solution containing 5 ng/mL of ISTD (Terfenadine), mixed well with shaking, and centrifuged at 4000 rpm for 15 minutes. The supernatant was diluted 5-fold with MeOH/water (1:1, v/v, 0.1% FA), and loaded to LC-MS/MS for quantification of plasma concentration by analytical methods and calculation of pharmacokinetic parameters.

### Experimental results:

The pharmacokinetic data of the example compounds of the present disclosure, derived from the above scheme, are shown in Table 3.

**Table 3**

| **No.** | **Clearance (mL/min/kg)** | **Half-life t_{1/2} (hr)** |
|---|---|---|
| Example 2-2 | 0.029 | 57.9 |
| Example 12-2 | 0.032 | 41.3 |
| Example 30-2 | 0.027 | 47.6 |
| AZD-5305 | 0.172 | 8.04 |

### Experimental conclusion:

The above data show that the example compounds of the present disclosure have good pharmacokinetic parameters in mice, with 5 to 6 times lower clearance and 5 to 7 times longer half-life compared to the reference compound AZD-5305.

## Claims

1. A compound of general formula (I), a prodrug thereof, a stereoisomer thereof, or a pharmaceutically acceptable salt thereof: 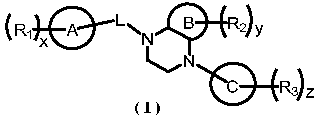 wherein
ring A is 3- to 18-membered heterocyclyl or 5- to 18-membered heteroaryl, wherein the 3- to 18-membered heterocyclyl and 5- to 18-membered heteroaryl are optionally further substituted;
ring B is C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted;
ring C is selected from C₃₋₁₂ cycloalkyl, 3- to 12-membered heterocyclyl, C₆₋₁₄ aryl, or 5-to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted;
R₁ is independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, 5- to 14-membered heteroaryloxy, C₃₋₁₂ cycloalkyl-C₁₋₆ alkyl, 3- to 12-membered heterocyclyl-C₁₋₆ alkyl, C₆₋₁₄ aryl-C₁₋₆ alkyl, or 5- to 14-membered heteroaryl-C₁₋₆ alkyl, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
or, any two R₁ together with the atom to which they are attached form C₃₋₁₂ cycloalkyl, 3-to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, and 5- to 14-membered heteroaryloxy;
R₂ is independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, 5- to 14-membered heteroaryloxy, C₃₋₁₂ cycloalkyl-C₁₋₆ alkyl, 3- to 12-membered heterocyclyl-C₁₋₆ alkyl, C₆₋₁₄ aryl-C₁₋₆ alkyl, or 5- to 14-membered heteroaryl-C₁₋₆ alkyl, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
R₃ is independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, 5- to 14-membered heteroaryloxy, -(CH₂)ₙ₁Rₐ, -(CH₂)ₙ₁ORₐ, -(CH₂)ₙ₁SRₐ, - (CH₂)ₙ₁NR_{b}Rₐ, -(CH₂)ₙ₁C(O)Rₐ, -(CH₂)ₙ₁C(O)NR_{b}Rₐ, -(CH₂)ₙ₁NR_{b}C(O)Rₐ, -(CH₂)ₙ₁S(O)ₘ₁Rₐ, -(CH₂)ₘS(O)ₘ₁NR_{b}Rₐ, -(CH₂)ₘS(O)(=NR_{b})Rₐ, -(CH₂)ₙ₁N=S(=O)RₐR_{b}, or-(CH₂)ₙ₁NR_{b}S(O)ₘ₁Rₐ, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, and 5- to 14-membered heteroaryloxy;
or, any two R₃ together with the atom to which they are attached form C₃₋₁₂ cycloalkyl, 3-to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, and 5- to 14-membered heteroaryloxy;
Rₐ and R_{b} are each independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, or 5- to 14-membered heteroaryloxy, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
L is -(CR₄R₅)ₙ₂-, -C(O)-, -C(O)NR₄-, or -(CR₄R₅)₂O;
R₄ and R₅ are each independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, or 5- to 14-membered heteroaryloxy, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
or, R₄ and R₅ together with the atom to which they are attached form C₃₋₁₂ cycloalkyl or 3- to 12-membered heterocyclyl, wherein the C₃₋₁₂ cycloalkyl and 3- to 12-membered heterocyclyl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
x, y, and z are each independently an integer of 0 to 10;
n1 is an integer of 0 to 10;
n2 is 0, 1, 2, or 3; and
m1 is 0, 1, or 2.

2. The compound, the prodrug thereof, the stereoisomer thereof, or the pharmaceutically acceptable salt thereof according to claim 1, wherein the ring A is 5- to 6-membered monocyclic heteroaryl, 6- to 10-membered bicyclic fused heterocyclyl, 8- to 10-membered bicyclic fused heteroaryl, 8- to 14-membered tricyclic fused heterocyclyl, or 10- to 14-membered tricyclic fused heteroaryl;
the ring A is optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy.

3. The compound, the prodrug thereof, the stereoisomer thereof, or the pharmaceutically acceptable salt thereof according to claim 1 or 2, wherein general formula (I) is further represented by general formula (II): 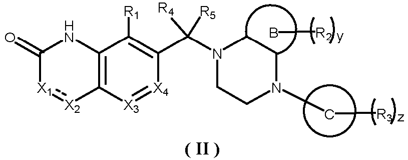 wherein
X₁ is O, S, N, NR₆, CR₆, or CR₆R₇;
X₂ is O, S, N, C(O), NR₈, CR₈, or CR₈R₉;
X₃ is N or CR₁₀;
X₄is N or CR₁₁;
R₆, R₇, R₈, R₉, R₁₀, and R₁₁ are each independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, 5- to 14-membered heteroaryloxy, or - (CH₂)ₙ₃R_{c}, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
or, any two of R₆, R₇, R₈, R₉, R₁₀, and R₁₁ together with the atom to which they are attached form C₃₋₁₂ cycloalkyl, 3- to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
R_{c} is hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, or 5- to 14-membered heteroaryloxy, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy; and
n3 is 0, 1, 2, 3, or 4;
ring B, ring C, R₁, R₂, R₃, R₄, R₅, y, and z are as described in claims 1 to 2.

4. The compound, the prodrug thereof, the stereoisomer thereof, or the pharmaceutically acceptable salt thereof according to claim 1, wherein the ring B is C₃₋₆ cycloalkyl or 3- to 6-membered heterocyclyl;
the ring B is optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy.

5. The compound, the prodrug thereof, the stereoisomer thereof, or the pharmaceutically acceptable salt thereof according to any one of claims 1 to 4, wherein general formula (I) is further represented by general formula (III): 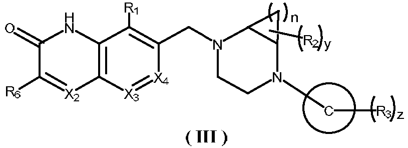 wherein
n is 1, 2, 3, 4, 5, or 6;
X₂ is N or CR₈;
R₆ and R₈ are each independently hydrogen, deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, 5- to 14-membered heteroaryloxy, or -(CH₂)ₙ₃R_{c}, wherein the amino, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy;
or, R₆ and R₈ together with the atom to which they are attached form C₃₋₁₂ cycloalkyl, 3-to 12-membered heterocyclyl, C₆₋₁₄ aryl, or 5- to 14-membered heteroaryl, wherein the C₃₋₁₂ cycloalkyl, 3- to 12-membered heterocyclyl, C₆₋₁₄ aryl, and 5- to 14-membered heteroaryl are optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ deuteroalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3-to 12-membered heterocyclyloxy, C₆₋₁₄ aryloxy, and 5- to 14-membered heteroaryloxy;
X₃, X₄, ring C, R₁, R₂, R₃, y, and z are as described in claims 1 to 4.

6. The compound, the prodrug thereof, the stereoisomer thereof, or the pharmaceutically acceptable salt thereof according to any one of claims 1 to 5, wherein general formula (I) is further represented by general formula (IIIA), general formula (IIIB), general formula (IIIC), or general formula (IIID): 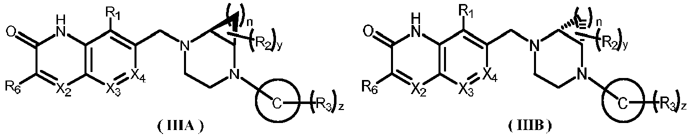 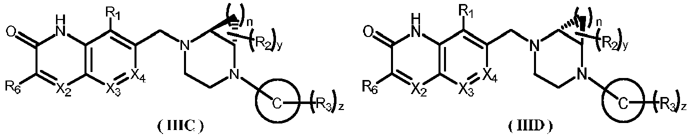 wherein
X₂, X₃, X₄, ring C, R₁, R₂, R₃, R₆, n, y, and z are as described in claim 5.

7. The compound, the prodrug thereof, the stereoisomer thereof, or the pharmaceutically acceptable salt thereof according to any one of claims 1 to 3, wherein general formula (I) is further represented by general formula (IV), general formula (V), general formula (VI), general formula (V), general formula (VI), general formula (VII), general formula (VIII), or general formula (IX): 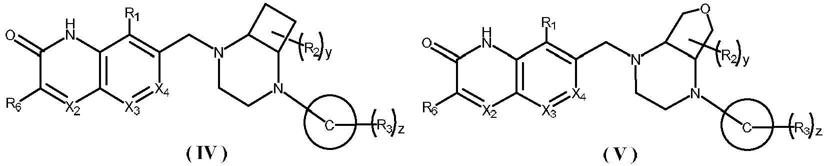 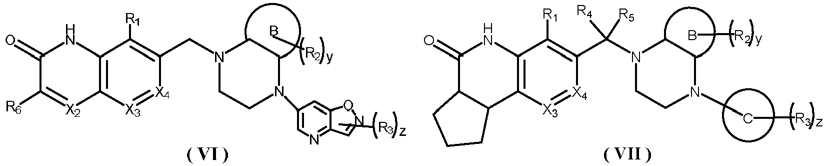 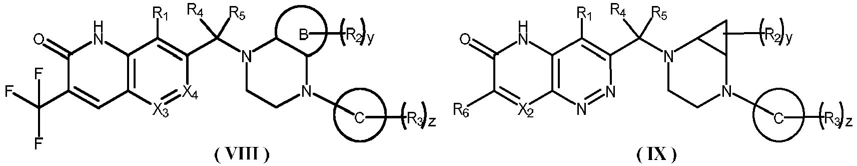 wherein
X₂, X₃, X₄, ring B, ring C, R₁, R₂, R₃, R₄, R₅, R₆, n, y, and z are as described in any one of claims 1 to 5;
preferably, general formula (IV) is represented by general formula (IVA), general formula (IVB), general formula (IVC), or general formula (IVD): 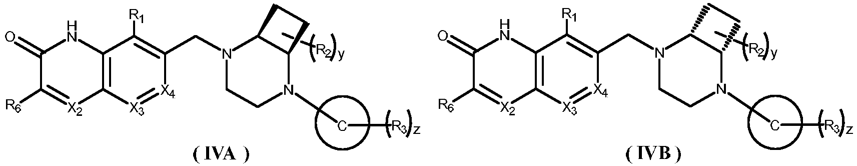 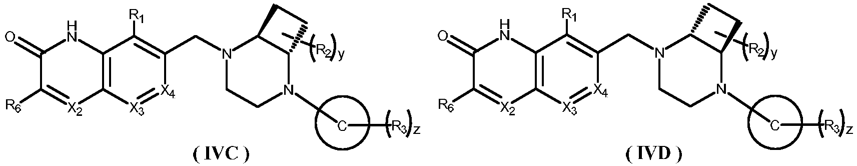 wherein
X₂, X₃, X₄, ring B, ring C, R₁, R₂, R₃, R₄, R₅, R₆, n, y, and z are as described in any one of claims 1 to 5.

8. The compound, the prodrug thereof, the stereoisomer thereof, or the pharmaceutically acceptable salt thereof according to any one of claims 1 to 7, wherein the ring C is phenyl, 3-to 6-membered nitrogen-, oxygen-, or sulfur-containing heterocyclyl, 5- to 6-membered nitrogen-containing heteroaryl, 5- to 6-membered nitrogen-containing heteroaryl-fused 5- to 6-membered heteroaryl, or 5- to 6-membered nitrogen-containing heteroaryl-fused 5- to 6-membered heterocyclyl; ring C is preferably 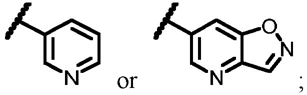 ring C is more preferably 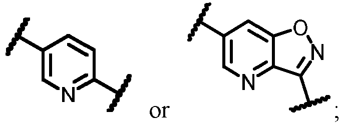 the ring C is optionally further substituted by one or more substituents selected from deuterium, halogen, hydroxyl, mercapto, nitro, cyano, amino, oxo, thio, C₁₋₆ alkyl, C₁₋₆ haloalkyl, C₁₋₆ alkoxy, C₁₋₆ alkylthio, C₁₋₆ hydroxyalkyl, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, C₃₋₁₂ cycloalkyloxy, 3- to 12-membered heterocyclyloxy, C₆₋₁₄ aryloxy, and 5- to 14-membered heteroaryloxy.

9. The compound, the prodrug thereof, the stereoisomer thereof, or the pharmaceutically acceptable salt thereof according to any one of claims 1 to 8, wherein the ring C is 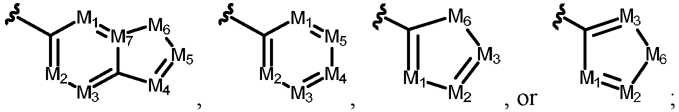
preferably, the ring C is 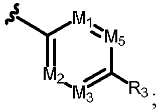
more preferably, the ring C is 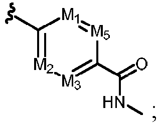
wherein
M₁, M₂, M₃, M₄, and M₅ are each independently N or CR₃;
each M₆ is independently N, NR₃, or CR₃R₃;
each M₇ is independently N or C;
R₃ is as described in claims 1 to 7.

10. The compound, the prodrug thereof, the stereoisomer thereof, or the pharmaceutically acceptable salt thereof according to any one of claims 1 to 9, wherein R₃ is independently hydrogen, deuterium, halogen, hydroxyl, mercapto, nitro, cyano, amino, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, 5- to 10-membered heteroaryl, -ORₐ, -SRₐ, -NR_{b}Rₐ, -C(O)Rₐ, -C(O)NR_{b}Rₐ, -NR_{b}C(O)Rₐ, -S(O)Rₐ, -S(O)₂Rₐ, - S(O)₂NR_{b}Rₐ, -S(O)(=NR_{b})Rₐ, -N=S(=O)RₐR_{b}, or -NR_{b}S(O)₂Rₐ, wherein the amino, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, and 5- to 10-membered heteroaryl are optionally further substituted by one or more substituents selected from deuterium, halogen, hydroxyl, mercapto, nitro, cyano, amino, oxo, thio, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, 5- to 10-membered heteroaryl, C₃₋₈ cycloalkyloxy, 3- to 8-membered heterocyclyloxy, C₆₋₁₀ aryloxy, and 5- to 10-membered heteroaryloxy;
or, any two R₃ together with the atom to which they are attached form C₃₋₈ cycloalkyl, 3-to 8-membered heterocyclyl, C₆₋₁₀ aryl, or 5- to 10-membered heteroaryl, wherein the C₃₋₈ cycloalkyl, 3- to 8-membered heterocyclyl, C₆₋₁₀ aryl, and 5- to 10-membered heteroaryl are optionally further substituted by one or more substituents selected from deuterium, halogen, hydroxyl, mercapto, nitro, cyano, amino, oxo, thio, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, 5- to 10-membered heteroaryl, C₃₋₈ cycloalkyloxy, 3- to 8-membered heterocyclyloxy, C₆₋₁₀ aryloxy, and 5- to 10-membered heteroaryloxy;
Rₐ and R_{b} are each independently hydrogen, deuterium, halogen, hydroxyl, mercapto, nitro, cyano, amino, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, or 5- to 10-membered heteroaryl, wherein the amino, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, and 5- to 10-membered heteroaryl are optionally further substituted by one or more substituents selected from deuterium, halogen, hydroxyl, mercapto, nitro, cyano, amino, oxo, thio, C₁₋₃ alkyl, C₁₋₃ haloalkyl, C₁₋₃ deuteroalkyl, C₁₋₃ alkoxy, C₁₋₃ alkylthio, C₁₋₃ hydroxyalkyl, C₂₋₃ alkenyl, C₂₋₃ alkynyl, C₃₋₈ cycloalkyl, 3- to 8-membered heterocyclyl, C₆₋₁₀ aryl, 5- to 10-membered heteroaryl, C₃₋₈ cycloalkyloxy, 3- to 8-membered heterocyclyloxy, C₆₋₁₀ aryloxy, and 5- to 10-membered heteroaryloxy.

11. The compound, the prodrug thereof, the stereoisomer thereof, or the pharmaceutically acceptable salt thereof according to any one of claims 1 to 10, wherein the compound satisfies one or more of the following conditions:
(1) the ring B is selected from C₄₋₁₂ cycloalkyl, 3- to 12-membered heterocyclyl, C₆₋₁₄ aryl, or 5- to 14-membered heteroaryl, wherein the C₄₋₁₂ cycloalkyl, 3- to 12-membered heterocyclyl, C₆₋₁₄ aryl, and 5- to 14-membered heteroaryl are optionally further substituted;
(2) the ring C is phenyl, furyl, thienyl, pyrrolyl, imidazolyl, pyrazolyl, triazolyl, tetrazolyl, oxazolyl, isoxazolyl, thiazolyl, isothiazolyl, pyridyl, pyrimidinyl, pyrazinyl, pyridazinyl, benzothiazolyl, benzisothiazolyl, benzoxazolyl, benzisoxazolyl, pyridopyrazolyl, pyridooxazolyl, pyridoisoxazolyl, pyridothiazolyl, pyridoisothiazolyl, or pyridopyrrolyl; preferably, ring C is phenyl, pyridyl, or pyridoisoxazolyl;
(3) R₁ is independently halogen, C₁₋₆ alkyl, C₁₋₆ alkyl substituted by one or more halogens, C₃₋₆ cycloalkyl, or C₁₋₆ alkoxy; or, any two R₁ together with the atom to which they are attached form C₃₋₆ cycloalkyl or 3- to 6-membered heterocyclyl, the heteroatom in the 3- to 6-membered heterocyclyl is N or O, and the number of heteroatoms is 1; preferably, R₁ is independently hydrogen, deuterium, halogen, C₁₋₆ alkyl, C₁₋₆ alkyl substituted by one or more halogens, C₃₋₆ cycloalkyl, or C₁₋₆ alkoxy; or, any two R₁ together with the atom to which they are attached form C₅ cycloalkyl or 6-membered heterocyclyl, the heteroatom in the 6-membered heterocyclyl is O, and the number of heteroatoms is 1; more preferably, R₁ is independently C₁₋₆ alkyl or C₃₋₆ cycloalkyl; or, any two R₁ together with the atom to which they are attached form C₅ cycloalkyl or 6-membered heterocyclyl, the heteroatom in the 6-membered heterocyclyl is O, and the number of heteroatoms is 1; for example, R₁ is independently C₁₋₆ alkyl or trifluoromethyl; or, any two R₁ together with the atom to which they are attached form C₅ cycloalkyl;
(4) R₂ is independently hydrogen or deuterium;
(5) R₃ is independently hydrogen, deuterium, halogen, C₁₋₆ alkyl, 5- to 6-membered heteroaryl, -(CH₂)ₙ₁C(O)NR_{b}Rₐ, or -(CH₂)ₙ₁NR_{b}Rₐ, wherein the 5- to 6-membered heteroaryl is optionally substituted by C₁₋₆ alkyl; the heteroatom in the 5- to 6-membered heterocyclyl is N and/or O, and the number of heteroatoms is 1 or 2; preferably, R₃ is independently hydrogen, halogen, or -(CH₂)ₙ₁C(O)NR_{b}Rₐ; for example, hydrogen or -(CH₂)ₙ₁C(O)NR_{b}Rₐ;
and (6) Rₐ and R_{b} are each independently hydrogen, C₁₋₆ alkyl, or C₃₋₆ cycloalkyl; preferably, Rₐ and R_{b} are each independently hydrogen or C₁₋₆ alkyl, such as hydrogen or methyl;
x, y, and z are each independently an integer of 0 to 3;
the n is 1, 2, 3, 4, 5, or 6, and, when X₂ is CH, X₃ is N, and X₄ is CH, then n is not 1.

12. The compound, the prodrug thereof, the stereoisomer thereof, or the pharmaceutically acceptable salt thereof according to any one of claims 1 to 11, wherein the compound satisfies one or more of the following conditions:
(1) R₁ is independently ethyl, methoxy, trifluoromethyl, difluoromethyl, cyclopropyl, fluorine, or methyl; or, any two R₁ together with the atom to which they are attached form cyclopentyl or 6-membered heterocyclyl; the heteroatom in the 6-membered heterocyclyl is N or O, and the number of heteroatoms is 1;
(2) R₃ is independently hydrogen, 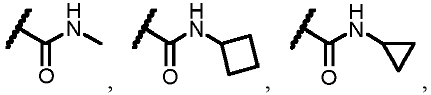 fluorine, methyl, 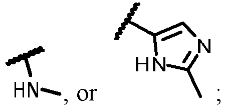
(3) x is an integer of 0 to 3;
(4) L is -(CR₄R₅)ₙ₂-; n2 is 1;
(5) 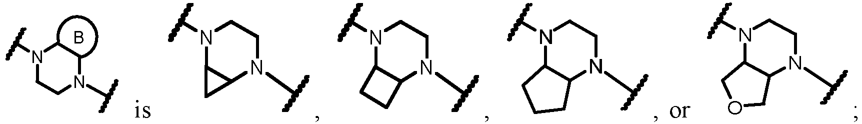
(6) y is 0;
(7) ring C is 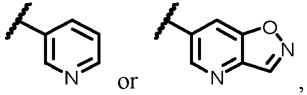 such as 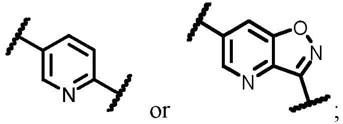
(8) z is 1 or 2;
(9) n1 is 0;
(10) ring A is 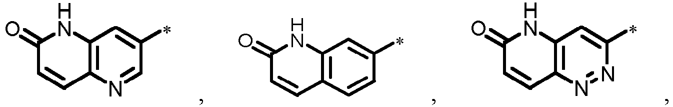 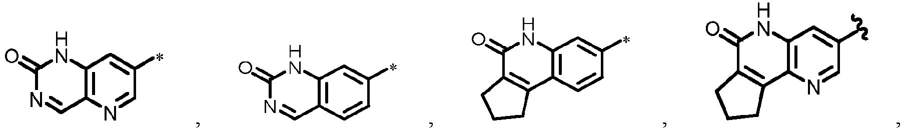 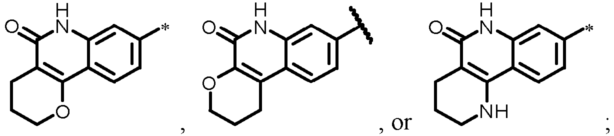
and (11) in a further preferred embodiment of the present disclosure, the compound of general formula (I) is not 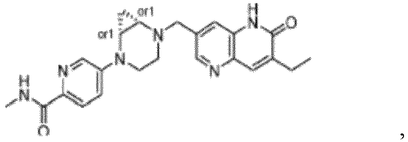 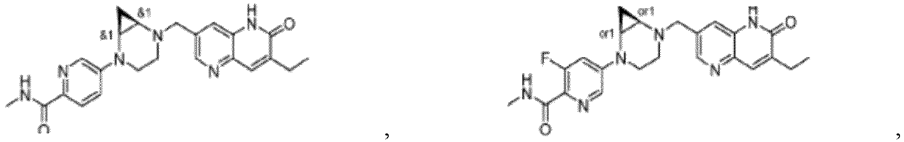 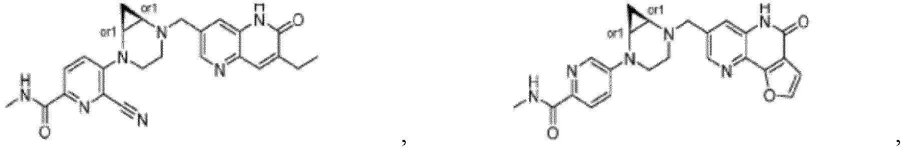 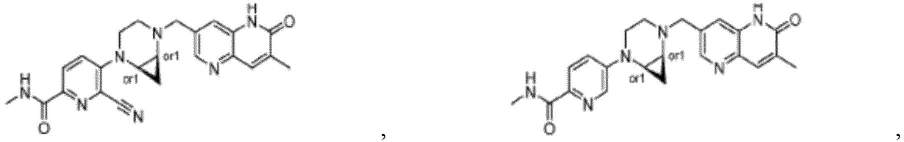 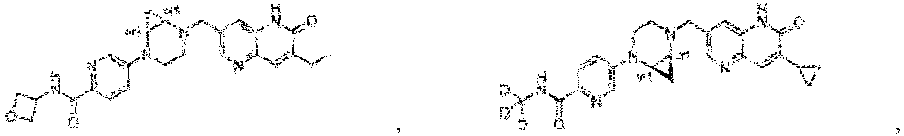 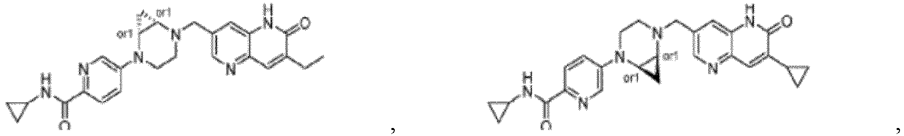 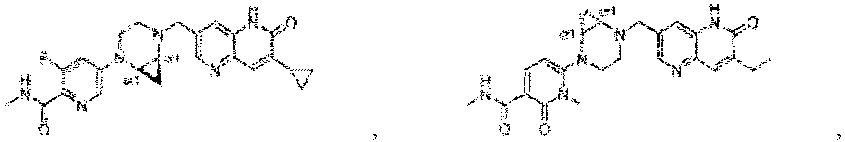 or 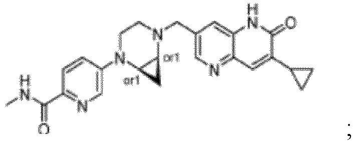
preferably, 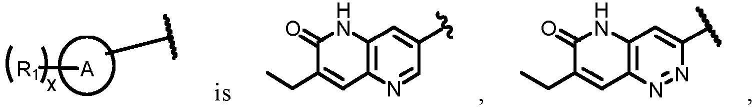 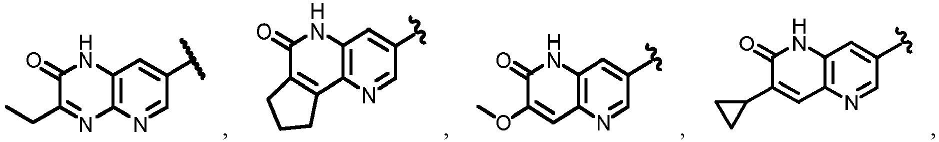 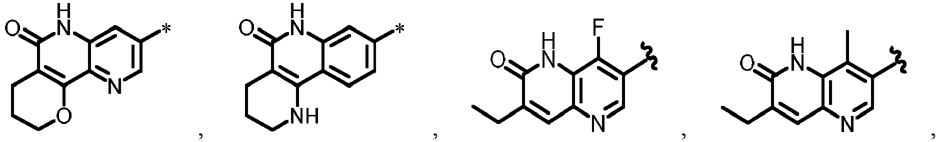 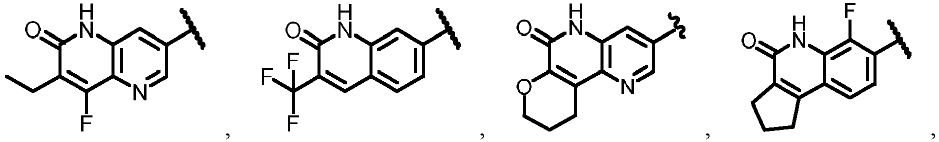 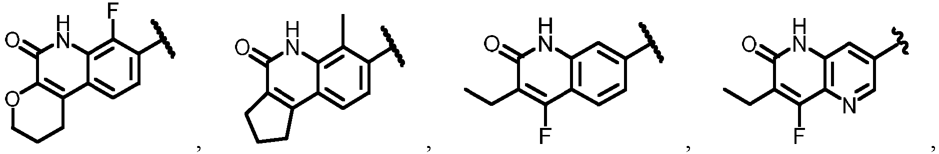 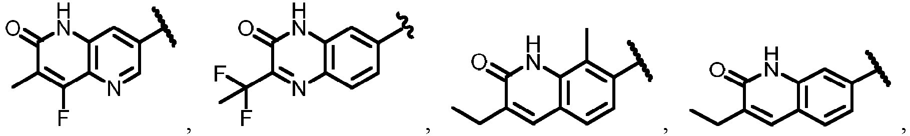 or 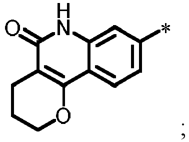 and/or,

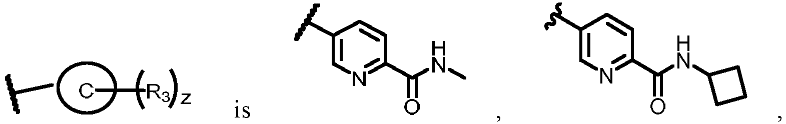

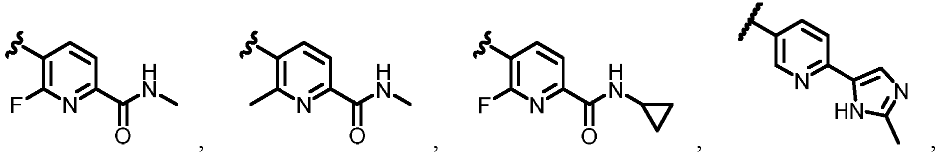

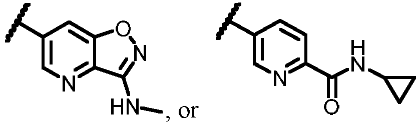

13. The compound, the prodrug thereof, the stereoisomer thereof, or the pharmaceutically acceptable salt thereof according to any one of claims 1 to 12, wherein ring A is 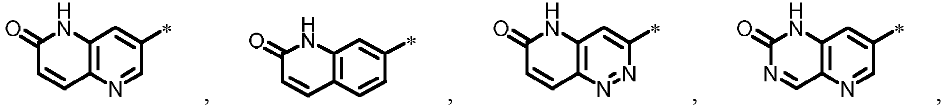 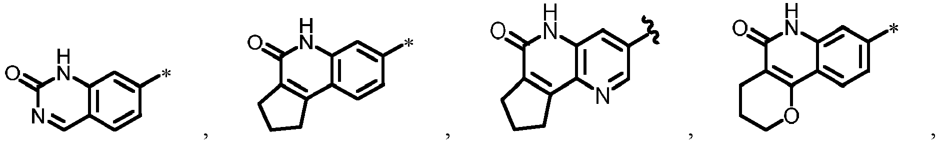 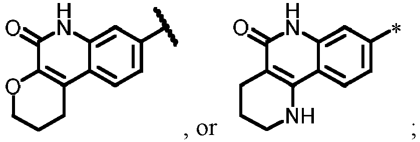
ring B is C₄₋₆ cycloalkyl or 5- to 6-membered heterocyclyl, the heteroatom in the 5- to 6-membered heterocyclyl is N, O, or S, and the number of heteroatoms is 1;
ring C is pyridyl or pyridoisoxazolyl;
R₁ is independently hydrogen, deuterium, halogen, C₁₋₆ alkyl, C₁₋₆ alkyl substituted by one or more halogens, C₃₋₆ cycloalkyl, or C₁₋₆ alkoxy;
or, any two R₁ together with the atom to which they are attached form C₃₋₆ cycloalkyl or 3- to 6-membered heterocyclyl, the heteroatom in the 3- to 6-membered heterocyclyl is N or O, and the number of heteroatoms is 1;
R₂ is independently hydrogen or deuterium;
R₃ is independently hydrogen, deuterium, halogen, C₁₋₆ alkyl, 5- to 6-membered heteroaryl, -(CH₂)ₙ₁C(O)NR_{b}Rₐ, or -(CH₂)ₙ₁NR_{b}Rₐ, wherein the 5- to 6-membered heteroaryl is optionally substituted by C₁₋₆ alkyl; the heteroatom in the 5- to 6-membered heterocyclyl is N and/or O, and the number of heteroatoms is 1 or 2;
Rₐ and R_{b} are each independently hydrogen, C₁₋₆ alkyl, or C₃₋₆ cycloalkyl;
L is -(CR₄R₅)ₙ₂-;
R₄ and R₅ are each independently hydrogen or deuterium;
x, y, and z are each independently an integer of 0 to 3;
n1 is an integer of 0 to 3;
n2 is 1; and
m1 is 0, 1, or 2.

14. The compound, the prodrug thereof, the stereoisomer thereof, or the pharmaceutically acceptable salt thereof according to any one of claims 1 to 13, wherein the general formula (I), general formula (II), general formula (III), general formula (IV), general formula (V), general formula (VI), general formula (V), general formula (VI), general formula (VII), general formula (VIII), and/or general formula (IX) is in a cis-configuration or trans-configuration, preferably cis-configuration.

15. The compound, the prodrug thereof, the stereoisomer thereof, or the pharmaceutically acceptable salt thereof according to any one of claims 1 to 14, wherein the compound, the prodrug thereof, the stereoisomer thereof, or the pharmaceutically acceptable salt thereof is selected from the following compounds:

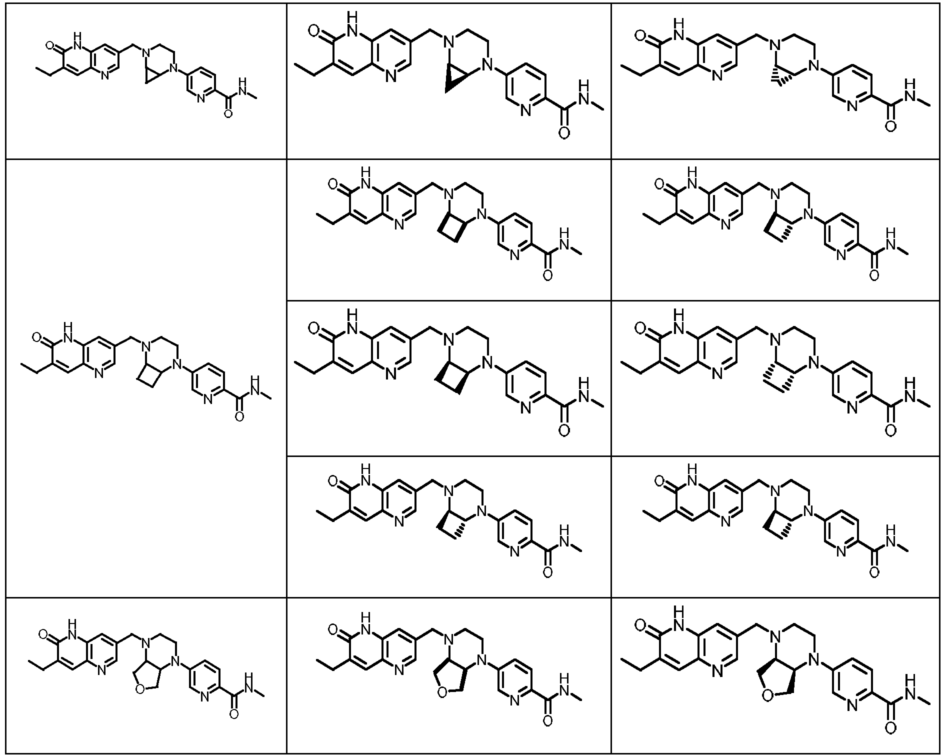

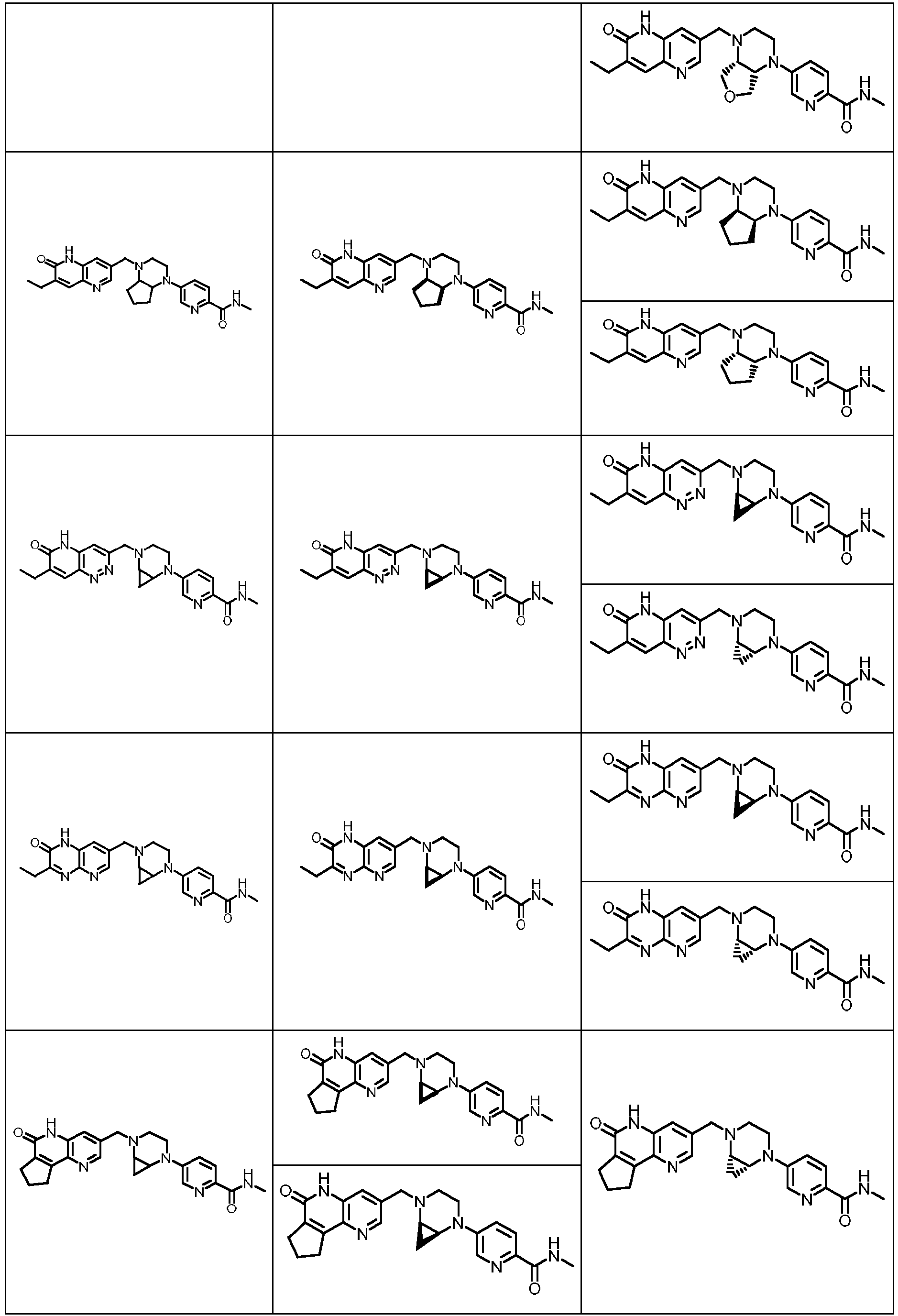

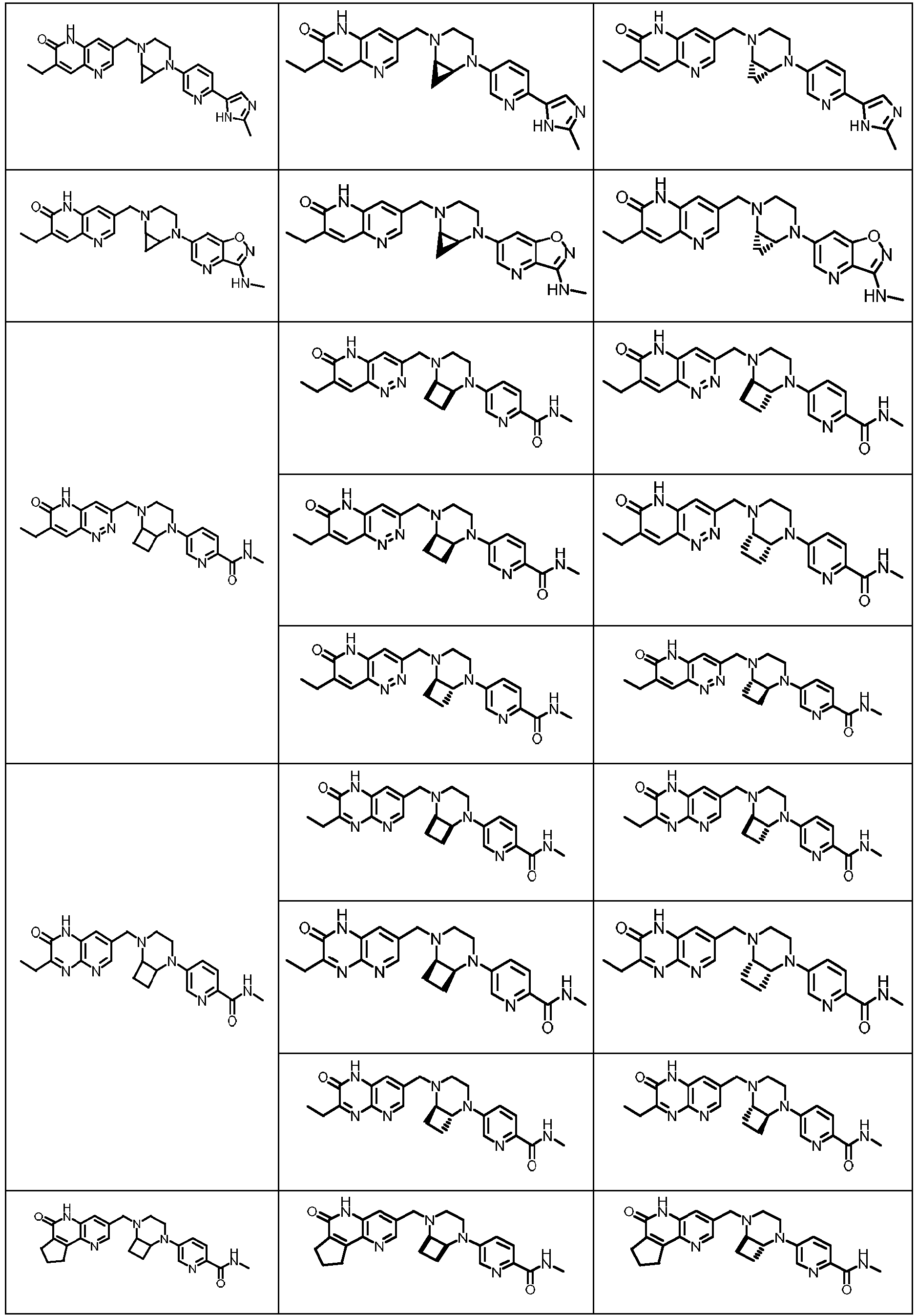

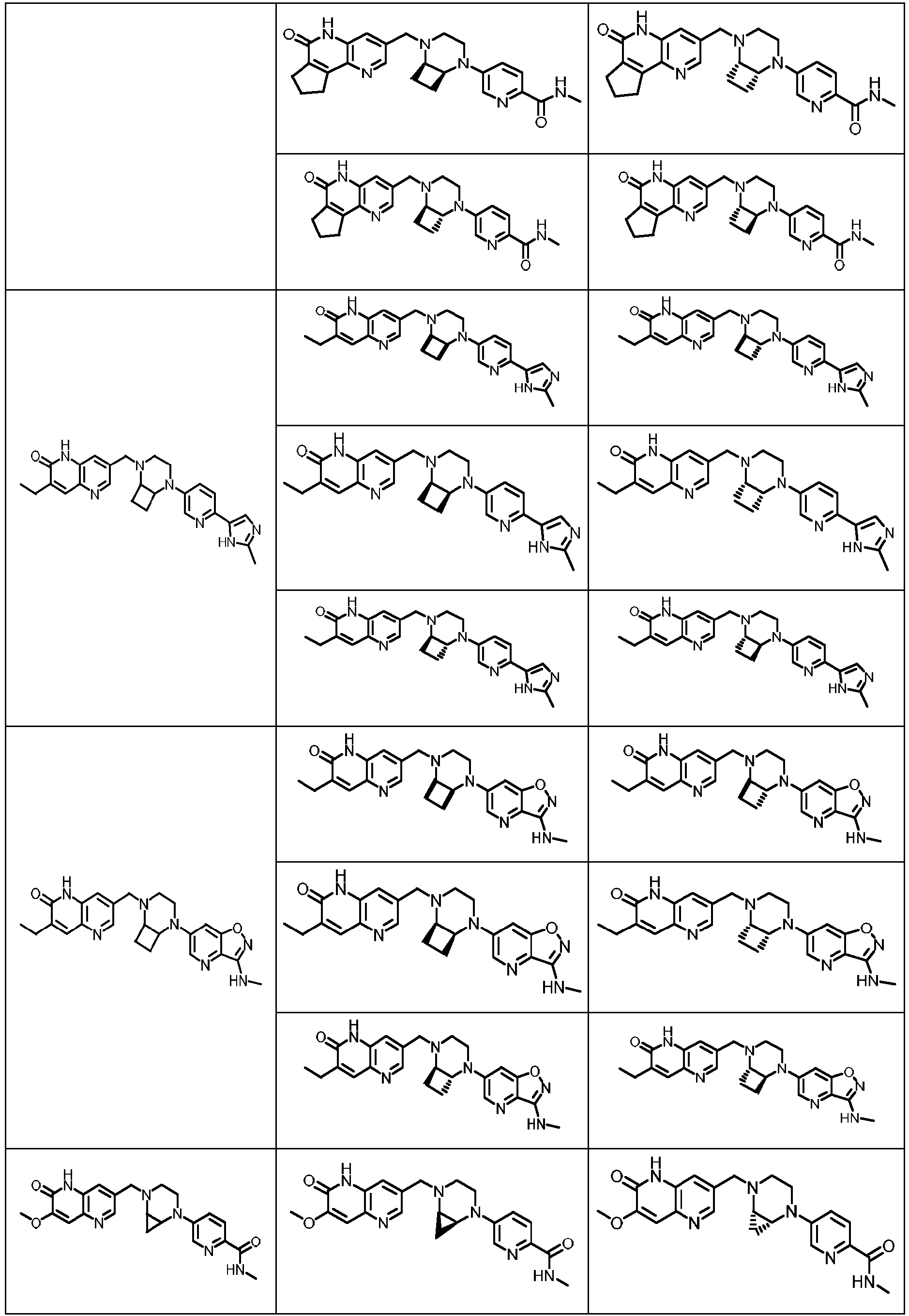

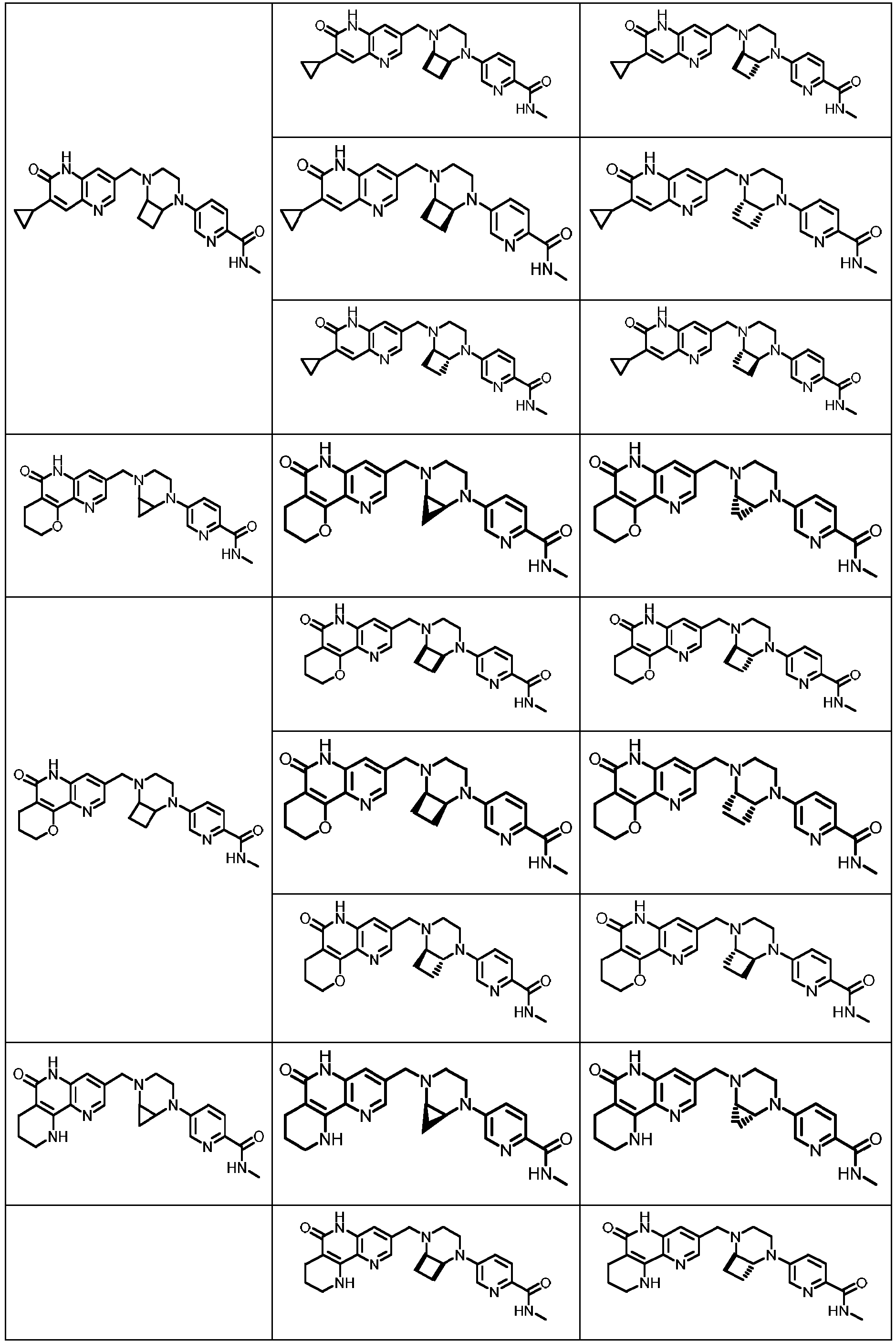

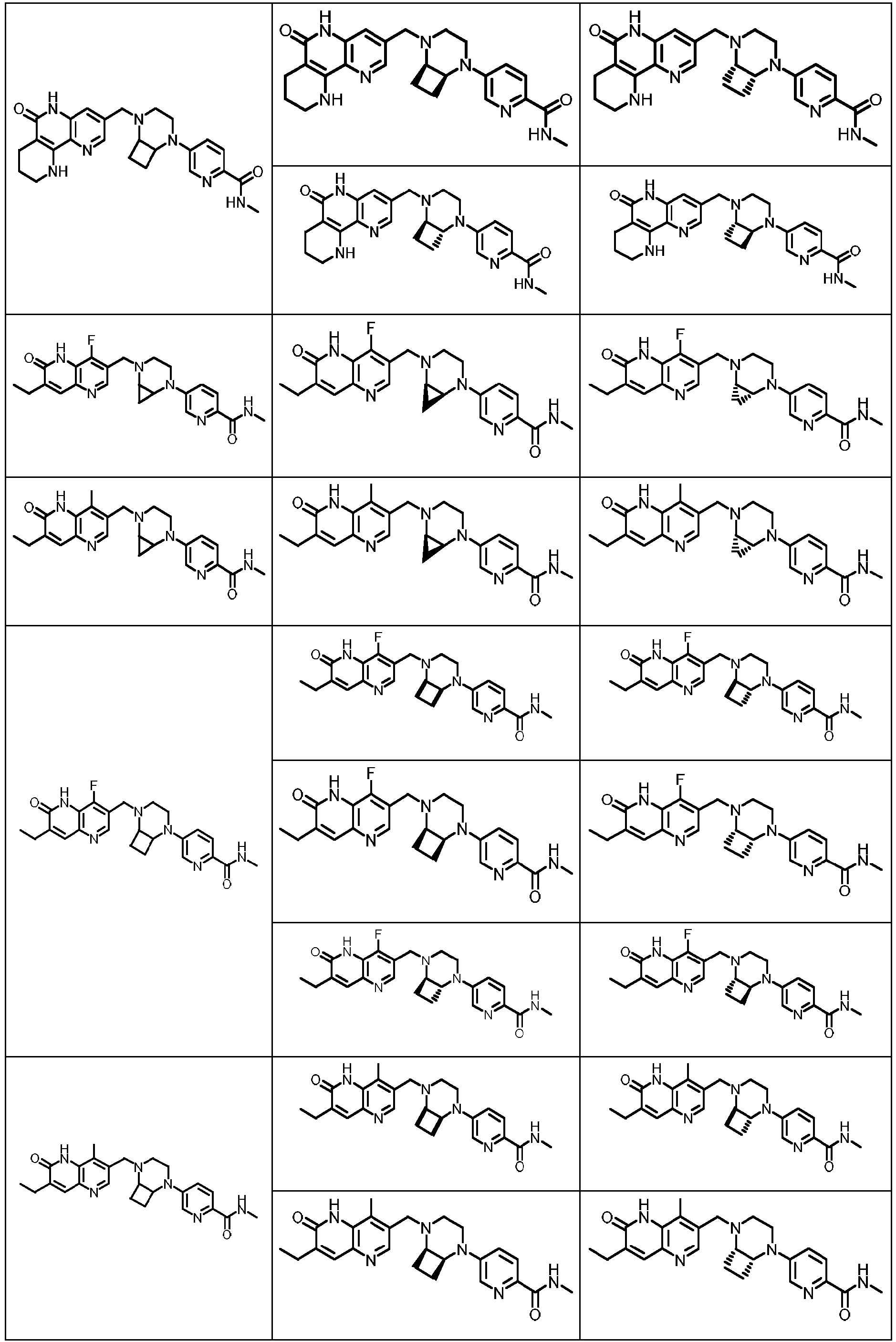

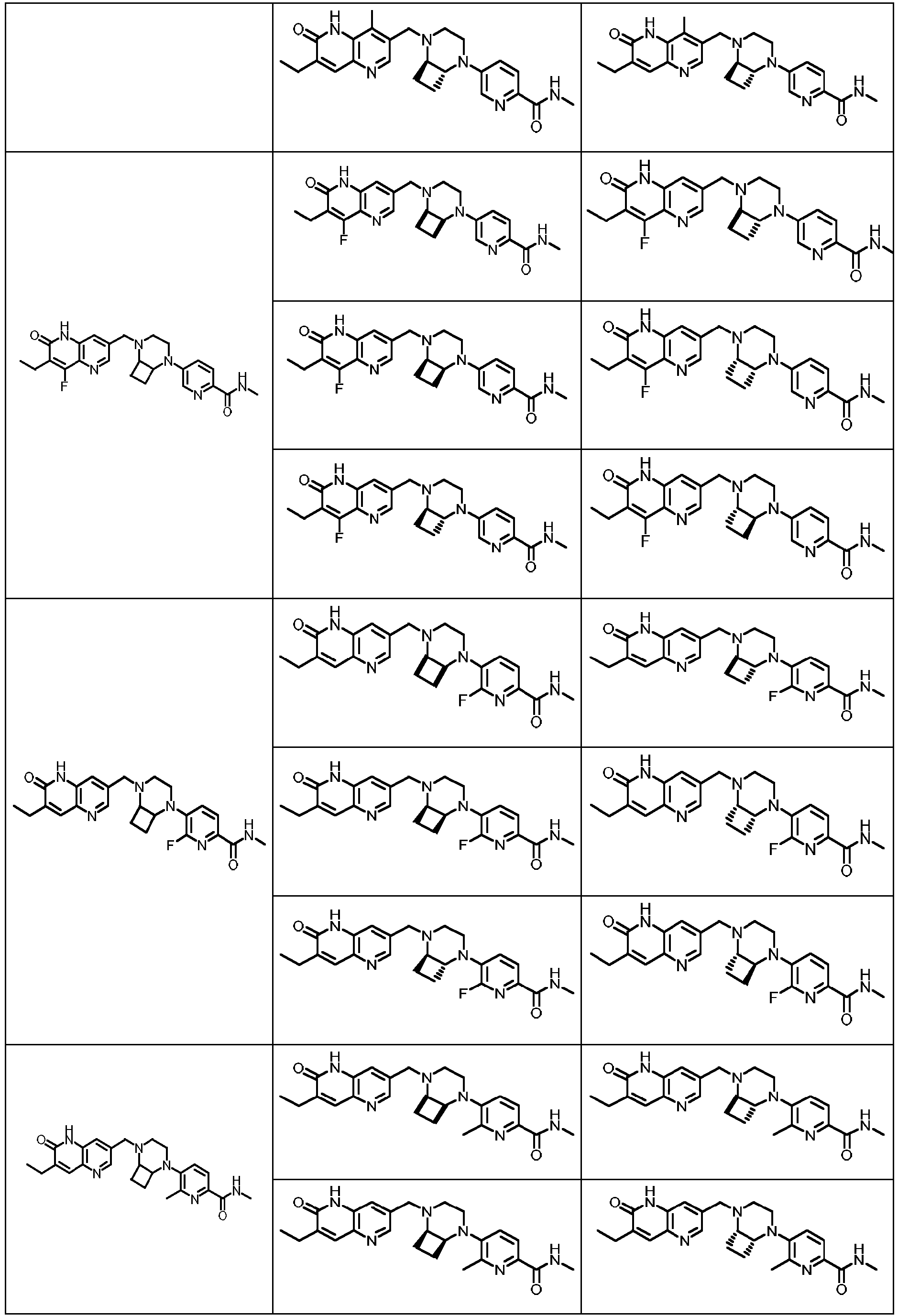

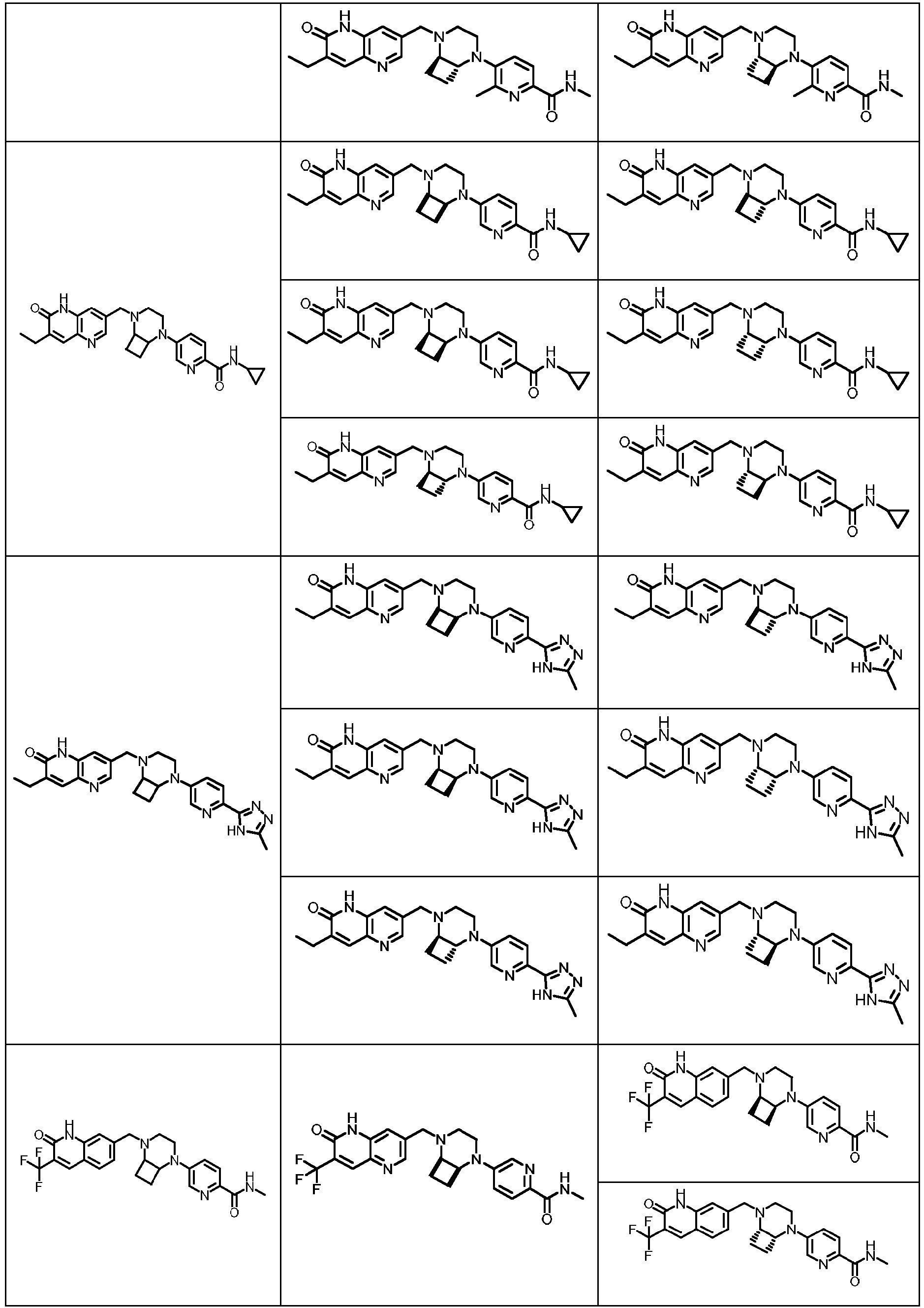

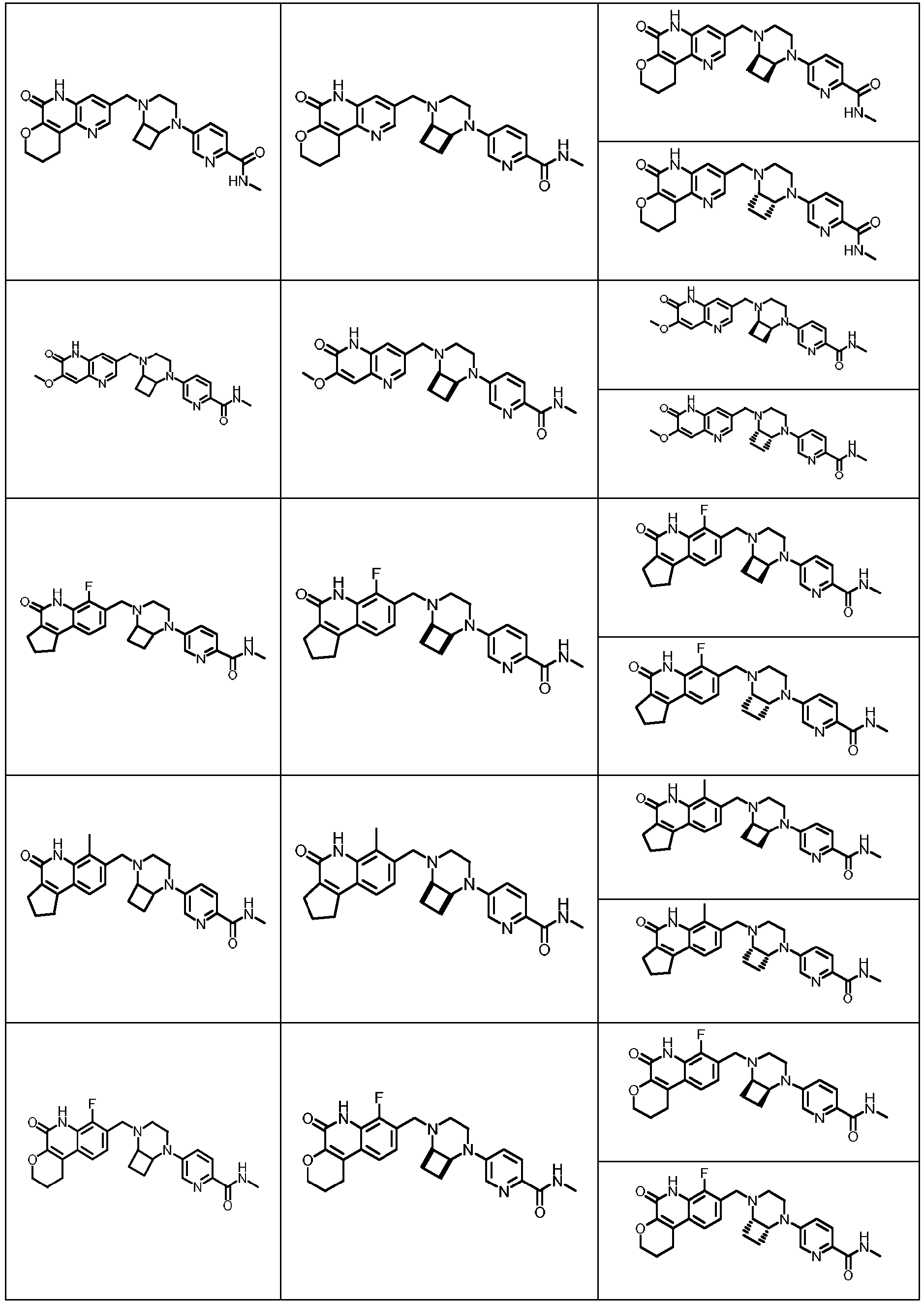

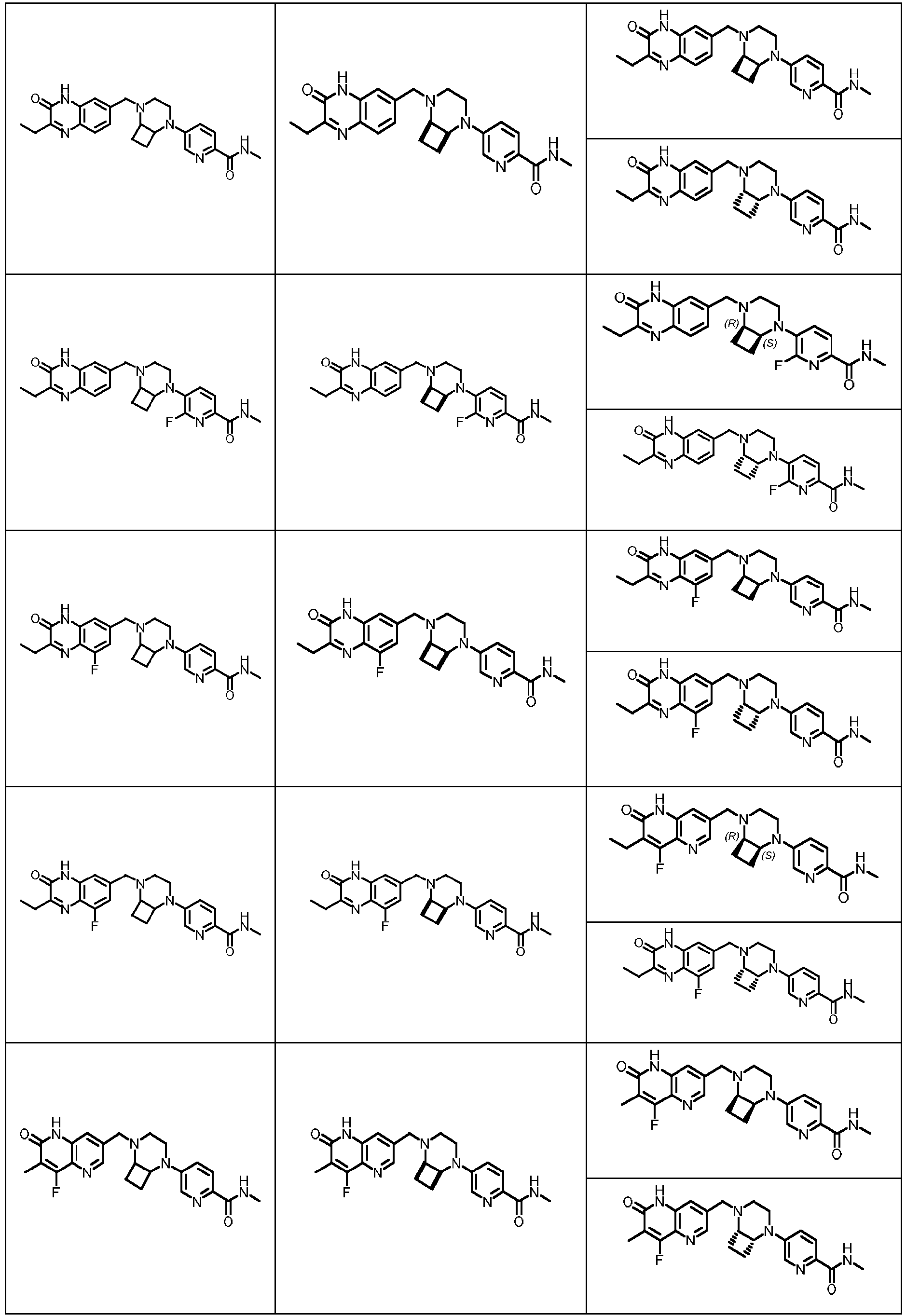

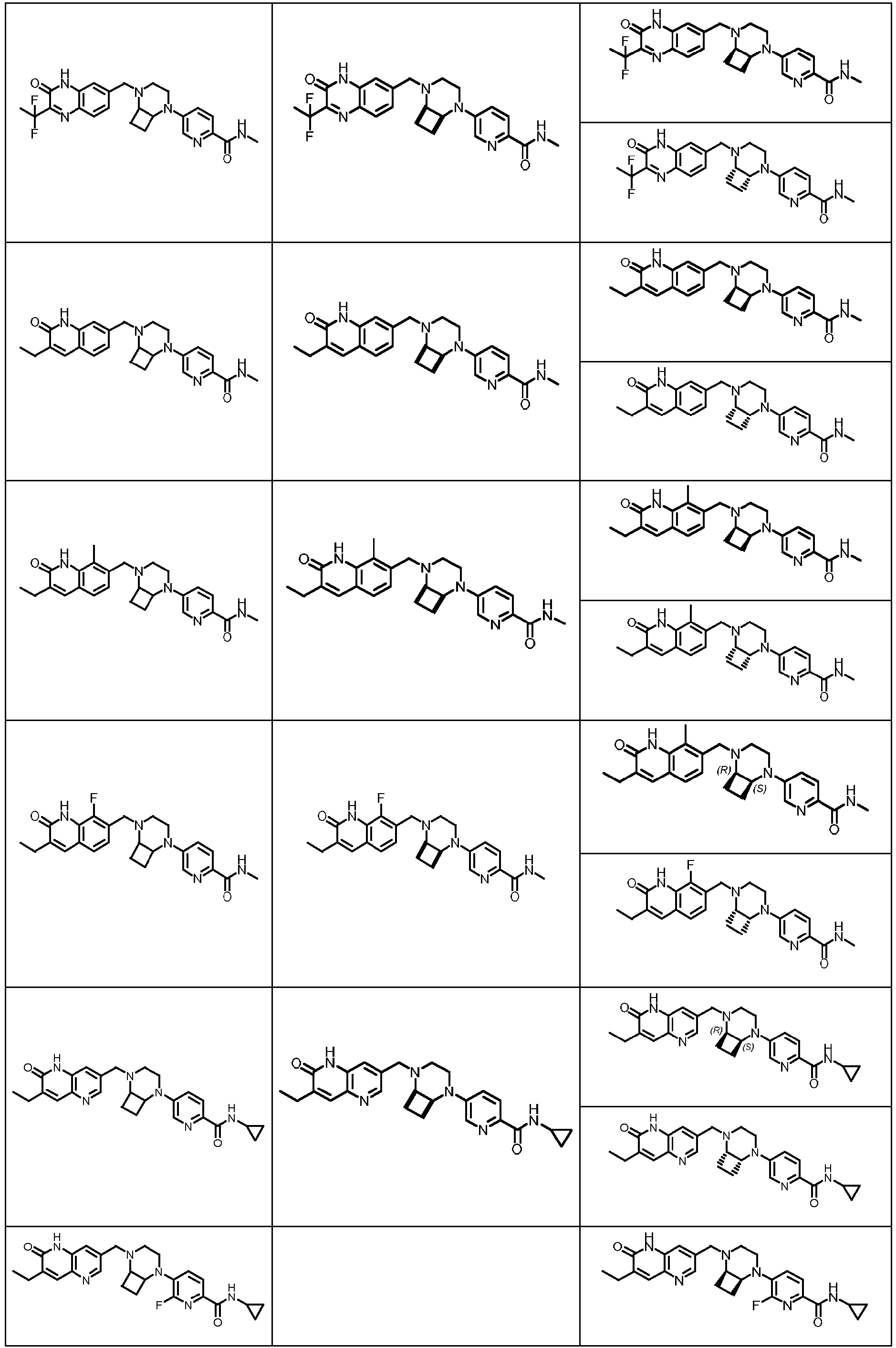

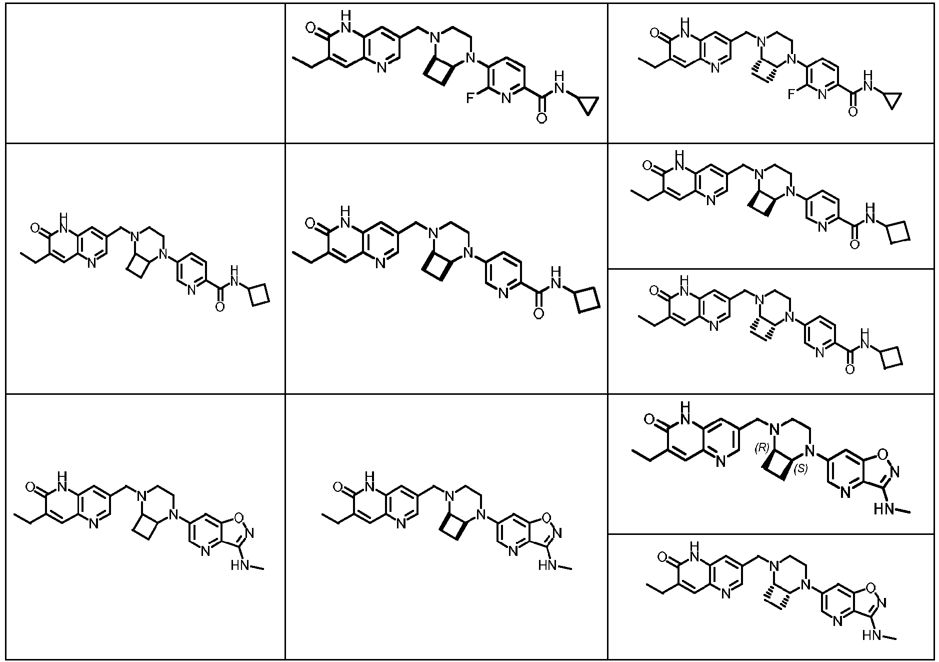

16. A method for preparing the compound of general formula (I), the prodrug thereof, the stereoisomer thereof, or the pharmaceutically acceptable salt thereof according to any one of claims 1 to 15, wherein the method comprises the following steps: 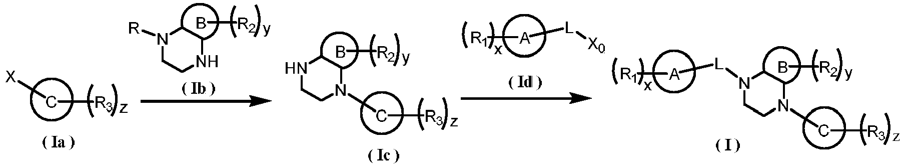
obtaining a compound of general formula (Ic), a prodrug thereof, a stereoisomer thereof, or a pharmaceutically acceptable salt thereof through a one-step or multi-step nucleophilic substitution reaction, coupling reaction, or other reaction between formula (Ia) and formula (Ib), either directly or through a further deprotection reaction;
obtaining the compound of general formula (I), the prodrug thereof, the stereoisomer thereof, or the pharmaceutically acceptable salt thereof through a one-step or multi-step nucleophilic substitution reaction, coupling reaction, or other reaction between formula (Ic) and formula (Id); optionally, the compound of general formula (I) is further subjected to chiral resolution to obtain a single-configuration compound, a prodrug thereof, a stereoisomer thereof, or a pharmaceutically acceptable salt thereof;
wherein
X is selected from halogen; preferably, X is fluorine, chlorine, bromine, or iodine;
X₀ is selected from halogen; preferably, X₀ is fluorine, chlorine, bromine, or iodine;
R is hydrogen or an amino protecting group; the amino protecting group including but not limited to, *tert*-butoxycarbonyl (Boc), benzyloxycarbonyl (Cbz), *etc.;*
when R is hydrogen, formula (Ia) reacts with formula (Ib) to obtain the compound of general formula (Ic), the prodrug thereof, the stereoisomer thereof, or the pharmaceutically acceptable salt thereof directly;
when R is an amino protecting group, formula (Ia) reacts with formula (Ib) followed by a further deprotection reaction to obtain the compound of general formula (Ic), the prodrug thereof, the stereoisomer thereof, or the pharmaceutically acceptable salt thereof;
ring A, ring B, ring C, L, R₁, R₂, R₃, x, y, and z are as described in claims 1 to 14.

17. A pharmaceutical composition comprising a therapeutically effective dose of the compound, the prodrug thereof, the stereoisomer thereof, or the pharmaceutically acceptable salt thereof according to any one of claims 1 to 15, and one or more pharmaceutically acceptable carriers or excipients.

18. A use of the compound, the prodrug thereof, the stereoisomer thereof, or the pharmaceutically acceptable salt thereof according to any one of claims 1 to 15, or the pharmaceutical composition according to claim 17 in the manufacture of a medicament for the treatment of a disease mediated by polyadenosine diphosphate ribose (ADP-ribose) polymerase (PARP); preferably in the manufacture of a medicament for the treatment of a disease related to a PARP1 inhibitor.

19. A use of the compound, the prodrug thereof, the stereoisomer thereof, or the pharmaceutically acceptable salt thereof according to any one of claims 1 to 15, or the pharmaceutical composition according to claim 17 in the manufacture of a medicament for the treatment of abnormal cell growth; preferably in the manufacture of a medicament for the treatment of cancer, inflammation, a metabolic disease, a cardiovascular disease, an immunological disease, a mental disorder, and a related disease.

20. A compound of formula (Ic), a stereoisomer thereof, or a salt thereof: 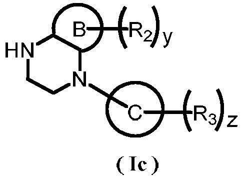 ring B, ring C, R₂, R₃, y, and z are as described in any one of claims 1 to 15.

21. A compound having any one of the following structures: 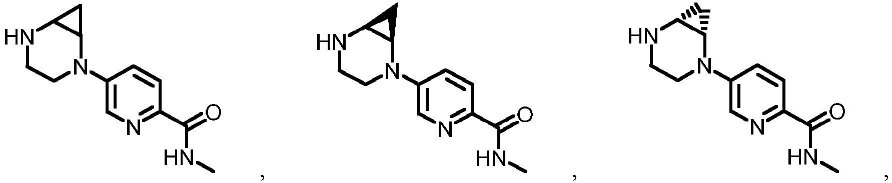 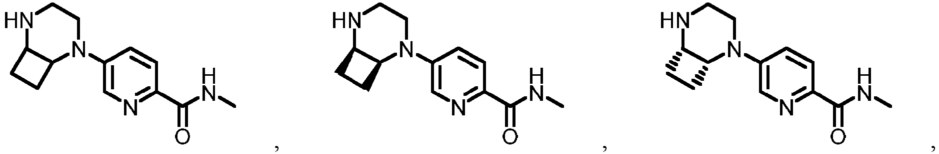 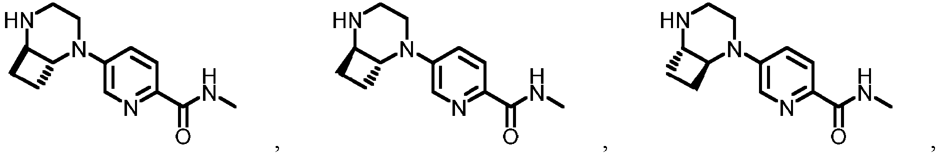 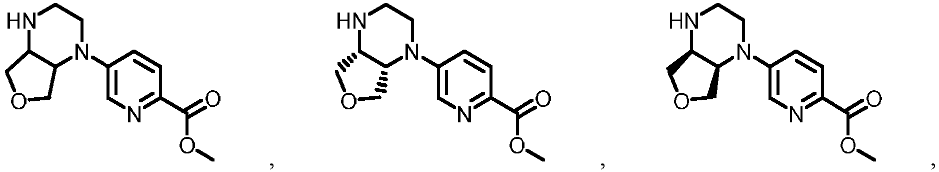 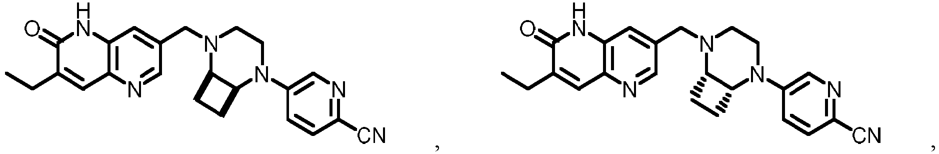 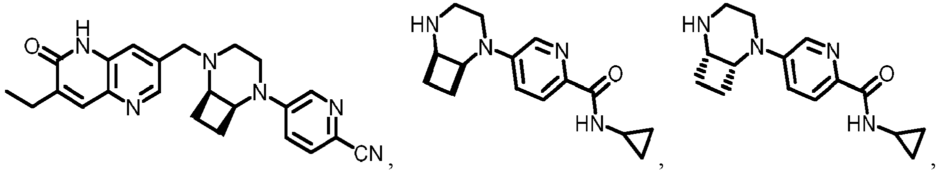 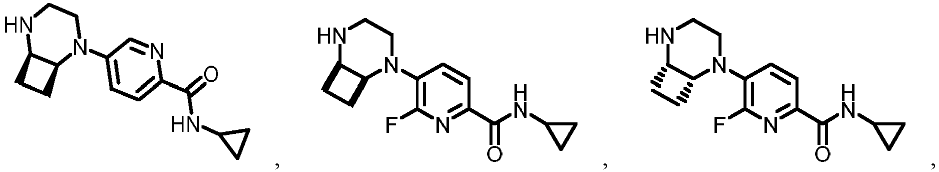 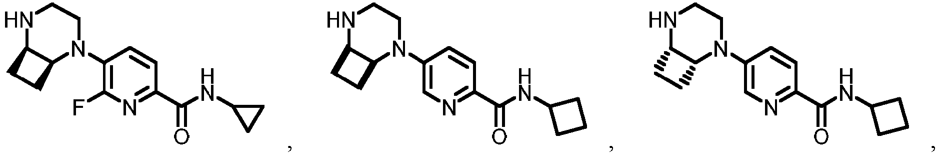 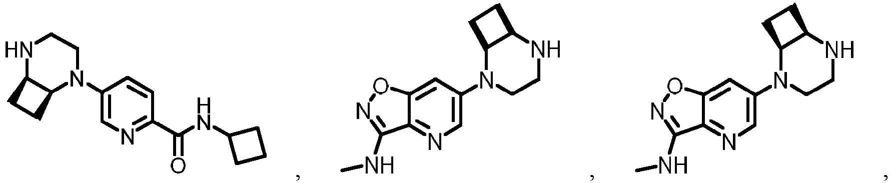 or
